# EUROPEAN PATENT SPECIFICATION

(11) **EP 3 312 267 B1**
(45) Date of publication and mention of the grant of the patent: **11.03.2020**
(21) Application number: 17001811.3
(22) Date of filing: 29.01.2013
(51) Int. Cl.: C12N 1/21, C12N 9/02, C12N 9/04, C12N 9/06, C12N 9/10, C12N 9/88, C12N 15/52, C12N 15/53, C12N 15/54, C12N 15/60, C12N 15/63, C12N 15/70, C07K 14/245, C12P 7/44, C12N 9/12

(54) **PRODUCTION OF MUCONIC ACID FROM GENETICALLY ENGINEERED MICROORGANISMS**
PRODUKTION VON MUCONSÄURE AUS GENETISCH MODIFIZIERTEN MIKROORGANISMEN
PRODUCTION D'ACIDE MUCONIQUE À PARTIR DE MICRO-ORGANISMES GÉNÉTIQUEMENT MODIFIÉS

(30) Priority: 30.01.2012 US 201261632777 P
(43) Date of publication of application: 25.04.2018
(62) Divisional of application: 13743123.5
(73) Proprietor: PTT Global Chemical Public Company Limited, Chatuchak, Bangkok 10900 (TH)
(72) Inventor: Yocum, R., Rogers, Lexington, MA 02420 (US); Gong, Wei, Newton, MA 02464 (US); Dole, Sudhanshu, Andover, MA 01810 (US); Sillers, Ryan, Reading, MA 01867 (US); Gandhi, Meghal, Waltham, MA 01845 (US); Pero, Janice, G., Lexington, MA 02420 (US); Spencer, Michelle, Pepperell, MA 01463 (US)
(74) Representative: Müller-Boré & Partner Patentanwälte PartG mbB

(56) References cited:
- WO-A1-2011/085311
- WO-A2-2004/033471
- US-A- 5 616 496
- ORITA I. ET AL: "Identification of mutation points inNCIMB 11599 and genetic reconstitution of glucose-utilization ability in wild strain H16 for polyhydroxyalkanoate production", JOURNAL OF BIOSCIENCE AND BIOENGINEERING, vol. 113, no. 1, 20 October 2011 (2011-10-20), pages 63-69, XP028123217, ISSN: 1389-1723, DOI: 10.1016/J.JBIOSC.2011.09.014
- CHANDRAN S.S. ET AL: "Phosphoenolpyruvate Availability and the Biosynthesis of Shikimic Acid", BIOTECHNOLOGY PROGRESS, vol. 19, no. 3, 6 June 2003 (2003-06-06), pages 808-814, XP055045223, ISSN: 8756-7938, DOI: 10.1021/bp025769p
- YI J. ET AL: "Altered Glucose Transport and Shikimate Pathway Product Yields in E. coli", BIOTECHNOLOGY PROGRESS, vol. 19, no. 5, 3 October 2003 (2003-10-03), pages 1450-1459, XP055045226, ISSN: 8756-7938, DOI: 10.1021/bp0340584
- ZHANG X. ET AL: "Metabolic evolution of energy-conserving pathways for succinate production in Escherichia coli", PROCEEDINGS OF THE NATIONAL ACADEMY OF SCIENCES, vol. 106, no. 48, 1 December 2009 (2009-12-01), pages 20180-20185, XP055010825, ISSN: 0027-8424, DOI: 10.1073/pnas.0905396106
- FLORES N. ET AL: "Growth recovery on glucose under aerobic conditions of an Escherichia coli strain carrying a phosphoenolpyruvate: Carbohydrate phosphotransferase system deletion by inactivating arcA and overexpressing the genes coding for glucokinase and galactose permease", JOURNAL OF MOLECULAR MICROBIOLOGY AND BIOTECHNOLOGY, vol. 13, no. 1-3, 1 August 2007 (2007-08-01), pages 105-116, XP009502783, ISSN: 1464-1801, DOI: 10.1159/000103602 [retrieved on 2007-08-16]
- NIU W. ET AL: "Benzene-free synthesis of adipic acid", BIOTECHNOLOGY PROGRESS, vol. 18, no. 2, 1 March 2002 (2002-03-01), pages 201-211, XP002568326, ISSN: 8756-7938, DOI: 10.1021/BP010179X [retrieved on 2002-02-12]
- GARNER C.C. ET AL: "Operator Mutations of the Escherichia coli aroF Gene", THE JOURNAL OF BIOLOGICAL CHEMISTRY, vol. 260, no. 6, 25 March 1985 (1985-03-25), pages 3820-3825, XP055238180,
- TANG J. ET AL: "Recruiting alternative glucose utilization pathways for improving succinate production", APPLIED MICROBIOLOGY AND BIOTECHNOLOGY, vol. 97, no. 6, 16 August 2012 (2012-08-16), pages 2513-2520, XP055093804, ISSN: 0175-7598, DOI: 10.1007/s00253-012-4344-1

### SEQUENCE LISTING

<110> MYRIANT CORPORATION
<120> PRODUCTION OF MUCONIC ACID FROM GENETICALLY ENGINEERED MICROORGANISMS
<130> M 6829EU-kl
<150> US 61/632,777
   <151> 2012-01-30
<160> 45
<170> PatentIn version 3.5
<210> 1
   <211> 195
   <212> DNA
   <213> Artificial Sequence
<220>
   <223> P15 promoter;
<400> 1
   gctattgacg acagctatgg ttcactgtcc accaaccaaa actgtgctca gtaccgccaa    60
   tatttctccc ttgaggggta caaagaggtg tccctagaag agatccacgc tgtgtaaaaa   120
   ttttacaaaa aggtattgac tttccctaca gggtgtgtaa taatttaatt acaggcgggg   180
   gcaaccccgc ctgtt                                                    195
<210> 2
   <211> 164
   <212> DNA
   <213> Artificial Sequence
<220>
   <223> P26 promoter;
<400> 2
   gcctacctag cttccaagaa agatatccta acagcacaag agcggaaaga tgttttgttc        60
   tacatccaga acaacctctg ctaaaattcc tgaaaaattt tgcaaaaagt tgttgacttt       120
   atctacaagg tgtggtataa taatcttaac aacagcagga cgct                        164
<210> 3
   <211> 91
   <212> DNA
   <213> Artificial Sequence
<220>
   <223> PR promoter;
<400> 3
   acgttaaatc tatcaccgca agggataaat atctaacacc gtgcgtgttg actattttac        60
   ctctggcggt gataatggtt gcatgtacaa g                                       91
<210> 4
   <211> 359
   <212> PRT
   <213> Artificial Sequence
<220>
   <223> 3-dehydroshikimate dehydratase encoded by the qa-4 gene;
<400> 4
   Met Pro Ser Lys Leu Ala Ile Ser Ser Met Ser Leu Gly Arg Cys Phe
   1               5                   10                  15
   Ala Gly His Ser Leu Asp Ser Lys Leu Asp Ala Ala Gln Arg Tyr Gly
               20                  25                  30
   Tyr Leu Gly Ile Glu Leu Phe Tyr Glu Asp Leu Val Asp Val Ala Glu
           35                  40                  45
   His Leu Ser Asn Glu Arg Pro Ser Pro Glu Gly Pro Phe Val Glu Ala
       50                  55                  60
   Gln Ile Ala Ala Ala Arg His Ile Leu Gln Met Cys Gln Ala Arg Gly
   65                  70                  75                  80
   Leu Glu Val Val Cys Leu Gln Pro Phe Met His Tyr Asp Gly Leu Asn
                   85                  90                  95
   Asp Arg Ala Glu His Glu Arg Arg Leu Glu Lys Leu Ala Leu Trp Ile
               100                 105                 110
   Glu Leu Ala His Glu Leu His Thr Asp Ile Ile Gln Ile Pro Ala Asn
           115                 120                 125
   Phe Leu Pro Ala Asn Gln Val Ser Asp Asn Leu Asp Leu Ile Val Ser
       130                 135                 140
   Asp Leu Cys Lys Val Ala Asp Ile Gly Ala Gln Ala Leu Pro Pro Ile
   145                 150                 155                 160
   Arg Phe Ala Tyr Glu Ser Leu Cys Trp Ser Thr Arg Val Asp Leu Trp
                   165                 170                 175
   Glu Arg Cys Trp Asp Ile Val Gln Arg Val Asp Arg Pro Asn Phe Gly
               180                 185                 190
   Ile Cys Leu Asp Thr Phe Asn Ile Leu Gly Arg Ile Tyr Ala Asp Pro
           195                 200                 205
   Thr Ser Pro Ser Gly Arg Thr Pro Asn Ala Lys Glu Ala Val Arg Lys
       210                 215                 220
   Ser Ile Ala Asn Leu Val Ser Arg Val Asp Val Ser Lys Val Phe Tyr
   225                 230                 235                 240
   Val Gln Val Val Asp Ala Glu Arg Leu Ser Lys Pro Leu Leu Pro Gly
                   245                 250                 255
   His Pro Tyr Tyr Asn Pro Glu Gln Pro Ala Arg Met Ser Trp Ser Arg
               260                 265                 270
   Asn Cys Arg Leu Phe Tyr Gly Glu Thr Glu Tyr Gly Ala Tyr Leu Pro
           275                 280                 285
   Val Lys Glu Val Ala Arg Ala Leu Phe His Gly Ile Gly Phe Glu Gly
       290                 295                 300
   Trp Val Ser Leu Glu Leu Phe Asn Arg Arg Met Ser Glu Glu Gly Pro
   305                 310                 315                 320
   Glu Val Pro Glu Glu Leu Ala Met Arg Gly Ala Ile Ser Trp Ala Lys
                   325                 330                 335
   Leu Val Gln Asp Leu Arg Ile Pro Val Glu Gly Pro Leu Val Thr Met
               340                 345                 350
   Pro Arg Val Ser Ala Ser Leu
           355
<210> 5
   <211> 2160
   <212> DNA
   <213> Artificial Sequence
<220>
   <223> qa-4 gene from Neurospora crassa plus surrounding sequences;
<400> 5
   gaattcggga aatggaatct tacctgggaa ccgaaatcac agtccgggta ggttatagag      60
   catatagtga actgtcaaag ttctagacct ggaccagcca cttggagtcg ttgttttagt     120
   tatacctaca ttcactcact gttgactttc aatcatactt acttagacgg agcaacgcgc     180
   cagaatccaa attgttgcat agttgcggta tcaccaagtg gcttcccata atagtttgcc     240
   attcgatgag acagctaact ggaagaccgg tactcgcagg ttgcacgatt acacggaagg     300
   attcggtatt ccgtgtttca tctgtcaaag tccctttcca tatgaatccg aggtactatg     360
   actggatctc gatacaagct ggccagcgag gtgcctgcct tgacaggctg tcaactgcgg     420
   gacggccggc taagtgttta acacgcaagg gtggaagatg tctcgtcccg tcatccaaga 480
   ccgtcaacat tcgaggccat ctgatcgttg aagagatgct aaatcttgtg aaacgctcat 540
   aggtcgctta ccttcggccc acccgttaat gctttattcc gctgagcaaa cttcggcttc 600
   catcccgcgg ttcaccgttt acatcactta tcgttgcggt tattggccga ttcttcgcaa 660
   accgaaacga tgacatcccg aatatctgca atacaccgcc acggccggcg tctttatcac 720
   acctcctatg ggagacgaaa gtgccttgat acccctagtc atttgaagat tcaggatggg 780
   agacggctgg ccgcttgcgg agttacgttc gagtcttggt cgcaggaacg cttgccgtat 840
   tgaatgagac cccgagaagg tcaaatcaaa tcttggaaga ccccaactgc ttcctcattg 900
   ccttcactcc ccatatcaat ggggcacatc ctgtgactac cttggtgctt tatttcctca 960
   ccatttggcg atacaagctc aaggacaccg aggtgatata cagttcttca aggacactat 1020
   ctcacctcaa tatcaagaac cagtctcatc atctcttatt tctccaggat ccccccacca 1080
   acaacatcgg cttttttttt tcccctattc tcaagaccca tcaagacgct cacttcgctg 1140
   agcctttcgc catgccgtca aagctagcca ttagttccat gtccctaggg cgctgctttg 1200
   ccggccactc tctggacagc aagcttgatg ccgctcaacg atacggctat cttggtatcg 1260
   agctttttta tgaggatctg gtcgacgttg cagagcattt gtcgaacgag cgtccctctc 1320
   ccgaaggccc ttttgtcgaa gctcagatag ccgccgctcg tcatattctc cagatgtgtc 1380
   aagccagggg gcttgaggtc gtctgcctcc agcctttcat gcactacgac ggccttaacg 1440
   acagggcaga acatgagcgt cgtctggaga agctagcact atggattgag ctcgctcatg 1500
   agcttcacac cgacatcatt cagatcccag ccaacttcct ccctgccaac caagtcagtg 1560
   acaacctcga cctgattgtc tcagatcttt gcaaggtggc cgatattgga gctcaagctt 1620
   tgccccctat ccgctttgcc tacgagagtc tttgctggag cacccgtgtc gacctctggg 1680
   agcgctgctg ggacatcgta caacgcgttg accgccccaa ctttggcatt tgccttgaca 1740
   ccttcaacat cctcggccgc atctatgccg accctacatc tcctagcggt aggacaccca 1800
   acgcaaaaga ggcagtcagg aagtccatcg ccaacttggt ctcgcgcgtg gatgtctcca 1860
   aagtcttcta cgtccaggtg gttgacgccg agaggctgag caagccacta ctgcccggtc 1920
   acccgtatta caatccagag cagccggcga ggatgagctg gtcgcgcaat tgtagactgt 1980
   tctacggcga aacagaatat ggtgcgtatc ttcccgtgaa ggaggttgct cgagcccttt 2040
   tccacggcat tggtttcgag ggctgggtca gtttggagct tttcaaccgc agaatgtctg 2100
   aggagggacc tgaagtgccg gaggaacttg ccatgagagg cgctatctcg tgggccaagt 2160
<210> 6
   <211> 348
   <212> PRT
   <213> Artificial Sequence
<220>
   <223> QutC from Aspergillus nidulans encoded by the qutC gene;
<400> 6
   Met Pro Ala Asn Leu Lys Ile Gly Ile Pro Thr Val Ser Leu Ser Lys
   1               5                   10                  15
   Pro Gly Leu His Ser Leu Asp His Lys Leu Arg Ser Ala Ala His Gly
               20                  25                  30
   Phe Ala Gly Ile Glu Leu Phe Ile Asp Asp Leu Ser His Phe Ala Ser
           35                  40                  45
   Ser Ser Phe Asn Gly Ser Leu Thr Gln Ala Ala Lys Tyr Ile Ser Ser
       50                  55                  60
   Leu Ala Lys Gln Leu Asn Leu Thr Phe Ile Cys Leu Gln Pro Phe Gly
   65                  70                  75                  80
   Phe Tyr Glu Gly Leu Val Asp Thr Asn Gln Ser Thr Tyr Leu Leu Thr
                   85                  90                  95
   Glu Lys Leu Pro Leu Trp Phe Ala Ile Ala Arg Ile Ile Gly Thr Asp
               100                 105                 110
   Leu Ile Gln Ile Pro Ala Asn Phe Leu Gln Asn Asp Pro Val Thr Gly
           115                 120                 125
   Ala Ala Arg Thr Ser Gly Asp Ile Arg Leu Ile Val Ser Asp Leu Gln
       130                 135                 140
   Thr Ile Ala Asp Ile Gly Val Lys Gln Gly Phe Arg Phe Val Tyr Glu
   145                 150                 155                 160
   Ala Leu Cys Trp Ser Thr His Val Asp Thr Trp Glu Ala Ala Trp Asn
                   165                 170                 175
   Val Val Lys Leu Val Asp Arg Glu Asn Phe Gly Ile Cys Leu Asp Ser
               180                 185                 190
   Phe Asn Thr Arg Thr Pro Leu Pro Ser Leu Gly Arg Arg Arg Met Leu
           195                 200                 205
   Ser Lys Pro Trp Pro Ser Pro Trp Arg Arg Ser Val Leu Ser Ser Pro
       210                 215                 220
   Val Glu Asn Trp Thr Ser Gly Lys Ser Ser Thr Ser Ser Leu Ser Met
   225                 230                 235                 240
   Ala Ser Gly Cys Arg Arg Arg Trp Thr Arg Ser Thr Pro Phe Met Trp
                   245                 250                 255
   Arg Ala Asn Pro Arg Arg Met Ser Trp Ser Arg Asn Ala Arg Leu Phe
               260                 265                 270
   Pro Cys Glu Glu Glu Arg Gly Gly Tyr Leu Pro Val Leu Glu Ile Ala
           275                 280                 285
   Arg Ala Phe Phe Glu Ile Gly Phe Glu Gly Trp Val Ser Leu Glu Leu
       290                 295                 300
   Phe Ser Arg Thr Cys Asn Asp Pro Asp Val Asn Thr Val Gly Glu His
   305                 310                 315                 320
   Ala Arg Arg Gly Met Asp Arg Arg Arg Arg Val Val Ala Ala Leu Gly
                   325                 330                 335
   Leu Asp Val Glu Val Pro Ala Arg Asn Cys Glu Cys
               340                 345
<210> 7
   <211> 3298
   <212> DNA
   <213> Artificial Sequence
<220>
   <223> qutC gene from Aspergillus nidulans plus surrounding sequences;
<400> 7
   aagcttggtt tcaagtgatg atatatagtt atgaggatat aatatgaacc gaaagacgat        60
   gtttcttgtg aatatttacg tgatagttgt ctgtctaata tggtacagca gtagaacaac       120
   tacatacggt cactacttac agccctagtc attccctccc tcgattgcct accatttata       180
   cactttgaac atccacaggc ttgcctccct ccatactctc cctaacagct tgaacaactc       240
   tgagcgccct caccccatct tcaacgccac agccaactcc tcgctcgcca tcctcaccct       300
   ttccactaac aacatcaaca aaatatcccc actgcgcatc aaacggcctc acatcagcat       360
   ccttcactga aatctgctgc atcgccaact ccgtattcca gcctttctct ttcccctgtc       420
   cacaagaaac atggtcatag ctccagcgcg tcatatcagg cacactgaga ctcgctctgg       480
   ttccaagaat tcgataacag tcgcttgcac tcttggaagg agcgggcgga atcgtaggat       540
   tctcgcccgt tcctgtttca aagttcaacg gcgaaggcgt cgcgtcgcag atgagaaatg       600
   tgcctactat cccagacgca aagcgcagcg tcacagcaca gccttcctcg gcggtatgtt       660
   ccgggttctg gcgcatgcgc tgcaggagtg taccctccgc gtagacccta ctgacgggcc       720
   caaacagaaa ctgcagcacg tcgatatcgt ggataagatt aatccccagc acgccgccct 780
   tcttcttatc tgcgcgccaa gaaccgagcg gcggcgcgaa gtaagaggcc ggcttcagaa 840
   gtgtccagag gccgttcact gcaacgacgg tgccgagtga gtctgtctct aacaaagact 900
   ttgtggtttg gatgtacgga ttgaagcggc ggtggtggcc gatctggatg ttgatcttcg 960
   catccttgcc tctcttctca tctttacatt tctgttcctt gacggtagcg aggaggtgct 1020
   cggccgactc cagatcgtca ctgatcggtt tctcaaggag gatattgcgg attccgttct 1080
   ccagcagctg gagcgtgacg tccacgtgcg tgtgattggg cgtgctcacg atcgccgcgt 1140
   ctggtttccc ggttgtctta ccgacaacgt ctaacataga cgtaatagaa tcatagcaag 1200
   gaacgccaaa tgattctgcg accgggattg cagagggtga agggtcaaca aaagcgatca 1260
   gctgggttcg tgggtgtcgt tgcacggatt gtgcgtgacg gggcccaata agtccggcac 1320
   cgacgatgac aatgaggata ttcttgtcct tttccttgct gcagggcacc attgtgcatg 1380
   tcggtggctg gaaataaaca gaacagggat atggtcaagt cggagaatcg gtgcaggata 1440
   gaccggctac ttgatgtagg acgacagtcg cgatctaccg agagcgtgag attcactgtg 1500
   ggactgattt atgtaatttg aggcgcagca gacttaggga cttgaaatgt ggctgtctgt 1560
   ggatgcattt gcggggtatg gagtacagag tgcatacagc tgtgtatatg gagttcctta 1620
   cggagagggt gacctggtat ggggagaacg ggcaaaatgc tcacccggca acctctcaaa 1680
   gcgtttaccc ggtatactcc tctgatatca atatttccaa tcagcaccta tatcatcacg 1740
   acgctctcct gaggattccg tagctaaccg ccctggatcc tacattaata aataagccat 1800
   ttgctttttc tgctgcgagt gtgattctca atacgattac gtatcacatg cagattgcct 1860
   ttacttcagc tgcatttgat cagccacagc tctaagagca aacataccct acctacctac 1920
   ctacttcgcc tagggtacat aatcaccgcc atctcctcct cgatcagtct tcaactcaat 1980
   cagctcattc attctattct taatataata tataccttta gatctccagc agagacccga 2040
   agagtcggca attcaaaatg cccgcaaacc tcaaaatcgg tatcccaacc gtgtccctgt 2100
   caaaaccggg cctgcactct cttgaccata agctccgctc ggccgctcat ggcttcgcgg 2160
   ggatcgagct gtttattgat gacctctccc atttcgcctc atcgtcattc aatggctccc 2220
   tcactcaagc ggcaaagtat atctcctcgc tcgccaagca acttaacctc acatttatct 2280
   gcctgcaacc attcggtttc tacgagggtc tggtggacac aaatcagtcg acgtacctgc 2340
   tcactgagaa actcccgctc tggtttgcga tcgcccgcat tataggcaca gatctcatcc 2400
   aaatccccgc aaatttcctc cagaatgacc ctgtcaccgg ggctgcacga acaagcggcg 2460
   acataaggct tatcgtctca gatctgcaga cgatcgcaga tatcggtgta aagcagggct 2520
   tccgctttgt gtacgaggcg ctctgctggt cgacgcatgt cgatacatgg gaagcagcgt 2580
   ggaatgtcgt caagctggtt gatagagaga atttcgggat ctgcctggat agcttcaaca 2640
   cgcggacccc gcttccgtca ctgggaagac gccggatgct gagcaagccg tggccaagtc 2700
   catggagacg ctccgttctc tcgtctccag tggagaactg gacatcagga aaatcttcta 2760
   catccagctt gtcgatggcg agcggttgtc ggcgccgctg gacgagaagc acccctttca 2820
   tgtggagggc caacccccga agaatgagct ggagtcgcaa tgcgcggtta ttcccctgtg 2880
   aagaggagag gggtgggtat cttcctgtgt tggagatcgc gagggcgttc tttgaaatcg 2940
   ggttcgaggg gtgggtgagt ctagagctgt tttcaaggac gtgtaatgat cccgatgtga 3000
   acacggtggg ggagcatgcg agacgtggga tggatagaag gaggagggtt gttgcggcgc 3060
   taggactcga tgttgaggtg ccagcacgta actgtgaatg ttagcatgaa cggcaaggag 3120
   agggtggagg tgcaggtgca ggaggagctg gctgttcagc atcggctgta ggtagtggta 3180
   tcttgaaagg acgatagggt ttgatctaga gatttttatt ttgtctaatt actggtaatg 3240
   atggcctcat gcacgctgtt gaacacgctg tacaacatca ctgttgaaga tgatacct 3298
<210> 8
   <211> 502
   <212> PRT
   <213> Artificial Sequence
<220>
   <223> AroY from Klebsiella pnemoniae ATCC25597;
<400> 8
   Met Thr Ala Pro Ile Gln Asp Leu Arg Asp Ala Ile Ala Leu Leu Gln
   1 5 10 15
   Gln His Asp Asn Gln Tyr Leu Glu Thr Asp His Pro Val Asp Pro Asn
   20 25 30
   Ala Glu Leu Ala Gly Val Tyr Arg His Ile Gly Ala Gly Gly Thr Val
   35 40 45
   Lys Arg Pro Thr Arg Ile Gly Pro Ala Met Met Phe Asn Asn Ile Lys
   50 55 60
   Gly Tyr Pro His Ser Arg Ile Leu Val Gly Met His Ala Ser Arg Gln
   65 70 75 80
   Arg Ala Ala Leu Leu Leu Gly Cys Glu Ala Ser Gln Leu Ala Leu Glu
   85 90 95
   Val Gly Lys Ala Val Lys Lys Pro Val Ala Pro Val Val Val Pro Ala
   100 105 110
   Ser Ser Ala Pro Cys Gln Glu Gln Ile Phe Leu Ala Asp Asp Pro Asp
           115                 120                 125
   Phe Asp Leu Arg Thr Leu Leu Pro Ala His Thr Asn Thr Pro Ile Asp
       130                 135                 140
   Ala Gly Pro Phe Phe Cys Leu Gly Leu Ala Leu Ala Ser Asp Pro Val
   145                 150                 155                 160
   Asp Ala Ser Leu Thr Asp Val Thr Ile His Arg Leu Cys Val Gln Gly
                   165                 170                 175
   Arg Asp Glu Leu Ser Met Phe Leu Ala Ala Gly Arg His Ile Glu Val
               180                 185                 190
   Phe Arg Gln Lys Ala Glu Ala Ala Gly Lys Pro Leu Pro Ile Thr Ile
           195                 200                 205
   Asn Met Gly Leu Asp Pro Ala Ile Tyr Ile Gly Ala Cys Phe Glu Ala
       210                 215                 220
   Pro Thr Thr Pro Phe Gly Tyr Asn Glu Leu Gly Val Ala Gly Ala Leu
   225                 230                 235                 240
   Arg Gln Arg Pro Val Glu Leu Val Gln Gly Val Ser Val Pro Glu Lys
                   245                 250                 255
   Ala Ile Ala Arg Ala Glu Ile Val Ile Glu Gly Glu Leu Leu Pro Gly
               260                 265                 270
   Val Arg Val Arg Glu Asp Gln His Thr Asn Ser Gly His Ala Met Pro
           275                 280                 285
   Glu Phe Pro Gly Tyr Cys Gly Gly Ala Asn Pro Ser Leu Pro Val Ile
       290                 295                 300
   Lys Val Lys Ala Val Thr Met Arg Asn Asn Ala Ile Leu Gln Thr Leu
   305                 310                 315                 320
   Val Gly Pro Gly Glu Glu His Thr Thr Leu Ala Gly Leu Pro Thr Glu
                   325                 330                 335
   Ala Ser Ile Trp Asn Ala Val Glu Ala Ala Ile Pro Gly Phe Leu Gln
               340                 345                 350
   Asn Val Tyr Ala His Thr Ala Gly Gly Gly Lys Phe Leu Gly Ile Leu
           355                 360                 365
   Gln Val Lys Lys Arg Gln Pro Ala Asp Glu Gly Arg Gln Gly Gln Ala
       370                 375                 380
   Ala Leu Leu Ala Leu Ala Thr Tyr Ser Glu Leu Lys Asn Ile Ile Leu
   385                 390                 395                 400
   Val Asp Glu Asp Val Asp Ile Phe Asp Ser Asp Asp Ile Leu Trp Ala
                   405                 410                 415
   Met Thr Thr Arg Met Gln Gly Asp Val Ser Ile Thr Thr Ile Pro Gly
               420                 425                 430
   Ile Arg Gly His Gln Leu Asp Pro Ser Gln Thr Pro Glu Tyr Ser Pro
           435                 440                 445
   Ser Ile Arg Gly Asn Gly Ile Ser Cys Lys Thr Ile Phe Asp Cys Thr
       450                 455                 460
   Val Pro Trp Ala Leu Lys Ser His Phe Glu Arg Ala Pro Phe Ala Asp
   465                 470                 475                 480
   Val Asp Pro Arg Pro Phe Ala Pro Glu Tyr Phe Ala Arg Leu Glu Lys
                   485                 490                 495
   Asn Gln Gly Ser Ala Lys
               500
<210> 9
   <211> 5502
   <212> DNA
   <213> Artificial Sequence
<220>
   <223> aroY gene of Klebsiella pneumoniae 342 plus 2 kilobases of
   surrounding DNA sequences;
<400> 9
   gcgacgccga ctgggcgatc cgtgaactgc tggcgcgtat gacccagcgt ctgcagggct 60
   gtgaaaccat agaggatgtg attaaggtgg cggagctgtt cgcgccgaac atcgccccga 120
   cgatccccgg taaactgtat attctggata ccgatccatg gcagatgcgc tgcgtggcgc 180
   agtggctgtc gcccgccggg gagacgacgt cctttgctcc cgacgactgc tgggcgatac 240
   ggcggggact cagccatccg ccggtgcagg gtgagcccga tatcacctgc tatcatctgc 300
   cggaggcgca cgccggccag tcgctctgcg taccgctcat cgcccagggc gaagcgatcg 360
   gtctgctgag ctttcagaac gtcaccgcca gtgacgcccc ttcccgggct tacctggagc 420
   tgatggccga agcgctgggg ctggcgctcg ccaatcagcg tttacgcagc gccctgctgg 480
   aaaaagcgtt gttcgattcg ctgaccggcc tgcgtaaccg ccatcatctt gatgaagcgc 540
   tgcactcgca gatggcgctg gcggtccata cccacacccc gctgagctgc ctgatgatcg 600
   acatcgatca cttcaaagcc atcaatgacc gctacggcca tgaagccggg gatctggtga 660
   ttaagagcgt cgcgaccatt gtgcagcgcg cggtgcgcga tatcggcatg gctttccgct 720
   acggcggcga ggagttttta gtgctgctcc ccgggattga cgaagccggg gcgcaccagt 780
   gcgccagcga gatctacacc caggtgcaca atatgacgct gcgcgatggc ctgacggaga 840
   taggccaggt ggatgtgtcg attggcatcg ccagctaccc gcagcacacc caaagcgaca 900
   gcctgctgcg cgcagcggac gccgcgctgt accgggcgaa agagctgggc cgttcaagga 960
   ttgtcagctt tggccgcctg aagacccgct aagcgggatt attgctcagc ggcattaagc 1020
   agcgagataa ctttccgcac caccgccgaa cggaaatggc ggtggtaaac catgctcagc 1080
   tcactctccg ccagccgatc ctgaagatcg atatacacca cgttatcaag gcgtaaggcc 1140
   ctcgccgaga ccggcaccag cgccaccccc accccggcgg agaccaggct aatcatcgag 1200
   gtgacatcgt taattcgctg caccacctgc ggcgtaaaac ccgcgacgcg acaggcgtca 1260
   ataaatacct gctccagtcc ggtgccctgc ggatcgtcaa gcgagatcca gttgtcagtg 1320
   cgcaacgagg ccagattgag cgcccccacg cctgccagcg gatgctgttg ataaagcgcc 1380
   aggcaaagtt tttcccgcac aaatggcctg accaccagcg cgtccggcgg tgacgccagc 1440
   ggcgcgcgga tgatggcgat atccagacgc agatccagca gcgcttcgta gagcatttgc 1500
   acatccccct gcaccagcga cagctcaatc cccggccagt cagcgcgcag ctcgcgcagg 1560
   agccccggca gtttgctgtc atacatcgca ctggagacat agcccagatg caatcgcccc 1620
   tgctcgcctc gtgcggtgcg ctgggcgtcc aggaccgcct gatcggccat ctccagcgcc 1680
   agccgcgtct tctgcaagaa ggcctcgccc gcggcggtga gggtcaggcg ccggttagcg 1740
   cgggagaaga gcaccacgcc caggcgctgc tcgagttgtt taatctgctg gctgagggcg 1800
   ggctgggcga tatgtaaccg ctctgccgcc cgatgcatat gtagttcttc agcaacgacc 1860
   acaaaatggc gtaacgctcg caaggacatg gccggactcc gcggagtaaa ttgataataa 1920
   aaatgttatc aataaagcat gaatgatgca attgataacc attagcctgc gagcatactg 1980
   tgcgcatcga cacgctaagg agaacatcat gaccgcaccg attcaggatc tgcgcgacgc 2040
   tatcgcgctg ctgcaacagc atgacaatca gtacctcgaa accgatcatc cggttgaccc 2100
   taacgctgag ctggccggcg tctatcgcca catcggcgcg ggcggcaccg tgaagcgccc 2160
   cacgcgcatc ggcccggcga tgatgtttaa caatattaag ggctatccgc actcgcgcat 2220
   tctggtgggc atgcacgcca gccgccagcg ggccgcgctg ctgctgggct gcgaagcctc 2280
   acagctggcg ctggaggtag gcaaagcggt gaaaaaaccg gtcgcgccgg tggtcgttcc 2340
   ggccagcagc gccccctgtc aggaacaggt ctttctggcc gacgatccgg attttgattt 2400
   gcgcaccctg ctcccggcgc ccaccaacac cccgatcgac gccggtccct tcttctgcct 2460
   gggcctggcg ctggccagcg atcccgacga cgcctcgctc accgacgtca ccatccaccg 2520
   cttgtgcgtc cagggccggg atgagctgtc gatgtttctc gccgctggcc gccatatcga 2580
   agtgtttcgt cagaaagccg aggccgctgg caaaccgctg ccgataacca tcaatatggg 2640
   actcgatccg gctatctata tcggcgcctg ctttgaagcg ccgaccacac gtttggctat 2700
   aacgaactgg gcgtcgccgg tgcgctgcgt cagcgtccgg tagagctggt acagggcgtc 2760
   agcgtcccgg agaaagccat cgctcgcgcc gagatcgtta tcgaagggga actgctgccg 2820
   ggggtacgcg tcagagaaga tcagcacaca acagcggcca tgcgatgccg gaatttcctg 2880
   gttactgcgg cggcgccaat ccgtcgctgc cggtcattaa agtcaaagcg gtgaccatgc 2940
   gaaacaatgc gattctgcag acgctggtag ggccgggcga agagcatacc accctcgccg 3000
   gattgccaac ggaagccagt atctggaatg ctgtcgaggc tgctatcccg ggctttttac 3060
   aaaatgtcta cgcccacacc gcgggcggcg gtaaattcct cgggatcctg caggtgaaaa 3120
   aacgccagcc cgccgacgaa gggcgtcagg ggcaggccgc gttgctggcg ctggcgacct 3180
   attccgagct gaaaaatatc attctggtcg atgaagatgt cgatatcttt gacagcgacg 3240
   atatcctgtg ggccatgacc acccgcatgc agggggatgt cagcatcacg acgatcccag 3300
   gcattcgtgg tcaccagctg gatccttccc agaccccggc ctacagcccg tcgatccgcg 3360
   gagagggtat cagttgcaag acgattttcg attgcacggt gccgtgggcg ctaaaatcac 3420
   acttcgagcg cgcaccgttt gccgatgtcg atccgcgtcc gtttgcgccg gagtattttg 3480
   cccggctgga aaaaaaccac ggtcagtaaa atcaggtgat agccgccgga gcacggcggc 3540
   atcttccggg ccagcatcac ctgcagcggg tggctgacgc agggttagtt gatcgcggcg 3600
   gagaggtctt ttttcacctg ctcacgctgc tcgggggtca acacctggct cacgtcgaag 3660
   tagtatttca cacgataata ccgcacctgc tggtccagct ggccaaaggc ggccagctgc 3720
   tgtttgacct tagcgtcatc ccatttcccg gagtgaataa cgtctgccag ggcaccatcc 3780
   tgatagccgc tgatttaatc tggcttacat tgttttcgaa tccctgacgc agcgcctgga 3840
   ttttggcgac ctgctcttca ctcagcttca ggtgctggac gaccggatcc tgcgagacag 3900
   acggtatatc ggcggaggtc gacgcctggc tggctgccgt aaagcaggtg gtcagcgcaa 3960
   tggcgagcag ggtgttacgc aagcgagtat tcacagtgaa tgatccttca aaaaagaaaa 4020
   tgagaggcga ttatcactgc gctaataaag actatctgta acaaagggtt aatttaaaac 4080
   tggataaaaa aaggatggta agaaacagaa atcagatccc gggtcagcag cacagaaaga 4140
   tatattcatc cttccagtaa cggccctgtc caatgatatc cccggcggcg ctgattaact 4200
   gtttttgctt ttggtttcaa tcccctcaac gatcacatgg ctggtcaggg tatgaataga 4260
   ttgcaacagc ccgggaaaag cggggtcgtt ttctttatcc cagaagtaat ctttatccac 4320
   tttgacgcaa tcgaagcgga agcgctccag cagcgggaag gacgtcgtcc gcggccaaaa 4380
   tcatccagcc agaccgggca aagcgccgcc agcgtgctca gcgccgtcag ctcacgtccg 4440
   gcgataaact cgtgaaagtt ctcatttatt tccagagcaa tgtgtttaca ggagcgcagg 4500
   aaatcacaga gatagcgatc cgtcagaata aagtggctta ataaatcatc aatattcagc 4560
   gatatcggtt tattatcgac ccgggcgaaa tcaaatacag aaagtaactc gatttgccga 4620
   ataaatagag caagcttatc gcgttcggta agcgtggaga aagaaaacgt cgacgcagag 4680
   gtggtatttt gtgcgggtgc tataatatct ttagtaagca actcccacga gtggtagctg 4740
   ccgtcatcgc taatagcggg ctccagaacg aagcgataag aggtattttt cacgttttct 4800
   tcaaccattt aaaaaatacc aaaaataaga aagggttaag catgtcatat attttccgcc 4860
   aacaaaaata gtttaaagtg atcgataata atcattcgat agttaaaaac tatcaagata 4920
   taatttattg atcggtaaat tgaattaata taaattagcc actgccgtaa ctccctctga 4980
   aaagtcaatt aaaatattgt ttcaaaccag ccagttacca gagtattctg cgtaaagcct 5040
   ggtcgtctca cgctttgtgc tgccaggtaa aaaaagagag gggtaataaa aatgaaaaat 5100
   acaagccgcc agttttagtc atatcattat gccgaatatg aataacgctg cgctgaggcg 5160
   ccgcttcgcc tggcatgcca tgagtcctca acaaaaaagt gtgactcagt cgacaaaacg 5220
   tcatattttc ccgctatcct gcagcgaaga agagtgaagt ggatgacagg cagtgaaaaa 5280
   aataaacgtg attccgctgg ggctgatgct attcatgctc atcgccagcg catggctggg 5340
   ccctgcgccg cggcacaccg gcagcatgca gtgcgtttgg tttgacgggg caatggtgag 5400
   ctgcctgccg aagcaacgac tgggcgaagg ctcgccgcat catttactgg tcagacgata 5460
   aaccggtact cgccgggtgg tgttgaacag attatcgctg gc 5502
<210> 10
   <211> 4629
   <212> DNA
   <213> Artificial Sequence
<220>
   <223> catA gene from Acinetobacter baylyi ADP1, including 410 bases of
   upstream sequence and two open reading frames downstream;
<400> 10
   atctgctcga ccatagtaat gatcacatta tgagctaaat ttacttttta aaatttaaat        60
   atattatata tatttgaatt ttattgtttt attttaattt ttagcttaga agtttttatt       120
   aagatttatt tttaaattag atgtcgaaaa aattagtata ccaaaaaagc atgaaaacat       180
   actctcttag gaattggagt cgccatgagt ttcagataca gttgatcagt atggaaggta       240
   tagaaacgac tatcgaaata aataagtttg tggtgtgtga agcaaggtaa agctcaaggc       300
   tgaggcaaac caagcaaagg ttaattgaac cgatatgcac aacacattca acgatagcgt 360
   cgacagataa gtttatcaaa tgatgttttg gcgatttcaa ggagaaagcc atggaagtta 420
   aaatattcaa tactcaggat gtgcaagatt ttttacgtgt tgcaagcgga cttgagcaag 480
   aaggtggcaa tccgcgtgta aagcagatca tccatcgtgt gctttcagat ttatataaag 540
   ccattgaaga tttgaatatc acttcagatg aatactgggc aggtgtggca tatttaaatc 600
   agctaggtgc caatcaagaa gctggtttac tctcgccagg cttgggtttt gaccattacc 660
   tcgatatgcg tatggatgcc gaagatgccg cactaggtat tgaaaatgcg acaccacgta 720
   ccattgaagg cccgctatac gtggcaggtg cgcctgaatc ggtaggttat gcgcgcatgg 780
   atgacggaag tgatccaaat ggtcataccc tgattctaca tggcacgatc tttgatgcag 840
   atggaaaacc tttacccaat gccaaagttg aaatctggca tgccaatacc aaaggctttt 900
   attcacactt cgacccaaca ggcgagcagc aggcgttcaa tatgcgccgt agtattatta 960
   ccgatgaaaa cggtcagtat cgcgttcgta ccattttgcc tgcgggttat ggttgcccac 1020
   cagaaggtcc aacgcaacag ttgctgaatc agttgggccg tcatggtaac cgccctgcgc 1080
   acattcacta ttttgtttct gccgatggac accgcaaact aactacgcaa attaatgtgg 1140
   ctggcgatcc gtacacctat gacgactttg cttatgcaac ccgtgaaggc ttggtggttg 1200
   atgcagtgga acacaccgat cctgaagcca ttaaggccaa tgatgttgaa ggcccattcg 1260
   ctgaaatggt tttcgatcta aaattgacgc gtttggttga tggtgtagat aaccaagttg 1320
   ttgatcgtcc acgtctagcg gtgtaataca ccaaaatggt tcaaaattat caggcgagtg 1380
   atcatgatca ctggcctgtt tttatttcag ggaagggtgg agacaattac gtggacaatc 1440
   aaatcattca ggaaaccgta gataaaattt taagcgtatt gccgaatcag gctgggcaat 1500
   tggcacgctt ggttcgtctg atgcagtttg cttgtgaccc caccattacc gtcattggta 1560
   aatataatca tggtaaaagc cgactactca atgagctgat cgggacagat attttttctg 1620
   ttgccgataa acgagagacg attcaactgg ccgaacataa acaagatcag gtgcgttggt 1680
   tggatgcacc cggactcgat gcagatgttg cggcagtgga tgatcgtcat gcttttgaag 1740
   cagtctggac acaggcagat attcgccttt ttgtgcattc agtccgagaa ggcgaactcg 1800
   atgcaactga gcatcatctt ttacaacaac ttattgaaga tgcagaccat agccggcgcc 1860
   aaaccatact ggtcttgacc cagatagatc agataccgga tcagacaatt ttaacccaga 1920
   ttaaaacctc aattgcacag caggtaccca aactcgatat ttgggctgtt tcggccactc 1980
   gccaccgtca gggtattgaa aatggaaaaa ccttgctgat cgaaaaaagt ggaatcggcg 2040
   cgttacgaca tacacttgag caggcacttg ctcaggttcc atctgcacga acgtatgaaa 2100
   agaatagatt gctgtctgac ttgcatcatc aacttaagca gttattactc gatcaaaaac 2160
   atgtacttca gcaactacaa cagacacagc agcagcaatt gcatgacttt gatacaggac 2220
   tcatcaacat actcgataag attcgagtag atcttgagcc cattgtaaat atagatggtc 2280
   aagaccaagc actcaatcca gattcatttg ccacgatgtt taaaaataca gcagccaagc 2340
   agcaacgtgc caaagtgcag attgcttact cacgtgcctg tatcgagatc aatagccatc 2400
   tcatacgtca cggtgtggtg ggtttacccg cagagcaaca aaccacgata aaaagtattg 2460
   atacggtcat tgttgcggtt tttggaattt cagtgaaatt tcgcgatcag ctacgtgcat 2520
   tgttttatac cgataccgaa cgacaacgct tgcaaagaga gtttcgattt tattttgaaa 2580
   agtcagcagg ccgaatgatt ttagctgcca agattgagca gacaatgcgg cagcaagggt 2640
   gtattcaaaa tgcaatgatg gcgttgcaac agatggagag tgcagcatga ccagcggcgg 2700
   acacattcaa ttgtttatcg aacacacccg gcagattgcg actgcccaag gggatataca 2760
   gttggcattg caatcgatgc agcaatggcg cgaagcattt gctacagcat taaaacaaaa 2820
   tacctttgat ttaacgggct ggtcaccgca gacaaagatc gccaatcaac tcaagcaatt 2880
   taaccataag cttacaacgc atgtatcgaa ttgggatacc gaatggcata cttttagtgc 2940
   tgctcaatcg gttgcagaag tatttcatga tcgggtgatg ttgcttgtat tcggtaagtt 3000
   taatgccgga aagagttcat tgtgtaactt actggccgaa tgctttcgtt ctcacgaaca 3060
   aaccgtgcaa tattttcatg ttcaaaatga acagatattt tataccgaat ctcacttacg 3120
   cgaaggtgca accgagacga cagcgcaact acagggcgta tgtctgggtg aaaaacttat 3180
   tttgctagat acaccaggtt tgcattctgg tactcagaaa aatgcagcgc tcacacaaaa 3240
   atttatcgac agtgcagatg gtgtgctgtg gctcagtagc gcaacttcac cgggtcaggt 3300
   gcaagagcta gatgcactgg ggcgcgagtt aaagcgtcat aaacctttat ttcctgttat 3360
   tacccgaagc gattttgtcg aagaagatga aattgatggt gagctatgta cagtgctttg 3420
   caataaaaat tcagaacaac gtgcgttgca agagtctgat gtattgatgc gtgcgaaaga 3480
   aaaactgcac atatgcaagt ggatgtgagt ttattaaagc cgcccgtgtc cgtttcaact 3540
   caaatggcgc gtgaagcaga tatgaaccca caagccatga acgaggctgg ttttgagcga 3600
   ttatttgcag cacttttggc tcttattgag cctgctttgc gctataagca gcgtaaacct 3660
   gccgaagtat tgttgcattt tttgcaagaa catatcattg aaggtttaag gttttacctg 3720
   caacccgatc tagagcaaat acaacaggac ctcaaacagg ctcaagatga tttacgacag 3780
   ctacacaccg atttagccga ggcagtctgg cgtagcgtat tgcctgagct accacaactt 3840
   cttgagcaac atgcaagtac acaaaatatt gatgccgtag tgaacagttt gaacgagtgg 3900
   ataaacgtcg cattcgaaca acagcttgca attcagcttg atgcttatgg tttaaatttg 3960
   gattcgctta gcaagatcga aaaaaccgaa aaaatgcagt atgaacgcat tgcgggaatg 4020
   gtggtgcatg atggcttgta cacgactctc acgcagcaga ttcaacaagc tgtcaaagct 4080
   tctacgagtg aattgattga tcagtgtcag gctcaacttg agcagtcaat caaacatgtt 4140
   caaacactcg atgaaacctt catcgattac agcgcagcac tcgatcaact cagccaagcg 4200
   ctacgcattg aataaagagc agtaaatttt tcagacatat tttattcgat gagtggcctg 4260
   atatggtgcg ttgcaaacac ctcctgtaca caggcgagaa ttttaggaat gtaattactg 4320
   tggtccatat ttcgcaccgc gagtgaaatt gggctatagg catcatcatc taaaattgga 4380
   atataaagta gattcttcac cccaatatcc atggcagacg ccggtacgat gcagacgcct 4440
   tcacctgctg ccaccaagcc gagtgccagt tgaatttctc gaatttcggt gagtttggat 4500
   ggtactaggc ctagttcggt aaagagtgac tgaataaagg tcgcaaaatt gggcttttga 4560
   gagactgggt acagcagcat cggttcatca ataatttgag agagatgaac ccctgttgct 4620
   gcaaactga 4629
<210> 11
   <211> 311
   <212> PRT
   <213> Artificial Sequence
<220>
   <223> CatA from Acinetobacter baylyi ADP1;
<400> 11
   Met Glu Val Lys Ile Phe Asn Thr Gln Asp Val Gln Asp Phe Leu Arg
   1 5 10 15
   Val Ala Ser Gly Leu Glu Gln Glu Gly Gly Asn Pro Arg Val Lys Gln
   20 25 30
   Ile Ile His Arg Val Leu Ser Asp Leu Tyr Lys Ala Ile Glu Asp Leu
   35 40 45
   Asn Ile Thr Ser Asp Glu Tyr Trp Ala Gly Val Ala Tyr Leu Asn Gln
   50 55 60
   Leu Gly Ala Asn Gln Glu Ala Gly Leu Leu Ser Pro Gly Leu Gly Phe
   65 70 75 80
   Asp His Tyr Leu Asp Met Arg Met Asp Ala Glu Asp Ala Ala Leu Gly
   85 90 95
   Ile Glu Asn Ala Thr Pro Arg Thr Ile Glu Gly Pro Leu Tyr Val Ala
   100 105 110
   Gly Ala Pro Glu Ser Val Gly Tyr Ala Arg Met Asp Asp Gly Ser Asp
   115 120 125
   Pro Asn Gly His Thr Leu Ile Leu His Gly Thr Ile Phe Asp Ala Asp
       130                 135                 140
   Gly Lys Pro Leu Pro Asn Ala Lys Val Glu Ile Trp His Ala Asn Thr
   145                 150                 155                 160
   Lys Gly Phe Tyr Ser His Phe Asp Pro Thr Gly Glu Gln Gln Ala Phe
                   165                 170                 175
   Asn Met Arg Arg Ser Ile Ile Thr Asp Glu Asn Gly Gln Tyr Arg Val
               180                 185                 190
   Arg Thr Ile Leu Pro Ala Gly Tyr Gly Cys Pro Pro Glu Gly Pro Thr
           195                 200                 205
   Gln Gln Leu Leu Asn Gln Leu Gly Arg His Gly Asn Arg Pro Ala His
       210                 215                 220
   Ile His Tyr Phe Val Ser Ala Asp Gly His Arg Lys Leu Thr Thr Gln
   225                 230                 235                 240
   Ile Asn Val Ala Gly Asp Pro Tyr Thr Tyr Asp Asp Phe Ala Tyr Ala
                   245                 250                 255
   Thr Arg Glu Gly Leu Val Val Asp Ala Val Glu His Thr Asp Pro Glu
               260                 265                 270
   Ala Ile Lys Ala Asn Asp Val Glu Gly Pro Phe Ala Glu Met Val Phe
           275                 280                 285
   Asp Leu Lys Leu Thr Arg Leu Val Asp Gly Val Asp Asn Gln Val Val
       290                 295                 300
   Asp Arg Pro Arg Leu Ala Val
   305                 310
<210> 12
   <211> 1461
   <212> DNA
   <213> Artificial Sequence
<220>
   <223> quiC from Acinetobacter sp. ADP1;
<400> 12
   atgaaattaa cttctttacg cgtatcttta ttggcgctgg gcttggtaac atcaggtttt        60
   gctgcggcag aaacttatac tgtagatcgt tatcaggatg atagtgaaaa aggctctttg       120
   cgttgggcaa ttgaacaatc taatgcaaat agcgcacaag agaatcagat tctgattcag       180
   gctgttggta aggcacctta tgtgatcaag gtggataaac cgttaccacc gattaaatca       240
   tctgtaaaaa ttattggtac agaatgggat aaaacgggcg aatttattgc gattgatggt       300
   tcaaactata tcaagggcga aggcgaaaaa gcatgtccag gtgcaaatcc aggacaatat       360
   ggtaccaatg ttcgtaccat gactttacca ggtttggttc tacaagatgt caatggtgtg       420
   accctgaaag gtcttgatgt tcatcgcttc tgtattggtg tactggtaaa tcgttcaagc       480
   aataatttga ttcagcataa ccgtatttca aataattacg gtggcgctgg tgtcatgatc       540
   acgggtgatg atggtaaagg taacccaacg tctaccacca ccaataacaa caaagtattg       600
   gataatgtgt ttattgacaa tggcgatggt cttgaactga cgcgtggagc agcattcaac       660
   ctgattgcta acaatctgtt tacatcgacc aaagccaatc cagagccgtc tcaaggcatt       720
   gaaattcttt gggggaatga caatgcagtg gtgggtaaca aatttgaaaa ctattcagat       780
   ggtctacaaa tcaactgggg taaacgtaat tacatcgctt ataacgaatt gaccaataac       840
   tctttgggtt tcaatcttac aggtgatgga aacatcttcg atagtaacaa agtgcatggc       900
   aatcgtattg gtatcgcaat tcgttctgaa aaagatgcaa atgcacgtat cacacttacc       960
   aaaaatcaga tttgggataa tggtaaagat atcaaacgct gtgaggctgg tggttcatgt      1020
   gttccaaacc aacgtttagg tgcaattgta tttggtgttc ctgcgcttga gcatgaaggt      1080
   tttgtaggct ctcgtggtgg cggtgtagtc attgaacctg caaaattaca aaaaacatgt      1140
   acacagccaa atcaacaaaa ctgtaatgcc attccgaacc aaggtattca ggcacctaaa      1200
   ctgactgtca gtaaaaaaca acttacagtt gaagttaaag gaacaccaaa ccagcgttac      1260
   aacgtagaat tttttggaaa tcgtaatgca tcttcttccg aagctgagca atatttaggt      1320
   tcaattgttg tagtgacaga tcatcaaggt cttgcaaaag caaactgggc accaaaagtc      1380
   agcatgccat ctgttactgc gaatgtaact gatcacttgg gcgccacttc agagttaagt      1440
   tctgcagtga aaatgagata a                                                1461
<210> 13
   <211> 1461
   <212> DNA
   <213> Artificial Sequence
<220>
   <223> Codon-optimized quiC gene from Acinetobacter sp. ADP1;
<400> 13
   atgaaactga ccagcctgcg tgttagcctg ctggcactgg gtctggttac cagcggtttt      60
   gcagcagcag aaacctatac cgttgatcgt tatcaggatg atagcgaaaa aggtagcctg     120
   cgttgggcaa ttgaacagag caatgcaaat agcgcacaag aaaaccagat tctgattcag     180
   gcagttggta aagcaccgta tgttatcaaa gttgataaac cgctgcctcc gattaaaagc     240
   agcgttaaaa tcattggcac cgagtgggat aaaaccggtg aatttattgc aattgatggc     300
   agcaactata tcaaaggcga aggtgaaaaa gcatgtccgg gtgcaaatcc gggtcagtat     360
   ggcaccaatg ttcgtaccat gaccctgcct ggtctggttc tgcaagatgt taatggtgtt     420
   accctgaaag gtctggatgt tcatcgtttt tgtattggtg ttctggttaa tcgcagcagc     480
   aataacctga ttcagcataa tcgtatcagc aacaattatg gtggtgccgg tgttatgatt     540
   accggtgatg atggtaaagg taatccgacc agcaccacca ccaataataa caaagttctg     600
   gataacgtgt tcatcgataa tggtgatggt ctggaactga cccgtggtgc agcatttaat     660
   ctgattgcaa ataacctgtt taccagcaca aaagccaatc cggaaccgag ccagggtatt     720
   gaaattctgt ggggtaatga taatgccgtg gtgggtaaca aattcgaaaa ctattcagat     780
   ggcctgcaaa tcaattgggg taaacgtaac tatatcgcct ataacgaact gaccaataac     840
   agcctgggtt tcaatctgac aggtgatggt aacattttcg acagcaataa agtgcatggt     900
   aaccgtattg gtattgccat tcgtagtgaa aaagatgcca atgcacgtat taccctgacc     960
   aaaaatcaga tttgggataa cggcaaagat atcaaacgtt gtgaagccgg tggtagctgt    1020
   gttccgaatc agcgtctggg tgcaattgtt tttggtgttc cggcactgga acatgaaggt    1080
   tttgttggta gccgtggcgg tggtgttgtt attgaaccgg caaaactgca aaaaacctgc    1140
   acccagccga accagcagaa ttgtaatgca attcctaatc agggtattca ggcaccgaaa    1200
   ctgacagtta gcaaaaaaca gctgaccgtt gaagttaaag gcacccctaa tcagcgttat    1260
   aatgtggaat tttttggcaa tcgtaatgcc agcagcagcg aagcagaaca gtatctgggt    1320
   agcattgttg ttgttaccga tcatcagggt ctggcaaaag caaattgggc tccgaaagtt    1380
   agcatgccga gcgttaccgc aaatgtgaca gatcatctgg gtgcgaccag cgaactgagc    1440
   agcgcagtta aaatgcgtta a                                              1461
<210> 14
   <211> 486
   <212> PRT
   <213> Artificial Sequence
<220>
   <223> QuiC from Acinetobacter sp. ADP1;
<400> 14
   Met Lys Leu Thr Ser Leu Arg Val Ser Leu Leu Ala Leu Gly Leu Val
   1               5                   10                  15
   Thr Ser Gly Phe Ala Ala Ala Glu Thr Tyr Thr Val Asp Arg Tyr Gln
               20                  25                  30
   Asp Asp Ser Glu Lys Gly Ser Leu Arg Trp Ala Ile Glu Gln Ser Asn
           35                  40                  45
   Ala Asn Ser Ala Gln Glu Asn Gln Ile Leu Ile Gln Ala Val Gly Lys
       50                  55                  60
   Ala Pro Tyr Val Ile Lys Val Asp Lys Pro Leu Pro Pro Ile Lys Ser
   65                  70                  75                  80
   Ser Val Lys Ile Ile Gly Thr Glu Trp Asp Lys Thr Gly Glu Phe Ile
                   85                  90                  95
   Ala Ile Asp Gly Ser Asn Tyr Ile Lys Gly Glu Gly Glu Lys Ala Cys
               100                 105                 110
   Pro Gly Ala Asn Pro Gly Gln Tyr Gly Thr Asn Val Arg Thr Met Thr
           115                 120                 125
   Leu Pro Gly Leu Val Leu Gln Asp Val Asn Gly Val Thr Leu Lys Gly
       130                 135                 140
   Leu Asp Val His Arg Phe Cys Ile Gly Val Leu Val Asn Arg Ser Ser
   145                 150                 155                 160
   Asn Asn Leu Ile Gln His Asn Arg Ile Ser Asn Asn Tyr Gly Gly Ala
                   165                 170                 175
   Gly Val Met Ile Thr Gly Asp Asp Gly Lys Gly Asn Pro Thr Ser Thr
               180                 185                 190
   Thr Thr Asn Asn Asn Lys Val Leu Asp Asn Val Phe Ile Asp Asn Gly
           195                 200                 205
   Asp Gly Leu Glu Leu Thr Arg Gly Ala Ala Phe Asn Leu Ile Ala Asn
       210                 215                 220
   Asn Leu Phe Thr Ser Thr Lys Ala Asn Pro Glu Pro Ser Gln Gly Ile
   225                 230                 235                 240
   Glu Ile Leu Trp Gly Asn Asp Asn Ala Val Val Gly Asn Lys Phe Glu
                   245                 250                 255
   Asn Tyr Ser Asp Gly Leu Gln Ile Asn Trp Gly Lys Arg Asn Tyr Ile
               260                 265                 270
   Ala Tyr Asn Glu Leu Thr Asn Asn Ser Leu Gly Phe Asn Leu Thr Gly
           275                 280                 285
   Asp Gly Asn Ile Phe Asp Ser Asn Lys Val His Gly Asn Arg Ile Gly
       290                 295                 300
   Ile Ala Ile Arg Ser Glu Lys Asp Ala Asn Ala Arg Ile Thr Leu Thr
   305                 310                 315                 320
   Lys Asn Gln Ile Trp Asp Asn Gly Lys Asp Ile Lys Arg Cys Glu Ala
                   325                 330                 335
   Gly Gly Ser Cys Val Pro Asn Gln Arg Leu Gly Ala Ile Val Phe Gly
               340                 345                 350
   Val Pro Ala Leu Glu His Glu Gly Phe Val Gly Ser Arg Gly Gly Gly
           355                 360                 365
   Val Val Ile Glu Pro Ala Lys Leu Gln Lys Thr Cys Thr Gln Pro Asn
       370                 375                 380
   Gln Gln Asn Cys Asn Ala Ile Pro Asn Gln Gly Ile Gln Ala Pro Lys
   385                 390                 395                 400
   Leu Thr Val Ser Lys Lys Gln Leu Thr Val Glu Val Lys Gly Thr Pro
                   405                 410                 415
   Asn Gln Arg Tyr Asn Val Glu Phe Phe Gly Asn Arg Asn Ala Ser Ser
               420                 425                 430
   Ser Glu Ala Glu Gln Tyr Leu Gly Ser Ile Val Val Val Thr Asp His
           435                 440                 445
   Gln Gly Leu Ala Lys Ala Asn Trp Ala Pro Lys Val Ser Met Pro Ser
       450                 455                 460
   Val Thr Ala Asn Val Thr Asp His Leu Gly Ala Thr Ser Glu Leu Ser
   465                 470                 475                 480
   Ser Ala Val Lys Met Arg
                   485
<210> 15
   <211> 9462
   <212> DNA
   <213> Artificial Sequence
<220>
   <223> Plasmid pAC21;
<220>
   <221> misc_feature
   <222> (5924)..(5924)
   <223> n is a, c, g, or t
<400> 15
   gttgacagta agacgggtaa gcctgttgat gataccgctg ccttactggg tgcattagcc    60
   agtctgaatg acctgtcacg ggataatccg aagtggtcag actggaaaat cagagggcag   120
   gaactgctga acagcaaaaa gtcagatagc accacatagc agacccgcca taaaacgccc   180
   tgagaagccc gtgacgggct tttcttgtat tatgggtagt ttccttgcat gaatccataa   240
   aaggcgcctg tagtgccatt tacccccatt cactgccaga gccgtgagcg cagcgaactg   300
   aatgtcacga aaaagacagc gactcaggtg cctgatggtc ggagacaaaa ggaatattca   360
   gcgatttgcc cgagcttgcg agggtgctac ttaagccttt agggttttaa ggtctgtttt   420
   gtagaggagc aaacagcgtt tgcgacatcc ttttgtaata ctgcggaact gactaaagta   480
   gtgagttata cacagggctg ggatctattc tttttatctt tttttattct ttctttattc   540
   tataaattat aaccacttga atataaacaa aaaaaacaca caaaggtcta gcggaattta   600
   cagagggtct agcagaattt acaagttttc cagcaaaggt ctagcagaat ttacagatac   660
   ccacaactca aaggaaaagg actagtaatt atcattgact agcccatctc aattggtata   720
   gtgattaaaa tcacctagac caattgagat gtatgtctga attagttgtt ttcaaagcaa   780
   atgaactagc gattagtcgc tatgacttaa cggagcatga aaccaagcta attttatgct   840
   gtgtggcact actcaacccc acgattgaaa accctacaag gaaagaacgg acggtatcgt   900
   tcacttataa ccaatacgct cagatgatga acatcagtag ggaaaatgct tatggtgtat   960
   tagctaaagc aaccagagag ctgatgacga gaactgtgga aatcaggaat cctttggtta  1020
   aaggctttga gattttccag tggacaaact atgccaagtt ctcaagcgaa aaattagaat  1080
   tagtttttag tgaagagata ttgccttatc ttttccagtt aaaaaaattc ataaaatata  1140
   atctggaaca tgttaagtct tttgaaaaca aatactctat gaggatttat gagtggttat  1200
   taaaagaact aacacaaaag aaaactcaca aggcaaatat agagattagc cttgatgaat  1260
   ttaagttcat gttaatgctt gaaaataact accatgagtt taaaaggctt aaccaatggg  1320
   ttttgaaacc aataagtaaa gatttaaaca cttacagcaa tatgaaattg gtggttgata  1380
   agcgaggccg cccgactgat acgttgattt tccaagttga actagataga caaatggatc  1440
   tcgtaaccga acttgagaac aaccagataa aaatgaatgg tgacaaaata ccaacaacca  1500
   ttacatcaga ttcctaccta cgtaacggac taagaaaaac actacacgat gctttaactg  1560
   caaaaattca gctcaccagt tttgaggcaa aatttttgag tgacatgcaa agtaagcatg  1620
   atctcaatgg ttcgttctca tggctcacgc aaaaacaacg aaccacacta gagaacatac  1680
   tggctaaata cggaaggatc tgaggttctt atggctcttg tatctatcag tgaagcatca  1740
   agactaacaa acaaaagtag aacaactgtt caccgttaga tatcaaaggg aaaactgtcc  1800
   atatgcacag atgaaaacgg tgtaaaaaag atagatacat cagagctttt acgagttttt  1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgcatgcctg 4440
   caggtcgact ctagaggatc ccccccgccg ccgacagagt aataggtttt acttaatagc 4500
   tcttcctgtc ccttccaggc agtgatccgc attccgttct catggcgagg caacatttcg 4560
   ggatggaaga taatgttctt tgctacagga aaatcaacaa tatgcgcacc agatgccact 4620
   ggcagccgcc cgctgcgcgt tactaactct ataaatgcag ggatctcatc aatgacaaca 4680
   tcctgcggac tgtttcctgc cagtcccatg atgatggcga catccgtggc atggcctttg 4740
   cccgtcagtg acaacgaccc gtacagatcg accacaatat ggctcgtcgc ggttaataag 4800
   ccgctacttt ccagccgatc aataaaactt tttccggcat tcattggccc cacggtatgc 4860
   gaactggagg gaccaatccc aattttgaaa atatcgaatg cactaatcat gtgacggaag 4920
   atcacttcgc agaataaata aatcctggtg tccctgttga taccgggaag ccctgggcca 4980
   acttttggcg aaaatgagac gttgatcggc acgtaagagg ttccaacttt caccataatg 5040
   aaataagatc actaccgggc gtattttttg agttatcgag attttcagga gctaaggaag 5100
   ctaaaatgga gaaaaaaatc actggatata ccaccgttga tatatcccaa tggcatcgta 5160
   aagaacattt tgaggcattt cagtcagttg ctcaatgtac ctataaccag accgttcagc 5220
   tggatattac ggccttttta aagaccgtaa agaaaaataa gcacaagttt tatccggcct 5280
   ttattcacat tcttgcccgc ctgatgaatg ctcatccgga attccgtatg gcaatgaaag 5340
   acggtgagct ggtgatatgg gatagtgttc acccttgtta caccgttttc catgagcaaa 5400
   ctgaaacgtt ttcatcgctc tggagtgaat accacgacga tttccggcag tttctacaca 5460
   tatattcgca agatgtggcg tgttacggtg aaaacctggc ctatttccct aaagggttta 5520
   ttgagaatat gtttttcgtc tcagccaatc cctgggtgag tttcaccagt tttgatttaa 5580
   acgtggccaa tatggacaac ttcttcgccc ccgttttcac catgggcaaa tattatacgc 5640
   aaggcgacaa ggtgctgatg ccgctggcga ttcaggttca tcatgccgtt tgtgatggct 5700
   tccatgtcgg cagaatgctt aatgaattac aacagtactg cgatgagtgg cagggcgggg 5760
   cgtaattttt ttaaggcagt tattggtgcc cttaaacgcc tggtgctacg cctgaataag 5820
   tgataataag cggatgaatg gcagaaattc gaaagcaaat tcgacccggt cgtcggttca 5880
   gggcagggtc gttaaatagc cgcttatgtc tattgctggt ttantcggta cccggggatc 5940
   gcggccgcgg accggatccc atcacatata cctgccgttc actattattt agtgaaatga 6000
   gatattatga tattttctga attgtgatta aaaaggcaac tttatgccca tgcaacagaa 6060
   actataaaaa atacagagaa tgaaaagaaa cagatagatt ttttagttct ttaggcccgt 6120
   agtctgcaaa tccttttatg attttctatc aaacaaaaga ggaaaataga ccagttgcaa 6180
   tccaaacgag agtctaatag aatgaggtcg aaaagtaaat cgcgcgggtt tgttactgat 6240
   aaagcaggca agacctaaaa tgtgtaaagg gcaaagtgta tactttggcg tcacccctta 6300
   catattttag gtcttttttt attgtgcgta actaacttgc catcttcaaa caggagggct 6360
   ggaagaagca gaccgctaac acagtacata aaaaaggaga catgaacgat gaacatcaaa 6420
   aagtttgcaa aacaagcaac agtattaacc tttactaccg cactgctggc aggaggcgca 6480
   actcaagcgt ttgcgaaaga aacgaaccaa aagccatata aggaaacata cggcatttcc 6540
   catattacac gccatgatat gctgcaaatc cctgaacagc aaaaaaatga aaaatatcaa 6600
   gttcctgaat tcgattcgtc cacaattaaa aatatctctt ctgcaaaagg cctggacgtt 6660
   tgggacagct ggccattaca aaacgctgac ggcactgtcg caaactatca cggctaccac 6720
   atcgtctttg cattagccgg agatcctaaa aatgcggatg acacatcgat ttacatgttc 6780
   tatcaaaaag tcggcgaaac ttctattgac agctggaaaa acgctggccg cgtctttaaa 6840
   gacagcgaca aattcgatgc aaatgattct atcctaaaag accaaacaca agaatggtca 6900
   ggttcagcca catttacatc tgacggaaaa atccgtttat tctacactga tttctccggt 6960
   aaacattacg gcaaacaaac actgacaact gcacaagtta acgtatcagc atcagacagc 7020
   tctttgaaca tcaacggtgt agaggattat aaatcaatct ttgacggtga cggaaaaacg 7080
   tatcaaaatg tacagcagtt catcgatgaa ggcaactaca gctcaggcga caaccatacg 7140
   ctgagagatc ctcactacgt agaagataaa ggccacaaat acttagtatt tgaagcaaac 7200
   actggaactg aagatggcta ccaaggcgaa gaatctttat ttaacaaagc atactatggc 7260
   aaaagcacat cattcttccg tcaagaaagt caaaaacttc tgcaaagcga taaaaaacgc 7320
   acggctgagt tagcaaacgg cgctctcggt atgattgagc taaacgatga ttacacactg 7380
   aaaaaagtga tgaaaccgct gattgcatct aacacagtaa cagatgaaat tgaacgcgcg 7440
   aacgtcttta aaatgaacgg caaatggtac ctgttcactg actcccgcgg atcaaaaatg 7500
   acgattgacg gcattacgtc taacgatatt tacatgcttg gttatgtttc taattcttta 7560
   actggcccat acaagccgct gaacaaaact ggccttgtgt taaaaatgga tcttgatcct 7620
   aacgatgtaa cctttactta ctcacacttc gctgtacctc aagcgaaagg aaacaatgtc 7680
   gtgattacaa gctatatgac aaacagagga ttctacgcag acaaacaatc aacgtttgcg 7740
   ccgagcttcc tgctgaacat caaaggcaag aaaacatctg ttgtcaaaga cagcatcctt 7800
   gaacaaggac aattaacagt taacaaataa aaacgcaaaa gaaaatgcca atatcctatt 7860
   ggcattttct tttatttctt ccatttaaat ggatgcatgc gctagcggag tgtatactgg 7920
   cttactatgt tggcactgat gagggtgtca gtgaagtgct tcagcctcgt gagcgggacg 7980
   gtcgtaaggt cgttccgctc cacttcactg aacggcaatc cgagggtgtg gatccaatta 8040
   aggccacgct gtcatttaaa ttccgttttt ccagttcaaa tgcaattgcc ttcaatgcac 8100
   cttcgtagct gtggtgagcc agcggtgctg gctctccccc atttacggat aagaatgcat 8160
   tttccgagtt aataccgtcg gcaatacctg acattaatac ttcacagtcg ctggcatcga 8220
   gtacggaaaa cttaatcgaa gacgaaccac agttaataac caaaacaacc ggaaattcat 8280
   tcatctcttt tctcatcctg agttacggat taaaacagtt tgtatacgat gttcaggatg 8340
   gtcagcagac caatcacggt aacaaacacg ttatccagac gaccacggta tttcgccaga 8400
   gacggcgctt tacggatggc atacatcggc aacaggcaca gcagggatgc gataatcggt 8460
   gcgcccatgg cttcaatcag gtcgaggatg ttcgggttgg cgtaggcaac aacccaggtg 8520
   gagcccatga tgaagatcat gctgagagta ttcagtttac ccagcgacac tttggttttg 8580
   tcacctttat aaccgaactt cagaatcaga ccattcaagc cttccagcgt ccccagatag 8640
   tgaccgaaga aagatttgaa gatagccacg agtgcgatga tggaagccgc atattccagt 8700
   gtaatcgcga acgttgtttt ggtaccggtc atggacgcaa agtggttagc cagataagaa 8760
   agcactggaa tattctgcgc tttggcttcc gccatgttgg ccggagacag agtaaacagg 8820
   cagctaaagg caaagaacat caccactgca accatcagca tgctggcacg agaaatgatt 8880
   tgggaacatt tacgttcggt gaagtcgcga ccgaagtctt tctcatactc ttcacgttta 8940
   gaaaccacga aggaagagac gattggcgag aagttaaagg agaaaaccat gatggaaatc 9000
   cccagccaga cagtgatcag gataccgtca tgaccggtta acgacagcga accgaggtca 9060
   acctggtcga taactgcaga gttccagtaa gggatcagcg acaaagaaat cagcaccagg 9120
   ctggcgataa acggccatac caggtagctc agggtaccga gctcgaattc actggccgtc 9180
   gttttacaac gtcgtgactg ggaaaaccct ggcgttaccc aacttaatcg ccttgcagca 9240
   catccccctt tcgccagctg gcgtaatagc gaagaggccc gcaccgatcg cccttcccaa 9300
   cagttgcgca gcctgaatgg cgaatggcgc ctgatgcggt attttctcct tacgcatctg 9360
   tgcggtattt cacaccgcat atggtgcact ctcagtacaa tctgctctga tgccgcatag 9420
   ttaagccagc cccgacaccc gccaacaccc gctgacgaat tc 9462
<210> 16
   <211> 9430
   <212> DNA
   <213> Artificial Sequence
<220>
   <223> Plasmid pAC19;
<400> 16
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt     900
   tcacttataa ccaatacgct cagatgatga acatcagtag ggaaaatgct tatggtgtat     960
   tagctaaagc aaccagagag ctgatgacga gaactgtgga aatcaggaat cctttggtta    1020
   aaggctttga gattttccag tggacaaact atgccaagtt ctcaagcgaa aaattagaat    1080
   tagtttttag tgaagagata ttgccttatc ttttccagtt aaaaaaattc ataaaatata    1140
   atctggaaca tgttaagtct tttgaaaaca aatactctat gaggatttat gagtggttat    1200
   taaaagaact aacacaaaag aaaactcaca aggcaaatat agagattagc cttgatgaat    1260
   ttaagttcat gttaatgctt gaaaataact accatgagtt taaaaggctt aaccaatggg    1320
   ttttgaaacc aataagtaaa gatttaaaca cttacagcaa tatgaaattg gtggttgata    1380
   agcgaggccg cccgactgat acgttgattt tccaagttga actagataga caaatggatc    1440
   tcgtaaccga acttgagaac aaccagataa aaatgaatgg tgacaaaata ccaacaacca    1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgcatgcctg 4440
   caggtcgact ctagaggatc ccccccgccg ccgacagagt aataggtttt acttaatagc 4500
   tcttcctgtc ccttccaggc agtgatccgc attccgttct catggcgagg caacatttcg 4560
   ggatggaaga taatgttctt tgctacagga aaatcaacaa tatgcgcacc agatgccact 4620
   ggcagccgcc cgctgcgcgt tactaactct ataaatgcag ggatctcatc aatgacaaca 4680
   tcctgcggac tgtttcctgc cagtcccatg atgatggcga catccgtggc atggcctttg 4740
   cccgtcagtg acaacgaccc gtacagatcg accacaatat ggctcgtcgc ggttaataag 4800
   ccgctacttt ccagccgatc aataaaactt tttccggcat tcattggccc cacggtatgc 4860
   gaactggagg gaccaatccc aattttgaaa atatcgaatg cactaatcat atccacaccc 4920
   tcggattgcc gttcagtgaa gtggagcgga acgaccttac gaccgtcccg ctcacgaggc 4980
   tttacgcact acgtactgcg atggcttcaa tttccagcgg gagggcggat ccactaatac 5040
   aaaatatatc aaaagttaat aataatatta ttcttactta agactttttt gtcttcattt 5100
   tttagtaaaa aatataaaaa aggccacctc ccgattttat cggaaggcag cctcttaaat 5160
   tcagttcata atattaaaaa atattattca acttcagaat atttgttggc ataggcagct 5220
   gccgcaccca acagtccagg ctgcggataa gtaatcaact taaccggaat cttggacatg 5280
   acgcgttcaa agcgtccttt tgaaacaaag cgctgacgga aaccagattc tggcaaatgg 5340
   gaagcgatac gaagaccgac accaccgcca ataacaacac tggttgcacc ctgtgccaaa 5400
   gcaagatcac cagcgatagc gccaaggctc aagcagaagc gatccaaagc ggcttcagca 5460
   aggttgtctt taccttccaa agccatctgc cataatttaa tatcatccag caagctgaac 5520
   ggaacgcctt caatggcagc cagtgcttcg tagatattac caagacccgg gccagaaata 5580
   atgcgttcga tagaaacgcg gcggaaacgt tcacgtaaac gtgccagaat tttgtcttca 5640
   agtctgtcaa gcggagcaaa gtcgatatga ccgccttcag tttcgatgac gaaataacgg 5700
   ccttcagtcc gcaacagatg ggcaacaccc aagcccgttc ccggaccaag aatagtgata 5760
   acaccatcgc taggaagcgc ttcatcagga ccacaaatat gatccagata agaagaatcc 5820
   atatgcgcaa ccgcgtgggc aaccgcgccg aagtcattga tcagaacatg cgtatcgatg 5880
   tccagctttt cattcagagt agctggtctt aatacccaag ggttattggt aagttttaaa 5940
   acttcaccat gaaccgggcc agcccatgca atagctgcgg cacgtggcag aggacgaccc 6000
   agtttttcac cgaaacgttc ccaagctaac tgcaagctag catgttctgc cgttttaaaa 6060
   gttgtttctt ctccaagaga aagaacccga ccattgctta cttccgcaat agagaaacgc 6120
   gcatgcgttc caccgatgtc aatcgcaaca atttccataa taattccttt ctgaaatcag 6180
   aaggctaccc aacaggtaaa ataagtccgc ccgctttata ccatcgttgt aaacaaaaag 6240
   tataattggt taagacttat ctaaaaaaga caaaaggatt cagccaaagc aagtttaact 6300
   acttctggga gcgccacatc tcctcgattt catccaggct ccgacctttg gtttccggca 6360
   cgaagcgagc aacaatcaag ccacctaaga tacttaatgc tgcgaaaacg agataggaga 6420
   aaccgtggtt gaaagtctga ttcaatgctg gagaaccatc ggcaacctta aacaggaagt 6480
   taaccaagat attagctaac cattgtccgg taacagcgat aggcatagct gcgcccttga 6540
   tggaactcgg gaacatttct gacagaacaa cccagcagac agggccccat gacataccaa 6600
   agactgcaat ataaagaagc acagaagcca aaggcaaaac accaccgact ttgaaccaga 6660
   aacagcagcc taaaacagcc atcattgcag ccataccgag agcaccccaa ataagcagag 6720
   gtttacggcc gaagcggtca acaacacggg aagcaatcat ggtgaagatg aagttcacaa 6780
   caccgataga gatggtctgc aataatgccg tatcagctcc aaaacctaaa ttctggaaca 6840
   tctgcggtgc ataatacagc acggcgttaa taccgactaa ctgctggaag gcagcaacgg 6900
   atacaccggc aaaaacaacg gtgataccaa aagcaaacaa acctgcgctg cttttgtcca 6960
   tggctttatc aaagccagct ttaatctttt gaatcgtcag attaggatcg gcttgcggtt 7020
   ccagacgagc aaggattttg ctagcctcgg aatgacgtcc cttcatcacc aaccaatgcg 7080
   gcgtatccgg tgcggttaac agcagcaata agaaggcaat accgatcagg ccttctgaag 7140
   ccggagacca gcaccaacca ctggcattaa cccaatcgat agaaccgaaa tgagccagta 7200
   accaggtaaa gatataaccg gttaaagcac ccgtcacaat ggccatctgc tgaccagaaa 7260
   ccatctgacc acgtttgtct ggcggagcaa tttcagcaat ataggttggg gtcaaggttg 7320
   aaacgacacc gatacctaaa ccggcaagaa accggaaaaa gcaaaaaatt tgtaaagccg 7380
   aaccaccggt tccaaataat ttttcggtta acgcagcacc aaaaccggcg gcgacgaaac 7440
   aaatggaact catcaacaat ccgccgcgac gaccgaagcg aataccaatc cagccagaca 7500
   gcaaagaacc ggtaacacaa ccgaccaaaa cagcaacaac gaccatccca gaaagggaag 7560
   ccgcagccgt agcagacagg tgacgagggg caataaaatg gatatcaacc ggtgtaccga 7620
   ttgcagcgat aaccgctgaa tcgtaaccga aaagcaagcc gcctatagca gcgattaggg 7680
   ctagtcgcgt gactagaccc tgactacttt cagaactcat ggcgattcct ctccctctag 7740
   agcgtcctgc tgttgttaag attattatac cacaccttgt agataaagtc aacaactttt 7800
   tgcaaaattt ttcaggaatt ttagcagagg ttgttctgga tgtagaacaa aacatctttc 7860
   cgctcttgtg ctgttaggat atctttcttg gaagctaggt aggcctcgag ttatggcagt 7920
   tggttaaaag gaaacaaaaa gaccgttttc acacaaaacg gtctttttcg atttcttttt 7980
   acagtcacag ccacttttgc accaattaag gccacgctgt catttaaact ccgtttttcc 8040
   agttcaaatg caattgcctt caatgcacct tcgtagctgt ggtgagccag cggtgctggc 8100
   tctcccccat ttacggataa gaatgcattt tccgagttaa taccgtcggc aatacctgac 8160
   attaatactt cacagtcgct ggcatcgagt acggaaaact taatcgaaga cgaaccacag 8220
   ttaataacca aaacaaccgg aaattcattc atctcttttc tcatcctgag ttacggatta 8280
   aaacagtttg tatacgatgt tcaggatggt cagcagacca atcacggtaa caaacacgtt 8340
   atccagacga ccacggtatt tcgccagaga cggcgcttta cggatggcat acatcggcaa 8400
   caggcacagc agggatgcga taatcggtgc gcccatggct tcaatcaggt cgaggatgtt 8460
   cgggttggcg taggcaacaa cccaggtgga gcccatgatg aagatcatgc tgagagtatt 8520
   cagtttaccc agcgacactt tggttttgtc acctttataa ccgaacttca gaatcagacc 8580
   attcaagcct tccagcgtcc ccagatagtg accgaagaaa gatttgaaga tagccacgag 8640
   tgcgatgatg gaagccgcat attccagtgt aatcgcgaac gttgttttgg taccggtcat 8700
   ggacgcaaag tggttagcca gataagaaag cactggaata ttctgcgctt tggcttccgc 8760
   catgttggcc ggagacagag taaacaggca gctaaaggca aagaacatca ccactgcaac 8820
   catcagcatg ctggcacgag aaatgatttg ggaacattta cgttcggtga agtcgcgacc 8880
   gaagtctttc tcatactctt cacgtttaga aaccacgaag gaagagacga ttggcgagaa 8940
   gttaaaggag aaaaccatga tggaaatccc cagccagaca gtgatcagga taccgtcatg 9000
   accggttaac gacagcgaac cgaggtcaac ctggtcgata actgcagagt tccagtaagg 9060
   gatcagcgac aaagaaatca gcaccaggct ggcgataaac ggccatacca ggtagctcag 9120
   ggtaccgagc tcgaattcac tggccgtcgt tttacaacgt cgtgactggg aaaaccctgg 9180
   cgttacccaa cttaatcgcc ttgcagcaca tccccctttc gccagctggc gtaatagcga 9240
   agaggcccgc accgatcgcc cttcccaaca gttgcgcagc ctgaatggcg aatggcgcct 9300
   gatgcggtat tttctcctta cgcatctgtg cggtatttca caccgcatat ggtgcactct 9360
   cagtacaatc tgctctgatg ccgcatagtt aagccagccc cgacacccgc caacacccgc 9420
   tgacgaattc 9430
<210> 17
   <211> 5768
   <212> DNA
   <213> Artificial Sequence
<220>
   <223> Plasmid pMH17F;
<400> 17
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt     900
   tcacttataa ccaatacgct cagatgatga acatcagtag ggaaaatgct tatggtgtat     960
   tagctaaagc aaccagagag ctgatgacga gaactgtgga aatcaggaat cctttggtta    1020
   aaggctttga gattttccag tggacaaact atgccaagtt ctcaagcgaa aaattagaat    1080
   tagtttttag tgaagagata ttgccttatc ttttccagtt aaaaaaattc ataaaatata    1140
   atctggaaca tgttaagtct tttgaaaaca aatactctat gaggatttat gagtggttat    1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagt 4380
   tacctagaga gggtgagaat tgccgaacat gcgcataagt ttcccggaca gatttcaggt 4440
   ggtcagcagc aacgcgttgc cattgcgcgt tcgctgtgta tgaagccgaa aattatgttg 4500
   tttgatgagc caacgtcggc gctcgatcct gagatggtga aagaggtgct ggatacgatg 4560
   attgggctgg cgcagtcggg tatgacaatg ttgtgtgtaa cacatgagat ggggtttgca 4620
   cgaaccgtcg ctgaccgggt aatttttatg gatcgtgggg aaatagtgga gcaagctgca 4680
   cctgatgaat tttttgcgca tcctaaatca gagcgtacga gggcattttt atcgcaggta 4740
   atccattaat tgaatgttag ttcgaaaagc aaaaaggcca tcctttcgga tggcctttcg 4800
   cttgatttga tgtctggcag tttatggcgg gcgtcctgcc cgccaccctc cgggccgttg 4860
   cttcgcaacg ttcaaatccg ctcccggcgg atttgtccta ctcgggagag tgttcaccga 4920
   caaacaacag ataaaacaaa aggcccagtc ttccgactga gccttttgtt ttatttgatg 4980
   tctggcagtt ccctactctc gcatggggag accccacact accatcggcg ctacggcggt 5040
   ttcacttctg agttcggcat ggggtcaggt gggaccaccg cgctactgcc gccagacaaa 5100
   ttcttttcta atctgccgaa ctttaaccta aaaagtggtg ctgataccca gagtcgaact 5160
   ggggacctca cccttaccaa gggtgcgctc taccaactga gccatatcag cacgctaaat 5220
   ttgatgcctg gcagttccct actctcgcat ggggagaccc cacactacca tcggcgctac 5280
   ggcgtttcac ttctgagttc ggcatggggt caggtgggac caccgcgcta cggccgccag 5340
   gcaaattctg ttttatcaga ccgcttctgc gttctgattt aatctgtatc aggctgaaaa 5400
   tcttctctca tccggataac aatttcacac aggaaacagc tatgaccatg attacgccaa 5460
   gctcgagctc gaattcactg gccgtcgttt tacaacgtcg tgactgggaa aaccctggcg 5520
   ttacccaact taatcgcctt gcagcacatc cccctttcgc cagctggcgt aatagcgaag 5580
   aggcccgcac cgatcgccct tcccaacagt tgcgcagcct gaatggcgaa tggcgcctga 5640
   tgcggtattt tctccttacg catctgtgcg gtatttcaca ccgcatatgg tgcactctca 5700
   gtacaatctg ctctgatgcc gcatagttaa gccagccccg acacccgcca acacccgctg 5760
   acgaattc 5768
<210> 18
   <211> 1053
   <212> DNA
   <213> Artificial Sequence
<220>
   <223> Coding region of the wild type aroG gene;
<400> 18
   atgaattatc agaacgacga tttacgcatc aaagaaatca aagagttact tcctcctgtc 60
   gcattgctgg aaaaattccc cgctactgaa aatgccgcga atacggttgc ccatgcccga 120
   aaagcgatcc ataagatcct gaaaggtaat gatgatcgcc tgttggttgt gattggccca 180
   tgctcaattc atgatcctgt cgcggcaaaa gagtatgcca ctcgcttgct ggcgctgcgt 240
   gaagagctga aagatgagct ggaaatcgta atgcgcgtct attttgaaaa gccgcgtacc 300
   acggtgggct ggaaagggct gattaacgat ccgcatatgg ataatagctt ccagatcaac 360
   gacggtctgc gtatagcccg taaattgctg cttgatatta acgacagcgg tctgccagcg 420
   gcaggtgagt ttctcgatat gatcacccca caatatctcg ctgacctgat gagctggggc 480
   gcaattggcg cacgtaccac cgaatcgcag gtgcaccgcg aactggcatc agggctttct 540
   tgtccggtcg gcttcaaaaa tggcaccgac ggtacgatta aagtggctat cgatgccatt 600
   aatgccgccg gtgcgccgca ctgcttcctg tccgtaacga aatgggggca ttcggcgatt 660
   gtgaatacca gcggtaacgg cgattgccat atcattctgc gcggcggtaa agagcctaac 720
   tacagcgcga agcacgttgc tgaagtgaaa gaagggctga acaaagcagg cctgccagca 780
   caggtgatga tcgatttcag ccatgctaac tcgtccaaac aattcaaaaa gcagatggat 840
   gtttgtgctg acgtttgcca gcagattgcc ggtggcgaaa aggccattat tggcgtgatg 900
   gtggaaagcc atctggtgga aggcaatcag agcctcgaga gcggggagcc gctggcctac 960
   ggtaagagca tcaccgatgc ctgcatcggc tgggaagata ccgatgctct gttacgtcaa 1020
   ctggcgaatg cagtaaaagc gcgtcgcggg taa 1053
<210> 19
   <211> 8820
   <212> DNA
   <213> Artificial Sequence
<220>
   <223> Plasmid pMH28F;
<220>
   <221> misc_feature
   <222> (5894)..(5894)
   <223> n is a, c, g, or t
<400> 19
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt     900
   tcacttataa ccaatacgct cagatgatga acatcagtag ggaaaatgct tatggtgtat     960
   tagctaaagc aaccagagag ctgatgacga gaactgtgga aatcaggaat cctttggtta    1020
   aaggctttga gattttccag tggacaaact atgccaagtt ctcaagcgaa aaattagaat    1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagt 4380
   tacctagaga gggtgagaat tgccgaacat gcgcataagt ttcccggaca gatttcaggt 4440
   ggtcagcagc aacgcgttgc cattgcgcgt tcgctgtgta tgaagccgaa aattatgttg 4500
   tttgatgagc caacgtcggc gctcgatcct gagatggtga aagaggtgct ggatacgatg 4560
   attgggctgg cgcagtcggg tatgacaatg ttgtgtgtaa cacatgagat ggggtttgca 4620
   cgaaccgtcg ctgaccgggt aatttttatg gatcgtgggg aaatagtgga gcaagctgca 4680
   cctgatgaat tttttgcgca tcctaaatca gagcgtacga gggcattttt atcgcaggta 4740
   atccattaat tgaatgttag ttcgaaaagc aaaaaggcca tcctttcgga tggcctttcg 4800
   cttgatttga tgtctggcag tttatggcgg gcgtcctgcc cgccaccctc cgggccgttg 4860
   cttcgcaacg ttcaaatccg gtgacggaag atcacttcgc agaataaata aatcctggtg 4920
   tccctgttga taccgggaag ccctgggcca acttttggcg aaaatgagac gttgatcggc 4980
   acgtaagagg ttccaacttt caccataatg aaataagatc actaccgggc gtattttttg 5040
   agttatcgag attttcagga gctaaggaag ctaaaatgga gaaaaaaatc actggatata 5100
   ccaccgttga tatatcccaa tggcatcgta aagaacattt tgaggcattt cagtcagttg 5160
   ctcaatgtac ctataaccag accgttcagc tggatattac ggccttttta aagaccgtaa 5220
   agaaaaataa gcacaagttt tatccggcct ttattcacat tcttgcccgc ctgatgaatg 5280
   ctcatccgga attccgtatg gcaatgaaag acggtgagct ggtgatatgg gatagtgttc 5340
   acccttgtta caccgttttc catgagcaaa ctgaaacgtt ttcatcgctc tggagtgaat 5400
   accacgacga tttccggcag tttctacaca tatattcgca agatgtggcg tgttacggtg 5460
   aaaacctggc ctatttccct aaagggttta ttgagaatat gtttttcgtc tcagccaatc 5520
   cctgggtgag tttcaccagt tttgatttaa acgtggccaa tatggacaac ttcttcgccc 5580
   ccgttttcac catgggcaaa tattatacgc aaggcgacaa ggtgctgatg ccgctggcga 5640
   ttcaggttca tcatgccgtt tgtgatggct tccatgtcgg cagaatgctt aatgaattac 5700
   aacagtactg cgatgagtgg cagggcgggg cgtaattttt ttaaggcagt tattggtgcc 5760
   cttaaacgcc tggtgctacg cctgaataag tgataataag cggatgaatg gcagaaattc 5820
   gaaagcaaat tcgacccggt cgtcggttca gggcagggtc gttaaatagc cgcttatgtc 5880
   tattgctggt ttantcggta cccggggatc gcggccgcgg accggatccc atcacatata 5940
   cctgccgttc actattattt agtgaaatga gatattatga tattttctga attgtgatta 6000
   aaaaggcaac tttatgccca tgcaacagaa actataaaaa atacagagaa tgaaaagaaa 6060
   cagatagatt ttttagttct ttaggcccgt agtctgcaaa tccttttatg attttctatc 6120
   aaacaaaaga ggaaaataga ccagttgcaa tccaaacgag agtctaatag aatgaggtcg 6180
   aaaagtaaat cgcgcgggtt tgttactgat aaagcaggca agacctaaaa tgtgtaaagg 6240
   gcaaagtgta tactttggcg tcacccctta catattttag gtcttttttt attgtgcgta 6300
   actaacttgc catcttcaaa caggagggct ggaagaagca gaccgctaac acagtacata 6360
   aaaaaggaga catgaacgat gaacatcaaa aagtttgcaa aacaagcaac agtattaacc 6420
   tttactaccg cactgctggc aggaggcgca actcaagcgt ttgcgaaaga aacgaaccaa 6480
   aagccatata aggaaacata cggcatttcc catattacac gccatgatat gctgcaaatc 6540
   cctgaacagc aaaaaaatga aaaatatcaa gttcctgaat tcgattcgtc cacaattaaa 6600
   aatatctctt ctgcaaaagg cctggacgtt tgggacagct ggccattaca aaacgctgac 6660
   ggcactgtcg caaactatca cggctaccac atcgtctttg cattagccgg agatcctaaa 6720
   aatgcggatg acacatcgat ttacatgttc tatcaaaaag tcggcgaaac ttctattgac 6780
   agctggaaaa acgctggccg cgtctttaaa gacagcgaca aattcgatgc aaatgattct 6840
   atcctaaaag accaaacaca agaatggtca ggttcagcca catttacatc tgacggaaaa 6900
   atccgtttat tctacactga tttctccggt aaacattacg gcaaacaaac actgacaact 6960
   gcacaagtta acgtatcagc atcagacagc tctttgaaca tcaacggtgt agaggattat 7020
   aaatcaatct ttgacggtga cggaaaaacg tatcaaaatg tacagcagtt catcgatgaa 7080
   ggcaactaca gctcaggcga caaccatacg ctgagagatc ctcactacgt agaagataaa 7140
   ggccacaaat acttagtatt tgaagcaaac actggaactg aagatggcta ccaaggcgaa 7200
   gaatctttat ttaacaaagc atactatggc aaaagcacat cattcttccg tcaagaaagt 7260
   caaaaacttc tgcaaagcga taaaaaacgc acggctgagt tagcaaacgg cgctctcggt 7320
   atgattgagc taaacgatga ttacacactg aaaaaagtga tgaaaccgct gattgcatct 7380
   aacacagtaa cagatgaaat tgaacgcgcg aacgtcttta aaatgaacgg caaatggtac 7440
   ctgttcactg actcccgcgg atcaaaaatg acgattgacg gcattacgtc taacgatatt 7500
   tacatgcttg gttatgtttc taattcttta actggcccat acaagccgct gaacaaaact 7560
   ggccttgtgt taaaaatgga tcttgatcct aacgatgtaa cctttactta ctcacacttc 7620
   gctgtacctc aagcgaaagg aaacaatgtc gtgattacaa gctatatgac aaacagagga 7680
   ttctacgcag acaaacaatc aacgtttgcg ccgagcttcc tgctgaacat caaaggcaag 7740
   aaaacatctg ttgtcaaaga cagcatcctt gaacaaggac aattaacagt taacaaataa 7800
   aaacgcaaaa gaaaatgcca atatcctatt ggcattttct tttatttctt ccatttaaat 7860
   ggatgcatgc gctagcggag tgtatactgg cttactatgt tggcactgat gagggtgtca 7920
   gtgaagtgct tcctcccggc ggatttgtcc tactcgggag agtgttcacc gacaaacaac 7980
   agataaaaca aaaggcccag tcttccgact gagccttttg ttttatttga tgtctggcag 8040
   ttccctactc tcgcatgggg agaccccaca ctaccatcgg cgctacggcg gtttcacttc 8100
   tgagttcggc atggggtcag gtgggaccac cgcgctactg ccgccagaca aattcttttc 8160
   taatctgccg aactttaacc taaaaagtgg tgctgatacc cagagtcgaa ctggggacct 8220
   cacccttacc aagggtgcgc tctaccaact gagccatatc agcacgctaa atttgatgcc 8280
   tggcagttcc ctactctcgc atggggagac cccacactac catcggcgct acggcgtttc 8340
   acttctgagt tcggcatggg gtcaggtggg accaccgcgc tacggccgcc aggcaaattc 8400
   tgttttatca gaccgcttct gcgttctgat ttaatctgta tcaggctgaa aatcttctct 8460
   catccggata acaatttcac acaggaaaca gctatgacca tgattacgcc aagctcgagc 8520
   tcgaattcac tggccgtcgt tttacaacgt cgtgactggg aaaaccctgg cgttacccaa 8580
   cttaatcgcc ttgcagcaca tccccctttc gccagctggc gtaatagcga agaggcccgc 8640
   accgatcgcc cttcccaaca gttgcgcagc ctgaatggcg aatggcgcct gatgcggtat 8700
   tttctcctta cgcatctgtg cggtatttca caccgcatat ggtgcactct cagtacaatc 8760
   tgctctgatg ccgcatagtt aagccagccc cgacacccgc caacacccgc tgacgaattc 8820
<210> 20
   <211> 4774
   <212> DNA
   <213> Artificial Sequence
<220>
   <223> Plasmid pCL1921;
<400> 20
   gttgacagta agacgggtaa gcctgttgat gataccgctg ccttactggg tgcattagcc 60
   agtctgaatg acctgtcacg ggataatccg aagtggtcag actggaaaat cagagggcag 120
   gaactgctga acagcaaaaa gtcagatagc accacatagc agacccgcca taaaacgccc 180
   tgagaagccc gtgacgggct tttcttgtat tatgggtagt ttccttgcat gaatccataa 240
   aaggcgcctg tagtgccatt tacccccatt cactgccaga gccgtgagcg cagcgaactg 300
   aatgtcacga aaaagacagc gactcaggtg cctgatggtc ggagacaaaa ggaatattca 360
   gcgatttgcc cgagcttgcg agggtgctac ttaagccttt agggttttaa ggtctgtttt 420
   gtagaggagc aaacagcgtt tgcgacatcc ttttgtaata ctgcggaact gactaaagta 480
   gtgagttata cacagggctg ggatctattc tttttatctt tttttattct ttctttattc 540
   tataaattat aaccacttga atataaacaa aaaaaacaca caaaggtcta gcggaattta 600
   cagagggtct agcagaattt acaagttttc cagcaaaggt ctagcagaat ttacagatac 660
   ccacaactca aaggaaaagg actagtaatt atcattgact agcccatctc aattggtata 720
   gtgattaaaa tcacctagac caattgagat gtatgtctga attagttgtt ttcaaagcaa 780
   atgaactagc gattagtcgc tatgacttaa cggagcatga aaccaagcta attttatgct 840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc tttttatttt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgcatgcctg 4440
   caggtcgact ctagaggatc cccgggtacc gagctcgaat tcactggccg tcgttttaca 4500
   acgtcgtgac tgggaaaacc ctggcgttac ccaacttaat cgccttgcag cacatccccc 4560
   tttcgccagc tggcgtaata gcgaagaggc ccgcaccgat cgcccttccc aacagttgcg 4620
   cagcctgaat ggcgaatggc gcctgatgcg gtattttctc cttacgcatc tgtgcggtat 4680
   ttcacaccgc atatggtgca ctctcagtac aatctgctct gatgccgcat agttaagcca 4740
   gccccgacac ccgccaacac ccgctgacga attc 4774
<210> 21
   <211> 6432
   <212> DNA
   <213> Artificial Sequence
<220>
   <223> Plasmid pMG27;
<400> 21
   gttgacagta agacgggtaa gcctgttgat gataccgctg ccttactggg tgcattagcc 60
   agtctgaatg acctgtcacg ggataatccg aagtggtcag actggaaaat cagagggcag 120
   gaactgctga acagcaaaaa gtcagatagc accacatagc agacccgcca taaaacgccc 180
   tgagaagccc gtgacgggct tttcttgtat tatgggtagt ttccttgcat gaatccataa 240
   aaggcgcctg tagtgccatt tacccccatt cactgccaga gccgtgagcg cagcgaactg 300
   aatgtcacga aaaagacagc gactcaggtg cctgatggtc ggagacaaaa ggaatattca 360
   gcgatttgcc cgagcttgcg agggtgctac ttaagccttt agggttttaa ggtctgtttt 420
   gtagaggagc aaacagcgtt tgcgacatcc ttttgtaata ctgcggaact gactaaagta 480
   gtgagttata cacagggctg ggatctattc tttttatctt tttttattct ttctttattc 540
   tataaattat aaccacttga atataaacaa aaaaaacaca caaaggtcta gcggaattta 600
   cagagggtct agcagaattt acaagttttc cagcaaaggt ctagcagaat ttacagatac 660
   ccacaactca aaggaaaagg actagtaatt atcattgact agcccatctc aattggtata 720
   gtgattaaaa tcacctagac caattgagat gtatgtctga attagttgtt ttcaaagcaa 780
   atgaactagc gattagtcgc tatgacttaa cggagcatga aaccaagcta attttatgct 840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat     1080
   tagtttttag tgaagagata ttgccttatc ttttccagtt aaaaaaattc ataaaatata     1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgcatgcctg 4440
   caggtcgacc gttaaatcta tcaccgcaag ggataaatat ctaacaccgt gcgtgttgac 4500
   tattttacct ctggcggtga taatggttgc atgtactaat ctagataagg aatatagcca 4560
   tgaccgcacc gattcaggat ctgcgtgatg caattgccct gctgcaacag catgataatc 4620
   agtatctgga aaccgatcat ccggttgatc cgaatgcaga actggcaggc gtttatcgtc 4680
   atattggtgc cggtggcacc gttaaacgtc cgacccgtat tggtccggca atgatgttta 4740
   ataacattaa aggttatccg cacagccgta ttctggttgg tatgcatgca agccgtcagc 4800
   gtgcagcact gctgctgggt tgtgaagcaa gtcagctggc actggaagtt ggtaaagcag 4860
   ttaaaaaacc ggttgcaccg gtggttgttc cggcaagcag cgcaccgtgt caagagcaga 4920
   tttttctggc agatgatccg gattttgatc tgcgtaccct gctgcctgca cataccaata 4980
   ccccgattga tgcaggtccg tttttttgtc tgggtctggc cctggcaagc gatccggtgg 5040
   atgcaagcct gaccgatgtt accattcatc gtctgtgtgt tcagggtcgt gatgaactga 5100
   gcatgttcct ggcagcaggt cgccatattg aagtttttcg tcagaaagca gaagcagcag 5160
   gtaaaccgct gccgattacc attaatatgg gtctggaccc agcaatctat attggcgcat 5220
   gttttgaagc accgaccacc ccgtttggtt ataatgaact gggtgttgcc ggtgcactgc 5280
   gtcagcgtcc ggttgaactg gttcagggtg ttagcgttcc ggaaaaagca attgcacgtg 5340
   ccgaaattgt tattgaaggt gaactgctgc ctggtgttcg tgttcgtgaa gatcagcata 5400
   ccaattcagg tcatgcaatg ccggaatttc cgggttattg tggtggtgca aatccgagcc 5460
   tgccggttat taaagttaaa gccgttacca tgcgcaataa cgcaattctg caaaccctgg 5520
   ttggtccggg tgaagaacat accaccctgg caggtctgcc gaccgaagca agcatttgga 5580
   atgcagttga agcagcaatt ccgggttttc tgcaaaatgt ttatgcccat accgcaggcg 5640
   gtggtaaatt tctgggtatt ctgcaagtga aaaaacgtca gcctgccgat gaaggtcgtc 5700
   agggtcaggc agccctgctg gcgctggcaa cctatagcga actgaaaaat atcattctgg 5760
   tggatgagga tgtggacatt tttgatagtg atgatattct gtgggcaatg accacccgta 5820
   tgcagggtga tgttagcatt accaccattc cgggtattcg cggtcatcag ctggacccga 5880
   gccagacacc ggaatattca ccgagcattc gtggtaatgg tattagctgc aaaaccatct 5940
   ttgattgtac cgttccgtgg gcactgaaaa gccattttga acgtgcaccg tttgcagatg 6000
   ttgatccgcg tccgtttgca cctgaatatt ttgcacgtct ggaaaaaaat cagggcagcg 6060
   caaaataagc taataacagg cctgctggta atcgcaggaa tttttatttg gatggatccc 6120
   cgggtaccga gctcgaattc actggccgtc gttttacaac gtcgtgactg ggaaaaccct 6180
   ggcgttaccc aacttaatcg ccttgcagca catccccctt tcgccagctg gcgtaatagc 6240
   gaagaggccc gcaccgatcg cccttcccaa cagttgcgca gcctgaatgg cgaatggcgc 6300
   ctgatgcggt attttctcct tacgcatctg tgcggtattt cacaccgcat atggtgcact 6360
   ctcagtacaa tctgctctga tgccgcatag ttaagccagc cccgacaccc gccaacaccc 6420
   gctgacgaat tc 6432
<210> 22
   <211> 7294
   <212> DNA
   <213> Artificial Sequence
<220>
   <223> Plasmid pMG31;
<400> 22
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc tttttatttt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgctattgac 4440
   gacagctatg gttcactgtc caccaaccaa aactgtgctc agtaccgcca atatttctcc 4500
   cttgaggggt acaaagaggt gtccctagaa gagatccacg ctgtgtaaaa attttacaaa 4560
   aaggtattga ctttccctac agggtgtgta ataatttaat tacaggcggg ggcaaccccg 4620
   cctgttctgc agaggaggaa tatagccatg gaagtgaaaa tcttcaacac ccaggatgtt 4680
   caggattttc tgcgtgttgc aagcggtctg gaacaagagg gtggtaatcc gcgtgttaaa 4740
   caaattattc atcgtgttct gagcgacctg tataaagcaa ttgaagatct gaatatcacc 4800
   agcgacgaat attgggcagg cgttgcatat ctgaatcagc tgggtgcaaa tcaagaagca 4860
   ggtctgctga gtccgggtct gggttttgat cattatctgg atatgcgtat ggatgcagaa 4920
   gatgcagcac tgggtattga aaatgcaaca ccgcgtacca ttgaaggtcc gctgtatgtt 4980
   gcgggtgcac cggaaagcgt tggttatgca cgcatggatg atggtagcga tccgaatggt 5040
   cataccctga ttctgcatgg caccattttt gatgcagatg gtaaaccgct gccgaatgca 5100
   aaagttgaaa tttggcatgc aaacaccaaa ggcttttata gccattttga tccgaccggt 5160
   gaacagcagg cctttaatat gcgtcgtagc attattaccg atgagaatgg tcagtatcgt 5220
   gttcgtacca ttctgcctgc cggttatggt tgtcctccgg aaggtccgac ccagcaactg 5280
   ctgaaccaac tgggtcgtca tggtaatcgt ccggcacata ttcattattt tgttagcgca 5340
   gatggtcacc gtaaactgac cacccagatt aatgttgccg gtgatccgta tacctatgat 5400
   gattttgcat atgccacccg tgaaggtctg gttgttgatg cagttgaaca taccgatccg 5460
   gaagcaatta aagccaatga tgtggaaggt ccttttgccg aaatggtgtt tgatctgaaa 5520
   ctgacccgtc tggttgatgg tgttgataat caggttgtgg atcgtccgcg tctggcagtt 5580
   taatacacca aaatggttca aaattatcag gcgagtgatc atgatcactg gcctgttttt 5640
   atttcaggga agggtggaga caattacgtg gataatcaga tcatccaaga aaccgtggat 5700
   aaaattctga gcgttctgcc gaatcaggca ggtcagctgg cacgtctggt gcgtctgatg 5760
   caatttgcat gcgatccgac cattaccgtt attggcaaat ataaccatgg taaaagccgt 5820
   ctgctgaatg aactgattgg caccgatatc tttagcgttg cagataaacg tgaaaccatt 5880
   cagctggccg aacataaaca ggatcaggtt cgttggctgg atgcacctgg tctggatgcc 5940
   gatgttgcag cagttgatga tcgtcatgca tttgaagcag tttggaccca ggcagatatt 6000
   cgtctgtttg ttcatagcgt tcgtgaaggt gaactggatg caaccgaaca ccatctgctg 6060
   caacagctga ttgaagatgc cgatcatagc cgtcgtcaga ccattctggt tctgacccag 6120
   attgatcaga ttccggatca gaccatcctg acacagatta aaaccagcat tgcacagcag 6180
   gttccgaaac tggatatttg ggcagttagc gcaacccgtc atcgtcaggg cattgaaaac 6240
   ggtaaaaccc tgctgatcga aaaaagcggt attggtgcac tgcgccatac cctggaacag 6300
   gcactggcac aggtgccgag cgcacgtacc tatgaaaaaa atcgtctgct gtcagatctg 6360
   caccatcagc tgaaacaact gctgctggat cagaaacatg ttctgcaaca actgcaacag 6420
   acacagcaac agcagctgca tgattttgat accggtctga ttaacattct ggacaaaatt 6480
   cgtgttgatc tggaaccgat tgtgaatatt gatggtcagg atcaagcact gaatccggat 6540
   agctttgcaa ccatgtttaa aaacaccgca gcaaaacagc agcgtgccaa agttcagatt 6600
   gcatatagcc gtgcatgcat tgaaatcaac agccatctga ttcgccatgg tgttgttggt 6660
   ctgcctgcgg aacagcagac caccattaaa agcattgata ccgtgattgt tgccgtgttt 6720
   ggtatcagcg ttaaatttcg tgatcagctg cgtgccctgt tttataccga taccgaacgt 6780
   cagcgtctgc aacgtgaatt tcgtttctat tttgaaaaaa gtgccggtcg catgattctg 6840
   gcagcaaaaa ttgaacagac catgcgtcag cagggctgta ttcagaatgc catgatggca 6900
   ctgcaacaaa tggaaagcgc agcataaaaa cacggacgcc gcaaacggcg tccgaatttc 6960
   ttggtcgact ctagaggatc cccgggtacc gagctcgaat tcactggccg tcgttttaca 7020
   acgtcgtgac tgggaaaacc ctggcgttac ccaacttaat cgccttgcag cacatccccc 7080
   tttcgccagc tggcgtaata gcgaagaggc ccgcaccgat cgcccttccc aacagttgcg 7140
   cagcctgaat ggcgaatggc gcctgatgcg gtattttctc cttacgcatc tgtgcggtat 7200
   ttcacaccgc atatggtgca ctctcagtac aatctgctct gatgccgcat agttaagcca 7260
   gccccgacac ccgccaacac ccgctgacga attc 7294
<210> 23
   <211> 8952
   <212> DNA
   <213> Artificial Sequence
<220>
   <223> Plasmid pMG33;
<400> 23
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct 840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgctattgac 4440
   gacagctatg gttcactgtc caccaaccaa aactgtgctc agtaccgcca atatttctcc 4500
   cttgaggggt acaaagaggt gtccctagaa gagatccacg ctgtgtaaaa attttacaaa 4560
   aaggtattga ctttccctac agggtgtgta ataatttaat tacaggcggg ggcaaccccg 4620
   cctgttctgc agaggaggaa tatagccatg gaagtgaaaa tcttcaacac ccaggatgtt 4680
   caggattttc tgcgtgttgc aagcggtctg gaacaagagg gtggtaatcc gcgtgttaaa 4740
   caaattattc atcgtgttct gagcgacctg tataaagcaa ttgaagatct gaatatcacc 4800
   agcgacgaat attgggcagg cgttgcatat ctgaatcagc tgggtgcaaa tcaagaagca 4860
   ggtctgctga gtccgggtct gggttttgat cattatctgg atatgcgtat ggatgcagaa 4920
   gatgcagcac tgggtattga aaatgcaaca ccgcgtacca ttgaaggtcc gctgtatgtt 4980
   gcgggtgcac cggaaagcgt tggttatgca cgcatggatg atggtagcga tccgaatggt 5040
   cataccctga ttctgcatgg caccattttt gatgcagatg gtaaaccgct gccgaatgca 5100
   aaagttgaaa tttggcatgc aaacaccaaa ggcttttata gccattttga tccgaccggt 5160
   gaacagcagg cctttaatat gcgtcgtagc attattaccg atgagaatgg tcagtatcgt 5220
   gttcgtacca ttctgcctgc cggttatggt tgtcctccgg aaggtccgac ccagcaactg 5280
   ctgaaccaac tgggtcgtca tggtaatcgt ccggcacata ttcattattt tgttagcgca 5340
   gatggtcacc gtaaactgac cacccagatt aatgttgccg gtgatccgta tacctatgat 5400
   gattttgcat atgccacccg tgaaggtctg gttgttgatg cagttgaaca taccgatccg 5460
   gaagcaatta aagccaatga tgtggaaggt ccttttgccg aaatggtgtt tgatctgaaa 5520
   ctgacccgtc tggttgatgg tgttgataat caggttgtgg atcgtccgcg tctggcagtt 5580
   taatacacca aaatggttca aaattatcag gcgagtgatc atgatcactg gcctgttttt 5640
   atttcaggga agggtggaga caattacgtg gataatcaga tcatccaaga aaccgtggat 5700
   aaaattctga gcgttctgcc gaatcaggca ggtcagctgg cacgtctggt gcgtctgatg 5760
   caatttgcat gcgatccgac cattaccgtt attggcaaat ataaccatgg taaaagccgt 5820
   ctgctgaatg aactgattgg caccgatatc tttagcgttg cagataaacg tgaaaccatt 5880
   cagctggccg aacataaaca ggatcaggtt cgttggctgg atgcacctgg tctggatgcc 5940
   gatgttgcag cagttgatga tcgtcatgca tttgaagcag tttggaccca ggcagatatt 6000
   cgtctgtttg ttcatagcgt tcgtgaaggt gaactggatg caaccgaaca ccatctgctg 6060
   caacagctga ttgaagatgc cgatcatagc cgtcgtcaga ccattctggt tctgacccag 6120
   attgatcaga ttccggatca gaccatcctg acacagatta aaaccagcat tgcacagcag 6180
   gttccgaaac tggatatttg ggcagttagc gcaacccgtc atcgtcaggg cattgaaaac 6240
   ggtaaaaccc tgctgatcga aaaaagcggt attggtgcac tgcgccatac cctggaacag 6300
   gcactggcac aggtgccgag cgcacgtacc tatgaaaaaa atcgtctgct gtcagatctg 6360
   caccatcagc tgaaacaact gctgctggat cagaaacatg ttctgcaaca actgcaacag 6420
   acacagcaac agcagctgca tgattttgat accggtctga ttaacattct ggacaaaatt 6480
   cgtgttgatc tggaaccgat tgtgaatatt gatggtcagg atcaagcact gaatccggat 6540
   agctttgcaa ccatgtttaa aaacaccgca gcaaaacagc agcgtgccaa agttcagatt 6600
   gcatatagcc gtgcatgcat tgaaatcaac agccatctga ttcgccatgg tgttgttggt 6660
   ctgcctgcgg aacagcagac caccattaaa agcattgata ccgtgattgt tgccgtgttt 6720
   ggtatcagcg ttaaatttcg tgatcagctg cgtgccctgt tttataccga taccgaacgt 6780
   cagcgtctgc aacgtgaatt tcgtttctat tttgaaaaaa gtgccggtcg catgattctg 6840
   gcagcaaaaa ttgaacagac catgcgtcag cagggctgta ttcagaatgc catgatggca 6900
   ctgcaacaaa tggaaagcgc agcataaaaa cacggacgcc gcaaacggcg tccgaatttc 6960
   ttggtcgacc gttaaatcta tcaccgcaag ggataaatat ctaacaccgt gcgtgttgac 7020
   tattttacct ctggcggtga taatggttgc atgtactaat ctagataagg aatatagcca 7080
   tgaccgcacc gattcaggat ctgcgtgatg caattgccct gctgcaacag catgataatc 7140
   agtatctgga aaccgatcat ccggttgatc cgaatgcaga actggcaggc gtttatcgtc 7200
   atattggtgc cggtggcacc gttaaacgtc cgacccgtat tggtccggca atgatgttta 7260
   ataacattaa aggttatccg cacagccgta ttctggttgg tatgcatgca agccgtcagc 7320
   gtgcagcact gctgctgggt tgtgaagcaa gtcagctggc actggaagtt ggtaaagcag 7380
   ttaaaaaacc ggttgcaccg gtggttgttc cggcaagcag cgcaccgtgt caagagcaga 7440
   tttttctggc agatgatccg gattttgatc tgcgtaccct gctgcctgca cataccaata 7500
   ccccgattga tgcaggtccg tttttttgtc tgggtctggc cctggcaagc gatccggtgg 7560
   atgcaagcct gaccgatgtt accattcatc gtctgtgtgt tcagggtcgt gatgaactga 7620
   gcatgttcct ggcagcaggt cgccatattg aagtttttcg tcagaaagca gaagcagcag 7680
   gtaaaccgct gccgattacc attaatatgg gtctggaccc agcaatctat attggcgcat 7740
   gttttgaagc accgaccacc ccgtttggtt ataatgaact gggtgttgcc ggtgcactgc 7800
   gtcagcgtcc ggttgaactg gttcagggtg ttagcgttcc ggaaaaagca attgcacgtg 7860
   ccgaaattgt tattgaaggt gaactgctgc ctggtgttcg tgttcgtgaa gatcagcata 7920
   ccaattcagg tcatgcaatg ccggaatttc cgggttattg tggtggtgca aatccgagcc 7980
   tgccggttat taaagttaaa gccgttacca tgcgcaataa cgcaattctg caaaccctgg 8040
   ttggtccggg tgaagaacat accaccctgg caggtctgcc gaccgaagca agcatttgga 8100
   atgcagttga agcagcaatt ccgggttttc tgcaaaatgt ttatgcccat accgcaggcg 8160
   gtggtaaatt tctgggtatt ctgcaagtga aaaaacgtca gcctgccgat gaaggtcgtc 8220
   agggtcaggc agccctgctg gcgctggcaa cctatagcga actgaaaaat atcattctgg 8280
   tggatgagga tgtggacatt tttgatagtg atgatattct gtgggcaatg accacccgta 8340
   tgcagggtga tgttagcatt accaccattc cgggtattcg cggtcatcag ctggacccga 8400
   gccagacacc ggaatattca ccgagcattc gtggtaatgg tattagctgc aaaaccatct 8460
   ttgattgtac cgttccgtgg gcactgaaaa gccattttga acgtgcaccg tttgcagatg 8520
   ttgatccgcg tccgtttgca cctgaatatt ttgcacgtct ggaaaaaaat cagggcagcg 8580
   caaaataagc taataacagg cctgctggta atcgcaggaa tttttatttg gatggatccc 8640
   cgggtaccga gctcgaattc actggccgtc gttttacaac gtcgtgactg ggaaaaccct 8700
   ggcgttaccc aacttaatcg ccttgcagca catccccctt tcgccagctg gcgtaatagc 8760
   gaagaggccc gcaccgatcg cccttcccaa cagttgcgca gcctgaatgg cgaatggcgc 8820
   ctgatgcggt attttctcct tacgcatctg tgcggtattt cacaccgcat atggtgcact 8880
   ctcagtacaa tctgctctga tgccgcatag ttaagccagc cccgacaccc gccaacaccc 8940
   gctgacgaat tc 8952
<210> 24
   <211> 10630
   <212> DNA
   <213> Artificial Sequence
<220>
   <223> Plasmid pMG37;
<400> 24
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt     900
   tcacttataa ccaatacgct cagatgatga acatcagtag ggaaaatgct tatggtgtat     960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgctattgac 4440
   gacagctatg gttcactgtc caccaaccaa aactgtgctc agtaccgcca atatttctcc 4500
   cttgaggggt acaaagaggt gtccctagaa gagatccacg ctgtgtaaaa attttacaaa 4560
   aaggtattga ctttccctac agggtgtgta ataatttaat tacaggcggg ggcaaccccg 4620
   cctgttctgc agaggaggaa tatagccatg gaagtgaaaa tcttcaacac ccaggatgtt 4680
   caggattttc tgcgtgttgc aagcggtctg gaacaagagg gtggtaatcc gcgtgttaaa 4740
   caaattattc atcgtgttct gagcgacctg tataaagcaa ttgaagatct gaatatcacc 4800
   agcgacgaat attgggcagg cgttgcatat ctgaatcagc tgggtgcaaa tcaagaagca 4860
   ggtctgctga gtccgggtct gggttttgat cattatctgg atatgcgtat ggatgcagaa 4920
   gatgcagcac tgggtattga aaatgcaaca ccgcgtacca ttgaaggtcc gctgtatgtt 4980
   gcgggtgcac cggaaagcgt tggttatgca cgcatggatg atggtagcga tccgaatggt 5040
   cataccctga ttctgcatgg caccattttt gatgcagatg gtaaaccgct gccgaatgca 5100
   aaagttgaaa tttggcatgc aaacaccaaa ggcttttata gccattttga tccgaccggt 5160
   gaacagcagg cctttaatat gcgtcgtagc attattaccg atgagaatgg tcagtatcgt 5220
   gttcgtacca ttctgcctgc cggttatggt tgtcctccgg aaggtccgac ccagcaactg 5280
   ctgaaccaac tgggtcgtca tggtaatcgt ccggcacata ttcattattt tgttagcgca 5340
   gatggtcacc gtaaactgac cacccagatt aatgttgccg gtgatccgta tacctatgat 5400
   gattttgcat atgccacccg tgaaggtctg gttgttgatg cagttgaaca taccgatccg 5460
   gaagcaatta aagccaatga tgtggaaggt ccttttgccg aaatggtgtt tgatctgaaa 5520
   ctgacccgtc tggttgatgg tgttgataat caggttgtgg atcgtccgcg tctggcagtt 5580
   taatacacca aaatggttca aaattatcag gcgagtgatc atgatcactg gcctgttttt 5640
   atttcaggga agggtggaga caattacgtg gataatcaga tcatccaaga aaccgtggat 5700
   aaaattctga gcgttctgcc gaatcaggca ggtcagctgg cacgtctggt gcgtctgatg 5760
   caatttgcat gcgatccgac cattaccgtt attggcaaat ataaccatgg taaaagccgt 5820
   ctgctgaatg aactgattgg caccgatatc tttagcgttg cagataaacg tgaaaccatt 5880
   cagctggccg aacataaaca ggatcaggtt cgttggctgg atgcacctgg tctggatgcc 5940
   gatgttgcag cagttgatga tcgtcatgca tttgaagcag tttggaccca ggcagatatt 6000
   cgtctgtttg ttcatagcgt tcgtgaaggt gaactggatg caaccgaaca ccatctgctg 6060
   caacagctga ttgaagatgc cgatcatagc cgtcgtcaga ccattctggt tctgacccag 6120
   attgatcaga ttccggatca gaccatcctg acacagatta aaaccagcat tgcacagcag 6180
   gttccgaaac tggatatttg ggcagttagc gcaacccgtc atcgtcaggg cattgaaaac 6240
   ggtaaaaccc tgctgatcga aaaaagcggt attggtgcac tgcgccatac cctggaacag 6300
   gcactggcac aggtgccgag cgcacgtacc tatgaaaaaa atcgtctgct gtcagatctg 6360
   caccatcagc tgaaacaact gctgctggat cagaaacatg ttctgcaaca actgcaacag 6420
   acacagcaac agcagctgca tgattttgat accggtctga ttaacattct ggacaaaatt 6480
   cgtgttgatc tggaaccgat tgtgaatatt gatggtcagg atcaagcact gaatccggat 6540
   agctttgcaa ccatgtttaa aaacaccgca gcaaaacagc agcgtgccaa agttcagatt 6600
   gcatatagcc gtgcatgcat tgaaatcaac agccatctga ttcgccatgg tgttgttggt 6660
   ctgcctgcgg aacagcagac caccattaaa agcattgata ccgtgattgt tgccgtgttt 6720
   ggtatcagcg ttaaatttcg tgatcagctg cgtgccctgt tttataccga taccgaacgt 6780
   cagcgtctgc aacgtgaatt tcgtttctat tttgaaaaaa gtgccggtcg catgattctg 6840
   gcagcaaaaa ttgaacagac catgcgtcag cagggctgta ttcagaatgc catgatggca 6900
   ctgcaacaaa tggaaagcgc agcataaaaa cacggacgcc gcaaacggcg tccgaatttc 6960
   ttggtcgacc gttaaatcta tcaccgcaag ggataaatat ctaacaccgt gcgtgttgac 7020
   tattttacct ctggcggtga taatggttgc atgtactaat ctagataagg aatatagcca 7080
   tgaccgcacc gattcaggat ctgcgtgatg caattgccct gctgcaacag catgataatc 7140
   agtatctgga aaccgatcat ccggttgatc cgaatgcaga actggcaggc gtttatcgtc 7200
   atattggtgc cggtggcacc gttaaacgtc cgacccgtat tggtccggca atgatgttta 7260
   ataacattaa aggttatccg cacagccgta ttctggttgg tatgcatgca agccgtcagc 7320
   gtgcagcact gctgctgggt tgtgaagcaa gtcagctggc actggaagtt ggtaaagcag 7380
   ttaaaaaacc ggttgcaccg gtggttgttc cggcaagcag cgcaccgtgt caagagcaga 7440
   tttttctggc agatgatccg gattttgatc tgcgtaccct gctgcctgca cataccaata 7500
   ccccgattga tgcaggtccg tttttttgtc tgggtctggc cctggcaagc gatccggtgg 7560
   atgcaagcct gaccgatgtt accattcatc gtctgtgtgt tcagggtcgt gatgaactga 7620
   gcatgttcct ggcagcaggt cgccatattg aagtttttcg tcagaaagca gaagcagcag 7680
   gtaaaccgct gccgattacc attaatatgg gtctggaccc agcaatctat attggcgcat 7740
   gttttgaagc accgaccacc ccgtttggtt ataatgaact gggtgttgcc ggtgcactgc 7800
   gtcagcgtcc ggttgaactg gttcagggtg ttagcgttcc ggaaaaagca attgcacgtg 7860
   ccgaaattgt tattgaaggt gaactgctgc ctggtgttcg tgttcgtgaa gatcagcata 7920
   ccaattcagg tcatgcaatg ccggaatttc cgggttattg tggtggtgca aatccgagcc 7980
   tgccggttat taaagttaaa gccgttacca tgcgcaataa cgcaattctg caaaccctgg 8040
   ttggtccggg tgaagaacat accaccctgg caggtctgcc gaccgaagca agcatttgga 8100
   atgcagttga agcagcaatt ccgggttttc tgcaaaatgt ttatgcccat accgcaggcg 8160
   gtggtaaatt tctgggtatt ctgcaagtga aaaaacgtca gcctgccgat gaaggtcgtc 8220
   agggtcaggc agccctgctg gcgctggcaa cctatagcga actgaaaaat atcattctgg 8280
   tggatgagga tgtggacatt tttgatagtg atgatattct gtgggcaatg accacccgta 8340
   tgcagggtga tgttagcatt accaccattc cgggtattcg cggtcatcag ctggacccga 8400
   gccagacacc ggaatattca ccgagcattc gtggtaatgg tattagctgc aaaaccatct 8460
   ttgattgtac cgttccgtgg gcactgaaaa gccattttga acgtgcaccg tttgcagatg 8520
   ttgatccgcg tccgtttgca cctgaatatt ttgcacgtct ggaaaaaaat cagggcagcg 8580
   caaaataagc taataacagg cctgctggta atcgcaggaa tttttatttg gatggatccg 8640
   cctacctagc ttccaagaaa gatatcctaa cagcacaaga gcggaaagat gttttgttct 8700
   acatccagaa caacctctgc taaaattcct gaaaaatttt gcaaaaagtt gttgacttta 8760
   tctacaaggt gtggtataat aatcttaaca acagcaggac gctcccgggt tgaggaaaac 8820
   ctaatgaaac tgaccagcct gcgtgttagc ctgctggcac tgggtctggt taccagcggt 8880
   tttgcagcag cagaaaccta taccgttgat cgttatcagg atgatagcga aaaaggtagc 8940
   ctgcgttggg caattgaaca gagcaatgca aatagcgcac aagaaaacca gattctgatt 9000
   caggcagttg gtaaagcacc gtatgttatc aaagttgata aaccgctgcc tccgattaaa 9060
   agcagcgtta aaatcattgg caccgagtgg gataaaaccg gtgaatttat tgcaattgat 9120
   ggcagcaact atatcaaagg cgaaggtgaa aaagcatgtc cgggtgcaaa tccgggtcag 9180
   tatggcacca atgttcgtac catgaccctg cctggtctgg ttctgcaaga tgttaatggt 9240
   gttaccctga aggtctggga tgttcatcgt ttttgtattg gtgttctggt taatcgcagc 9300
   agcaataacc tgattcagca taatcgtatc agcaacaatt atggtggtgc cggtgttatg 9360
   attaccggtg atgatggtaa aggtaatccg accagcacca ccaccaataa taacaaagtt 9420
   ctggataacg tgttcatcga taatggtgat ggtctggaac tgacccgtgg tgcagcattt 9480
   aatctgattg caaataacct gtttaccagc acaaaagcca atccggaacc gagccagggt 9540
   attgaaattc tgtggggtaa tgataatgcc gtggtgggta acaaattcga aaactattca 9600
   gatggcctgc aaatcaattg gggtaaacgt aactatatcg cctataacga actgaccaat 9660
   aacagcctgg gtttcaatct gacaggtgat ggtaacattt tcgacagcaa taaagtgcat 9720
   ggtaaccgta ttggtattgc cattcgtagt gaaaaagatg ccaatgcacg tattaccctg 9780
   accaaaaatc agatttggga taacggcaaa gatatcaaac gttgtgaagc cggtggtagc 9840
   tgtgttccga atcagcgtct gggtgcaatt gtttttggtg ttccggcact ggaacatgaa 9900
   ggttttgttg gtagccgtgg cggtggtgtt gttattgaac cggcaaaact gcaaaaaacc 9960
   tgcacccagc cgaaccagca gaattgtaat gcaattccta atcagggtat tcaggcaccg 10020
   aaactgacag ttagcaaaaa acagctgacc gttgaagtta aaggcacccc taatcagcgt 10080
   tataatgtgg aatttttggc caatcgtaat gccagcagca gcgaagcaga acagtatctg 10140
   ggtagcattg ttgttgttac cgatcatcag ggtctggcaa aagcaaattg ggctccgaaa 10200
   gttagcatgc cgagcgttac cgcaaatgtg acagatcatc tgggtgcgac cagcgaactg 10260
   agcagcgcag ttaaaatgcg ttaaatgcat gcgcgccgcg ttcgcgcggc gctttttttt 10320
   ggtaccgagc tcgaattcac tggccgtcgt tttacaacgt cgtgactggg aaaaccctgg 10380
   cgttacccaa cttaatcgcc ttgcagcaca tccccctttc gccagctggc gtaatagcga 10440
   agaggcccgc accgatcgcc cttcccaaca gttgcgcagc ctgaatggcg aatggcgcct 10500
   gatgcggtat tttctcctta cgcatctgtg cggtatttca caccgcatat ggtgcactct 10560
   cagtacaatc tgctctgatg ccgcatagtt aagccagccc cgacacccgc caacacccgc 10620
   tgacgaattc 10630
<210> 25
   <211> 6452
   <212> DNA
   <213> Artificial Sequence
<220>
   <223> Plasmid pMG39;
<400> 25
   gttgacagta agacgggtaa gcctgttgat gataccgctg ccttactggg tgcattagcc 60
   agtctgaatg acctgtcacg ggataatccg aagtggtcag actggaaaat cagagggcag 120
   gaactgctga acagcaaaaa gtcagatagc accacatagc agacccgcca taaaacgccc 180
   tgagaagccc gtgacgggct tttcttgtat tatgggtagt ttccttgcat gaatccataa 240
   aaggcgcctg tagtgccatt tacccccatt cactgccaga gccgtgagcg cagcgaactg 300
   aatgtcacga aaaagacagc gactcaggtg cctgatggtc ggagacaaaa ggaatattca 360
   gcgatttgcc cgagcttgcg agggtgctac ttaagccttt agggttttaa ggtctgtttt 420
   gtagaggagc aaacagcgtt tgcgacatcc ttttgtaata ctgcggaact gactaaagta 480
   gtgagttata cacagggctg ggatctattc tttttatctt tttttattct ttctttattc 540
   tataaattat aaccacttga atataaacaa aaaaaacaca caaaggtcta gcggaattta 600
   cagagggtct agcagaattt acaagttttc cagcaaaggt ctagcagaat ttacagatac 660
   ccacaactca aaggaaaagg actagtaatt atcattgact agcccatctc aattggtata 720
   gtgattaaaa tcacctagac caattgagat gtatgtctga attagttgtt ttcaaagcaa 780
   atgaactagc gattagtcgc tatgacttaa cggagcatga aaccaagcta attttatgct 840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgcatgcctg 4440
   caggtcgact ctagaggatc cgcctaccta gcttccaaga aagatatcct aacagcacaa 4500
   gagcggaaag atgttttgtt ctacatccag aacaacctct gctaaaattc ctgaaaaatt 4560
   ttgcaaaaag ttgttgactt tatctacaag gtgtggtata ataatcttaa caacagcagg 4620
   acgctcccgg gttgaggaaa acctaatgaa actgaccagc ctgcgtgtta gcctgctggc 4680
   actgggtctg gttaccagcg gttttgcagc agcagaaacc tataccgttg atcgttatca 4740
   ggatgatagc gaaaaaggta gcctgcgttg ggcaattgaa cagagcaatg caaatagcgc 4800
   acaagaaaac cagattctga ttcaggcagt tggtaaagca ccgtatgtta tcaaagttga 4860
   taaaccgctg cctccgatta aaagcagcgt taaaatcatt ggcaccgagt gggataaaac 4920
   cggtgaattt attgcaattg atggcagcaa ctatatcaaa ggcgaaggtg aaaaagcatg 4980
   tccgggtgca aatccgggtc agtatggcac caatgttcgt accatgaccc tgcctggtct 5040
   ggttctgcaa gatgttaatg gtgttaccct gaaaggtctg gatgttcatc gtttttgtat 5100
   tggtgttctg gttaatcgca gcagcaataa cctgattcag cataatcgta tcagcaacaa 5160
   ttatggtggt gccggtgtta tgattaccgg tgatgatggt aaaggtaatc cgaccagcac 5220
   caccaccaat aataacaaag ttctggataa cgtgttcatc gataatggtg atggtctgga 5280
   actgacccgt ggtgcagcat ttaatctgat tgcaaataac ctgtttacca gcacaaaagc 5340
   caatccggaa ccgagccagg gtattgaaat tctgtggggt aatgataatg ccgtggtggg 5400
   taacaaattc gaaaactatt cagatggcct gcaaatcaat tggggtaaac gtaactatat 5460
   cgcctataac gaactgacca ataacagcct gggtttcaat ctgacaggtg atggtaacat 5520
   tttcgacagc aataaagtgc atggtaaccg tattggtatt gccattcgta gtgaaaaaga 5580
   tgccaatgca cgtattaccc tgaccaaaaa tcagatttgg gataacggca aagatatcaa 5640
   acgttgtgaa gccggtggta gctgtgttcc gaatcagcgt ctgggtgcaa ttgtttttgg 5700
   tgttccggca ctggaacatg aaggttttgt tggtagccgt ggcggtggtg ttgttattga 5760
   accggcaaaa ctgcaaaaaa cctgcaccca gccgaaccag cagaattgta atgcaattcc 5820
   taatcagggt attcaggcac cgaaactgac agttagcaaa aaacagctga ccgttgaagt 5880
   taaaggcacc cctaatcagc gttataatgt ggaatttttt ggcaatcgta atgccagcag 5940
   cagcgaagca gaacagtatc tgggtagcat tgttgttgtt accgatcatc agggtctggc 6000
   aaaagcaaat tgggctccga aagttagcat gccgagcgtt accgcaaatg tgacagatca 6060
   tctgggtgcg accagcgaac tgagcagcgc agttaaaatg cgttaaatgc atgcgcgccg 6120
   cgttcgcgcg gcgctttttt ttggtaccga gctcgaattc actggccgtc gttttacaac 6180
   gtcgtgactg ggaaaaccct ggcgttaccc aacttaatcg ccttgcagca catccccctt 6240
   tcgccagctg gcgtaatagc gaagaggccc gcaccgatcg cccttcccaa cagttgcgca 6300
   gcctgaatgg cgaatggcgc ctgatgcggt attttctcct tacgcatctg tgcggtattt 6360
   cacaccgcat atggtgcact ctcagtacaa tctgctctga tgccgcatag ttaagccagc 6420
   cccgacaccc gccaacaccc gctgacgaat tc 6452
<210> 26
   <211> 10012
   <212> DNA
   <213> Artificial Sequence
<220>
   <223> Plasmid pMG47F;
<400> 26
   gttgacagta agacgggtaa gcctgttgat gataccgctg ccttactggg tgcattagcc 60
   agtctgaatg acctgtcacg ggataatccg aagtggtcag actggaaaat cagagggcag 120
   gaactgctga acagcaaaaa gtcagatagc accacatagc agacccgcca taaaacgccc 180
   tgagaagccc gtgacgggct tttcttgtat tatgggtagt ttccttgcat gaatccataa 240
   aaggcgcctg tagtgccatt tacccccatt cactgccaga gccgtgagcg cagcgaactg 300
   aatgtcacga aaaagacagc gactcaggtg cctgatggtc ggagacaaaa ggaatattca 360
   gcgatttgcc cgagcttgcg agggtgctac ttaagccttt agggttttaa ggtctgtttt 420
   gtagaggagc aaacagcgtt tgcgacatcc ttttgtaata ctgcggaact gactaaagta 480
   gtgagttata cacagggctg ggatctattc tttttatctt tttttattct ttctttattc 540
   tataaattat aaccacttga atataaacaa aaaaaacaca caaaggtcta gcggaattta 600
   cagagggtct agcagaattt acaagttttc cagcaaaggt ctagcagaat ttacagatac 660
   ccacaactca aaggaaaagg actagtaatt atcattgact agcccatctc aattggtata 720
   gtgattaaaa tcacctagac caattgagat gtatgtctga attagttgtt ttcaaagcaa 780
   atgaactagc gattagtcgc tatgacttaa cggagcatga aaccaagcta attttatgct 840
   gtgtggcact actcaacccc acgattgaaa accctacaag gaaagaacgg acggtatcgt 900
   tcacttataa ccaatacgct cagatgatga acatcagtag ggaaaatgct tatggtgtat 960
   tagctaaagc aaccagagag ctgatgacga gaactgtgga aatcaggaat cctttggtta 1020
   aaggctttga gattttccag tggacaaact atgccaagtt ctcaagcgaa aaattagaat 1080
   tagtttttag tgaagagata ttgccttatc ttttccagtt aaaaaaattc ataaaatata 1140
   atctggaaca tgttaagtct tttgaaaaca aatactctat gaggatttat gagtggttat 1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgctattgac 4440
   gacagctatg gttcactgtc caccaaccaa aactgtgctc agtaccgcca atatttctcc 4500
   cttgaggggt acaaagaggt gtccctagaa gagatccacg ctgtgtaaaa attttacaaa 4560
   aaggtattga ctttccctac agggtgtgta ataatttaat tacaggcggg ggcaaccccg 4620
   cctgttctgc agaggaggaa tatagccatg gaagtgaaaa tcttcaacac ccaggatgtt 4680
   caggattttc tgcgtgttgc aagcggtctg gaacaagagg gtggtaatcc gcgtgttaaa 4740
   caaattattc atcgtgttct gagcgacctg tataaagcaa ttgaagatct gaatatcacc 4800
   agcgacgaat attgggcagg cgttgcatat ctgaatcagc tgggtgcaaa tcaagaagca 4860
   ggtctgctga gtccgggtct gggttttgat cattatctgg atatgcgtat ggatgcagaa 4920
   gatgcagcac tgggtattga aaatgcaaca ccgcgtacca ttgaaggtcc gctgtatgtt 4980
   gcgggtgcac cggaaagcgt tggttatgca cgcatggatg atggtagcga tccgaatggt 5040
   cataccctga ttctgcatgg caccattttt gatgcagatg gtaaaccgct gccgaatgca 5100
   aaagttgaaa tttggcatgc aaacaccaaa ggcttttata gccattttga tccgaccggt 5160
   gaacagcagg cctttaatat gcgtcgtagc attattaccg atgagaatgg tcagtatcgt 5220
   gttcgtacca ttctgcctgc cggttatggt tgtcctccgg aaggtccgac ccagcaactg 5280
   ctgaaccaac tgggtcgtca tggtaatcgt ccggcacata ttcattattt tgttagcgca 5340
   gatggtcacc gtaaactgac cacccagatt aatgttgccg gtgatccgta tacctatgat 5400
   gattttgcat atgccacccg tgaaggtctg gttgttgatg cagttgaaca taccgatccg 5460
   gaagcaatta aagccaatga tgtggaaggt ccttttgccg aaatggtgtt tgatctgaaa 5520
   ctgacccgtc tggttgatgg tgttgataat caggttgtgg atcgtccgcg tctggcagtt 5580
   taatacacca aaatggttca aaattatcag gcgagtgatc atgatcactg gcctgttttt 5640
   atttcaggga agggtggaga caattacgtg gataatcaga tcatccaaga aaccgtggat 5700
   aaaattctga gcgttctgcc gaatcaggca ggtcagctgg cacgtctggt gcgtctgatg 5760
   caatttgcat gcgatccgac cattaccgtt attggcaaat ataaccatgg taaaagccgt 5820
   ctgctgaatg aactgattgg caccgatatc tttagcgttg cagataaacg tgaaaccatt 5880
   cagctggccg aacataaaca ggatcaggtt cgttggctgg atgcacctgg tctggatgcc 5940
   gatgttgcag cagttgatga tcgtcatgca tttgaagcag tttggaccca ggcagatatt 6000
   cgtctgtttg ttcatagcgt tcgtgaaggt gaactggatg caaccgaaca ccatctgctg 6060
   caacagctga ttgaagatgc cgatcatagc cgtcgtcaga ccattctggt tctgacccag 6120
   attgatcaga ttccggatca gaccatcctg acacagatta aaaccagcat tgcacagcag 6180
   gttccgaaac tggatatttg ggcagttagc gcaacccgtc atcgtcaggg cattgaaaac 6240
   ggtaaaaccc tgctgatcga aaaaagcggt attggtgcac tgcgccatac cctggaacag 6300
   gcactggcac aggtgccgag cgcacgtacc tatgaaaaaa atcgtctgct gtcagatctg 6360
   caccatcagc tgaaacaact gctgctggat cagaaacatg ttctgcaaca actgcaacag 6420
   acacagcaac agcagctgca tgattttgat accggtctga ttaacattct ggacaaaatt 6480
   cgtgttgatc tggaaccgat tgtgaatatt gatggtcagg atcaagcact gaatccggat 6540
   agctttgcaa ccatgtttaa aaacaccgca gcaaaacagc agcgtgccaa agttcagatt 6600
   gcatatagcc gtgcatgcat tgaaatcaac agccatctga ttcgccatgg tgttgttggt 6660
   ctgcctgcgg aacagcagac caccattaaa agcattgata ccgtgattgt tgccgtgttt 6720
   ggtatcagcg ttaaatttcg tgatcagctg cgtgccctgt tttataccga taccgaacgt 6780
   cagcgtctgc aacgtgaatt tcgtttctat tttgaaaaaa gtgccggtcg catgattctg 6840
   gcagcaaaaa ttgaacagac catgcgtcag cagggctgta ttcagaatgc catgatggca 6900
   ctgcaacaaa tggaaagcgc agcataaaaa cacggacgcc gcaaacggcg tccgaatttc 6960
   ttggtcgacc gttaaatcta tcaccgcaag ggataaatat ctaacaccgt gcgtgttgac 7020
   tattttacct ctggcggtga taatggttgc atgtactaat ctagataagg aatatagcca 7080
   tgaccgcacc gattcaggat ctgcgtgatg caattgccct gctgcaacag catgataatc 7140
   agtatctgga aaccgatcat ccggttgatc cgaatgcaga actggcaggc gtttatcgtc 7200
   atattggtgc cggtggcacc gttaaacgtc cgacccgtat tggtccggca atgatgttta 7260
   ataacattaa aggttatccg cacagccgta ttctggttgg tatgcatgca agccgtcagc 7320
   gtgcagcact gctgctgggt tgtgaagcaa gtcagctggc actggaagtt ggtaaagcag 7380
   ttaaaaaacc ggttgcaccg gtggttgttc cggcaagcag cgcaccgtgt caagagcaga 7440
   tttttctggc agatgatccg gattttgatc tgcgtaccct gctgcctgca cataccaata 7500
   ccccgattga tgcaggtccg tttttttgtc tgggtctggc cctggcaagc gatccggtgg 7560
   atgcaagcct gaccgatgtt accattcatc gtctgtgtgt tcagggtcgt gatgaactga 7620
   gcatgttcct ggcagcaggt cgccatattg aagtttttcg tcagaaagca gaagcagcag 7680
   gtaaaccgct gccgattacc attaatatgg gtctggaccc agcaatctat attggcgcat 7740
   gttttgaagc accgaccacc ccgtttggtt ataatgaact gggtgttgcc ggtgcactgc 7800
   gtcagcgtcc ggttgaactg gttcagggtg ttagcgttcc ggaaaaagca attgcacgtg 7860
   ccgaaattgt tattgaaggt gaactgctgc ctggtgttcg tgttcgtgaa gatcagcata 7920
   ccaattcagg tcatgcaatg ccggaatttc cgggttattg tggtggtgca aatccgagcc 7980
   tgccggttat taaagttaaa gccgttacca tgcgcaataa cgcaattctg caaaccctgg 8040
   ttggtccggg tgaagaacat accaccctgg caggtctgcc gaccgaagca agcatttgga 8100
   atgcagttga agcagcaatt ccgggttttc tgcaaaatgt ttatgcccat accgcaggcg 8160
   gtggtaaatt tctgggtatt ctgcaagtga aaaaacgtca gcctgccgat gaaggtcgtc 8220
   agggtcaggc agccctgctg gcgctggcaa cctatagcga actgaaaaat atcattctgg 8280
   tggatgagga tgtggacatt tttgatagtg atgatattct gtgggcaatg accacccgta 8340
   tgcagggtga tgttagcatt accaccattc cgggtattcg cggtcatcag ctggacccga 8400
   gccagacacc ggaatattca ccgagcattc gtggtaatgg tattagctgc aaaaccatct 8460
   ttgattgtac cgttccgtgg gcactgaaaa gccattttga acgtgcaccg tttgcagatg 8520
   ttgatccgcg tccgtttgca cctgaatatt ttgcacgtct ggaaaaaaat cagggcagcg 8580
   caaaataagc taataacagg cctgctggta atcgcaggaa tttttatttg gatggatccg 8640
   cctacctagc ttccaagaaa gatatcctaa cagcacaaga gcggaaagat gttttgttct 8700
   acatccagaa caacctctgc taaaattcct gaaaaatttt gcaaaaagtt gttgacttta 8760
   tctacaaggt gtggtataat aatcttaaca acagcaggac gctcccgggt tgaggaaaac 8820
   ctaatgaaat atagcctgtg caccattagc tttcgtcacc agctgattag ctttaccgat 8880
   attgttcagt ttgcctatga aaacggcttt gaaggtattg aactgtgggg cacccatgca 8940
   cagaatctgt atatgcaaga atatgaaacc accgaacgtg aactgaattg cctgaaagat 9000
   aaaaccctgg aaattaccat gatcagcgat tatctggata ttagcctgag cgcagatttt 9060
   gaaaaaacca tcgaaaaatg tgaacagctg gcaattctgg ccaattggtt taaaacgaac 9120
   aaaattcgta cctttgccgg tcagaaaggt agtgcagatt ttagccagca agaacgtcaa 9180
   gagtatgtga atcgtattcg catgatttgt gaactgtttg cccagcataa tatgtatgtt 9240
   ctgctggaaa cccatccgaa taccctgacc gataccctgc cgagcaccct ggaactgctg 9300
   ggtgaagttg atcatccgaa tctgaaaatc aacctggatt ttctgcatat ctgggaaagc 9360
   ggtgcagatc cggttgatag ctttcagcag ctgcgtccgt ggattcagca ttatcacttt 9420
   aaaaacatta gcagcgcaga ctatctgcat gtgtttgaac cgaataatgt ttatgcagca 9480
   gcaggtaatc gtaccggtat ggttccgctg tttgaaggca ttgttaacta tgatgaaatc 9540
   atccaagaag tgcgcgatac cgatcatttt gcaagcctgg aatggtttgg tcataacgca 9600
   aaagatattc tgaaagccga aatgaaagtg ctgaccaatc gtaatctgga agttgttacc 9660
   agctaaatgc atgcgcgccg cgttcgcgcg gcgctttttt ttggtaccga gctcgaattc     9720
   actggccgtc gttttacaac gtcgtgactg ggaaaaccct ggcgttaccc aacttaatcg     9780
   ccttgcagca catccccctt tcgccagctg gcgtaatagc gaagaggccc gcaccgatcg     9840
   cccttcccaa cagttgcgca gcctgaatgg cgaatggcgc ctgatgcggt attttctcct     9900
   tacgcatctg tgcggtattt cacaccgcat atggtgcact ctcagtacaa tctgctctga     9960
   tgccgcatag ttaagccagc cccgacaccc gccaacaccc gctgacgaat tc            10012
<210> 27
   <211> 10249
   <212> DNA
   <213> Artificial Sequence
<220>
   <223> Plasmid pMG70;
<400> 27
   gttgacagta agacgggtaa gcctgttgat gataccgctg ccttactggg tgcattagcc      60
   agtctgaatg acctgtcacg ggataatccg aagtggtcag actggaaaat cagagggcag     120
   gaactgctga acagcaaaaa gtcagatagc accacatagc agacccgcca taaaacgccc     180
   tgagaagccc gtgacgggct tttcttgtat tatgggtagt ttccttgcat gaatccataa     240
   aaggcgcctg tagtgccatt tacccccatt cactgccaga gccgtgagcg cagcgaactg     300
   aatgtcacga aaaagacagc gactcaggtg cctgatggtc ggagacaaaa ggaatattca     360
   gcgatttgcc cgagcttgcg agggtgctac ttaagccttt agggttttaa ggtctgtttt     420
   gtagaggagc aaacagcgtt tgcgacatcc ttttgtaata ctgcggaact gactaaagta     480
   gtgagttata cacagggctg ggatctattc tttttatctt tttttattct ttctttattc     540
   tataaattat aaccacttga atataaacaa aaaaaacaca caaaggtcta gcggaattta     600
   cagagggtct agcagaattt acaagttttc cagcaaaggt ctagcagaat ttacagatac     660
   ccacaactca aaggaaaagg actagtaatt atcattgact agcccatctc aattggtata     720
   gtgattaaaa tcacctagac caattgagat gtatgtctga attagttgtt ttcaaagcaa     780
   atgaactagc gattagtcgc tatgacttaa cggagcatga aaccaagcta attttatgct     840
   gtgtggcact actcaacccc acgattgaaa accctacaag gaaagaacgg acggtatcgt     900
   tcacttataa ccaatacgct cagatgatga acatcagtag ggaaaatgct tatggtgtat     960
   tagctaaagc aaccagagag ctgatgacga gaactgtgga aatcaggaat cctttggtta    1020
   aaggctttga gattttccag tggacaaact atgccaagtt ctcaagcgaa aaattagaat    1080
   tagtttttag tgaagagata ttgccttatc ttttccagtt aaaaaaattc ataaaatata    1140
   atctggaaca tgttaagtct tttgaaaaca aatactctat gaggatttat gagtggttat    1200
   taaaagaact aacacaaaag aaaactcaca aggcaaatat agagattagc cttgatgaat 1260
   ttaagttcat gttaatgctt gaaaataact accatgagtt taaaaggctt aaccaatggg 1320
   ttttgaaacc aataagtaaa gatttaaaca cttacagcaa tatgaaattg gtggttgata 1380
   agcgaggccg cccgactgat acgttgattt tccaagttga actagataga caaatggatc 1440
   tcgtaaccga acttgagaac aaccagataa aaatgaatgg tgacaaaata ccaacaacca 1500
   ttacatcaga ttcctaccta cgtaacggac taagaaaaac actacacgat gctttaactg 1560
   caaaaattca gctcaccagt tttgaggcaa aatttttgag tgacatgcaa agtaagcatg 1620
   atctcaatgg ttcgttctca tggctcacgc aaaaacaacg aaccacacta gagaacatac 1680
   tggctaaata cggaaggatc tgaggttctt atggctcttg tatctatcag tgaagcatca 1740
   agactaacaa acaaaagtag aacaactgtt caccgttaga tatcaaaggg aaaactgtcc 1800
   atatgcacag atgaaaacgg tgtaaaaaag atagatacat cagagctttt acgagttttt 1860
   ggtgcattta aagctgttca ccatgaacag atcgacaatg taacagatga acagcatgta 1920
   acacctaata gaacaggtga aaccagtaaa acaaagcaac tagaacatga aattgaacac 1980
   ctgagacaac ttgttacagc tcaacagtca cacatagaca gcctgaaaca ggcgatgctg 2040
   cttatcgaat caaagctgcc gacaacacgg gagccagtga cgcctcccgt ggggaaaaaa 2100
   tcatggcaat tctggaagaa atagcgcttt cagccggcaa acctgaagcc ggatctgcga 2160
   ttctgataac aaactagcaa caccagaaca gcccgtttgc gggcagcaaa acccgttatg 2220
   cttgtaaacc gttttgtgaa aaaattttta aaataaaaaa ggggacctct agggtcccca 2280
   attaattagt aatataatct attaaaggtc attcaaaagg tcatccaccg gatcaattcc 2340
   cctgctcgcg caggctgggt gccaagctct cgggtaacat caaggcccga tccttggagc 2400
   ccttgccctc ccgcacgatg atcgtgccgt gatcgaaatc cagatccttg acccgcagtt 2460
   gcaaaccctc actgatccgc atgcccgttc catacagaag ctgggcgaac aaacgatgct 2520
   cgccttccag aaaaccgagg atgcgaacca cttcatccgg ggtcagcacc accggcaagc 2580
   gccgcgacgg ccgaggtctt ccgatctcct gaagccaggg cagatccgtg cacagcacct 2640
   tgccgtagaa gaacagcaag gccgccaatg cctgacgatg cgtggagacc gaaaccttgc 2700
   gctcgttcgc cagccaggac agaaatgcct cgacttcgct gctgcccaag gttgccgggt 2760
   gacgcacacc gtggaaacgg atgaaggcac gaacccagtg gacataagcc tgttcggttc 2820
   gtaagctgta atgcaagtag cgtatgcgct cacgcaactg gtccagaacc ttgaccgaac 2880
   gcagcggtgg taacggcgca gtggcggttt tcatggcttg ttatgactgt ttttttgggg 2940
   tacagtctat gcctcgggca tccaagcagc aagcgcgtta cgccgtgggt cgatgtttga 3000
   tgttatggag cagcaacgat gttacgcagc agggcagtcg ccctaaaaca aagttaaaca 3060
   tcatgaggga agcggtgatc gccgaagtat cgactcaact atcagaggta gttggcgtca 3120
   tcgagcgcca tctcgaaccg acgttgctgg ccgtacattt gtacggctcc gcagtggatg 3180
   gcggcctgaa gccacacagt gatattgatt tgctggttac ggtgaccgta aggcttgatg 3240
   aaacaacgcg gcgagctttg atcaacgacc ttttggaaac ttcggcttcc cctggagaga 3300
   gcgagattct ccgcgctgta gaagtcacca ttgttgtgca cgacgacatc attccgtggc 3360
   gttatccagc taagcgcgaa ctgcaatttg gagaatggca gcgcaatgac attcttgcag 3420
   gtatcttcga gccagccacg atcgacattg atctggctat cttgctgaca aaagcaagag 3480
   aacatagcgt tgccttggta ggtccagcgg cggaggaact ctttgatccg gttcctgaac 3540
   aggatctatt tgaggcgcta aatgaaacct taacgctatg gaactcgccg cccgactggg 3600
   ctggcgatga gcgaaatgta gtgcttacgt tgtcccgcat ttggtacagc gcagtaaccg 3660
   gcaaaatcgc gccgaaggat gtcgctgccg actgggcaat ggagcgcctg ccggcccagt 3720
   atcagcccgt catacttgaa gctagacagg cttatcttgg acaagaagaa gatcgcttgg 3780
   cctcgcgcgc agatcagttg gaagaatttg tccactacgt gaaaggcgag atcaccaagg 3840
   tagtcggcaa ataatgtcta acaattcgtt caagccgacg ccgcttcgcg gcgcggctta 3900
   actcaagcgt tagatgcact aagcacataa ttgctcacag ccaaactatc aggtcaagtc 3960
   tgcttttatt atttttaagc gtgcataata agccctacac aaattgggag atatatcatg 4020
   aaaggctggc tttttcttgt tatcgcaata gttggcgaag taatcgcaac atccgcatta 4080
   aaatctagcg agggctttac taagctgatc cggtggatga ccttttgaat gacctttaat 4140
   agattatatt actaattaat tggggaccct agaggtcccc ttttttattt taaaaatttt 4200
   ttcacaaaac ggtttacaag catacgttgg ccgattcatt aatgcagctg gcacgacagg 4260
   tttcccgact ggaaagcggg cagtgagcgc aacgcaatta atgtgagtta gctcactcat 4320
   taggcacccc aggctttaca ctttatgctt ccggctcgta tgttgtgtgg aattgtgagc 4380
   ggataacaat ttcacacagg aaacagctat gaccatgatt acgccaagct tgctattgac 4440
   gacagctatg gttcactgtc caccaaccaa aactgtgctc agtaccgcca atatttctcc 4500
   cttgaggggt acaaagaggt gtccctagaa gagatccacg ctgtgtaaaa attttacaaa 4560
   aaggtattga ctttccctac agggtgtgta ataatttaat tacaggcggg ggcaaccccg 4620
   cctgttctgc agaggaggaa tatagccatg gaagtgaaaa tcttcaacac ccaggatgtt 4680
   caggattttc tgcgtgttgc aagcggtctg gaacaagagg gtggtaatcc gcgtgttaaa 4740
   caaattattc atcgtgttct gagcgacctg tataaagcaa ttgaagatct gaatatcacc 4800
   agcgacgaat attgggcagg cgttgcatat ctgaatcagc tgggtgcaaa tcaagaagca 4860
   ggtctgctga gtccgggtct gggttttgat cattatctgg atatgcgtat ggatgcagaa 4920
   gatgcagcac tgggtattga aaatgcaaca ccgcgtacca ttgaaggtcc gctgtatgtt 4980
   gcgggtgcac cggaaagcgt tggttatgca cgcatggatg atggtagcga tccgaatggt 5040
   cataccctga ttctgcatgg caccattttt gatgcagatg gtaaaccgct gccgaatgca 5100
   aaagttgaaa tttggcatgc aaacaccaaa ggcttttata gccattttga tccgaccggt 5160
   gaacagcagg cctttaatat gcgtcgtagc attattaccg atgagaatgg tcagtatcgt 5220
   gttcgtacca ttctgcctgc cggttatggt tgtcctccgg aaggtccgac ccagcaactg 5280
   ctgaaccaac tgggtcgtca tggtaatcgt ccggcacata ttcattattt tgttagcgca 5340
   gatggtcacc gtaaactgac cacccagatt aatgttgccg gtgatccgta tacctatgat 5400
   gattttgcat atgccacccg tgaaggtctg gttgttgatg cagttgaaca taccgatccg 5460
   gaagcaatta aagccaatga tgtggaaggt ccttttgccg aaatggtgtt tgatctgaaa 5520
   ctgacccgtc tggttgatgg tgttgataat caggttgtgg atcgtccgcg tctggcagtt 5580
   taatacacca aaatggttca aaattatcag gcgagtgatc atgatcactg gcctgttttt 5640
   atttcaggga agggtggaga caattacgtg gataatcaga tcatccaaga aaccgtggat 5700
   aaaattctga gcgttctgcc gaatcaggca ggtcagctgg cacgtctggt gcgtctgatg 5760
   caatttgcat gcgatccgac cattaccgtt attggcaaat ataaccatgg taaaagccgt 5820
   ctgctgaatg aactgattgg caccgatatc tttagcgttg cagataaacg tgaaaccatt 5880
   cagctggccg aacataaaca ggatcaggtt cgttggctgg atgcacctgg tctggatgcc 5940
   gatgttgcag cagttgatga tcgtcatgca tttgaagcag tttggaccca ggcagatatt 6000
   cgtctgtttg ttcatagcgt tcgtgaaggt gaactggatg caaccgaaca ccatctgctg 6060
   caacagctga ttgaagatgc cgatcatagc cgtcgtcaga ccattctggt tctgacccag 6120
   attgatcaga ttccggatca gaccatcctg acacagatta aaaccagcat tgcacagcag 6180
   gttccgaaac tggatatttg ggcagttagc gcaacccgtc atcgtcaggg cattgaaaac 6240
   ggtaaaaccc tgctgatcga aaaaagcggt attggtgcac tgcgccatac cctggaacag 6300
   gcactggcac aggtgccgag cgcacgtacc tatgaaaaaa atcgtctgct gtcagatctg 6360
   caccatcagc tgaaacaact gctgctggat cagaaacatg ttctgcaaca actgcaacag 6420
   acacagcaac agcagctgca tgattttgat accggtctga ttaacattct ggacaaaatt 6480
   cgtgttgatc tggaaccgat tgtgaatatt gatggtcagg atcaagcact gaatccggat 6540
   agctttgcaa ccatgtttaa aaacaccgca gcaaaacagc agcgtgccaa agttcagatt 6600
   gcatatagcc gtgcatgcat tgaaatcaac agccatctga ttcgccatgg tgttgttggt 6660
   ctgcctgcgg aacagcagac caccattaaa agcattgata ccgtgattgt tgccgtgttt 6720
   ggtatcagcg ttaaatttcg tgatcagctg cgtgccctgt tttataccga taccgaacgt 6780
   cagcgtctgc aacgtgaatt tcgtttctat tttgaaaaaa gtgccggtcg catgattctg 6840
   gcagcaaaaa ttgaacagac catgcgtcag cagggctgta ttcagaatgc catgatggca 6900
   ctgcaacaaa tggaaagcgc agcataaaaa cacggacgcc gcaaacggcg tccgaatttc 6960
   ttggtcgacc gttaaatcta tcaccgcaag ggataaatat ctaacaccgt gcgtgttgac 7020
   tattttacct ctggcggtga taatggttgc atgtactaat ctagataagg aatatagcca 7080
   tgaccgcacc gattcaggat ctgcgtgatg caattgccct gctgcaacag catgataatc 7140
   agtatctgga aaccgatcat ccggttgatc cgaatgcaga actggcaggc gtttatcgtc 7200
   atattggtgc cggtggcacc gttaaacgtc cgacccgtat tggtccggca atgatgttta 7260
   ataacattaa aggttatccg cacagccgta ttctggttgg tatgcatgca agccgtcagc 7320
   gtgcagcact gctgctgggt tgtgaagcaa gtcagctggc actggaagtt ggtaaagcag 7380
   ttaaaaaacc ggttgcaccg gtggttgttc cggcaagcag cgcaccgtgt caagagcaga 7440
   tttttctggc agatgatccg gattttgatc tgcgtaccct gctgcctgca cataccaata 7500
   ccccgattga tgcaggtccg tttttttgtc tgggtctggc cctggcaagc gatccggtgg 7560
   atgcaagcct gaccgatgtt accattcatc gtctgtgtgt tcagggtcgt gatgaactga 7620
   gcatgttcct ggcagcaggt cgccatattg aagtttttcg tcagaaagca gaagcagcag 7680
   gtaaaccgct gccgattacc attaatatgg gtctggaccc agcaatctat attggcgcat 7740
   gttttgaagc accgaccacc ccgtttggtt ataatgaact gggtgttgcc ggtgcactgc 7800
   gtcagcgtcc ggttgaactg gttcagggtg ttagcgttcc ggaaaaagca attgcacgtg 7860
   ccgaaattgt tattgaaggt gaactgctgc ctggtgttcg tgttcgtgaa gatcagcata 7920
   ccaattcagg tcatgcaatg ccggaatttc cgggttattg tggtggtgca aatccgagcc 7980
   tgccggttat taaagttaaa gccgttacca tgcgcaataa cgcaattctg caaaccctgg 8040
   ttggtccggg tgaagaacat accaccctgg caggtctgcc gaccgaagca agcatttgga 8100
   atgcagttga agcagcaatt ccgggttttc tgcaaaatgt ttatgcccat accgcaggcg 8160
   gtggtaaatt tctgggtatt ctgcaagtga aaaaacgtca gcctgccgat gaaggtcgtc 8220
   agggtcaggc agccctgctg gcgctggcaa cctatagcga actgaaaaat atcattctgg 8280
   tggatgagga tgtggacatt tttgatagtg atgatattct gtgggcaatg accacccgta 8340
   tgcagggtga tgttagcatt accaccattc cgggtattcg cggtcatcag ctggacccga 8400
   gccagacacc ggaatattca ccgagcattc gtggtaatgg tattagctgc aaaaccatct 8460
   ttgattgtac cgttccgtgg gcactgaaaa gccattttga acgtgcaccg tttgcagatg 8520
   ttgatccgcg tccgtttgca cctgaatatt ttgcacgtct ggaaaaaaat cagggcagcg 8580
   caaaataagc taataacagg cctgctggta atcgcaggaa tttttatttg gatggatccg 8640
   cctacctagc ttccaagaaa gatatcctaa cagcacaaga gcggaaagat gttttgttct 8700
   acatccagaa caacctctgc taaaattcct gaaaaatttt gcaaaaagtt gttgacttta 8760
   tctacaaggt gtggtataat aatcttaaca acagcaggac gctcccgggt tgaggaaaac 8820
   ctaatgccga gcaaactggc aattagcagc atgagcctgg gtcgttgttt tgcaggtcat 8880
   agcctggata gtaaactgga tgcagcacag cgttatggtt atctgggtat tgaactgttt 8940
   tatgaggatc tggttgatgt tgcagaacat ctgagcaatg aacgtccgag tccggaaggt 9000
   ccgtttgttg aagcacagat tgcagcagca cgtcatattc tgcaaatgtg tcaggcacgt 9060
   ggtctggaag ttgtttgtct gcaaccgttt atgcattatg atggtctgaa tgatcgtgcc 9120
   gaacatgaac gtcgtctgga aaaactggca ctgtggattg aactggcaca tgaactgcat 9180
   accgatatta ttcagattcc ggcaaatttt ctgcctgcaa atcaggttag cgataatctg 9240
   gatctgattg ttagcgatct gtgtaaagtt gcagatattg gtgcacaggc actgcctccg 9300
   attcgttttg catatgaaag cctgtgttgg agcacccgtg ttgatctgtg ggaacgttgt 9360
   tgggatattg ttcagcgtgt ggatcgtccg aattttggta tttgtctgga tacctttaac 9420
   atcctgggtc gcatttatgc agatccgacc agcccgagcg gtcgtacccc gaatgcaaaa 9480
   gaagcagttc gtaaaagcat tgccaatctg gttagccgtg tggatgttag caaagttttt 9540
   tatgttcagg ttgtggatgc cgaacgtctg agtaaaccgc tgctgcctgg tcatccgtat 9600
   tataacccgg aacagcctgc acgtatgagc tggtcacgta attgtcgtct gttctatggt 9660
   gaaaccgaat atggtgcata tctgccggtt aaagaagttg cacgcgcact gtttcatggt 9720
   attggttttg aaggttgggt tagcctggaa ctgtttaatc gtcgtatgag cgaagaaggt 9780
   ccggaagttc ctgaagaact ggccatgcgt ggtgcaatta gctgggcaaa actggttcag 9840
   gatctgcgta ttccggttga aggtccgctg gttaccatgc ctcgtgttag cgcaagcctg 9900
   taaatgcatg cgcgccgcgt tcgcgcggcg cttttttttg gtaccgagct cgaattcact 9960
   ggccgtcgtt ttacaacgtc gtgactggga aaaccctggc gttacccaac ttaatcgcct 10020
   tgcagcacat ccccctttcg ccagctggcg taatagcgaa gaggcccgca ccgatcgccc 10080
   ttcccaacag ttgcgcagcc tgaatggcga atggcgcctg atgcggtatt ttctccttac 10140
   gcatctgtgc ggtatttcac accgcatatg gtgcactctc agtacaatct gctctgatgc 10200
   cgcatagtta agccagcccc gacacccgcc aacacccgct gacgaattc 10249
<210> 28
   <211> 7623
   <212> DNA
   <213> Artificial Sequence
<220>
   <223> Plasmid pCP32AMP;
<400> 28
   acatgaatgg tcttcggttt ccgtgtttcg taaagtctgg aaacgcggaa gtcagcgccc 60
   tgcaccatta tgttccggat ctgcatcgca ggatgctgct ggctaccctg tggaacacct 120
   acatctgtat taacgaagcg ctggcattga ccctgagtga tttttctctg gtcccgccgc 180
   atccataccg ccagttgttt accctcacaa cgttccagta accgggcatg ttcatcatca 240
   gtaacccgta tcgtgagcat cctctctcgt ttcatcggta tcattacccc catgaacaga 300
   aattccccct tacacggagg catcaagtga ccaaacagga aaaaaccgcc cttaacatgg 360
   cccgctttat cagaagccag acattaacgc ttctggagaa actcaacgag ctggacgcgg 420
   atgaacaggc agacatctgt gaatcgcttc acgaccacgc tgatgagctt taccgcagct 480
   gcctcgcgcg tttcggtgat gacggtgaaa acctctgaca catgcagctc ccggagacgg 540
   tcacagcttg tctgtaagcg gatgccggga gcagacaagc ccgtcagggc gcgtcagcgg 600
   gtgttggcgg gtgtcggggc gcagccatga cccagtcacg tagcgatagc ggagtgtata 660
   ctggcttaac tatgcggcat cagagcagat tgtactgaga gtgcaccata tgcggtgtga 720
   aataccgcac agatgcgtaa ggagaaaata ccgcatcagg cgctcttccg cttcctcgct 780
   cactgactcg ctgcgctcgg tcgttcggct gcggcgagcg gtatcagctc actcaaaggc 840
   ggtaatacgg ttatccacag aatcagggga taacgcagga aagaacatgt gagcaaaagg 900
   ccagcaaaag gccaggaacc gtaaaaaggc cgcgttgctg gcgtttttcc ataggctccg 960
   cccccctgac gagcatcaca aaaatcgacg ctcaagtcag aggtggcgaa acccgacagg 1020
   actataaaga taccaggcgt ttccccctgg aagctccctc gtgcgctctc ctgttccgac 1080
   cctgccgctt accggatacc tgtccgcctt tctcccttcg ggaagcgtgg cgctttctca 1140
   tagctcacgc tgtaggtatc tcagttcggt gtaggtcgtt cgctccaagc tgggctgtgt 1200
   gcacgaaccc cccgttcagc ccgaccgctg cgccttatcc ggtaactatc gtcttgagtc 1260
   caacccggta agacacgact tatcgccact ggcagcagcc actggtaaca ggattagcag 1320
   agcgaggtat gtaggcggtg ctacagagtt cttgaagtgg tggcctaact acggctacac 1380
   tagaaggaca gtatttggta tctgcgctct gctgaagcca gttaccttcg gaaaaagagt 1440
   tggtagctct tgatccggca aacaaaccac cgctggtagc ggtggttttt ttgtttgcaa 1500
   gcagcagatt acgcgcagaa aaaaaggatc tcaagaagat cctttgatct tttctacggg 1560
   gtctgacgct cagtggaacg aaaactcacg ttaagggatt ttggtcatga gattatcaaa 1620
   aaggatcttc acctagatcc ttttaaatta aaaatgaagt tttaaatcaa tctaaagtat 1680
   atatgagtaa acttggtctg acagttacca atgcttaatc agtgaggcac ctatctcagc 1740
   gatctgtcta tttcgttcat ccatagttgc ctgactcccc gtcgtgtaga taactacgat 1800
   acgggagggc ttaccatctg gccccagtgc tgcaatgata ccgcgagacc cacgctcacc 1860
   ggctccagat ttatcagcaa taaaccagcc agccggaagg gccgagcgca gaagtggtcc 1920
   tgcaacttta tccgcctcca tccagtctat taattgttgc cgggaagcta gagtaagtag 1980
   ttcgccagtt aatagtttgc gcaacgttgt tgccattgct gcaggcatcg tggtgtcacg 2040
   ctcgtcgttt ggtatggctt cattcagctc cggttcccaa cgatcaaggc gagttacatg 2100
   atcccccatg ttgtgcaaaa aagcggttag ctccttcggt cctccgatcg ttgtcagaag 2160
   taagttggcc gcagtgttat cactcatggt tatggcagca ctgcataatt ctcttactgt 2220
   catgccatcc gtaagatgct tttctgtgac tggtgagtac tcaaccaagt cattctgaga 2280
   atagtgtatg cggcgaccga gttgctcttg cccggcgtca acacgggata ataccgcgcc 2340
   acatagcaga actttaaaag tgctcatcat tggaaaacgt tcttcggggc gaaaactctc 2400
   aaggatctta ccgctgttga gatccagttc gatgtaaccc actcgtgcac ccaactgatc 2460
   ttcagcatct tttactttca ccagcgtttc tgggtgagca aaaacaggaa ggcaaaatgc 2520
   cgcaaaaaag ggaataaggg cgacacggaa atgttgaata ctcatactct tcctttttca 2580
   atattattga agcatttatc agggttattg tctcatgagc ggatacatat ttgaatgtat 2640
   ttagaaaaat aaacaaatag gggttccgcg cacatttccc cgaaaagtgc cacctgacgt 2700
   ctaagaaacc attattatca tgacattaac ctataaaaat aggcgtatca cgaggccctt 2760
   tcgtcttcaa gaattctgaa ccagtcctaa aacgagtaaa taggaccggc aattcttcaa 2820
   gcaataaaca ggaataccaa ttattaaaag ataacttagt cagatcgtac aataaagctt 2880
   tgaagaaaaa tgcgccttat tcaatctttg ctataaaaaa tggcccaaaa tctcacattg 2940
   gaagacattt gatgacctca tttctttcaa tgaagggcct aacggagttg actaatgttg 3000
   tgggaaattg gagcgataag cgtgcttctg ccgtggccag gacaacgtat actcatcaga 3060
   taacagcaat acctgatcac tacttcgcac tagtttctcg gtactatgca tatgatccaa 3120
   tatcaaagga aatgatagca ttgaaggatg agactaatcc aattgaggag tggcagcata 3180
   tagaacagct aaagggtagt gctgaaggaa gcatacgata ccccgcatgg aatgggataa 3240
   tatcacagga ggtactagac tacctttcat cctacataaa tagacgcata taagtacgca 3300
   tttaagcata aacacgcact atgccgttct tctcatgtat atatatatac aggcaacacg 3360
   cagatatagg tgcgacgtga acagtgagct gtatgtgcgc agctcgcgtt gcattttcgg 3420
   aagcgctcgt tttcggaaac gctttgaagt tcctattccg aagttcctat tctctagaaa 3480
   gtataggaac ttcagagcgc ttttgaaaac caaaagcgct ctgaagacgc actttcaaaa 3540
   aaccaaaaac gcaccggact gtaacgagct actaaaatat tgcgaatacc gcttccacaa 3600
   acattgctca aaagtatctc tttgctatat atctctgtgc tatatcccta tataacctac 3660
   ccatccacct ttcgctcctt gaacttgcat ctaaactcga cctctacatt ttttatgttt 3720
   atctctagta ttactcttta gacaaaaaaa ttgtagtaag aactattcat agagtgaatc 3780
   gaaaacaata cgaaaatgta aacatttcct atacgtagta tatagagaca aaatagaaga 3840
   aaccgttcat aattttctga ccaatgaaga atcatcaacg ctatcacttt ctgttcacaa 3900
   agtatgcgca atccacatcg gtatagaata taatcgggga tgcctttatc ttgaaaaaat 3960
   gcacccgcag cttcgctagt aatcagtaaa cgcgggaagt ggagtcaggc tttttttatg 4020
   gaagagaaaa tagacaccaa agtagccttc ttctaacctt aacggaccta cagtgcaaaa 4080
   agttatcaag agactgcatt atagagcgca caaaggagaa aaaaagtaat ctaagatgct 4140
   ttgttagaaa aatagcgctc tcgggatgca tttttgtaga acaaaaaaga agtatagatt 4200
   ctttgttggt aaaatagcgc tctcgcgttg catttctgtt ctgtaaaaat gcagctcaga 4260
   ttctttgttt gaaaaattag cgctctcgcg ttgcattttt gttttacaaa aatgaagcac 4320
   agattcttcg ttggtaaaat agcgctttcg cgttgcattt ctgttctgta aaaatgcagc 4380
   tcagattctt tgtttgaaaa attagcgctc tcgcgttgca tttttgttct acaaaatgaa 4440
   gcacagatgc ttcgttaaca aagatatgct attgaagtgc aagatggaaa cgcagaaaat 4500
   gaaccgggga tgcgacgtgc aagattacct atgcaataga tgcaatagtt tctccaggaa 4560
   ccgaaataca tacattgtct tccgtaaagc gctagactat atattattat acaggttcaa 4620
   atatactatc tgtttcaggg aaaactccca ggttcggatg ttcaaaattc aatgatgggt 4680
   aacaagtacg atcgtaaatc tgtaaaacag tttgtcggat attaggctgt atctcctcaa 4740
   agcgtattcg aatatcattg agaagctgca gcgtcacatc ggataataat gatggcagcc 4800
   attgtagaag tgccttttgc atttctagtc tctttctcgg tctagctagt tttactacat 4860
   cgcgaagata gaatcttaga tcacactgcc tttgctgagc tggatcaata gagtaacaaa 4920
   agagtggtaa ggcctcgtta aaggacaagg acctgagcgg aagtgtatcg tacagtagac 4980
   ggagtatact agtatagtct atagtccgtg gaattctcat gtttgacagc ttatcatcga 5040
   taagcttttc aattcaattc atcatttttt ttttattctt ttttttgatt tcggtttctt 5100
   tgaaattttt ttgattcggt aatctccgaa cagaaggaag aacgaaggaa ggagcacaga 5160
   cttagattgg tatatatacg catatgtagt gttgaagaaa catgaaattg cccagtattc 5220
   ttaacccaac tgcacagaac aaaaacctgc aggaaacgaa gataaatcat gtcgaaagct 5280
   acatataagg aacgtgctgc tactcatcct agtcctgttg ctgccaagct atttaatatc 5340
   atgcacgaaa agcaaacaaa cttgtgtgct tcattggatg ttcgtaccac caaggaatta 5400
   ctggagttag ttgaagcatt aggtcccaaa atttgtttac taaaaacaca tgtggatatc 5460
   ttgactgatt tttccatgga gggcacagtt aagccgctaa aggcattatc cgccaagtac 5520
   aattttttac tcttcgaaga cagaaaattt gctgacattg gtaatacagt caaattgcag 5580
   tactctgcgg gtgtatacag aatagcagaa tgggcagaca ttacgaatgc acacggtgtg 5640
   gtgggcccag gtattgttag cggtttgaag caggcggcag aagaagtaac aaaggaacct 5700
   agaggccttt tgatgttagc agaattgtca tgcaagggct ccctatctac tggagaatat 5760
   actaagggta ctgttgacat tgcgaagagc gacaaagatt ttgttatcgg ctttattgct 5820
   caaagagaca tgggtggaag agatgaaggt tacgattggt tgattatgac acccggtgtg 5880
   ggtttagatg acaagggaga cgcattgggt caacagtata gaaccgtgga tgatgtggtc 5940
   tctacaggat ctgacattat tattgttgga agaggactat ttgcaaaggg aagggatgct 6000
   aaggtagagg gtgaacgtta cagaaaagca ggctgggaag catatttgag aagatgcggc 6060
   cagcaaaact aaaaaactgt attataagta aatgcatgta tactaaactc acaaattaga 6120
   gcttcaattt aattatatca gttattaccc gggaatctcg gtcgtaatga cttgaaataa 6180
   ttaacaaaca aaggagttac agttagaaat tgtaggagag atctcgtttt tcgcgacaat 6240
   ctggcgtttt tcttgctaat tccaggatta atccgttcat agtgtaaaac cccgtttaca 6300
   cattctgacg gaagatatag attggaagta ttgcattcac taagataagt atggcaacac 6360
   tggaacagac atgaattatc agaacgacga tttacgcatc aaagaaatca aagagttact 6420
   tcctcctgtc gcattgctgg aaaaattccc cgctactgaa aatgccgcga atacggttgc 6480
   ccatgcccga aaagcgatcc ataagatcct gaaaggtaat gatgatcgcc tgttggttgt 6540
   gattggccca tgctcaattc atgatcctgt cgcggcaaaa gagtatgcca ctcgcttgct 6600
   ggcgctgcgt gaagagctga aagatgagct ggaaatcgta atgcgcgtct attttgaaaa 6660
   gccgcgtacc acggtgggct ggaaagggct gattaacgat ccgcatatgg ataatagctt 6720
   ccagatcaac gacggtctgc gtatagcccg taaattgctg cttgatatta acgacagcgg 6780
   tctgccagcg gcaggtgagt ttctcgatat gatcacccca caatatctcg ctgacctgat 6840
   gagctggggc gcaattggcg cacgtaccac cgaatcgcag gtgcaccgcg aactggcatc 6900
   agggctttct tgtccggtcg gcttcaaaaa tggcaccgac ggtacgatta aagtggctat 6960
   cgatgccatt aatgccgccg gtgcgccgca ctgcttcctg tccgtaacga aatgggggca 7020
   ttcggcgatt gtgaatacca gcggtaacgg cgattgccat atcattctgc gcggcggtaa 7080
   agagcctaac tacagcgcga agcacgttgc tgaagtgaaa gaagggctga acaaagcagg 7140
   cctgccagca caggtgatga tcgatttcag ccatgctaac tcgtccaaac aattcaaaaa 7200
   gcagatggat gtttgtgctg acgtttgcca gcagattgcc ggtggcgaaa aggccattat 7260
   tggcgtgatg gtggaaagcc atctggtgga aggcaatcag agcctcgaga gcggggagcc 7320
   gctggcctac ggtaagagca tcaccgatgc ctgcatcggc tgggaagata ccgatgctct 7380
   gttacgtcaa ctggcgaatg cagtaaaagc gcgtcgcggg taaggtttaa ttgtcggatg 7440
   cgccgtcaga gtggcgtatc cgatgaatca ccacaggcct gataagtcgc gcagcgtcgc 7500
   atcaggcaat gtgctccatt gttagcaaca aaaaagccga ctcacttgca gtcggctttc 7560
   tcattttaaa cgaatgacgt ttacttcgct ttaccctggt ttgcaaccgc cgctgctttc 7620
   gct 7623
<210> 29
   <211> 7630
   <212> DNA
   <213> Artificial Sequence
<220>
   <223> Plasmid pCP14;
<400> 29
   ctcgaggcta ttgacgacag ctatggttca ctgtccacca accaaaactg tgctcagtac 60
   cgccaatatt tctcccttga ggggtacaaa gaggtgtccc tagaagagat ccacgctgtg 120
   taaaaatttt acaaaaaggt attgactttc cctacagggt gtgtaataat ttaattacag 180
   gcgggggcaa ccccgcctgt tctagaggag gaggaatcgc catggagagg attgtcgtta 240
   ctctcgggga acgtagttac ccaattacca tcgcatctgg tttgtttaat gaaccagctt 300
   cattcttacc gctgaaatcg ggcgagcagg tcatgttggt caccaacgaa accctggctc 360
   ctctgtatct cgataaggtc cgcggcgtac ttgaacaggc gggtgttaac gtcgatagcg 420
   ttatcctccc tgacggcgag cagtataaaa gcctggctgt actcgatacc gtctttacgg 480
   cgttgttaca aaagccgcat ggtcgcgata ctacgctggt ggcgcttggc ggcggcgtag 540
   tgggcgatct gaccggcttc gcggcggcga gttatcagcg cggtgttcgt ttcattcaag 600
   tcccgacgac gttactgtcg caggtcgatt cctccgttgg cggcaaaact gcggtcaacc 660
   atcccctcgg taaaaacatg attggcgcgt tctaccagcc tgcttcagtg gtggtggatc 720
   tcgactgtct gaaaacgctt cccccgcgtg agttagcgtc ggggctggca gaagtcatca 780
   aatacggcat tattcttgac ggtgcgtttt tcaactggct ggaagagaat ctggatgcgt 840
   tgttgcgtct ggacggtccg gcaatggcgt actgtattcg ccgttgttgt gaactgaagg 900
   cagaagttgt cgccgccgac gagcgcgaaa ccgggttacg tgctttactg aatctgggac 960
   acacctttgg tcatgccatt gaagctgaaa tggggtatgg caattggtta catggtgaag 1020
   cggtcgctgc gggtatggtg atggcggcgc ggacgtcgga acgtctcggg cagtttagtt 1080
   ctgccgaaac gcagcgtatt ataaccctgc tcacgcgggc tgggttaccg gtcaatgggc 1140
   cgcgcgaaat gtccgcgcag gcgtatttac cgcatatgct gcgtgacaag aaagtccttg 1200
   cgggagagat gcgcttaatt cttccgttgg caattggtaa gagtgaagtt cgcagcggcg 1260
   tttcgcacga gcttgttctt aacgccattg ccgattgtca atcagcgtaa tcatcgttca 1320
   tgcctgatgc cgctatgtag gccggataag gcgttcacgc cgcatccggc aaccgatgcc 1380
   tgatgcgacg cggtcgcgtc ttatcaggcc tacaggtcga tgccgatatg tacatcgtat 1440
   tcggcaatta atacatagca acatgaatgg tcttcggttt ccgtgtttcg taaagtctgg 1500
   aaacgcggaa gtcagcgccc tgcaccatta tgttccggat ctgcatcgca ggatgctgct 1560
   ggctaccctg tggaacacct acatctgtat taacgaagcg ctggcattga ccctgagtga 1620
   tttttctctg gtcccgccgc atccataccg ccagttgttt accctcacaa cgttccagta 1680
   accgggcatg ttcatcatca gtaacccgta tcgtgagcat cctctctcgt ttcatcggta 1740
   tcattacccc catgaacaga aattccccct tacacggagg catcaagtga ccaaacagga 1800
   aaaaaccgcc cttaacatgg cccgctttat cagaagccag acattaacgc ttctggagaa 1860
   actcaacgag ctggacgcgg atgaacaggc agacatctgt gaatcgcttc acgaccacgc 1920
   tgatgagctt taccgcagct gcctcgcgcg tttcggtgat gacggtgaaa acctctgaca 1980
   catgcagctc ccggagacgg tcacagcttg tctgtaagcg gatgccggga gcagacaagc 2040
   ccgtcagggc gcgtcagcgg gtgttggcgg gtgtcggggc gcagccatga cccagtcacg 2100
   tagcgatagc ggagtgtata ctggcttaac tatgcggcat cagagcagat tgtactgaga 2160
   gtgcaccata tgcggtgtga aataccgcac agatgcgtaa ggagaaaata ccgcatcagg 2220
   cgctcttccg cttcctcgct cactgactcg ctgcgctcgg tcgttcggct gcggcgagcg 2280
   gtatcagctc actcaaaggc ggtaatacgg ttatccacag aatcagggga taacgcagga 2340
   aagaacatgt gagcaaaagg ccagcaaaag gccaggaacc gtaaaaaggc cgcgttgctg 2400
   gcgtttttcc ataggctccg cccccctgac gagcatcaca aaaatcgacg ctcaagtcag 2460
   aggtggcgaa acccgacagg actataaaga taccaggcgt ttccccctgg aagctccctc 2520
   gtgcgctctc ctgttccgac cctgccgctt accggatacc tgtccgcctt tctcccttcg 2580
   ggaagcgtgg cgctttctca tagctcacgc tgtaggtatc tcagttcggt gtaggtcgtt 2640
   cgctccaagc tgggctgtgt gcacgaaccc cccgttcagc ccgaccgctg cgccttatcc 2700
   ggtaactatc gtcttgagtc caacccggta agacacgact tatcgccact ggcagcagcc 2760
   actggtaaca ggattagcag agcgaggtat gtaggcggtg ctacagagtt cttgaagtgg 2820
   tggcctaact acggctacac tagaaggaca gtatttggta tctgcgctct gctgaagcca 2880
   gttaccttcg gaaaaagagt tggtagctct tgatccggca aacaaaccac cgctggtagc 2940
   ggtggttttt ttgtttgcaa gcagcagatt acgcgcagaa aaaaaggatc tcaagaagat 3000
   cctttgatct tttctacggg gtctgacgct cagtggaacg aaaactcacg ttaagggatt 3060
   ttggtcatga gattatcaaa aaggatcttc acctagatcc ttttaaatta aaaatgaagt 3120
   tttaaatcaa tctaaagtat atatgagtaa acttggtctg acagttacca atgcttaatc 3180
   agtgaggcac ctatctcagc gatctgtcta tttcgttcat ccatagttgc ctgactcccc 3240
   gtcgtgtaga taactacgat acgggagggc ttaccatctg gccccagtgc tgcaatgata 3300
   ccgcgagacc cacgctcacc ggctccagat ttatcagcaa taaaccagcc agccggaagg 3360
   gccgagcgca gaagtggtcc tgcaacttta tccgcctcca tccagtctat taattgttgc 3420
   cgggaagcta gagtaagtag ttcgccagtt aatagtttgc gcaacgttgt tgccattgct 3480
   gcaggcatcg tggtgtcacg ctcgtcgttt ggtatggctt cattcagctc cggttcccaa 3540
   cgatcaaggc gagttacatg atcccccatg ttgtgcaaaa aagcggttag ctccttcggt 3600
   cctccgatcg ttgtcagaag taagttggcc gcagtgttat cactcatggt tatggcagca 3660
   ctgcataatt ctcttactgt catgccatcc gtaagatgct tttctgtgac tggtgagtac 3720
   tcaaccaagt cattctgaga atagtgtatg cggcgaccga gttgctcttg cccggcgtca 3780
   acacgggata ataccgcgcc acatagcaga actttaaaag tgctcatcat tggaaaacgt 3840
   tcttcggggc gaaaactctc aaggatctta ccgctgttga gatccagttc gatgtaaccc 3900
   actcgtgcac ccaactgatc ttcagcatct tttactttca ccagcgtttc tgggtgagca 3960
   aaaacaggaa ggcaaaatgc cgcaaaaaag ggaataaggg cgacacggaa atgttgaata 4020
   ctcatactct tcctttttca atattattga agcatttatc agggttattg tctcatgagc 4080
   ggatacatat ttgaatgtat ttagaaaaat aaacaaatag gggttccgcg cacatttccc 4140
   cgaaaagtgc cacctgacgt ctaagaaacc attattatca tgacattaac ctataaaaat 4200
   aggcgtatca cgaggccctt tcgtcttcaa gaattctgaa ccagtcctaa aacgagtaaa 4260
   taggaccggc aattcttcaa gcaataaaca ggaataccaa ttattaaaag ataacttagt 4320
   cagatcgtac aataaagctt tgaagaaaaa tgcgccttat tcaatctttg ctataaaaaa 4380
   tggcccaaaa tctcacattg gaagacattt gatgacctca tttctttcaa tgaagggcct 4440
   aacggagttg actaatgttg tgggaaattg gagcgataag cgtgcttctg ccgtggccag 4500
   gacaacgtat actcatcaga taacagcaat acctgatcac tacttcgcac tagtttctcg 4560
   gtactatgca tatgatccaa tatcaaagga aatgatagca ttgaaggatg agactaatcc 4620
   aattgaggag tggcagcata tagaacagct aaagggtagt gctgaaggaa gcatacgata 4680
   ccccgcatgg aatgggataa tatcacagga ggtactagac tacctttcat cctacataaa 4740
   tagacgcata taagtacgca tttaagcata aacacgcact atgccgttct tctcatgtat 4800
   atatatatac aggcaacacg cagatatagg tgcgacgtga acagtgagct gtatgtgcgc 4860
   agctcgcgtt gcattttcgg aagcgctcgt tttcggaaac gctttgaagt tcctattccg 4920
   aagttcctat tctctagaaa gtataggaac ttcagagcgc ttttgaaaac caaaagcgct 4980
   ctgaagacgc actttcaaaa aaccaaaaac gcaccggact gtaacgagct actaaaatat 5040
   tgcgaatacc gcttccacaa acattgctca aaagtatctc tttgctatat atctctgtgc 5100
   tatatcccta tataacctac ccatccacct ttcgctcctt gaacttgcat ctaaactcga 5160
   cctctacatt ttttatgttt atctctagta ttactcttta gacaaaaaaa ttgtagtaag 5220
   aactattcat agagtgaatc gaaaacaata cgaaaatgta aacatttcct atacgtagta 5280
   tatagagaca aaatagaaga aaccgttcat aattttctga ccaatgaaga atcatcaacg 5340
   ctatcacttt ctgttcacaa agtatgcgca atccacatcg gtatagaata taatcgggga 5400
   tgcctttatc ttgaaaaaat gcacccgcag cttcgctagt aatcagtaaa cgcgggaagt 5460
   ggagtcaggc tttttttatg gaagagaaaa tagacaccaa agtagccttc ttctaacctt 5520
   aacggaccta cagtgcaaaa agttatcaag agactgcatt atagagcgca caaaggagaa 5580
   aaaaagtaat ctaagatgct ttgttagaaa aatagcgctc tcgggatgca tttttgtaga 5640
   acaaaaaaga agtatagatt ctttgttggt aaaatagcgc tctcgcgttg catttctgtt 5700
   ctgtaaaaat gcagctcaga ttctttgttt gaaaaattag cgctctcgcg ttgcattttt 5760
   gttttacaaa aatgaagcac agattcttcg ttggtaaaat agcgctttcg cgttgcattt 5820
   ctgttctgta aaaatgcagc tcagattctt tgtttgaaaa attagcgctc tcgcgttgca 5880
   tttttgttct acaaaatgaa gcacagatgc ttcgttaaca aagatatgct attgaagtgc 5940
   aagatggaaa cgcagaaaat gaaccgggga tgcgacgtgc aagattacct atgcaataga 6000
   tgcaatagtt tctccaggaa ccgaaataca tacattgtct tccgtaaagc gctagactat 6060
   atattattat acaggttcaa atatactatc tgtttcaggg aaaactccca ggttcggatg 6120
   ttcaaaattc aatgatgggt aacaagtacg atcgtaaatc tgtaaaacag tttgtcggat 6180
   attaggctgt atctcctcaa agcgtattcg aatatcattg agaagctgca gcgtcacatc 6240
   ggataataat gatggcagcc attgtagaag tgccttttgc atttctagtc tctttctcgg 6300
   tctagctagt tttactacat cgcgaagata gaatcttaga tcacactgcc tttgctgagc 6360
   tggatcaata gagtaacaaa agagtggtaa ggcctcgtta aaggacaagg acctgagcgg 6420
   aagtgtatcg tacagtagac ggagtatact agtatagtct atagtccgtg gaattctcat 6480
   gtttgacagc ttatcatcga taagcttttc aattcaattc atcatttttt ttttattctt 6540
   ttttttgatt tcggtttctt tgaaattttt ttgattcggt aatctccgaa cagaaggaag 6600
   aacgaaggaa ggagcacaga cttagattgg tatatatacg catatgtagt gttgaagaaa 6660
   catgaaattg cccagtattc ttaacccaac tgcacagaac aaaaacctgc aggaaacgaa 6720
   gataaatcat gtcgaaagct acatataagg aacgtgctgc tactcatcct agtcctgttg 6780
   ctgccaagct atttaatatc atgcacgaaa agcaaacaaa cttgtgtgct tcattggatg 6840
   ttcgtaccac caaggaatta ctggagttag ttgaagcatt aggtcccaaa atttgtttac 6900
   taaaaacaca tgtggatatc ttgactgatt tttccatgga gggcacagtt aagccgctaa 6960
   aggcattatc cgccaagtac aattttttac tcttcgaaga cagaaaattt gctgacattg 7020
   gtaatacagt caaattgcag tactctgcgg gtgtatacag aatagcagaa tgggcagaca 7080
   ttacgaatgc acacggtgtg gtgggcccag gtattgttag cggtttgaag caggcggcag 7140
   aagaagtaac aaaggaacct agaggccttt tgatgttagc agaattgtca tgcaagggct 7200
   ccctatctac tggagaatat actaagggta ctgttgacat tgcgaagagc gacaaagatt 7260
   ttgttatcgg ctttattgct caaagagaca tgggtggaag agatgaaggt tacgattggt      7320
   tgattatgac acccggtgtg ggtttagatg acaagggaga cgcattgggt caacagtata      7380
   gaaccgtgga tgatgtggtc tctacaggat ctgacattat tattgttgga agaggactat      7440
   ttgcaaaggg aagggatgct aaggtagagg gtgaacgtta cagaaaagca ggctgggaag      7500
   catatttgag aagatgcggc cagcaaaact aaaaaactgt attataagta aatgcatgta      7560
   tactaaactc acaaattaga gcttcaattt aattatatca gttattaccc gggaatctcg      7620
   gtcgtaatga                                                             7630
<210> 30
   <211> 10015
   <212> DNA
   <213> Artificial Sequence
<220>
   <223> Plasmid pCP50;
<400> 30
   cttgaaataa ttaacaaaca aaggagttac agttagaaat tgtaggagag atctcgtttt      60
   tcgcgacaat ctggcgtttt tcttgctaat tccaggatta atccgttcat agtgtaaaac     120
   cccgtttaca cattctgacg gaagatatag attggaagta ttgcattcac taagataagt     180
   atggcaacac tggaacagac atgaattatc agaacgacga tttacgcatc aaagaaatca     240
   aagagttact tcctcctgtc gcattgctgg aaaaattccc cgctactgaa aatgccgcga     300
   atacggttgc ccatgcccga aaagcgatcc ataagatcct gaaaggtaat gatgatcgcc     360
   tgttggttgt gattggccca tgctcaattc atgatcctgt cgcggcaaaa gagtatgcca     420
   ctcgcttgct ggcgctgcgt gaagagctga aagatgagct ggaaatcgta atgcgcgtct     480
   attttgaaaa gccgcgtacc acggtgggct ggaaagggct gattaacgat ccgcatatgg     540
   ataatagctt ccagatcaac gacggtctgc gtatagcccg taaattgctg cttgatatta     600
   acgacagcgg tctgccagcg gcaggtgagt ttctcgatat gatcacccca caatatctcg     660
   ctgacctgat gagctggggc gcaattggcg cacgtaccac cgaatcgcag gtgcaccgcg     720
   aactggcatc agggctttct tgtccggtcg gcttcaaaaa tggcaccgac ggtacgatta     780
   aagtggctat cgatgccatt aatgccgccg gtgcgccgca ctgcttcctg tccgtaacga     840
   aatgggggca ttcggcgatt gtgaatacca gcggtaacgg cgattgccat atcattctgc     900
   gcggcggtaa agagcctaac tacagcgcga agcacgttgc tgaagtgaaa gaagggctga     960
   acaaagcagg cctgccagca caggtgatga tcgatttcag ccatgctaac tcgtccaaac    1020
   aattcaaaaa gcagatggat gtttgtgctg acgtttgcca gcagattgcc ggtggcgaaa    1080
   aggccattat tggcgtgatg gtggaaagcc atctggtgga aggcaatcag agcctcgaga    1140
   gcggggagcc gctggcctac ggtaagagca tcaccgatgc ctgcatcggc tgggaagata     1200
   ccgatgctct gttacgtcaa ctggcgaatg cagtaaaagc gcgtcgcggg taaggtttaa     1260
   ttgtcggatg cgccgtcaga gtggcgtatc cgatgaatca ccacaggcct gataagtcgc     1320
   gcagcgtcgc atcaggcaat gtgctccatt gttagcaaca aaaaagccga ctcacttgca     1380
   gtcggctttc tcattttaaa cgaatgacgt ttacttcgct ttaccctggt ttgcaaccgc     1440
   cgctgctttc gctacatgaa tggtcttcgg tttccgtgtt tcgtaaagtc tggaaacgcg     1500
   gaagtcagcg ccctgcacca ttatgttccg gatctgcatc gcaggatgct gctggctacc     1560
   ctgtggaaca cctacatctg tattaacgaa gcgctggcat tgaccctgag tgatttttct     1620
   ctggtcccgc cgcatccata ccgccagttg tttaccctca caacgttcca gtaaccgggc     1680
   atgttcatca tcagtaaccc gtatcgtgag catcctctct cgtttcatcg gtatcattac     1740
   ccccatgaac agaaattccc ccttacacgg aggcatcaag tgaccaaaca ggaaaaaacc     1800
   gcccttaaca tggcccgctt tatcagaagc cagacattaa cgcttctgga gaaactcaac     1860
   gagctggacg cggatgaaca ggcagacatc tgtgaatcgc ttcacgacca cgctgatgag     1920
   ctttaccgca gctgcctcgc gcgtttcggt gatgacggtg aaaacctctg acacatgcag     1980
   ctcccggaga cggtcacagc ttgtctgtaa gcggatgccg ggagcagaca agcccgtcag     2040
   ggcgcgtcag cgggtgttgg cgggtgtcgg ggcgcagcca tgacccagtc acgtagcgat     2100
   agcggagtgt atactggctt aactatgcgg catcagagca gattgtactg agagtgcacc     2160
   atatgcggtg tgaaataccg cacagatgcg taaggagaaa ataccgcatc aggcgctctt     2220
   ccgcttcctc gctcactgac tcgctgcgct cggtcgttcg gctgcggcga gcggtatcag     2280
   ctcactcaaa ggcggtaata cggttatcca cagaatcagg ggataacgca ggaaagaaca     2340
   tgtgagcaaa aggccagcaa aaggccagga accgtaaaaa ggccgcgttg ctggcgtttt     2400
   tccataggct ccgcccccct gacgagcatc acaaaaatcg acgctcaagt cagaggtggc     2460
   gaaacccgac aggactataa agataccagg cgtttccccc tggaagctcc ctcgtgcgct     2520
   ctcctgttcc gaccctgccg cttaccggat acctgtccgc ctttctccct tcgggaagcg     2580
   tggcgctttc tcatagctca cgctgtaggt atctcagttc ggtgtaggtc gttcgctcca     2640
   agctgggctg tgtgcacgaa ccccccgttc agcccgaccg ctgcgcctta tccggtaact     2700
   atcgtcttga gtccaacccg gtaagacacg acttatcgcc actggcagca gccactggta     2760
   acaggattag cagagcgagg tatgtaggcg gtgctacaga gttcttgaag tggtggccta     2820
   actacggcta cactagaagg acagtatttg gtatctgcgc tctgctgaag ccagttacct     2880
   tcggaaaaag agttggtagc tcttgatccg gcaaacaaac caccgctggt agcggtggtt     2940
   tttttgtttg caagcagcag attacgcgca gaaaaaaagg atctcaagaa gatcctttga     3000
   tcttttctac ggggtctgac gctcagtgga acgaaaactc acgttaaggg attttggtca     3060
   tgagattatc aaaaaggatc ttcacctaga tccttttaaa ttaaaaatga agttttaaat 3120
   caatctaaag tatatatgag taaacttggt ctgacagtta ccaatgctta atcagtgagg 3180
   cacctatctc agcgatctgt ctatttcgtt catccatagt tgcctgactc cccgtcgtgt 3240
   agataactac gatacgggag ggcttaccat ctggccccag tgctgcaatg ataccgcgag 3300
   acccacgctc accggctcca gatttatcag caataaacca gccagccgga agggccgagc 3360
   gcagaagtgg tcctgcaact ttatccgcct ccatccagtc tattaattgt tgccgggaag 3420
   ctagagtaag tagttcgcca gttaatagtt tgcgcaacgt tgttgccatt gctgcaggca 3480
   tcgtggtgtc acgctcgtcg tttggtatgg cttcattcag ctccggttcc caacgatcaa 3540
   ggcgagttac atgatccccc atgttgtgca aaaaagcggt tagctccttc ggtcctccga 3600
   tcgttgtcag aagtaagttg gccgcagtgt tatcactcat ggttatggca gcactgcata 3660
   attctcttac tgtcatgcca tccgtaagat gcttttctgt gactggtgag tactcaacca 3720
   agtcattctg agaatagtgt atgcggcgac cgagttgctc ttgcccggcg tcaacacggg 3780
   ataataccgc gccacatagc agaactttaa aagtgctcat cattggaaaa cgttcttcgg 3840
   ggcgaaaact ctcaaggatc ttaccgctgt tgagatccag ttcgatgtaa cccactcgtg 3900
   cacccaactg atcttcagca tcttttactt tcaccagcgt ttctgggtga gcaaaaacag 3960
   gaaggcaaaa tgccgcaaaa aagggaataa gggcgacacg gaaatgttga atactcatac 4020
   tcttcctttt tcaatattat tgaagcattt atcagggtta ttgtctcatg agcggataca 4080
   tatttgaatg tatttagaaa aataaacaaa taggggttcc gcgcacattt ccccgaaaag 4140
   tgccacctga cgtctaagaa accattatta tcatgacatt aacctataaa aataggcgta 4200
   tcacgaggcc ctttcgtctt caagaattct gaaccagtcc taaaacgagt aaataggacc 4260
   ggcaattctt caagcaataa acaggaatac caattattaa aagataactt agtcagatcg 4320
   tacaataaag ctttgaagaa aaatgcgcct tattcaatct ttgctataaa aaatggccca 4380
   aaatctcaca ttggaagaca tttgatgacc tcatttcttt caatgaaggg cctaacggag 4440
   ttgactaatg ttgtgggaaa ttggagcgat aagcgtgctt ctgccgtggc caggacaacg 4500
   tatactcatc agataacagc aatacctgat cactacttcg cactagtttc tcggtactat 4560
   gcatatgatc caatatcaaa ggaaatgata gcattgaagg atgagactaa tccaattgag 4620
   gagtggcagc atatagaaca gctaaagggt agtgctgaag gaagcatacg ataccccgca 4680
   tggaatggga taatatcaca ggaggtacta gactaccttt catcctacat aaatagacgc 4740
   atataagtac gcatttaagc ataaacacgc actatgccgt tcttctcatg tatatatata 4800
   tacaggcaac acgcagatat aggtgcgacg tgaacagtga gctgtatgtg cgcagctcgc 4860
   gttgcatttt cggaagcgct cgttttcgga aacgctttga agttcctatt ccgaagttcc 4920
   tattctctag aaagtatagg aacttcagag cgcttttgaa aaccaaaagc gctctgaaga 4980
   cgcactttca aaaaaccaaa aacgcaccgg actgtaacga gctactaaaa tattgcgaat 5040
   accgcttcca caaacattgc tcaaaagtat ctctttgcta tatatctctg tgctatatcc 5100
   ctatataacc tacccatcca cctttcgctc cttgaacttg catctaaact cgacctctac 5160
   attttttatg tttatctcta gtattactct ttagacaaaa aaattgtagt aagaactatt 5220
   catagagtga atcgaaaaca atacgaaaat gtaaacattt cctatacgta gtatatagag 5280
   acaaaataga agaaaccgtt cataattttc tgaccaatga agaatcatca acgctatcac 5340
   tttctgttca caaagtatgc gcaatccaca tcggtataga atataatcgg ggatgccttt 5400
   atcttgaaaa aatgcacccg cagcttcgct agtaatcagt aaacgcggga agtggagtca 5460
   ggcttttttt atggaagaga aaatagacac caaagtagcc ttcttctaac cttaacggac 5520
   ctacagtgca aaaagttatc aagagactgc attatagagc gcacaaagga gaaaaaaagt 5580
   aatctaagat gctttgttag aaaaatagcg ctctcgggat gcatttttgt agaacaaaaa 5640
   agaagtatag attctttgtt ggtaaaatag cgctctcgcg ttgcatttct gttctgtaaa 5700
   aatgcagctc agattctttg tttgaaaaat tagcgctctc gcgttgcatt tttgttttac 5760
   aaaaatgaag cacagattct tcgttggtaa aatagcgctt tcgcgttgca tttctgttct 5820
   gtaaaaatgc agctcagatt ctttgtttga aaaattagcg ctctcgcgtt gcatttttgt 5880
   tctacaaaat gaagcacaga tgcttcgtta acaaagatat gctattgaag tgcaagatgg 5940
   aaacgcagaa aatgaaccgg ggatgcgacg tgcaagatta cctatgcaat agatgcaata 6000
   gtttctccag gaaccgaaat acatacattg tcttccgtaa agcgctagac tatatattat 6060
   tatacaggtt caaatatact atctgtttca gggaaaactc ccaggttcgg atgttcaaaa 6120
   ttcaatgatg ggtaacaagt acgatcgtaa atctgtaaaa cagtttgtcg gatattaggc 6180
   tgtatctcct caaagcgtat tcgaatatca ttgagaagct gcagcgtcac atcggataat 6240
   aatgatggca gccattgtag aagtgccttt tgcatttcta gtctctttct cggtctagct 6300
   agttttacta catcgcgaag atagaatctt agatcacact gcctttgctg agctggatca 6360
   atagagtaac aaaagagtgg taaggcctcg ttaaaggaca aggacctgag cggaagtgta 6420
   tcgtacagta gacggagtat actagtatag tctatagtcc gtggaattct catgtttgac 6480
   agcttatcat cgataagctt ttcaattcaa ttcatcattt tttttttatt cttttttttg 6540
   atttcggttt ctttgaaatt tttttgattc ggtaatctcc gaacagaagg aagaacgaag 6600
   gaaggagcac agacttagat tggtatatat acgcatatgt agtgttgaag aaacatgaaa 6660
   ttgcccagta ttcttaaccc aactgcacag aacaaaaacc tgcaggaaac gaagataaat 6720
   catgtcgaaa gctacatata aggaacgtgc tgctactcat cctagtcctg ttgctgccaa 6780
   gctatttaat atcatgcacg aaaagcaaac aaacttgtgt gcttcattgg atgttcgtac 6840
   caccaaggaa ttactggagt tagttgaagc attaggtccc aaaatttgtt tactaaaaac 6900
   acatgtggat atcttgactg atttttccat ggagggcaca gttaagccgc taaaggcatt 6960
   atccgccaag tacaattttt tactcttcga agacagaaaa tttgctgaca ttggtaatac 7020
   agtcaaattg cagtactctg cgggtgtata cagaatagca gaatgggcag acattacgaa 7080
   tgcacacggt gtggtgggcc caggtattgt tagcggtttg aagcaggcgg cagaagaagt 7140
   aacaaaggaa cctagaggcc ttttgatgtt agcagaattg tcatgcaagg gctccctatc 7200
   tactggagaa tatactaagg gtactgttga cattgcgaag agcgacaaag attttgttat 7260
   cggctttatt gctcaaagag acatgggtgg aagagatgaa ggttacgatt ggttgattat 7320
   gacacccggt gtgggtttag atgacaaggg agacgcattg ggtcaacagt atagaaccgt 7380
   ggatgatgtg gtctctacag gatctgacat tattattgtt ggaagaggac tatttgcaaa 7440
   gggaagggat gctaaggtag agggtgaacg ttacagaaaa gcaggctggg aagcatattt 7500
   gagaagatgc ggccagcaaa actaaaaaac tgtattataa gtaaatgcat gtatactaaa 7560
   ctcacaaatt agagcttcaa tttaattata tcagttatta cccgggaatc tcggtcgtaa 7620
   tgaaaggaaa agcgcaacgg acgggcgagt agattgcgca acatgcgagc atgatccaga 7680
   gatttctgaa gcagcaaaag gatgttccat gtacatgacg cgcggcttgc ggtaaattgt 7740
   tggcaaattt tccggcgtag cccaaaacgc gctgtcgtca agtcgttaag ggcgtgccct 7800
   tcatcatccg atctggagtc aaaatgtcct cacgtaaaga gcttgccaat gctattcgtg 7860
   cgctgagcat ggacgcagta cagaaagcca aatccggtca cccgggtgcc cctatgggta 7920
   tggctgacat tgccgaagtc ctgtggcgtg atttcctgaa acacaacccg cagaatccgt 7980
   cctgggctga ccgtgaccgc ttcgtgctgt ccaacggcca cggctccatg ctgatctaca 8040
   gcctgctgca cctcaccggt tacgatctgc cgatggaaga actgaaaaac ttccgtcagc 8100
   tgcactctaa aactccgggc cacccggaag taggttatac cgctggtgtg gaaaccacca 8160
   ccggtccgct gggtcagggt attgccaacg cagtcggtat ggcgattgca gaaaaaacgc 8220
   tggcggcgca gtttaaccgt ccaggtcacg acattgtcga ccactacacc tacgccttca 8280
   tgggcgacgg ctgcatgatg gaaggcatct cccacgaagt ttgctctctg gcgggtacgc 8340
   tgaagctggg taaactgatt gcgttctacg atgacaacgg tatctcaatc gatggtcacg 8400
   ttgaaggctg gttcactgac gacaccgcaa tgcgtttcga agcttacggc tggcacgtta 8460
   ttcgcgacat cgacggtcat gacgcggcat ccatcaaacg cgcagtagaa gaagcgcgcg 8520
   cagtgactga caaaccgtcc ctgctgatgt gcaaaaccat catcggtttc ggttccccga 8580
   acaaagccgg tacccacgac tcccacggtg cgccgctggg cgacgctgaa attgccctga 8640
   cccgcgaaca gctgggctgg aaatacgcgc cgttcgaaat cccgtctgaa atctatgctc 8700
   agtgggatgc gaaagaagca ggccaggcga aagaatctgc atggaatgag aagtttgcgg 8760
   cttacgcgaa agcttatccg caggaagcgg ctgaatttac ccgccgtatg aaaggcgaaa 8820
   tgccgtctga cttcgacgcc aaagcgaaag agtttatcgc taaactgcag gctaatccgg 8880
   cgaaaatcgc cagccgtaaa gcgtcgcaga atgctatcga agcgttcggc ccgctgttgc 8940
   ctgaattcct cggcggctct gctgacctgg caccgtctaa cctgaccctg tggtctggtt 9000
   ctaaagcaat caacgaagat gctgcaggta actacatcca ctacggtgtt cgcgagttcg 9060
   gtatgaccgc gattgctaac ggtatctccc tgcacggtgg tttcctgccg tacacctcca 9120
   ccttcctgat gttcgtggaa tacgcacgta acgccgtacg tatggctgcg ctgatgaaac 9180
   agcgtcaggt gatggtttac acccacgact ccatcggtct gggcgaagat ggcccgactc 9240
   accagccggt tgagcaggtc gcttctctgc gcgtgacccc gaacatgtct acatggcgtc 9300
   cgtgtgacca ggttgaatcc gcggtcgcgt ggaaatacgg cgttgagcgt caggacggcc 9360
   cgactgcgct tatcctctcc cgtcagaacc tggcgcagca ggaacgaact gaagagcaac 9420
   tggcaaacat cgcgcgcggt ggttatgtgc tgaaagactg cgccggtcag ccggaactga 9480
   ttttcatcgc taccggttca gaagttgaac tggctgttgc tgcctacgaa aaactgactg 9540
   ccgaaggcgt gaaagcgcgc gtggtgtcca tgccgtctac cgacgcattt gacaagcagg 9600
   atgctgctta ccgtgaatcc gtactgccga aagcggttac tgcacgcgtt gctgtagaag 9660
   cgggtattgc tgactactgg tacaagtatg ttggcctgaa cggtgctatc gtcggtatga 9720
   ccaccttcgg tgaatctgct ccggcagagc tgctgtttga agagttcggc ttcactgttg 9780
   ataacgttgt tgcgaaagca aaagaactgc tgtaattagc atttcgggta aaaaggtcgc 9840
   ttcggcgacc ttttttatta ccttgatatg tccgtttgcg gacaagcaat agataaagcg 9900
   tgttgtagat cacaaatatt tatatgcaat aaatatcaat tatgtaatat gcatcacgat 9960
   atgcgtattg acatttgttg ttataactat aactcaatgt tatataagaa attaa 10015
<210> 31
   <211> 9065
   <212> DNA
   <213> Artificial Sequence
<220>
   <223> Plasmid pCP54;
<400> 31
   acatgaatgg tcttcggttt ccgtgtttcg taaagtctgg aaacgcggaa gtcagcgccc        60
   tgcaccatta tgttccggat ctgcatcgca ggatgctgct ggctaccctg tggaacacct       120
   acatctgtat taacgaagcg ctggcattga ccctgagtga tttttctctg gtcccgccgc       180
   atccataccg ccagttgttt accctcacaa cgttccagta accgggcatg ttcatcatca       240
   gtaacccgta tcgtgagcat cctctctcgt ttcatcggta tcattacccc catgaacaga       300
   aattccccct tacacggagg catcaagtga ccaaacagga aaaaaccgcc cttaacatgg      360
   cccgctttat cagaagccag acattaacgc ttctggagaa actcaacgag ctggacgcgg      420
   atgaacaggc agacatctgt gaatcgcttc acgaccacgc tgatgagctt taccgcagct      480
   gcctcgcgcg tttcggtgat gacggtgaaa acctctgaca catgcagctc ccggagacgg      540
   tcacagcttg tctgtaagcg gatgccggga gcagacaagc ccgtcagggc gcgtcagcgg      600
   gtgttggcgg gtgtcggggc gcagccatga cccagtcacg tagcgatagc ggagtgtata      660
   ctggcttaac tatgcggcat cagagcagat tgtactgaga gtgcaccata tgcggtgtga      720
   aataccgcac agatgcgtaa ggagaaaata ccgcatcagg cgctcttccg cttcctcgct      780
   cactgactcg ctgcgctcgg tcgttcggct gcggcgagcg gtatcagctc actcaaaggc      840
   ggtaatacgg ttatccacag aatcagggga taacgcagga aagaacatgt gagcaaaagg      900
   ccagcaaaag gccaggaacc gtaaaaaggc cgcgttgctg gcgtttttcc ataggctccg      960
   cccccctgac gagcatcaca aaaatcgacg ctcaagtcag aggtggcgaa acccgacagg     1020
   actataaaga taccaggcgt ttccccctgg aagctccctc gtgcgctctc ctgttccgac     1080
   cctgccgctt accggatacc tgtccgcctt tctcccttcg ggaagcgtgg cgctttctca     1140
   tagctcacgc tgtaggtatc tcagttcggt gtaggtcgtt cgctccaagc tgggctgtgt     1200
   gcacgaaccc cccgttcagc ccgaccgctg cgccttatcc ggtaactatc gtcttgagtc     1260
   caacccggta agacacgact tatcgccact ggcagcagcc actggtaaca ggattagcag     1320
   agcgaggtat gtaggcggtg ctacagagtt cttgaagtgg tggcctaact acggctacac     1380
   tagaaggaca gtatttggta tctgcgctct gctgaagcca gttaccttcg gaaaaagagt     1440
   tggtagctct tgatccggca aacaaaccac cgctggtagc ggtggttttt ttgtttgcaa     1500
   gcagcagatt acgcgcagaa aaaaaggatc tcaagaagat cctttgatct tttctacggg     1560
   gtctgacgct cagtggaacg aaaactcacg ttaagggatt ttggtcatga gattatcaaa     1620
   aaggatcttc acctagatcc ttttaaatta aaaatgaagt tttaaatcaa tctaaagtat     1680
   atatgagtaa acttggtctg agtggcggtt ttcatggctt gttatgactg tttttttggg     1740
   gtacagtcta tgcctcgggc atccaagcag caagcgcgtt acgccgtggg tcgatgtttg     1800
   atgttatgga gcagcaacga tgttacgcag cagggcagtc gccctaaaac aaagttaaac     1860
   atcatgaggg aagcggtgat cgccgaagta tcgactcaac tatcagaggt agttggcgtc     1920
   atcgagcgcc atctcgaacc gacgttgctg gccgtacatt tgtacggctc cgcagtggat     1980
   ggcggcctga agccacacag tgatattgat ttgctggtta cggtgaccgt aaggcttgat     2040
   gaaacaacgc ggcgagcttt gatcaacgac cttttggaaa cttcggcttc ccctggagag     2100
   agcgagattc tccgcgctgt agaagtcacc attgttgtgc acgacgacat cattccgtgg     2160
   cgttatccag ctaagcgcga actgcaattt ggagaatggc agcgcaatga cattcttgca 2220
   ggtatcttcg agccagccac gatcgacatt gatctggcta tcttgctgac aaaagcaaga 2280
   gaacatagcg ttgccttggt aggtccagcg gcggaggaac tctttgatcc ggttcctgaa 2340
   caggatctat ttgaggcgct aaatgaaacc ttaacgctat ggaactcgcc gcccgactgg 2400
   gctggcgatg agcgaaatgt agtgcttacg ttgtcccgca tttggtacag cgcagtaacc 2460
   ggcaaaatcg cgccgaagga tgtcgctgcc gactgggcaa tggagcgcct gccggcccag 2520
   tatcagcccg tcatacttga agctagacag gcttatcttg gacaagaaga agatcgcttg 2580
   gcctcgcgcg cagatcagtt ggaagaattt gtccactacg tgaaaggcga gatcaccaag 2640
   gtagtcggca aataatgtct aacaattcgt tcaagccgac gccgcttcgc ggcgcggctt 2700
   aactcaagcg ttagatgcac taagcacata attgctcaca gccaaactat cagaattctg 2760
   aaccagtcct aaaacgagta aataggaccg gcaattcttc aagcaataaa caggaatacc 2820
   aattattaaa agataactta gtcagatcgt acaataaagc tttgaagaaa aatgcgcctt 2880
   attcaatctt tgctataaaa aatggcccaa aatctcacat tggaagacat ttgatgacct 2940
   catttctttc aatgaagggc ctaacggagt tgactaatgt tgtgggaaat tggagcgata 3000
   agcgtgcttc tgccgtggcc aggacaacgt atactcatca gataacagca atacctgatc 3060
   actacttcgc actagtttct cggtactatg catatgatcc aatatcaaag gaaatgatag 3120
   cattgaagga tgagactaat ccaattgagg agtggcagca tatagaacag ctaaagggta 3180
   gtgctgaagg aagcatacga taccccgcat ggaatgggat aatatcacag gaggtactag 3240
   actacctttc atcctacata aatagacgca tataagtacg catttaagca taaacacgca 3300
   ctatgccgtt cttctcatgt atatatatat acaggcaaca cgcagatata ggtgcgacgt 3360
   gaacagtgag ctgtatgtgc gcagctcgcg ttgcattttc ggaagcgctc gttttcggaa 3420
   acgctttgaa gttcctattc cgaagttcct attctctaga aagtatagga acttcagagc 3480
   gcttttgaaa accaaaagcg ctctgaagac gcactttcaa aaaaccaaaa acgcaccgga 3540
   ctgtaacgag ctactaaaat attgcgaata ccgcttccac aaacattgct caaaagtatc 3600
   tctttgctat atatctctgt gctatatccc tatataacct acccatccac ctttcgctcc 3660
   ttgaacttgc atctaaactc gacctctaca ttttttatgt ttatctctag tattactctt 3720
   tagacaaaaa aattgtagta agaactattc atagagtgaa tcgaaaacaa tacgaaaatg 3780
   taaacatttc ctatacgtag tatatagaga caaaatagaa gaaaccgttc ataattttct 3840
   gaccaatgaa gaatcatcaa cgctatcact ttctgttcac aaagtatgcg caatccacat 3900
   cggtatagaa tataatcggg gatgccttta tcttgaaaaa atgcacccgc agcttcgcta 3960
   gtaatcagta aacgcgggaa gtggagtcag gcttttttta tggaagagaa aatagacacc 4020
   aaagtagcct tcttctaacc ttaacggacc tacagtgcaa aaagttatca agagactgca 4080
   ttatagagcg cacaaaggag aaaaaaagta atctaagatg ctttgttaga aaaatagcgc 4140
   tctcgggatg catttttgta gaacaaaaaa gaagtataga ttctttgttg gtaaaatagc 4200
   gctctcgcgt tgcatttctg ttctgtaaaa atgcagctca gattctttgt ttgaaaaatt 4260
   agcgctctcg cgttgcattt ttgttttaca aaaatgaagc acagattctt cgttggtaaa 4320
   atagcgcttt cgcgttgcat ttctgttctg taaaaatgca gctcagattc tttgtttgaa 4380
   aaattagcgc tctcgcgttg catttttgtt ctacaaaatg aagcacagat gcttcgttaa 4440
   caaagatatg ctattgaagt gcaagatgga aacgcagaaa atgaaccggg gatgcgacgt 4500
   gcaagattac ctatgcaata gatgcaatag tttctccagg aaccgaaata catacattgt 4560
   cttccgtaaa gcgctagact atatattatt atacaggttc aaatatacta tctgtttcag 4620
   ggaaaactcc caggttcgga tgttcaaaat tcaatgatgg gtaacaagta cgatcgtaaa 4680
   tctgtaaaac agtttgtcgg atattaggct gtatctcctc aaagcgtatt cgaatatcat 4740
   tgagaagctg cagcgtcaca tcggataata atgatggcag ccattgtaga agtgcctttt 4800
   gcatttctag tctctttctc ggtctagcta gttttactac atcgcgaaga tagaatctta 4860
   gatcacactg cctttgctga gctggatcaa tagagtaaca aaagagtggt aaggcctcgt 4920
   taaaggacaa ggacctgagc ggaagtgtat cgtacagtag acggagtata ctagtatagt 4980
   ctatagtccg tggaattctc atgtttgaca gcttatcatc gataagcttt tcaattcaat 5040
   tcatcatttt ttttttattc ttttttttga tttcggtttc tttgaaattt ttttgattcg 5100
   gtaatctccg aacagaagga agaacgaagg aaggagcaca gacttagatt ggtatatata 5160
   cgcatatgta gtgttgaaga aacatgaaat tgcccagtat tcttaaccca actgcacaga 5220
   acaaaaacct gcaggaaacg aagataaatc atgtcgaaag ctacatataa ggaacgtgct 5280
   gctactcatc ctagtcctgt tgctgccaag ctatttaata tcatgcacga aaagcaaaca 5340
   aacttgtgtg cttcattgga tgttcgtacc accaaggaat tactggagtt agttgaagca 5400
   ttaggtccca aaatttgttt actaaaaaca catgtggata tcttgactga tttttccatg 5460
   gagggcacag ttaagccgct aaaggcatta tccgccaagt acaatttttt actcttcgaa 5520
   gacagaaaat ttgctgacat tggtaataca gtcaaattgc agtactctgc gggtgtatac 5580
   agaatagcag aatgggcaga cattacgaat gcacacggtg tggtgggccc aggtattgtt 5640
   agcggtttga agcaggcggc agaagaagta acaaaggaac ctagaggcct tttgatgtta 5700
   gcagaattgt catgcaaggg ctccctatct actggagaat atactaaggg tactgttgac 5760
   attgcgaaga gcgacaaaga ttttgttatc ggctttattg ctcaaagaga catgggtgga 5820
   agagatgaag gttacgattg gttgattatg acacccggtg tgggtttaga tgacaaggga 5880
   gacgcattgg gtcaacagta tagaaccgtg gatgatgtgg tctctacagg atctgacatt 5940
   attattgttg gaagaggact atttgcaaag ggaagggatg ctaaggtaga gggtgaacgt 6000
   tacagaaaag caggctggga agcatatttg agaagatgcg gccagcaaaa ctaaaaaact 6060
   gtattataag taaatgcatg tatactaaac tcacaaatta gagcttcaat ttaattatat 6120
   cagttattac ccgggaatct cggtcgtaat gacttgaaat aattaacaaa caaaggagtt 6180
   acagttagaa attgtaggag agatctcgtt tttcgcgaca atctggcgtt tttcttgcta 6240
   attccaggat taatccgttc atagtgtaaa accccgttta cacattctga cggaagatat 6300
   agattggaag tattgcattc actaagataa gtatggcaac actggaacag acatgaatta 6360
   tcagaacgac gatttacgca tcaaagaaat caaagagtta cttcctcctg tcgcattgct 6420
   ggaaaaattc cccgctactg aaaatgccgc gaatacggtt gcccatgccc gaaaagcgat 6480
   ccataagatc ctgaaaggta atgatgatcg cctgttggtt gtgattggcc catgctcaat 6540
   tcatgatcct gtcgcggcaa aagagtatgc cactcgcttg ctggcgctgc gtgaagagct 6600
   gaaagatgag ctggaaatcg taatgcgcgt ctattttgaa aagccgcgta ccacggtggg 6660
   ctggaaaggg ctgattaacg atccgcatat ggataatagc ttccagatca acgacggtct 6720
   gcgtatagcc cgtaaattgc tgcttgatat taacgacagc ggtctgccag cggcaggtga 6780
   gtttctcgat atgatcaccc cacaatatct cgctgacctg atgagctggg gcgcaattgg 6840
   cgcacgtacc accgaatcgc aggtgcaccg cgaactggca tcagggcttt cttgtccggt 6900
   cggcttcaaa aatggcaccg acggtacgat taaagtggct atcgatgcca ttaatgccgc 6960
   cggtgcgccg cactgcttcc tgtccgtaac gaaatggggg cattcggcga ttgtgaatac 7020
   cagcggtaac ggcgattgcc atatcattct gcgcggcggt aaagagccta actacagcgc 7080
   gaagcacgtt gctgaagtga aagaagggct gaacaaagca ggcctgccag cacaggtgat 7140
   gatcgatttc agccatgcta actcgtccaa acaattcaaa aagcagatgg atgtttgtgc 7200
   tgacgtttgc cagcagattg ccggtggcga aaaggccatt attggcgtga tggtggaaag 7260
   ccatctggtg gaaggcaatc agagcctcga gagcggggag ccgctggcct acggtaagag 7320
   catcaccgat gcctgcatcg gctgggaaga taccgatgct ctgttacgtc aactggcgaa 7380
   tgcagtaaaa gcgcgtcgcg ggtaaggttt aattgtcgga tgcgccgtca gagtggcgta 7440
   tccgatgaat caccacaggc ctgataagtc gcgcagcgtc gcatcaggca atgtgctcca 7500
   ttgttagcaa caaaaaagcc gactcacttg cagtcggctt tctcatttta aacgaatgac 7560
   gtttacttcg ctttaccctg gtttgcaacc gccgctgctt tcgctctcga ggctattgac 7620
   gacagctatg gttcactgtc caccaaccaa aactgtgctc agtaccgcca atatttctcc 7680
   cttgaggggt acaaagaggt gtccctagaa gagatccacg ctgtgtaaaa attttacaaa 7740
   aaggtattga ctttccctac agggtgtgta ataatttaat tacaggcggg ggcaaccccg 7800
   cctgttctag aggaggagga atcgccatgg agaggattgt cgttactctc ggggaacgta 7860
   gttacccaat taccatcgca tctggtttgt ttaatgaacc agcttcattc ttaccgctga 7920
   aatcgggcga gcaggtcatg ttggtcacca acgaaaccct ggctcctctg tatctcgata 7980
   aggtccgcgg cgtacttgaa caggcgggtg ttaacgtcga tagcgttatc ctccctgacg 8040
   gcgagcagta taaaagcctg gctgtactcg ataccgtctt tacggcgttg ttacaaaagc 8100
   cgcatggtcg cgatactacg ctggtggcgc ttggcggcgg cgtagtgggc gatctgaccg 8160
   gcttcgcggc ggcgagttat cagcgcggtg ttcgtttcat tcaagtcccg acgacgttac 8220
   tgtcgcaggt cgattcctcc gttggcggca aaactgcggt caaccatccc ctcggtaaaa 8280
   acatgattgg cgcgttctac cagcctgctt cagtggtggt ggatctcgac tgtctgaaaa 8340
   cgcttccccc gcgtgagtta gcgtcggggc tggcagaagt catcaaatac ggcattattc 8400
   ttgacggtgc gtttttcaac tggctggaag agaatctgga tgcgttgttg cgtctggacg 8460
   gtccggcaat ggcgtactgt attcgccgtt gttgtgaact gaaggcagaa gttgtcgccg 8520
   ccgacgagcg cgaaaccggg ttacgtgctt tactgaatct gggacacacc tttggtcatg 8580
   ccattgaagc tgaaatgggg tatggcaatt ggttacatgg tgaagcggtc gctgcgggta 8640
   tggtgatggc ggcgcggacg tcggaacgtc tcgggcagtt tagttctgcc gaaacgcagc 8700
   gtattataac cctgctcacg cgggctgggt taccggtcaa tgggccgcgc gaaatgtccg 8760
   cgcaggcgta tttaccgcat atgctgcgtg acaagaaagt ccttgcggga gagatgcgct 8820
   taattcttcc gttggcaatt ggtaagagtg aagttcgcag cggcgtttcg cacgagcttg 8880
   ttcttaacgc cattgccgat tgtcaatcag cgtaatcatc gttcatgcct gatgccgcta 8940
   tgtaggccgg ataaggcgtt cacgccgcat ccggcaaccg atgcctgatg cgacgcggtc 9000
   gcgtcttatc aggcctacag gtcgatgccg atatgtacat cgtattcggc aattaataca 9060
   tagca 9065
<210> 32
   <211> 11475
   <212> DNA
   <213> Artificial Sequence
<220>
   <223> Plasmid pCP55;
<400> 32
   acatgaatgg tcttcggttt ccgtgtttcg taaagtctgg aaacgcggaa gtcagcgccc     60
   tgcaccatta tgttccggat ctgcatcgca ggatgctgct ggctaccctg tggaacacct    120
   acatctgtat taacgaagcg ctggcattga ccctgagtga tttttctctg gtcccgccgc    180
   atccataccg ccagttgttt accctcacaa cgttccagta accgggcatg ttcatcatca    240
   gtaacccgta tcgtgagcat cctctctcgt ttcatcggta tcattacccc catgaacaga    300
   aattccccct tacacggagg catcaagtga ccaaacagga aaaaaccgcc cttaacatgg 360
   cccgctttat cagaagccag acattaacgc ttctggagaa actcaacgag ctggacgcgg 420
   atgaacaggc agacatctgt gaatcgcttc acgaccacgc tgatgagctt taccgcagct 480
   gcctcgcgcg tttcggtgat gacggtgaaa acctctgaca catgcagctc ccggagacgg 540
   tcacagcttg tctgtaagcg gatgccggga gcagacaagc ccgtcagggc gcgtcagcgg 600
   gtgttggcgg gtgtcggggc gcagccatga cccagtcacg tagcgatagc ggagtgtata 660
   ctggcttaac tatgcggcat cagagcagat tgtactgaga gtgcaccata tgcggtgtga 720
   aataccgcac agatgcgtaa ggagaaaata ccgcatcagg cgctcttccg cttcctcgct 780
   cactgactcg ctgcgctcgg tcgttcggct gcggcgagcg gtatcagctc actcaaaggc 840
   ggtaatacgg ttatccacag aatcagggga taacgcagga aagaacatgt gagcaaaagg 900
   ccagcaaaag gccaggaacc gtaaaaaggc cgcgttgctg gcgtttttcc ataggctccg 960
   cccccctgac gagcatcaca aaaatcgacg ctcaagtcag aggtggcgaa acccgacagg 1020
   actataaaga taccaggcgt ttccccctgg aagctccctc gtgcgctctc ctgttccgac 1080
   cctgccgctt accggatacc tgtccgcctt tctcccttcg ggaagcgtgg cgctttctca 1140
   tagctcacgc tgtaggtatc tcagttcggt gtaggtcgtt cgctccaagc tgggctgtgt 1200
   gcacgaaccc cccgttcagc ccgaccgctg cgccttatcc ggtaactatc gtcttgagtc 1260
   caacccggta agacacgact tatcgccact ggcagcagcc actggtaaca ggattagcag 1320
   agcgaggtat gtaggcggtg ctacagagtt cttgaagtgg tggcctaact acggctacac 1380
   tagaaggaca gtatttggta tctgcgctct gctgaagcca gttaccttcg gaaaaagagt 1440
   tggtagctct tgatccggca aacaaaccac cgctggtagc ggtggttttt ttgtttgcaa 1500
   gcagcagatt acgcgcagaa aaaaaggatc tcaagaagat cctttgatct tttctacggg 1560
   gtctgacgct cagtggaacg aaaactcacg ttaagggatt ttggtcatga gattatcaaa 1620
   aaggatcttc acctagatcc ttttaaatta aaaatgaagt tttaaatcaa tctaaagtat 1680
   atatgagtaa acttggtctg acagttacca atgcttaatc agtgaggcac ctatctcagc 1740
   gatctgtcta tttcgttcat ccatagttgc ctgactcccc gtcgtgtaga taactacgat 1800
   acgggagggc ttaccatctg gccccagtgc tgcaatgata ccgcgagacc cacgctcacc 1860
   ggctccagat ttatcagcaa taaaccagcc agccggaagg gccgagcgca gaagtggtcc 1920
   tgcaacttta tccgcctcca tccagtctat taattgttgc cgggaagcta gagtaagtag 1980
   ttcgccagtt aatagtttgc gcaacgttgt tgccattgct gcaggcatcg tggtgtcacg 2040
   ctcgtcgttt ggtatggctt cattcagctc cggttcccaa cgatcaaggc gagttacatg 2100
   atcccccatg ttgtgcaaaa aagcggttag ctccttcggt cctccgatcg ttgtcagaag 2160
   taagttggcc gcagtgttat cactcatggt tatggcagca ctgcataatt ctcttactgt 2220
   catgccatcc gtaagatgct tttctgtgac tggtgagtac tcaaccaagt cattctgaga 2280
   atagtgtatg cggcgaccga gttgctcttg cccggcgtca acacgggata ataccgcgcc 2340
   acatagcaga actttaaaag tgctcatcat tggaaaacgt tcttcggggc gaaaactctc 2400
   aaggatctta ccgctgttga gatccagttc gatgtaaccc actcgtgcac ccaactgatc 2460
   ttcagcatct tttactttca ccagcgtttc tgggtgagca aaaacaggaa ggcaaaatgc 2520
   cgcaaaaaag ggaataaggg cgacacggaa atgttgaata ctcatactct tcctttttca 2580
   atattattga agcatttatc agggttattg tctcatgagc ggatacatat ttgaatgtat 2640
   ttagaaaaat aaacaaatag gggttccgcg cacatttccc cgaaaagtgc cacctgacgt 2700
   ctaagaaacc attattatca tgacattaac ctataaaaat aggcgtatca cgaggccctt 2760
   tcgtcttcaa gaattctgaa ccagtcctaa aacgagtaaa taggaccggc aattcttcaa 2820
   gcaataaaca ggaataccaa ttattaaaag ataacttagt cagatcgtac aataaagctt 2880
   tgaagaaaaa tgcgccttat tcaatctttg ctataaaaaa tggcccaaaa tctcacattg 2940
   gaagacattt gatgacctca tttctttcaa tgaagggcct aacggagttg actaatgttg 3000
   tgggaaattg gagcgataag cgtgcttctg ccgtggccag gacaacgtat actcatcaga 3060
   taacagcaat acctgatcac tacttcgcac tagtttctcg gtactatgca tatgatccaa 3120
   tatcaaagga aatgatagca ttgaaggatg agactaatcc aattgaggag tggcagcata 3180
   tagaacagct aaagggtagt gctgaaggaa gcatacgata ccccgcatgg aatgggataa 3240
   tatcacagga ggtactagac tacctttcat cctacataaa tagacgcata taagtacgca 3300
   tttaagcata aacacgcact atgccgttct tctcatgtat atatatatac aggcaacacg 3360
   cagatatagg tgcgacgtga acagtgagct gtatgtgcgc agctcgcgtt gcattttcgg 3420
   aagcgctcgt tttcggaaac gctttgaagt tcctattccg aagttcctat tctctagaaa 3480
   gtataggaac ttcagagcgc ttttgaaaac caaaagcgct ctgaagacgc actttcaaaa 3540
   aaccaaaaac gcaccggact gtaacgagct actaaaatat tgcgaatacc gcttccacaa 3600
   acattgctca aaagtatctc tttgctatat atctctgtgc tatatcccta tataacctac 3660
   ccatccacct ttcgctcctt gaacttgcat ctaaactcga cctctacatt ttttatgttt 3720
   atctctagta ttactcttta gacaaaaaaa ttgtagtaag aactattcat agagtgaatc 3780
   gaaaacaata cgaaaatgta aacatttcct atacgtagta tatagagaca aaatagaaga 3840
   aaccgttcat aattttctga ccaatgaaga atcatcaacg ctatcacttt ctgttcacaa 3900
   agtatgcgca atccacatcg gtatagaata taatcgggga tgcctttatc ttgaaaaaat 3960
   gcacccgcag cttcgctagt aatcagtaaa cgcgggaagt ggagtcaggc tttttttatg 4020
   gaagagaaaa tagacaccaa agtagccttc ttctaacctt aacggaccta cagtgcaaaa 4080
   agttatcaag agactgcatt atagagcgca caaaggagaa aaaaagtaat ctaagatgct 4140
   ttgttagaaa aatagcgctc tcgggatgca tttttgtaga acaaaaaaga agtatagatt 4200
   ctttgttggt aaaatagcgc tctcgcgttg catttctgtt ctgtaaaaat gcagctcaga 4260
   ttctttgttt gaaaaattag cgctctcgcg ttgcattttt gttttacaaa aatgaagcac 4320
   agattcttcg ttggtaaaat agcgctttcg cgttgcattt ctgttctgta aaaatgcagc 4380
   tcagattctt tgtttgaaaa attagcgctc tcgcgttgca tttttgttct acaaaatgaa 4440
   gcacagatgc ttcgttaaca aagatatgct attgaagtgc aagatggaaa cgcagaaaat 4500
   gaaccgggga tgcgacgtgc aagattacct atgcaataga tgcaatagtt tctccaggaa 4560
   ccgaaataca tacattgtct tccgtaaagc gctagactat atattattat acaggttcaa 4620
   atatactatc tgtttcaggg aaaactccca ggttcggatg ttcaaaattc aatgatgggt 4680
   aacaagtacg atcgtaaatc tgtaaaacag tttgtcggat attaggctgt atctcctcaa 4740
   agcgtattcg aatatcattg agaagctgca gcgtcacatc ggataataat gatggcagcc 4800
   attgtagaag tgccttttgc atttctagtc tctttctcgg tctagctagt tttactacat 4860
   cgcgaagata gaatcttaga tcacactgcc tttgctgagc tggatcaata gagtaacaaa 4920
   agagtggtaa ggcctcgtta aaggacaagg acctgagcgg aagtgtatcg tacagtagac 4980
   ggagtatact agtatagtct atagtccgtg gaattctcat gtttgacagc ttatcatcga 5040
   taagcttttc aattcaattc atcatttttt ttttattctt ttttttgatt tcggtttctt 5100
   tgaaattttt ttgattcggt aatctccgaa cagaaggaag aacgaaggaa ggagcacaga 5160
   cttagattgg tatatatacg catatgtagt gttgaagaaa catgaaattg cccagtattc 5220
   ttaacccaac tgcacagaac aaaaacctgc aggaaacgaa gataaatcat gtcgaaagct 5280
   acatataagg aacgtgctgc tactcatcct agtcctgttg ctgccaagct atttaatatc 5340
   atgcacgaaa agcaaacaaa cttgtgtgct tcattggatg ttcgtaccac caaggaatta 5400
   ctggagttag ttgaagcatt aggtcccaaa atttgtttac taaaaacaca tgtggatatc 5460
   ttgactgatt tttccatgga gggcacagtt aagccgctaa aggcattatc cgccaagtac 5520
   aattttttac tcttcgaaga cagaaaattt gctgacattg gtaatacagt caaattgcag 5580
   tactctgcgg gtgtatacag aatagcagaa tgggcagaca ttacgaatgc acacggtgtg 5640
   gtgggcccag gtattgttag cggtttgaag caggcggcag aagaagtaac aaaggaacct 5700
   agaggccttt tgatgttagc agaattgtca tgcaagggct ccctatctac tggagaatat 5760
   actaagggta ctgttgacat tgcgaagagc gacaaagatt ttgttatcgg ctttattgct 5820
   caaagagaca tgggtggaag agatgaaggt tacgattggt tgattatgac acccggtgtg 5880
   ggtttagatg acaagggaga cgcattgggt caacagtata gaaccgtgga tgatgtggtc 5940
   tctacaggat ctgacattat tattgttgga agaggactat ttgcaaaggg aagggatgct 6000
   aaggtagagg gtgaacgtta cagaaaagca ggctgggaag catatttgag aagatgcggc 6060
   cagcaaaact aaaaaactgt attataagta aatgcatgta tactaaactc acaaattaga 6120
   gcttcaattt aattatatca gttattaccc gggaatctcg gtcgtaatga aaggaaaagc 6180
   gcaacggacg ggcgagtaga ttgcgcaaca tgcgagcatg atccagagat ttctgaagca 6240
   gcaaaaggat gttccatgta catgacgcgc ggcttgcggt aaattgttgg caaattttcc 6300
   ggcgtagccc aaaacgcgct gtcgtcaagt cgttaagggc gtgcccttca tcatccgatc 6360
   tggagtcaaa atgtcctcac gtaaagagct tgccaatgct attcgtgcgc tgagcatgga 6420
   cgcagtacag aaagccaaat ccggtcaccc gggtgcccct atgggtatgg ctgacattgc 6480
   cgaagtcctg tggcgtgatt tcctgaaaca caacccgcag aatccgtcct gggctgaccg 6540
   tgaccgcttc gtgctgtcca acggccacgg ctccatgctg atctacagcc tgctgcacct 6600
   caccggttac gatctgccga tggaagaact gaaaaacttc cgtcagctgc actctaaaac 6660
   tccgggccac ccggaagtag gttataccgc tggtgtggaa accaccaccg gtccgctggg 6720
   tcagggtatt gccaacgcag tcggtatggc gattgcagaa aaaacgctgg cggcgcagtt 6780
   taaccgtcca ggtcacgaca ttgtcgacca ctacacctac gccttcatgg gcgacggctg 6840
   catgatggaa ggcatctccc acgaagtttg ctctctggcg ggtacgctga agctgggtaa 6900
   actgattgcg ttctacgatg acaacggtat ctcaatcgat ggtcacgttg aaggctggtt 6960
   cactgacgac accgcaatgc gtttcgaagc ttacggctgg cacgttattc gcgacatcga 7020
   cggtcatgac gcggcatcca tcaaacgcgc agtagaagaa gcgcgcgcag tgactgacaa 7080
   accgtccctg ctgatgtgca aaaccatcat cggtttcggt tccccgaaca aagccggtac 7140
   ccacgactcc cacggtgcgc cgctgggcga cgctgaaatt gccctgaccc gcgaacagct 7200
   gggctggaaa tacgcgccgt tcgaaatccc gtctgaaatc tatgctcagt gggatgcgaa 7260
   agaagcaggc caggcgaaag aatctgcatg gaatgagaag tttgcggctt acgcgaaagc 7320
   ttatccgcag gaagcggctg aatttacccg ccgtatgaaa ggcgaaatgc cgtctgactt 7380
   cgacgccaaa gcgaaagagt ttatcgctaa actgcaggct aatccggcga aaatcgccag 7440
   ccgtaaagcg tcgcagaatg ctatcgaagc gttcggcccg ctgttgcctg aattcctcgg 7500
   cggctctgct gacctggcac cgtctaacct gaccctgtgg tctggttcta aagcaatcaa 7560
   cgaagatgct gcaggtaact acatccacta cggtgttcgc gagttcggta tgaccgcgat 7620
   tgctaacggt atctccctgc acggtggttt cctgccgtac acctccacct tcctgatgtt 7680
   cgtggaatac gcacgtaacg ccgtacgtat ggctgcgctg atgaaacagc gtcaggtgat 7740
   ggtttacacc cacgactcca tcggtctggg cgaagatggc ccgactcacc agccggttga 7800
   gcaggtcgct tctctgcgcg tgaccccgaa catgtctaca tggcgtccgt gtgaccaggt 7860
   tgaatccgcg gtcgcgtgga aatacggcgt tgagcgtcag gacggcccga ctgcgcttat 7920
   cctctcccgt cagaacctgg cgcagcagga acgaactgaa gagcaactgg caaacatcgc 7980
   gcgcggtggt tatgtgctga aagactgcgc cggtcagccg gaactgattt tcatcgctac 8040
   cggttcagaa gttgaactgg ctgttgctgc ctacgaaaaa ctgactgccg aaggcgtgaa 8100
   agcgcgcgtg gtgtccatgc cgtctaccga cgcatttgac aagcaggatg ctgcttaccg 8160
   tgaatccgta ctgccgaaag cggttactgc acgcgttgct gtagaagcgg gtattgctga 8220
   ctactggtac aagtatgttg gcctgaacgg tgctatcgtc ggtatgacca ccttcggtga 8280
   atctgctccg gcagagctgc tgtttgaaga gttcggcttc actgttgata acgttgttgc 8340
   gaaagcaaaa gaactgctgt aattagcatt tcgggtaaaa aggtcgcttc ggcgaccttt 8400
   tttattacct tgatatgtcc gtttgcggac aagcaataga taaagcgtgt tgtagatcac 8460
   aaatatttat atgcaataaa tatcaattat gtaatatgca tcacgatatg cgtattgaca 8520
   tttgttgtta taactataac tcaatgttat ataagaaatt aacttgaaat aattaacaaa 8580
   caaaggagtt acagttagaa attgtaggag agatctcgtt tttcgcgaca atctggcgtt 8640
   tttcttgcta attccaggat taatccgttc atagtgtaaa accccgttta cacattctga 8700
   cggaagatat agattggaag tattgcattc actaagataa gtatggcaac actggaacag 8760
   acatgaatta tcagaacgac gatttacgca tcaaagaaat caaagagtta cttcctcctg 8820
   tcgcattgct ggaaaaattc cccgctactg aaaatgccgc gaatacggtt gcccatgccc 8880
   gaaaagcgat ccataagatc ctgaaaggta atgatgatcg cctgttggtt gtgattggcc 8940
   catgctcaat tcatgatcct gtcgcggcaa aagagtatgc cactcgcttg ctggcgctgc 9000
   gtgaagagct gaaagatgag ctggaaatcg taatgcgcgt ctattttgaa aagccgcgta 9060
   ccacggtggg ctggaaaggg ctgattaacg atccgcatat ggataatagc ttccagatca 9120
   acgacggtct gcgtatagcc cgtaaattgc tgcttgatat taacgacagc ggtctgccag 9180
   cggcaggtga gtttctcgat atgatcaccc cacaatatct cgctgacctg atgagctggg 9240
   gcgcaattgg cgcacgtacc accgaatcgc aggtgcaccg cgaactggca tcagggcttt 9300
   cttgtccggt cggcttcaaa aatggcaccg acggtacgat taaagtggct atcgatgcca 9360
   ttaatgccgc cggtgcgccg cactgcttcc tgtccgtaac gaaatggggg cattcggcga 9420
   ttgtgaatac cagcggtaac ggcgattgcc atatcattct gcgcggcggt aaagagccta 9480
   actacagcgc gaagcacgtt gctgaagtga aagaagggct gaacaaagca ggcctgccag 9540
   cacaggtgat gatcgatttc agccatgcta actcgtccaa acaattcaaa aagcagatgg 9600
   atgtttgtgc tgacgtttgc cagcagattg ccggtggcga aaaggccatt attggcgtga 9660
   tggtggaaag ccatctggtg gaaggcaatc agagcctcga gagcggggag ccgctggcct 9720
   acggtaagag catcaccgat gcctgcatcg gctgggaaga taccgatgct ctgttacgtc 9780
   aactggcgaa tgcagtaaaa gcgcgtcgcg ggtaaggttt aattgtcgga tgcgccgtca 9840
   gagtggcgta tccgatgaat caccacaggc ctgataagtc gcgcagcgtc gcatcaggca 9900
   atgtgctcca ttgttagcaa caaaaaagcc gactcacttg cagtcggctt tctcatttta 9960
   aacgaatgac gtttacttcg ctttaccctg gtttgcaacc gccgctgctt tcgctctcga 10020
   ggctattgac gacagctatg gttcactgtc caccaaccaa aactgtgctc agtaccgcca 10080
   atatttctcc cttgaggggt acaaagaggt gtccctagaa gagatccacg ctgtgtaaaa 10140
   attttacaaa aaggtattga ctttccctac agggtgtgta ataatttaat tacaggcggg 10200
   ggcaaccccg cctgttctag aggaggagga atcgccatgg agaggattgt cgttactctc 10260
   ggggaacgta gttacccaat taccatcgca tctggtttgt ttaatgaacc agcttcattc 10320
   ttaccgctga aatcgggcga gcaggtcatg ttggtcacca acgaaaccct ggctcctctg 10380
   tatctcgata aggtccgcgg cgtacttgaa caggcgggtg ttaacgtcga tagcgttatc 10440
   ctccctgacg gcgagcagta taaaagcctg gctgtactcg ataccgtctt tacggcgttg 10500
   ttacaaaagc cgcatggtcg cgatactacg ctggtggcgc ttggcggcgg cgtagtgggc 10560
   gatctgaccg gcttcgcggc ggcgagttat cagcgcggtg ttcgtttcat tcaagtcccg 10620
   acgacgttac tgtcgcaggt cgattcctcc gttggcggca aaactgcggt caaccatccc 10680
   ctcggtaaaa acatgattgg cgcgttctac cagcctgctt cagtggtggt ggatctcgac 10740
   tgtctgaaaa cgcttccccc gcgtgagtta gcgtcggggc tggcagaagt catcaaatac 10800
   ggcattattc ttgacggtgc gtttttcaac tggctggaag agaatctgga tgcgttgttg 10860
   cgtctggacg gtccggcaat ggcgtactgt attcgccgtt gttgtgaact gaaggcagaa 10920
   gttgtcgccg ccgacgagcg cgaaaccggg ttacgtgctt tactgaatct gggacacacc 10980
   tttggtcatg ccattgaagc tgaaatgggg tatggcaatt ggttacatgg tgaagcggtc 11040
   gctgcgggta tggtgatggc ggcgcggacg tcggaacgtc tcgggcagtt tagttctgcc 11100
   gaaacgcagc gtattataac cctgctcacg cgggctgggt taccggtcaa tgggccgcgc 11160
   gaaatgtccg cgcaggcgta tttaccgcat atgctgcgtg acaagaaagt ccttgcggga 11220
   gagatgcgct taattcttcc gttggcaatt ggtaagagtg aagttcgcag cggcgtttcg 11280
   cacgagcttg ttcttaacgc cattgccgat tgtcaatcag cgtaatcatc gttcatgcct 11340
   gatgccgcta tgtaggccgg ataaggcgtt cacgccgcat ccggcaaccg atgcctgatg 11400
   cgacgcggtc gcgtcttatc aggcctacag gtcgatgccg atatgtacat cgtattcggc 11460
   aattaataca tagca 11475
<210> 33
   <211> 7769
   <212> DNA
   <213> Artificial Sequence
<220>
   <223> Plasmid pYEP24;
<400> 33
   gaattctgaa ccagtcctaa aacgagtaaa taggaccggc aattcttcaa gcaataaaca      60
   ggaataccaa ttattaaaag ataacttagt cagatcgtac aataaagctt tgaagaaaaa     120
   tgcgccttat tcaatctttg ctataaaaaa tggcccaaaa tctcacattg gaagacattt     180
   gatgacctca tttctttcaa tgaagggcct aacggagttg actaatgttg tgggaaattg     240
   gagcgataag cgtgcttctg ccgtggccag gacaacgtat actcatcaga taacagcaat     300
   acctgatcac tacttcgcac tagtttctcg gtactatgca tatgatccaa tatcaaagga     360
   aatgatagca ttgaaggatg agactaatcc aattgaggag tggcagcata tagaacagct     420
   aaagggtagt gctgaaggaa gcatacgata ccccgcatgg aatgggataa tatcacagga     480
   ggtactagac tacctttcat cctacataaa tagacgcata taagtacgca tttaagcata     540
   aacacgcact atgccgttct tctcatgtat atatatatac aggcaacacg cagatatagg     600
   tgcgacgtga acagtgagct gtatgtgcgc agctcgcgtt gcattttcgg aagcgctcgt     660
   tttcggaaac gctttgaagt tcctattccg aagttcctat tctctagaaa gtataggaac     720
   ttcagagcgc ttttgaaaac caaaagcgct ctgaagacgc actttcaaaa aaccaaaaac     780
   gcaccggact gtaacgagct actaaaatat tgcgaatacc gcttccacaa acattgctca     840
   aaagtatctc tttgctatat atctctgtgc tatatcccta tataacctac ccatccacct     900
   ttcgctcctt gaacttgcat ctaaactcga cctctacatt ttttatgttt atctctagta     960
   ttactcttta gacaaaaaaa ttgtagtaag aactattcat agagtgaatc gaaaacaata    1020
   cgaaaatgta aacatttcct atacgtagta tatagagaca aaatagaaga aaccgttcat    1080
   aattttctga ccaatgaaga atcatcaacg ctatcacttt ctgttcacaa agtatgcgca    1140
   atccacatcg gtatagaata taatcgggga tgcctttatc ttgaaaaaat gcacccgcag    1200
   cttcgctagt aatcagtaaa cgcgggaagt ggagtcaggc tttttttatg gaagagaaaa    1260
   tagacaccaa agtagccttc ttctaacctt aacggaccta cagtgcaaaa agttatcaag    1320
   agactgcatt atagagcgca caaaggagaa aaaaagtaat ctaagatgct ttgttagaaa    1380
   aatagcgctc tcgggatgca tttttgtaga acaaaaaaga agtatagatt ctttgttggt    1440
   aaaatagcgc tctcgcgttg catttctgtt ctgtaaaaat gcagctcaga ttctttgttt    1500
   gaaaaattag cgctctcgcg ttgcattttt gttttacaaa aatgaagcac agattcttcg    1560
   ttggtaaaat agcgctttcg cgttgcattt ctgttctgta aaaatgcagc tcagattctt    1620
   tgtttgaaaa attagcgctc tcgcgttgca tttttgttct acaaaatgaa gcacagatgc    1680
   ttcgttaaca aagatatgct attgaagtgc aagatggaaa cgcagaaaat gaaccgggga    1740
   tgcgacgtgc aagattacct atgcaataga tgcaatagtt tctccaggaa ccgaaataca 1800
   tacattgtct tccgtaaagc gctagactat atattattat acaggttcaa atatactatc 1860
   tgtttcaggg aaaactccca ggttcggatg ttcaaaattc aatgatgggt aacaagtacg 1920
   atcgtaaatc tgtaaaacag tttgtcggat attaggctgt atctcctcaa agcgtattcg 1980
   aatatcattg agaagctgca gcgtcacatc ggataataat gatggcagcc attgtagaag 2040
   tgccttttgc atttctagtc tctttctcgg tctagctagt tttactacat cgcgaagata 2100
   gaatcttaga tcacactgcc tttgctgagc tggatcaata gagtaacaaa agagtggtaa 2160
   ggcctcgtta aaggacaagg acctgagcgg aagtgtatcg tacagtagac ggagtatact 2220
   agtatagtct atagtccgtg gaattctcat gtttgacagc ttatcatcga taagcttttc 2280
   aattcaattc atcatttttt ttttattctt ttttttgatt tcggtttctt tgaaattttt 2340
   ttgattcggt aatctccgaa cagaaggaag aacgaaggaa ggagcacaga cttagattgg 2400
   tatatatacg catatgtagt gttgaagaaa catgaaattg cccagtattc ttaacccaac 2460
   tgcacagaac aaaaacctgc aggaaacgaa gataaatcat gtcgaaagct acatataagg 2520
   aacgtgctgc tactcatcct agtcctgttg ctgccaagct atttaatatc atgcacgaaa 2580
   agcaaacaaa cttgtgtgct tcattggatg ttcgtaccac caaggaatta ctggagttag 2640
   ttgaagcatt aggtcccaaa atttgtttac taaaaacaca tgtggatatc ttgactgatt 2700
   tttccatgga gggcacagtt aagccgctaa aggcattatc cgccaagtac aattttttac 2760
   tcttcgaaga cagaaaattt gctgacattg gtaatacagt caaattgcag tactctgcgg 2820
   gtgtatacag aatagcagaa tgggcagaca ttacgaatgc acacggtgtg gtgggcccag 2880
   gtattgttag cggtttgaag caggcggcag aagaagtaac aaaggaacct agaggccttt 2940
   tgatgttagc agaattgtca tgcaagggct ccctatctac tggagaatat actaagggta 3000
   ctgttgacat tgcgaagagc gacaaagatt ttgttatcgg ctttattgct caaagagaca 3060
   tgggtggaag agatgaaggt tacgattggt tgattatgac acccggtgtg ggtttagatg 3120
   acaagggaga cgcattgggt caacagtata gaaccgtgga tgatgtggtc tctacaggat 3180
   ctgacattat tattgttgga agaggactat ttgcaaaggg aagggatgct aaggtagagg 3240
   gtgaacgtta cagaaaagca ggctgggaag catatttgag aagatgcggc cagcaaaact 3300
   aaaaaactgt attataagta aatgcatgta tactaaactc acaaattaga gcttcaattt 3360
   aattatatca gttattaccc gggaatctcg gtcgtaatga tttttataat gacgaaaaaa 3420
   aaaaaattgg aaagaaaaag ctttaatgcg gtagtttatc acagttaaat tgctaacgca 3480
   gtcaggcacc gtgtatgaaa tctaacaatg cgctcatcgt catcctcggc accgtcaccc 3540
   tggatgctgt aggcataggc ttggttatgc cggtactgcc gggcctcttg cgggatatcg 3600
   tccattccga cagcatcgcc agtcactatg gcgtgctgct agcgctatat gcgttgatgc 3660
   aatttctatg cgcacccgtt ctcggagcac tgtccgaccg ctttggccgc cgcccagtcc 3720
   tgctcgcttc gctacttgga gccactatcg actacgcgat catggcgacc acacccgtcc 3780
   tgtggatcct ctacgccgga cgcatcgtgg ccggcatcac cggcgccaca ggtgcggttg 3840
   ctggcgccta tatcgccgac atcaccgatg gggaagatcg ggctcgccac ttcgggctca 3900
   tgagcgcttg tttcggcgtg ggtatggtgg caggccccgt ggccggggga ctgttgggcg 3960
   ccatctcctt gcatgcacca ttccttgcgg cggcggtgct caacggcctc aacctactac 4020
   tgggctgctt cctaatgcag gagtcgcata agggagagcg tcgaccgatg cccttgagag 4080
   ccttcaaccc agtcagctcc ttccggtggg cgcggggcat gactatcgtc gccgcactta 4140
   tgactgtctt ctttatcatg caactcgtag gacaggtgcc ggcagcgctc tgggtcattt 4200
   tcggcgagga ccgctttcgc tggagcgcga cgatgatcgg cctgtcgctt gcggtattcg 4260
   gaatcttgca cgccctcgct caagccttcg tcactggtcc cgccaccaaa cgtttcggcg 4320
   agaagcaggc cattatcgcc ggcatggcgg ccgacgcgct gggctacgtc ttgctggcgt 4380
   tcgcgacgcg aggctggatg gccttcccca ttatgattct tctcgcttcc ggcggcatcg 4440
   ggatgcccgc gttgcaggcc atgctgtcca ggcaggtaga tgacgaccat cagggacagc 4500
   ttcaaggatc gctcgcggct cttaccagcc taacttcgat cactggaccg ctgatcgtca 4560
   cggcgattta tgccgcctcg gcgagcacat ggaacgggtt ggcatggatt gtaggcgccg 4620
   ccctatacct tgtctgcctc cccgcgttgc gtcgcggtgc atggagccgg gccacctcga 4680
   cctgaatgga agccggcggc acctcgctaa cggattcacc actccaagaa ttggagccaa 4740
   tcaattcttg cggagaactg tgaatgcgca aaccaaccct tggcagaaca tatccatcgc 4800
   gtccgccatc tccagcagcc gcacgcggcg catctcgggc agcgttgggt cctggccacg 4860
   ggtgcgcatg atcgtgctcc tgtcgttgag gacccggcta ggctggcggg gttgccttac 4920
   tggttagcag aatgaatcac cgatacgcga gcgaacgtga agcgactgct gctgcaaaac 4980
   gtctgcgacc tgagcaacaa catgaatggt cttcggtttc cgtgtttcgt aaagtctgga 5040
   aacgcggaag tcagcgccct gcaccattat gttccggatc tgcatcgcag gatgctgctg 5100
   gctaccctgt ggaacaccta catctgtatt aacgaagcgc tggcattgac cctgagtgat 5160
   ttttctctgg tcccgccgca tccataccgc cagttgttta ccctcacaac gttccagtaa 5220
   ccgggcatgt tcatcatcag taacccgtat cgtgagcatc ctctctcgtt tcatcggtat 5280
   cattaccccc atgaacagaa attccccctt acacggaggc atcaagtgac caaacaggaa 5340
   aaaaccgccc ttaacatggc ccgctttatc agaagccaga cattaacgct tctggagaaa 5400
   ctcaacgagc tggacgcgga tgaacaggca gacatctgtg aatcgcttca cgaccacgct 5460
   gatgagcttt accgcagctg cctcgcgcgt ttcggtgatg acggtgaaaa cctctgacac 5520
   atgcagctcc cggagacggt cacagcttgt ctgtaagcgg atgccgggag cagacaagcc 5580
   cgtcagggcg cgtcagcggg tgttggcggg tgtcggggcg cagccatgac ccagtcacgt 5640
   agcgatagcg gagtgtatac tggcttaact atgcggcatc agagcagatt gtactgagag 5700
   tgcaccatat gcggtgtgaa ataccgcaca gatgcgtaag gagaaaatac cgcatcaggc 5760
   gctcttccgc ttcctcgctc actgactcgc tgcgctcggt cgttcggctg cggcgagcgg 5820
   tatcagctca ctcaaaggcg gtaatacggt tatccacaga atcaggggat aacgcaggaa 5880
   agaacatgtg agcaaaaggc cagcaaaagg ccaggaaccg taaaaaggcc gcgttgctgg 5940
   cgtttttcca taggctccgc ccccctgacg agcatcacaa aaatcgacgc tcaagtcaga 6000
   ggtggcgaaa cccgacagga ctataaagat accaggcgtt tccccctgga agctccctcg 6060
   tgcgctctcc tgttccgacc ctgccgctta ccggatacct gtccgccttt ctcccttcgg 6120
   gaagcgtggc gctttctcat agctcacgct gtaggtatct cagttcggtg taggtcgttc 6180
   gctccaagct gggctgtgtg cacgaacccc ccgttcagcc cgaccgctgc gccttatccg 6240
   gtaactatcg tcttgagtcc aacccggtaa gacacgactt atcgccactg gcagcagcca 6300
   ctggtaacag gattagcaga gcgaggtatg taggcggtgc tacagagttc ttgaagtggt 6360
   ggcctaacta cggctacact agaaggacag tatttggtat ctgcgctctg ctgaagccag 6420
   ttaccttcgg aaaaagagtt ggtagctctt gatccggcaa acaaaccacc gctggtagcg 6480
   gtggtttttt tgtttgcaag cagcagatta cgcgcagaaa aaaaggatct caagaagatc 6540
   ctttgatctt ttctacgggg tctgacgctc agtggaacga aaactcacgt taagggattt 6600
   tggtcatgag attatcaaaa aggatcttca cctagatcct tttaaattaa aaatgaagtt 6660
   ttaaatcaat ctaaagtata tatgagtaaa cttggtctga cagttaccaa tgcttaatca 6720
   gtgaggcacc tatctcagcg atctgtctat ttcgttcatc catagttgcc tgactccccg 6780
   tcgtgtagat aactacgata cgggagggct taccatctgg ccccagtgct gcaatgatac 6840
   cgcgagaccc acgctcaccg gctccagatt tatcagcaat aaaccagcca gccggaaggg 6900
   ccgagcgcag aagtggtcct gcaactttat ccgcctccat ccagtctatt aattgttgcc 6960
   gggaagctag agtaagtagt tcgccagtta atagtttgcg caacgttgtt gccattgctg 7020
   caggcatcgt ggtgtcacgc tcgtcgtttg gtatggcttc attcagctcc ggttcccaac 7080
   gatcaaggcg agttacatga tcccccatgt tgtgcaaaaa agcggttagc tccttcggtc 7140
   ctccgatcgt tgtcagaagt aagttggccg cagtgttatc actcatggtt atggcagcac 7200
   tgcataattc tcttactgtc atgccatccg taagatgctt ttctgtgact ggtgagtact 7260
   caaccaagtc attctgagaa tagtgtatgc ggcgaccgag ttgctcttgc ccggcgtcaa 7320
   cacgggataa taccgcgcca catagcagaa ctttaaaagt gctcatcatt ggaaaacgtt 7380
   cttcggggcg aaaactctca aggatcttac cgctgttgag atccagttcg atgtaaccca 7440
   ctcgtgcacc caactgatct tcagcatctt ttactttcac cagcgtttct gggtgagcaa 7500
   aaacaggaag gcaaaatgcc gcaaaaaagg gaataagggc gacacggaaa tgttgaatac 7560
   tcatactctt cctttttcaa tattattgaa gcatttatca gggttattgt ctcatgagcg 7620
   gatacatatt tgaatgtatt tagaaaaata aacaaatagg ggttccgcgc acatttcccc 7680
   gaaaagtgcc acctgacgtc taagaaacca ttattatcat gacattaacc tataaaaata 7740
   ggcgtatcac gaggcccttt cgtcttcaa 7769
<210> 34
   <211> 2000
   <212> DNA
   <213> Artificial Sequence
<220>
   <223> Sequence of the deleted aroE region;
<400> 34
   actacgtccg tcctctgaaa tcttcagcgg atggacatat cgtcaaagtt ctggaggggc      60
   aggtttgccc tgcatgtggc gcaaatctgg tattacgcca gggacgcttt ggtatgttta     120
   ttggttgcat taactaccct gaatgcgaac ataccgaact tatcgataaa ccggacgaaa     180
   cagcaattac atgcccccaa tgtcggacgg gccatctggt ccagcgccgc tcccgttatg     240
   gcaaaacatt tcactcttgt gatcgctacc cggagtgtca atttgccatt aacttcaaac     300
   ccatagctgg agaatgccct gagtgtcatt atccgctact catcgaaaag aaaaccgcgc     360
   agggtgtaaa acacttttgt gccagtaaac aatgtggaaa gccggtttcg gcggaataat     420
   aacgtgaata ataacctgca aagagacgct atcgcagctg cgatagatgt tctcaatgaa     480
   gaacgtgtca tcgcctatcc aacggaagcc gttttcggtg ttgggtgcga tcctgatagc     540
   gaaacagcag tgatgcgact gttggagtta aaacagcgtc cggttgataa ggggctgatt     600
   ttaatcgcag caaattacga gcagcttaaa ccctatattg atgacaccat gttgactgac     660
   gtgcagcgtg aaaccatttt ttcccgctgg ccaggtcctg tcacctttgt ctttcccgcg     720
   cctgcgacaa caccgcgctg gttgacgggc cgctttgatt cgcttgctgt acgagtcacc     780
   gaccatccgt tggtggttgc tttgtgccag gcttatggta aaccgctggt ttctaccagt     840
   gccaacttga gtggattgcc accttgtcga acagtagacg aagttcgcgc acaatttggc     900
   gcggcgttcc cggttgtgcc tggtgaaacg ggggggcgtt taaatccttc agaaatccgc     960
   gatgccctga cgggtgaact gtttcgacag gggtaacata atcaggccat ccagtttccg     1020
   gacagggaag agtgggacga gaataaaaaa tgtgtatgtt ttcccgctct cgtgaatggt    1080
   atgcaactga catgcgcgat ctctggcgag agtctggcgt atcgctttac tggagatacg    1140
   ccagaacagt ggttagcgag ttttcgtcag catcgctggg acctggaaga agaagcggaa    1200
   aacttaattc aggaacaaag tgaagatgat caaggctggg tctggttacc ctgatccaga 1260
   tattcgtcct tccatttcac gtaattattc gcggaatagc gtaacccagc cttctcttca 1320
   tcacttaacg ggcggatctg tttgacgggg ctaccgagat acagatatcc gctctccagc 1380
   cgtttatttt gtgggaccag actacccgca ccaatcatca catcatcttc tactattgcg 1440
   ccatcaagta aaattgagcc catcccaacc aaaactcgat tgccaatggt gcagccgtgg 1500
   agcatcacct tgtgaccaac agtgacatct tcgccaatgg ttaatgggtt gccatctggg 1560
   ttgtacgagg atttatgagt gacatgcaac atactgccat cctggatatt ggtgcgtgct 1620
   ccgatctgta cataatgtac atctccacga atcacaacga gcggccagat ccccacatca 1680
   tcagccagac gaacgtcacc aatcacgaca ctgctatcgt cgatcattac gcgctgaccg 1740
   atttgtggaa aaagatcgcg gtatgggcgt aaaacatcag acatacttac ctcagcaata 1800
   aatgatttac taatgacttt gggggcatta ttggccttgt gcaagtcttt tagtatgcaa 1860
   aaaagcaccg ttttgtgtgc gattgcagca aaaagggtga aaaaacaaca aacagaaaaa 1920
   aagatcaaaa aaatacttgt gcaaaaaatt gggatcccta taatgcgcct ccgttgagac 1980
   gacaacgtga aacacttcac 2000
<210> 35
   <211> 2460
   <212> DNA
   <213> Artificial Sequence
<220>
   <223> Sequence of the P15aroB cassette integrated at deleted ack locus;
<400> 35
   gatcggcggc ataaaacgga tcgcataacg cgtcatcttg ataacgcgat tttcgacaaa      60
   gaccggggca aggcgttttt ccagcggcca cgtctttgag taatgctgtc cccggcgaaa     120
   caagctaaaa aaattaacag aacgattatc cggcgttgac atgcttcacc tcaacttcac     180
   atataaagat tcaaaaattt gtgcaaattc acaactcagc gggacaacgt tcaaaacatt     240
   ttgtcttcca tacccactat caggtatcct ttagcagcct gaaggcctaa gtagtacata     300
   ttcattgagt cgtcaaattc atatacatta tgccattggc tgaaaattac gcaaaatggc     360
   atagactcaa gatatttctt ccatcatgca aaaaaaaatt tgcagtgcat gatgttaatc     420
   ataaatgtcg gtgtcatcat gcgctacgct ctatggctcc ctgacgtttt tttagccacg     480
   tatcaattat aggtacttcc ctcgaggcta ttgacgacag ctatggttca ctgtccacca     540
   accaaaactg tgctcagtac cgccaatatt tctcccttga ggggtacaaa gaggtgtccc     600
   tagaagagat ccacgctgtg taaaaatttt acaaaaaggt attgactttc cctacagggt     660
   gtgtaataat ttaattacag gcgggggcaa ccccgcctgt tctagaggag gaggaatcgc     720
   catggagagg attgtcgtta ctctcgggga acgtagttac ccaattacca tcgcatctgg 780
   tttgtttaat gaaccagctt cattcttacc gctgaaatcg ggcgagcagg tcatgttggt 840
   caccaacgaa accctggctc ctctgtatct cgataaggtc cgcggcgtac ttgaacaggc 900
   gggtgttaac gtcgatagcg ttatcctccc tgacggcgag cagtataaaa gcctggctgt 960
   actcgatacc gtctttacgg cgttgttaca aaagccgcat ggtcgcgata ctacgctggt 1020
   ggcgcttggc ggcggcgtag tgggcgatct gaccggcttc gcggcggcga gttatcagcg 1080
   cggtgttcgt ttcattcaag tcccgacgac gttactgtcg caggtcgatt cctccgttgg 1140
   cggcaaaact gcggtcaacc atcccctcgg taaaaacatg attggcgcgt tctaccagcc 1200
   tgcttcagtg gtggtggatc tcgactgtct gaaaacgctt cccccgcgtg agttagcgtc 1260
   ggggctggca gaagtcatca aatacggcat tattcttgac ggtgcgtttt tcaactggct 1320
   ggaagagaat ctggatgcgt tgttgcgtct ggacggtccg gcaatggcgt actgtattcg 1380
   ccgttgttgt gaactgaagg cagaagttgt cgccgccgac gagcgcgaaa ccgggttacg 1440
   tgctttactg aatctgggac acacctttgg tcatgccatt gaagctgaaa tggggtatgg 1500
   caattggtta catggtgaag cggtcgctgc gggtatggtg atggcggcgc ggacgtcgga 1560
   acgtctcggg cagtttagtt ctgccgaaac gcagcgtatt ataaccctgc tcacgcgggc 1620
   tgggttaccg gtcaatgggc cgcgcgaaat gtccgcgcag gcgtatttac cgcatatgct 1680
   gcgtgacaag aaagtccttg cgggagagat gcgcttaatt cttccgttgg caattggtaa 1740
   gagtgaagtt cgcagcggcg tttcgcacga gcttgttctt aacgccattg ccgattgtca 1800
   atcagcgtaa tcatcgttca tgcctgatgc cgctatgtag gccggataag gcgttcacgc 1860
   cgcatccggc aaccgatgcc tgatgcgacg cggtcgcgtc ttatcaggcc tacaggtcga 1920
   tgccgatatg tacatcgtat tcggcaatta atacatagca tttcacaccg ccagctcagc 1980
   tggcggtgct gttttgtaac ccgccaaatc ggcggtaacg aaagaggata aaccgtgtcc 2040
   cgtattatta tgctgatccc taccggaacc agcgtcggtc tgaccagcgt cagccttggc 2100
   gtgatccgtg caatggaacg caaaggcgtt cgtctgagcg ttttcaaacc tatcgctcag 2160
   ccgcgtaccg gtggcgatgc gcccgatcag actacgacta tcgtgcgtgc gaactcttcc 2220
   accacgacgg ccgctgaacc gctgaaaatg agctacgttg aaggtctgct ttccagcaat 2280
   cagaaagatg tgctgatgga agagatcgtc gcaaactacc acgctaacac caaagacgct 2340
   gaagtcgttc tggttgaagg tctggtcccg acacgtaagc accagtttgc ccagtctctg 2400
   aactacgaaa tcgctaaaac gctgaatgcg gaaatcgtct tcgttatgtc tcagggcact 2460
<210> 36
   <211> 1000
   <212> DNA
   <213> Artificial Sequence
<220>
   <223> Sequence of the ?deleted poxB region;
<400> 36
   gcggcccggc tccgtatatg gattgggtag agcaggaagt gaaagcgctc ggcgtgacgc      60
   gtttctttaa agagaaattc ttcaccccag tagcggaagc agcgaccagc ggtctgaaat     120
   tcaccaaact gcaaccggca cgagaatttt acgccccggt tggcaccacg ctactggagg     180
   cgctggaaag caataacgtt ccggttgtcg ccgcctgccg tgcgggtgtt tgcggctgct     240
   gtaagacaaa agtggtttcc ggtgaatata cggtgagcag cacaatgacg ctgaccgacg     300
   ccgaaatcgc tgaaggttac gtactggcct gctcctgcca tccgcagggg gatttggttc     360
   tcgcataatc gccttatgcc cgatgatatt cctttcatcg ggctatttaa ccgttagtgc     420
   ctcctttctc tcccatccct tccccctccg tcagatgaac taaacttgtt accgttatca     480
   cattcaggag atggagaacc aaagggtggc atttcccgtc ataataagga catgccatga     540
   ttgatttacg cagtgatacc gttacccgac cgagccgcgc catgctcgaa gcaatgatgg     600
   ccgccccggt tggggacgac gtttacggag acgaccctac cgttaatgct ctgcaggact     660
   acgccgcaga gctttccggt aaagaagccg ccatttttct gccgaccggc actcaggcca     720
   acctggtcgc tctgctcagt cactgcgaac gtggcgaaga gtatattgtc ggtcaggccg     780
   cgcataacta tctgtttgaa gccggtggcg cagcggtgct gggcagtatt cagccgcaac     840
   ccatcgacgc ggctgccgac ggcacgctac cgctggataa agtggcgatg aaaatcaaac     900
   ccgacgatat ccatttcgcc cgcaccaaat tactcagtct ggaaaacacc cacaacggca     960
   aagtgctgcc gcgtgaatac ctgaaagaag catgggaatt                          1000
<210> 37
   <211> 3392
   <212> DNA
   <213> Artificial Sequence
<220>
   <223> tktA cassette at the deleted poxB locus;
<400> 37
   gcggcccggc tccgtatatg gattgggtag agcaggaagt gaaagcgctc ggcgtgacgc 60
   gtttctttaa agagaaattc ttcaccccag tagcggaagc agcgaccagc ggtctgaaat 120
   tcaccaaact gcaaccggca cgagaatttt acgccccggt tggcaccacg ctactggagg 180
   cgctggaaag caataacgtt ccggttgtcg ccgcctgccg tgcgggtgtt tgcggctgct 240
   gtaagacaaa agtggtttcc ggtgaatata cggtgagcag cacaatgacg ctgaccgacg 300
   ccgaaatcgc tgaaggttac gtactggcct gctcctgcca tccgcagggg gatttggttc 360
   tcgcataatc gccttatgcc cgatgatatt cctttcatcg ggctatttaa ccgttagtgc 420
   ctcctttctc tcccatccct tccccctccg tcagatgaac taaacttgtt accgttatca 480
   cattcaggag atggagaacc aaggaaaagc gcaacggacg ggcgagtaga ttgcgcaaca 540
   tgcgagcatg atccagagat ttctgaagca gcaaaaggat gttccatgta catgacgcgc 600
   ggcttgcggt aaattgttgg caaattttcc ggcgtagccc aaaacgcgct gtcgtcaagt 660
   cgttaagggc gtgcccttca tcatccgatc tggagtcaaa atgtcctcac gtaaagagct 720
   tgccaatgct attcgtgcgc tgagcatgga cgcagtacag aaagccaaat ccggtcaccc 780
   gggtgcccct atgggtatgg ctgacattgc cgaagtcctg tggcgtgatt tcctgaaaca 840
   caacccgcag aatccgtcct gggctgaccg tgaccgcttc gtgctgtcca acggccacgg 900
   ctccatgctg atctacagcc tgctgcacct caccggttac gatctgccga tggaagaact 960
   gaaaaacttc cgtcagctgc actctaaaac tccgggccac ccggaagtag gttataccgc 1020
   tggtgtggaa accaccaccg gtccgctggg tcagggtatt gccaacgcag tcggtatggc 1080
   gattgcagaa aaaacgctgg cggcgcagtt taaccgtcca ggtcacgaca ttgtcgacca 1140
   ctacacctac gccttcatgg gcgacggctg catgatggaa ggcatctccc acgaagtttg 1200
   ctctctggcg ggtacgctga agctgggtaa actgattgcg ttctacgatg acaacggtat 1260
   ctcaatcgat ggtcacgttg aaggctggtt cactgacgac accgcaatgc gtttcgaagc 1320
   ttacggctgg cacgttattc gcgacatcga cggtcatgac gcggcatcca tcaaacgcgc 1380
   agtagaagaa gcgcgcgcag tgactgacaa accgtccctg ctgatgtgca aaaccatcat 1440
   cggtttcggt tccccgaaca aagccggtac ccacgactcc cacggtgcgc cgctgggcga 1500
   cgctgaaatt gccctgaccc gcgaacagct gggctggaaa tacgcgccgt tcgaaatccc 1560
   gtctgaaatc tatgctcagt gggatgcgaa agaagcaggc caggcgaaag aatctgcatg 1620
   gaatgagaag tttgcggctt acgcgaaagc ttatccgcag gaagcggctg aatttacccg 1680
   ccgtatgaaa ggcgaaatgc cgtctgactt cgacgccaaa gcgaaagagt ttatcgctaa 1740
   actgcaggct aatccggcga aaatcgccag ccgtaaagcg tcgcagaatg ctatcgaagc 1800
   gttcggcccg ctgttgcctg aattcctcgg cggctctgct gacctggcac cgtctaacct 1860
   gaccctgtgg tctggttcta aagcaatcaa cgaagatgct gcaggtaact acatccacta 1920
   cggtgttcgc gagttcggta tgaccgcgat tgctaacggt atctccctgc acggtggttt 1980
   cctgccgtac acctccacct tcctgatgtt cgtggaatac gcacgtaacg ccgtacgtat 2040
   ggctgcgctg atgaaacagc gtcaggtgat ggtttacacc cacgactcca tcggtctggg 2100
   cgaagatggc ccgactcacc agccggttga gcaggtcgct tctctgcgcg tgaccccgaa 2160
   catgtctaca tggcgtccgt gtgaccaggt tgaatccgcg gtcgcgtgga aatacggcgt 2220
   tgagcgtcag gacggcccga ctgcgcttat cctctcccgt cagaacctgg cgcagcagga 2280
   acgaactgaa gagcaactgg caaacatcgc gcgcggtggt tatgtgctga aagactgcgc 2340
   cggtcagccg gaactgattt tcatcgctac cggttcagaa gttgaactgg ctgttgctgc     2400
   ctacgaaaaa ctgactgccg aaggcgtgaa agcgcgcgtg gtgtccatgc cgtctaccga     2460
   cgcatttgac aagcaggatg ctgcttaccg tgaatccgta ctgccgaaag cggttactgc     2520
   acgcgttgct gtagaagcgg gtattgctga ctactggtac aagtatgttg gcctgaacgg     2580
   tgctatcgtc ggtatgacca ccttcggtga atctgctccg gcagagctgc tgtttgaaga     2640
   gttcggcttc actgttgata acgttgttgc gaaagcaaaa gaactgctgt aattagcatt     2700
   tcgggtaaaa aggtcgcttc ggcgaccttt tttattacct tgatatgtcc gtttgcggac     2760
   aagcaataga taaagcgtgt tgtagatcac aaatatttat atgcaataaa tatcaattat     2820
   gtaatatgca tcacgatatg cgtattgaca tttgttgtta taactataac tcaatgttat     2880
   ataagaaatt aaaaagggtg gcatttcccg tcataataag gacatgccat gattgattta     2940
   cgcagtgata ccgttacccg accgagccgc gccatgctcg aagcaatgat ggccgccccg     3000
   gttggggacg acgtttacgg agacgaccct accgttaatg ctctgcagga ctacgccgca     3060
   gagctttccg gtaaagaagc cgccattttt ctgccgaccg gcactcaggc caacctggtc     3120
   gctctgctca gtcactgcga acgtggcgaa gagtatattg tcggtcaggc cgcgcataac     3180
   tatctgtttg aagccggtgg cgcagcggtg ctgggcagta ttcagccgca acccatcgac     3240
   gcggctgccg acggcacgct accgctggat aaagtggcga tgaaaatcaa acccgacgat     3300
   atccatttcg cccgcaccaa attactcagt ctggaaaaca cccacaacgg caaagtgctg     3360
   ccgcgtgaat acctgaaaga agcatgggaa tt                                   3392
<210> 38
   <211> 1045
   <212> DNA
   <213> Artificial Sequence
<220>
   <223> ptsHI deletion sequence;
<400> 38
   gaagatgaaa gctttaccaa caagaatatt gtggttattc taccatcatc gggtgagcgt 60
   tatttaagca ccgcattgtt tgccgatctc ttcactgaga aagaattgca acagtaatgc 120
   cagcttgtta aaaatgcgta aaaaagcacc tttttaggtg cttttttgtg gcctgcttca 180
   aactttcgcc cctcctggca ttgattcagc ctgtcggaac tggtatttaa ccagactaat 240
   tattttgatg cgcgaaatta atcgttacag gaaaagccaa agctgaatcg attttatgat 300
   ttggttcaat tcttccttta gcggcataat gtttaatgac gtacgaaacg tcagcggtca 360
   acacccgcca gcaatggact gtattgcgct cttcgtgcgt cgcgtctgtt aaaaactggc 420
   gctaacaata caggctaaag tcgaaccgcc aggctagact ttagttccac aacactaaac 480
   ctataagttg gggaaataca atgttccagc aagaagttac cattaccgct ccgacaatct 540
   gctaatccac gagatgcggc ccaatttact gcttaggaga agatcatggg tttgttcgat 600
   aaactgaaat ctctggtttc cgacgacaag aaggataccg gaactattga gatcattgct 660
   ccgctctctg gcgagatcgt caatatcgaa gacgtgccgg atgtcgtttt tgcggaaaaa 720
   atcgttggtg atggtattgc tatcaaacca acgggtaaca aaatggtcgc gccagtagac 780
   ggcaccattg gtaaaatctt tgaaaccaac cacgcattct ctatcgaatc tgatagcggc 840
   gttgaactgt tcgtccactt cggtatcgac accgttgaac tgaaaggcga aggcttcaag 900
   cgtattgctg aagaaggtca gcgcgtgaaa gttggcgata ctgtcattga atttgatctg 960
   ccgctgctgg aagagaaagc caagtctacc ctgactccgg ttgttatctc caacatggac 1020
   gaaatcaaag aactgatcaa actgt 1045
<210> 39
   <211> 4595
   <212> DNA
   <213> Artificial Sequence
<220>
   <223> glf-glk cassette integrated at the deleted tdc locus;
<400> 39
   ctgatttctt tgtcgctgat cccttactgg aactctgcag ttatcgacca ggttgacctc      60
   ggttcgctgt cgttaaccgg tcatgacggt atcctgatca ctgtctggct ggggatttcc     120
   atcatggttt tctcctttaa cttctcgcca atcgtctctt ccttcgtggt ttctaaacgt     180
   gaagagtatg agaaagactt cggtcgcgac ttcaccgaac gtaaatgttc ccaaatcatt     240
   tctcgtgcca gcatgctgat ggttgcagtg gtgatgttct ttgcctttag ctgcctgttt     300
   actctgtctc cggccaacat ggcggaagcc aaagcgcaga atattccagt gctttcttat     360
   ctggctaacc actttgcgtc catgaccggt accaaaacaa cgttcgcgat tacactggaa     420
   tatgcggctt ccatcatcgc actcgtggct atcttcaaat ctttcttcgg tcactatctg     480
   gggacgctgg aaggcttgaa tggtctgatt ctgaagttcg gttataaagg tgacaaaacc     540
   aaagtgtcgc tgggtaaact gaatactctc agcatgatct tcatcatggg ctccacctgg     600
   gttgttgcct acgccaaccc gaacatcctc gacctgattg aagccatggg cgcaccgatt     660
   atcgcatccc tgctgtgcct gttgccgatg tatgccatcc gtaaagcgcc gtctctggcg     720
   aaataccgtg gtcgtctgga taacgtgttt gttaccgtga ttggtctgct gaccatcctg     780
   aacatcgtat acaaactgtt ttaatccgta actcaggatg agaaaagaga tgaatgaatt     840
   tccggttgtt ttggttatta actgtggttc gtcttcgatt aagttttccg tactcgatgc     900
   cagcgactgt gaagtattaa tgtcaggtat tgccgacggt attaactcgg aaaatgcatt     960
   cttatccgta aatgggggag agccagcacc gctggctcac cacagctacg aaggtgcatt    1020
   gaaggcaatt gcatttgaac tggaaaaacg gagtttaaat gacagcgtgg ccttaattgg    1080
   tgcaaaagtg gctgtgactg taaaaagaaa tcgaaaaaga ccgttttgtg tgaaaacggt    1140
   ctttttgttt ccttttaacc aactgccata actcgaggcc tacctagctt ccaagaaaga    1200
   tatcctaaca gcacaagagc ggaaagatgt tttgttctac atccagaaca acctctgcta    1260
   aaattcctga aaaattttgc aaaaagttgt tgactttatc tacaaggtgt ggtataataa    1320
   tcttaacaac agcaggacgc tctagaggga gaggaatcgc catgagttct gaaagtagtc    1380
   agggtctagt cacgcgacta gccctaatcg ctgctatagg cggcttgctt ttcggttacg    1440
   attcagcggt tatcgctgca atcggtacac cggttgatat ccattttatt gcccctcgtc    1500
   acctgtctgc tacggctgcg gcttcccttt ctgggatggt cgttgttgct gttttggtcg    1560
   gttgtgttac cggttctttg ctgtctggct ggattggtat tcgcttcggt cgtcgcggcg    1620
   gattgttgat gagttccatt tgtttcgtcg ccgccggttt tggtgctgcg ttaaccgaaa    1680
   aattatttgg aaccggtggt tcggctttac aaattttttg ctttttccgg tttcttgccg    1740
   gtttaggtat cggtgtcgtt tcaaccttga ccccaaccta tattgctgaa attgctccgc    1800
   cagacaaacg tggtcagatg gtttctggtc agcagatggc cattgtgacg ggtgctttaa    1860
   ccggttatat ctttacctgg ttactggctc atttcggttc tatcgattgg gttaatgcca    1920
   gtggttggtg ctggtctccg gcttcagaag gcctgatcgg tattgccttc ttattgctgc    1980
   tgttaaccgc accggatacg ccgcattggt tggtgatgaa gggacgtcat tccgaggcta    2040
   gcaaaatcct tgctcgtctg gaaccgcaag ccgatcctaa tctgacgatt caaaagatta    2100
   aagctggctt tgataaagcc atggacaaaa gcagcgcagg tttgtttgct tttggtatca    2160
   ccgttgtttt tgccggtgta tccgttgctg ccttccagca gttagtcggt attaacgccg    2220
   tgctgtatta tgcaccgcag atgttccaga atttaggttt tggagctgat acggcattat    2280
   tgcagaccat ctctatcggt gttgtgaact tcatcttcac catgattgct tcccgtgttg    2340
   ttgaccgctt cggccgtaaa cctctgctta tttggggtgc tctcggtatg gctgcaatga    2400
   tggctgtttt aggctgctgt ttctggttca aagtcggtgg tgttttgcct ttggcttctg    2460
   tgcttcttta tattgcagtc tttggtatgt catggggccc tgtctgctgg gttgttctgt    2520
   cagaaatgtt cccgagttcc atcaagggcg cagctatgcc tatcgctgtt accggacaat    2580
   ggttagctaa tatcttggtt aacttcctgt ttaaggttgc cgatggttct ccagcattga    2640
   atcagacttt caaccacggt ttctcctatc tcgttttcgc agcattaagt atcttaggtg    2700
   gcttgattgt tgctcgcttc gtgccggaaa ccaaaggtcg gagcctggat gaaatcgagg    2760
   agatgtggcg ctcccagaag tagttaaact tgctttggct gaatcctttt gtctttttta     2820
   gataagtctt aaccaattat actttttgtt tacaacgatg gtataaagcg ggcggactta    2880
   ttttacctgt tgggtagcct tctgatttca gaaaggaatt attatggaaa ttgttgcgat 2940
   tgacatcggt ggaacgcatg cgcgtttctc tattgcggaa gtaagcaatg gtcgggttct 3000
   ttctcttgga gaagaaacaa cttttaaaac ggcagaacat gctagcttgc agttagcttg 3060
   ggaacgtttc ggtgaaaaac tgggtcgtcc tctgccacgt gccgcagcta ttgcatgggc 3120
   tggcccggtt catggtgaag ttttaaaact taccaataac ccttgggtat taagaccagc 3180
   tactctgaat gaaaagctgg acatcgatac gcatgttctg atcaatgact tcggcgcggt 3240
   tgcccacgcg gttgcgcata tggattcttc ttatctggat catatttgtg gtcctgatga 3300
   agcgcttcct agcgatggtg ttatcactat tcttggtccg ggaacgggct tgggtgttgc 3360
   ccatctgttg cggactgaag gccgttattt cgtcatcgaa actgaaggcg gtcatatcga 3420
   ctttgctccg cttgacagac ttgaagacaa aattctggca cgtttacgtg aacgtttccg 3480
   ccgcgtttct atcgaacgca ttatttctgg cccgggtctt ggtaatatct acgaagcact 3540
   ggctgccatt gaaggcgttc cgttcagctt gctggatgat attaaattat ggcagatggc 3600
   tttggaaggt aaagacaacc ttgctgaagc cgctttggat cgcttctgct tgagccttgg 3660
   cgctatcgct ggtgatcttg ctttggcaca gggtcgaacc agtgttgtta ttggcggtgg 3720
   tgtcggtctt cgtatcgctt cccatttgcc agaatctggt ttccgtcagc gctttgtttc 3780
   aaaaggacgc tttgaacgcg tcatgtccaa gattccggtt aagttgatta cttatccgca 3840
   gcctggactg ttgggtgcgc agctgcctat gccaacaaat attctgaagt tgaataatat 3900
   tttttaatat tatgaactga atttaagagg ctgccttccg ataaaatcgg gaggtggcct 3960
   tttttatatt ttttactaaa aaatgaagac aaaaaagtct taagtaagaa taatattatt 4020
   attaactttt gatatatttt gtattagtgg atccgccctc ccgctggaaa ttgaagccat 4080
   cgcagtacgt agtgcgtaaa gcctcgtgag cgggacggtc gtaaggtcgt tccgctccac 4140
   ttcactgaac ggcaatccga gggtgtggat atgattagtg cattcgatat tttcaaaatt 4200
   gggattggtc cctccagttc gcataccgtg gggccaatga atgccggaaa aagttttatt 4260
   gatcggctgg aaagtagcgg cttattaacc gcgacgagcc atattgtggt cgatctgtac 4320
   gggtcgttgt cactgacggg caaaggccat gccacggatg tcgccatcat catgggactg 4380
   gcaggaaaca gtccgcagga tgttgtcatt gatgagatcc ctgcatttat agagttagta 4440
   acgcgcagcg ggcggctgcc agtggcatct ggtgcgcata ttgttgattt tcctgtagca 4500
   aagaacatta tcttccatcc cgaaatgttg cctcgccatg agaacggaat gcggatcact 4560
   gcctggaagg gacaggaaga gctattaagt aaaac 4595
<210> 40
   <211> 1069
   <212> DNA
   <213> Artificial Sequence
<220>
   <223> galP deletion region;
<400> 40
   actttggtcg tgaacatttc ccgtgggaaa aaaccgacaa agcgcagctg ctgcgcgatg        60
   ctgccggtct gaagtaatct ttcttcacct gcgttcaaag gccagcctcg cgctggcctt       120
   tttcttttgg ataggcgttc acgccgcatc cggcaaaaaa accgcccgca caataacatc       180
   attcttcctg atcacgtttc accgcagatt atcatcacaa ctgaaaccga ttacaccaac       240
   cacaacagac aaagatttgt aatattttca tattattatt cggttttcac agttgttaca       300
   tttcttttca gtaaagtctt aattgcagat aacagcgttt aatctatgat gatataactc       360
   aattattttc atgcacttaa atcataacta agataaatgt tagtgtaagc gattacactg       420
   atgtgatttg cttcacatct ttttacgtcg tactcaccta tcttaattca caataaaaaa       480
   taaccatatt ggagggcatc atgcctgacg ctaaaaaaca ggggcggtca aacaaggcaa       540
   tgacgtttga aataggcgct cacgattaat ctccccaagc ttcctcccat cgcggaggaa       600
   gccacctctt gcagtcatct tttcttcgct ctatcctctg ccgctatgaa aacatcccgt       660
   ctccctatcg ccatccaaca ggccgttatg cgtcgcctgc gggaaaaact cgcccaggcc       720
   aacctgaagc tagggcgtaa ctacccggag ccaaaactct cttacaccca gcgcggaacc       780
   tccgccggaa cggcctggct ggaaagctat gaaattcgcc tcaatcccgt tttgctgttg       840
   gaaaacagtg aagcttttat tgaagaagtg gtaccgcacg aactggcaca tttgctggta       900
   tggaaacatt tcggccgcgt agcgccacat ggcaaagagt ggaagtggat gatggaaaac       960
   gtgctgggtg ttcccgcccg tcgtacgcat cagttcgaac tgcaatccgt gcgtcgcaac      1020
   accttcccct accgctgcaa gtgccaggag catcagctta ccgtacgcc                  1069
<210> 41
   <211> 6100
   <212> DNA
   <213> Artificial Sequence
<220>
   <223> P15-catAX +PR-aroY+P26-quiC cassette at adhE locus;
<400> 41
   ttgattttca taggttaagc aaatcatcac cgcactgact atactctcgt attcgagcag        60
   atgatttact aaaaaagttt aacattatca ggagagcatt agcttgctat tgacgacagc       120
   tatggttcac tgtccaccaa ccaaaactgt gctcagtacc gccaatattt ctcccttgag       180
   gggtacaaag aggtgtccct agaagagatc cacgctgtgt aaaaatttta caaaaaggta       240
   ttgactttcc ctacagggtg tgtaataatt taattacagg cgggggcaac cccgcctgtt       300
   ctgcagagga ggaatatagc catggaagtg aaaatcttca acacccagga tgttcaggat       360
   tttctgcgtg ttgcaagcgg tctggaacaa gagggtggta atccgcgtgt taaacaaatt 420
   attcatcgtg ttctgagcga cctgtataaa gcaattgaag atctgaatat caccagcgac 480
   gaatattggg caggcgttgc atatctgaat cagctgggtg caaatcaaga agcaggtctg 540
   ctgagtccgg gtctgggttt tgatcattat ctggatatgc gtatggatgc agaagatgca 600
   gcactgggta ttgaaaatgc aacaccgcgt accattgaag gtccgctgta tgttgcgggt 660
   gcaccggaaa gcgttggtta tgcacgcatg gatgatggta gcgatccgaa tggtcatacc 720
   ctgattctgc atggcaccat ttttgatgca gatggtaaac cgctgccgaa tgcaaaagtt 780
   gaaatttggc atgcaaacac caaaggcttt tatagccatt ttgatccgac cggtgaacag 840
   caggccttta atatgcgtcg tagcattatt accgatgaga atggtcagta tcgtgttcgt 900
   accattctgc ctgccggtta tggttgtcct ccggaaggtc cgacccagca actgctgaac 960
   caactgggtc gtcatggtaa tcgtccggca catattcatt attttgttag cgcagatggt 1020
   caccgtaaac tgaccaccca gattaatgtt gccggtgatc cgtataccta tgatgatttt 1080
   gcatatgcca cccgtgaagg tctggttgtt gatgcagttg aacataccga tccggaagca 1140
   attaaagcca atgatgtgga aggtcctttt gccgaaatgg tgtttgatct gaaactgacc 1200
   cgtctggttg atggtgttga taatcaggtt gtggatcgtc cgcgtctggc agtttaatac 1260
   accaaaatgg ttcaaaatta tcaggcgagt gatcatgatc actggcctgt ttttatttca 1320
   gggaagggtg gagacaatta cgtggataat cagatcatcc aagaaaccgt ggataaaatt 1380
   ctgagcgttc tgccgaatca ggcaggtcag ctggcacgtc tggtgcgtct gatgcaattt 1440
   gcatgcgatc cgaccattac cgttattggc aaatataacc atggtaaaag ccgtctgctg 1500
   aatgaactga ttggcaccga tatctttagc gttgcagata aacgtgaaac cattcagctg 1560
   gccgaacata aacaggatca ggttcgttgg ctggatgcac ctggtctgga tgccgatgtt 1620
   gcagcagttg atgatcgtca tgcatttgaa gcagtttgga cccaggcaga tattcgtctg 1680
   tttgttcata gcgttcgtga aggtgaactg gatgcaaccg aacaccatct gctgcaacag 1740
   ctgattgaag atgccgatca tagccgtcgt cagaccattc tggttctgac ccagattgat 1800
   cagattccgg atcagaccat cctgacacag attaaaacca gcattgcaca gcaggttccg 1860
   aaactggata tttgggcagt tagcgcaacc cgtcatcgtc agggcattga aaacggtaaa 1920
   accctgctga tcgaaaaaag cggtattggt gcactgcgcc ataccctgga acaggcactg 1980
   gcacaggtgc cgagcgcacg tacctatgaa aaaaatcgtc tgctgtcaga tctgcaccat 2040
   cagctgaaac aactgctgct ggatcagaaa catgttctgc aacaactgca acagacacag 2100
   caacagcagc tgcatgattt tgataccggt ctgattaaca ttctggacaa aattcgtgtt 2160
   gatctggaac cgattgtgaa tattgatggt caggatcaag cactgaatcc ggatagcttt 2220
   gcaaccatgt ttaaaaacac cgcagcaaaa cagcagcgtg ccaaagttca gattgcatat 2280
   agccgtgcat gcattgaaat caacagccat ctgattcgcc atggtgttgt tggtctgcct 2340
   gcggaacagc agaccaccat taaaagcatt gataccgtga ttgttgccgt gtttggtatc 2400
   agcgttaaat ttcgtgatca gctgcgtgcc ctgttttata ccgataccga acgtcagcgt 2460
   ctgcaacgtg aatttcgttt ctattttgaa aaaagtgccg gtcgcatgat tctggcagca 2520
   aaaattgaac agaccatgcg tcagcagggc tgtattcaga atgccatgat ggcactgcaa 2580
   caaatggaaa gcgcagcata aaaacacgga cgccgcaaac ggcgtccgaa tttcttggtc 2640
   gaccgttaaa tctatcaccg caagggataa atatctaaca ccgtgcgtgt tgactatttt 2700
   acctctggcg gtgataatgg ttgcatgtac taatctagat aaggaatata gccatgaccg 2760
   caccgattca ggatctgcgt gatgcaattg ccctgctgca acagcatgat aatcagtatc 2820
   tggaaaccga tcatccggtt gatccgaatg cagaactggc aggcgtttat cgtcatattg 2880
   gtgccggtgg caccgttaaa cgtccgaccc gtattggtcc ggcaatgatg tttaataaca 2940
   ttaaaggtta tccgcacagc cgtattctgg ttggtatgca tgcaagccgt cagcgtgcag 3000
   cactgctgct gggttgtgaa gcaagtcagc tggcactgga agttggtaaa gcagttaaaa 3060
   aaccggttgc accggtggtt gttccggcaa gcagcgcacc gtgtcaagag cagatttttc 3120
   tggcagatga tccggatttt gatctgcgta ccctgctgcc tgcacatacc aataccccga 3180
   ttgatgcagg tccgtttttt tgtctgggtc tggccctggc aagcgatccg gtggatgcaa 3240
   gcctgaccga tgttaccatt catcgtctgt gtgttcaggg tcgtgatgaa ctgagcatgt 3300
   tcctggcagc aggtcgccat attgaagttt ttcgtcagaa agcagaagca gcaggtaaac 3360
   cgctgccgat taccattaat atgggtctgg acccagcaat ctatattggc gcatgttttg 3420
   aagcaccgac caccccgttt ggttataatg aactgggtgt tgccggtgca ctgcgtcagc 3480
   gtccggttga actggttcag ggtgttagcg ttccggaaaa agcaattgca cgtgccgaaa 3540
   ttgttattga aggtgaactg ctgcctggtg ttcgtgttcg tgaagatcag cataccaatt 3600
   caggtcatgc aatgccggaa tttccgggtt attgtggtgg tgcaaatccg agcctgccgg 3660
   ttattaaagt taaagccgtt accatgcgca ataacgcaat tctgcaaacc ctggttggtc 3720
   cgggtgaaga acataccacc ctggcaggtc tgccgaccga agcaagcatt tggaatgcag 3780
   ttgaagcagc aattccgggt tttctgcaaa atgtttatgc ccataccgca ggcggtggta 3840
   aatttctggg tattctgcaa gtgaaaaaac gtcagcctgc cgatgaaggt cgtcagggtc 3900
   aggcagccct gctggcgctg gcaacctata gcgaactgaa aaatatcatt ctggtggatg 3960
   aggatgtgga catttttgat agtgatgata ttctgtgggc aatgaccacc cgtatgcagg 4020
   gtgatgttag cattaccacc attccgggta ttcgcggtca tcagctggac ccgagccaga 4080
   caccggaata ttcaccgagc attcgtggta atggtattag ctgcaaaacc atctttgatt 4140
   gtaccgttcc gtgggcactg aaaagccatt ttgaacgtgc accgtttgca gatgttgatc 4200
   cgcgtccgtt tgcacctgaa tattttgcac gtctggaaaa aaatcagggc agcgcaaaat 4260
   aagctaataa caggcctgct ggtaatcgca ggaattttta tttggatgga tccgcctacc 4320
   tagcttccaa gaaagatatc ctaacagcac aagagcggaa agatgttttg ttctacatcc 4380
   agaacaacct ctgctaaaat tcctgaaaaa ttttgcaaaa agttgttgac tttatctaca 4440
   aggtgtggta taataatctt aacaacagca ggacgctccc gggttgagga aaacctaatg 4500
   aaactgacca gcctgcgtgt tagcctgctg gcactgggtc tggttaccag cggttttgca 4560
   gcagcagaaa cctataccgt tgatcgttat caggatgata gcgaaaaagg tagcctgcgt 4620
   tgggcaattg aacagagcaa tgcaaatagc gcacaagaaa accagattct gattcaggca 4680
   gttggtaaag caccgtatgt tatcaaagtt gataaaccgc tgcctccgat taaaagcagc 4740
   gttaaaatca ttggcaccga gtgggataaa accggtgaat ttattgcaat tgatggcagc 4800
   aactatatca aggcgaaggt tgaaaaagca tgtccgggtg caaatccggg tcagtatggc 4860
   accaatgttc gtaccatgac cctgcctggt ctggttctgc aagatgttaa tggtgttacc 4920
   ctgaaaggtc tggatgttca tcgtttttgt attggtgttc tggttaatcg cagcagcaat 4980
   aacctgattc agcataatcg tatcagcaac aattatggtg gtgccggtgt tatgattacc 5040
   ggtgatgatg gtaaaggtaa tccgaccagc accaccacca ataataacaa agttctggat 5100
   aacgtgttca tcgataatgg tgatggtctg gaactgaccc gtggtgcagc atttaatctg 5160
   attgcaaata acctgtttac cagcacaaaa gccaatccgg aaccgagcca gggtattgaa 5220
   attctgtggg gtaatgataa tgccgtggtg ggtaacaaat tcgaaaacta ttcagatggc 5280
   ctgcaaatca attggggtaa acgtaactat atcgcctata acgaactgac caataacagc 5340
   ctgggtttca atctgacagg tgatggtaac attttcgaca gcaataaagt gcatggtaac 5400
   cgtattggta ttgccattcg tagtgaaaaa gatgccaatg cacgtattac cctgaccaaa 5460
   aatcagattt gggataacgg caaagatatc aaacgttgtg aagccggtgg tagctgtgtt 5520
   ccgaatcagc gtctgggtgc aattgttttt ggtgttccgg cactggaaca tgaaggtttt 5580
   gttggtagcc gtggcggtgg tgttgttatt gaaccggcaa aactgcaaaa aacctgcacc 5640
   cagccgaacc agcagaattg taatgcaatt cctaatcagg gtattcaggc accgaaactg 5700
   acagttagca aaaaacagct gaccgttgaa gttaaaggca cccctaatca gcgttataat 5760
   gtggaatttt ttggcaatcg taatgccagc agcagcgaag cagaacagta tctgggtagc 5820
   attgttgttg ttaccgatca tcagggtctg gcaaaagcaa attgggctcc gaaagttagc 5880
   atgccgagcg ttaccgcaaa tgtgacagat catctgggtg cgaccagcga actgagcagc 5940
   gcagttaaaa tgcgttaaat gcatgcgcgc cgcgttcgcg cggcgctttt ttttggtact 6000
   cagtagcgct gtctggcaac ataaacggcc ccttctgggc aatgccgatc agttaaggat 6060
   tagttgaccg atccttaaac tgaggcacta taacggcttc 6100
<210> 42
   <211> 14
   <212> DNA
   <213> Artificial Sequence
<220>
   <223> RBS
<400> 42
   cggagtatta catg 14
<210> 43
   <211> 14
   <212> DNA
   <213> Artificial Sequence
<220>
   <223> mutated RBS
<400> 43
   caaagtatta catg 14
<210> 44
   <211> 14
   <212> DNA
   <213> Artificial Sequence
<220>
   <223> mutated RBS
<400> 44
   caatgtatta catg 14
<210> 45
   <211> 14
   <212> DNA
   <213> Artificial Sequence
<220>
   <223> mutated RBS
<400> 45
   caatatatta catg 14

## Description

The present invention is in the field of producing renewable chemical feedstocks using biocatalysts that have been genetically engineered to increase their ability to convert renewable carbon resources into useful compounds. More specifically, the present invention provides a genetically engineered *Escherichia coli* in which the PEP-dependent phosphotransferase system and the GalP-based system for glucose import are eliminated, and which comprises an exogenous glucose facilitator system comprising a protein encoded by an exogenous *glf* gene.

Adipic acid is a large volume chemical used in the manufacture of Nylon 66. Adipic acid is currently made from petrochemicals, but the synthesis is not environmentally friendly (Niu et al., 2002). Alternatively, adipic acid can be made from any of the three isomers of muconic acid (cis, cis; cis, trans; trans, trans) by chemical hydrogenation. It would be desirable to produce muconic acid from renewable resources by fermentation with a microorganism, followed by hydrogenation to adipic acid, since such a route to adipic acid would be more environmentally friendly than the traditional petrochemical route.

Current efforts towards microbial production of muconic acid can be grouped under three categories namely: (1) An aromatic degradation pathway for muconic acid production, in which various aromatic compounds are fed, and the benzene ring portion of aromatic compounds are oxidatively cleaved open; (2) A muconate buildup pathway, in which the muconic acid backbone is built up from various C2, C3, C4, compounds or lysine; and (3) An aromatic amino acid biosynthetic muconic acid pathway, in which muconic acid is built from 3-dehydroshikimate, an intermediate in the aromatic amino acid biosynthetic pathway in many organisms.

Many microorganisms are capable of degrading aromatic compounds containing a benzene ring, such as phenol, catechol, and benzoic acid, using pathways that cleave the aromatic ring to give terminal or intermediate compounds that are non-aromatic compounds such as cis, cis-muconic acid, or 3-carboxy-cis, cis-muconic acid (Niu et al., 2002; Perez-Pantoja et al., 2008). In the past, a number of groups have attempted to exploit this ability of microbes in the production of cis, cis-muconic acid at the industrial level (Mizuno et al, 1988; Yoshikawa et al, 1990; Choi et al, 1997). In the late 1980s, Celgene Corporation of USA and Mitsubishi Chemical Industries of Japan were active in developing a process for manufacturing muconic acid from toluene and benzoic acid respectively, as evidenced by a number of granted United States and Japanese patents in this area.

A number of microbial organisms have been reported to produce cis, cis-muconic acid using toluene, benzoic acid, benzene or catechol. For example, with catechol as the source of carbon, cis, cis-muconic acid production can be achieved with an almost 100% molar conversion yield using a recombinant *E. coli* cells expressing the *catA* gene, which encodes the *Pseudomonas putida* mt-2 catechol 1,2-dioxygenase responsible for catalyzing ortho-clevage of catechol, as biocatalyst (Kaneko et al, 2011). Bioreactors for the continuous production of muconic acid using this system have been described.

This approach of microbial production of cis, cis-muconic acid using cyclic C6 carbon compounds never became a commercial reality; however, there has been continuous academic interest in understanding the functioning of the enzymes in the degradation pathway for muconic acid production in microbes.

A recently, published international patent application (WO 2011/017560) claims biocatalysts having a muconate pathway and a method for producing muconic acid using these biocatalysts. In brief, this published patent application discloses four different pathways for producing muconic acid. The first pathway for muconic acid production starts with succinyl-CoA and acetyl-CoA. The second pathway for muconic acid production begins with pyruvate and malonate semialdehyde. The third pathway for muconic acid production starts with pyruvate and succinic semialdehyde. The fourth pathway for muconic acid production starts with lysine. All these pathways for muconic acid production proposed in this patent application are based on computer modeling and it is yet to be seen whether such biocatalysts can ever be created with commercially acceptable productivity and yield for muconic acid.

A fermentation route to cis, cis-muconic acid using a genetically engineered *E*. *coli* system has been described in the scientific literature (Niu et al., 2002) and in patent literature (US 5,616,496; US 5,487,987; WO 2011/085311 A1), but the prior art processes need to be substantially improved with respect to titer, yield, and suitability for large scale commercial production in order to be economically attractive. There have been two reports of *Saccharomyces cerevisiae* yeasts that were genetically engineered to produce cis, cis-muconic acid from glucose, but the published titers were only 1.5 mg/l and 140 mg/l (Weber et al., 2012; Curran et al., 2012). At these titers, neither of these yeast processes would be attractive for commercial production. Described herein is a process for producing cis, cis-muconic acid or cis, trans-muconic acid, by fermentation that is substantially improved compared to the published processes well known in the art with respect to suitability for large scale commercial production.

One of the objectives of the invention disclosed herein is to produce cis, cis-muconic acid by fermentation of a microorganism starting from a renewable, non-aromatic carbon source, such as a sugar or other simple carbon compound that can be derived from photosynthetic plants, using a genetically engineered organism that is suitable for large scale commercial production.

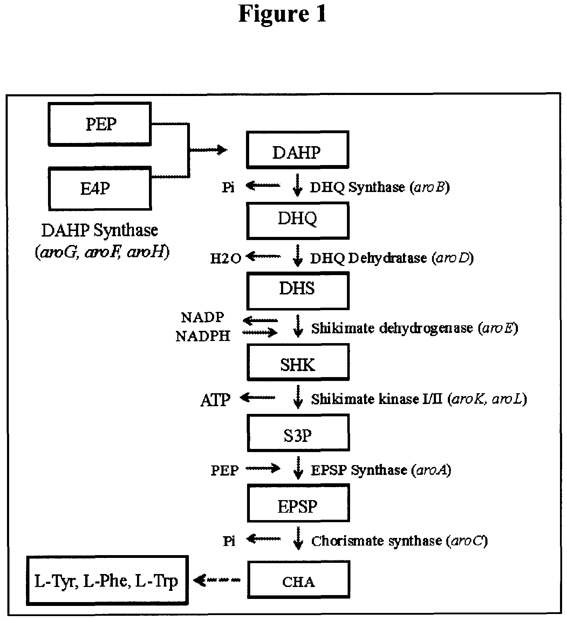

In 2002, Niu et al published a "benzene free" route to produce adipic acid that used a fermentation process to produce cis, cis-muconic acid, and then a catalytic chemical process to convert the cis, cis-muconic acid to adipic acid. This process was patented, but as far as the present inventors know, this process has not been commercialized, (US 5,487,987; US 5,616,496). The fermentation portion of this published process used genetically engineered strains of *E. coli,* the best of which was named WN1/pWN2.248. The pathway uses a portion of the native aromatic amino acid biosynthetic pathway (also known as the "shikimic acid pathway", the "shikimate pathway", the "chorismate pathway", the "common aromatic pathway", the "central aromatic pathway", or simply the "aromatic pathway", part of which is illustrated in Figure 1. In this specification, any biochemical step downstream from a carbon source fed to an organism, for example glucose, and which leads directly or indirectly to chorismate, is considered to be part of the shikimic acid pathway, including, for example, the steps catalyzed by, Glf, Glk, Zwf, TktA, TalB, and Pps. In addition, the published process uses three heterologous enzymes that convert 3-dehydroshikimate (an intermediate in the aromatic pathway) to cis, cis-muconic acid through the intermediates protocatechuate and catechol. The engineered host strain is a derivative *of E. coli* K-12 that has a genotype of *aroE353, serA::(aroB, aroZ), lacZ::(tktA, aroZ),* where *aroB* encodes 3-dehydroquinate synthase (hereinafter named AroB), *tktA* encodes transketolase, and *aroZ* is a heterologous gene from *Klebsiella pneumoniae* that encodes 3-dehydroshikimate dehydratase (hereinafter called "AroZ"). The engineered strain contains a multicopy plasmid, pWN2.248, which is derived from pBR322 and contains gene cassettes for expressing *catA, catX, aroY, aroF* (feedback resistant), *serA, lacI^{q},* and ampicillin resistance. The heterologous genes *catA* and *catX* were from *Acinetobacter calcoaceticus* and encode catechol 1,2-dioxygenase (hereinafter named "CatA"), and a protein ("CatX") of unknown function that might enhance CatA activity. In the literature, the *catX* gene is also called "orfl" (Neidle and Ornston, 1986). In this patent specification, we shall refer to the *catA* plus *catX* gene pair from *Acinetobacter* as *"catAX."* The heterologous gene *aroY* was from *Klebseilla pneumoniae* and encodes protocatechuate decarboxylase (hereinafter called "AroY").

A more recent patent application, related to the issued patents described above, has been published (WO 2011/085311 A1). In this application, the same strain as mentioned above, WN1/pWN2.248, was used to produce cis, cis-muconic acid, which was then isomerized into cis, trans-muconic acid,

However, the strain WN1/pWN2.248 is not well suited for large scale commercial production, so there is a need for a much improved process. The present invention provides improved biocatalysts for the fermentative production of cis, cis-muconic acid.

The process described in WO 2011/085311 A1 has several other features that make it impractical for implementation on a large commercial scale. The *aroE3*53 mutation that was included in the biocatalyst WN1/pWN2.248 used in the fermentative production of cis, cis-muconic acid functions to block flow of carbon into the lower part of the shikimate pathway so as to maximize flow into the desired pathway to cis, cis-muconic acid. However the *aroE* mutation is a "null" mutation (a mutation that renders the gene inactive for all practical purposes), which has the effect of turning the strain into an auxotroph for the aromatic amino acids (phenylalanine, tyrosine, and tryptophan) and aromatic vitamins or vitamin-like intermediates made from the shikimate pathway (p-hydroxy benzoic acid, p-amino benzoic acid, and 2,3-dihydroxy benzoic acid). The aromatic amino acids are relatively expensive, and their requirement would add a large burden to the cost of producing cis, cis-muconic acid. Thus, there is also a need for a process that does not require these expensive nutrients to be added to the growth medium.

Yet another problem associated with the currently available biocatalyst for the production of cis, cis-muconic acid is related to the need for maintaining a multicopy plasmid to express some of the necessary genes (Niu et al., 2002). Multicopy plasmids are often too unstable to be used in large scale industrial processes. Moreover, at least one of the genes on the plasmids is expressed from a promoter, P_{tac}, that requires either isopropylthiogalactoside (IPTG) or lactose for induction, and those two chemicals are too expensive to allow an economically attractive process. Thus there is a need for more stable strains that have expression cassettes stably integrated into the chromosome of the production strain, and there is a need for high levels of expression from constitutive promoters so as to alleviate the need for chemical inducers for the promoters.

The present invention relates to the following items:
1. A genetically engineered *Escherichia coli* in which the PEP-dependent phosphotransferase system and the GalP-based system for glucose import are eliminated, and which comprises an exogenous glucose facilitator system comprising a protein encoded by an exogenous *glf* gene.
2. A genetically engineered *Escherichia coli* as in item 1 further comprising a *pck* gene coding for phosphoenol pyruvate carboxykinase enzyme with increased activity.
3. A genetically engineered *Escherichia coli* as in item 1 further comprising a deletion in the *pykA* gene coding for pyruvate kinase enzyme activity.
4. A genetically engineered *Escherichia coli* as in item 1 further comprising a deletion in the *pykF* gene coding for pyruvate kinase enzyme activity.
5. A genetically engineered *Escherichia coli* as in item 1 further comprising one or more exogenous genes selected from the group consisting of *aroZ, qa-4, asbF, aroY, quiC* and *catAX,* wherein said one or more exogenous genes code for proteins functional in a muconic acid pathway.
6. A genetically engineered *Escherichia coli* as in item 1 further comprising one or more exogenous genes selected from the group consisting of *aroB, aroD, aroF, aroG, aroH, tktA, talB, rpe,* and *rpi,* wherein said one or more exogenous genes code for proteins functional in a shikimic acid pathway.
7. A genetically engineered *Escherichia coli* as in item 1 wherein the activity of a negative regulator protein of aromatic amino acid biosynthesis encoded by a *tyrR* gene or its homolog is substantially reduced or eliminated.
8. A genetically engineered *Escherichia coli* as in item 1 further comprising an *aroG** gene which codes for a DAHP synthase enzyme that is substantially resistant to inhibition by phenylalanine.
9. A genetically engineered *Escherichia coli* of item 8 further comprising an *aroG** gene that codes for a DAHP synthase enzyme that is selected from the group consisting of *aroG*20-893, aroG*20-897, aroG*20-899, aroG*20-901, aroG*111, aroG*211, aroG*212, aroG*311, aroG*312, aroG*411, aroG*412,* and *aroG*511.*
10. A genetically engineered *Escherichia coli* of item 1 further comprising an exogenous gene that codes for a QuiC enzyme of a bacterium of the genus *Acinetobacter,* or a homolog of said QuiC enzyme.

This present invention provides genetically engineered microorganisms that produce cis, cis-muconic acid starting from non-aromatic carbon sources. The genetically engineered microorganisms do not need to contain any exogenous plasmids in order to produce muconic acid, although they have certain exogenous or heterologous genes necessary to achieve the desired phenotype. The exogenous genes introduced into the microorganisms are stably integrated into the chromosomal DNA. As a result of this chromosomal DNA integration of the exogenous genes, the need for the use of antibiotics or other selective methods to maintain the plasmids carrying exogenous DNA is totally eliminated. In addition, strong promoters that do not require chemical inducers are used to express genes necessary for the pathway from carbon source, such as glucose, to cis, cis-muconic acid.

In one embodiment described herein, the activity of a negative regulator of aromatic amino acid biosynthesis is genetically manipulated. In one aspect, the activity of the negative regulator TyrR is substantially reduced by means of controlling the expression of the *tyrR* gene coding for the TyrR protein. In another aspect, the activity of the negative regulator TyrR is totally eliminated by means of deleting or inactivating the *tyrR* gene from the chromosomal DNA of the microorganism.

In another embodiment described herein, the feedback inhibition of certain enzymes in the aromatic amino acid pathway due to certain metabolites is overcome through genetic manipulations. In most wild type *E. coli* strains, deoxyarabino-heptulosonate 7-phosphate (DAHP) synthase occurs as three different isozymes which are known to be encoded by three different genes namely *aroG, aroF* and *aroH.* The proteins encoded by each of these three genes are subjected to feedback inhibition by one or more metabolites of shikimic acid pathway responsible for aromatic amino acid biosynthesis. In one aspect, the wild type *aroG* gene is replaced by a modified *aroG* gene which codes for an AroG protein that is resistant to feedback inhibition by one or more metabolites of the aromatic amino acid pathway within the microbial cell. In another aspect, the wild type *aroF* gene is replaced by an *aroF* gene which codes for an AroF protein that is resistant to feedback inhibition by one or more metabolites of the aromatic amino acid pathway within the microbial cell. In yet another aspect, the wild type *aroH* gene is replaced by an *aroH* gene which codes for an AroH protein that is resistant to feedback inhibition by one or more metabolites of the aromatic amino acid pathway within the microbial cell. In yet another embodiment described herein, the biocatalyst selected for the commercial production of cis, cis-muconic acid may have more than one feedback resistant isozyme for deoxyarabino-heptulosonate 7-phosphate (DAHP) synthase.

In another embodiment described herein, the activity of one or more of the enzymes involved in the flow of carbon through the aromatic amino acid pathway within the microbial cell is enhanced. In one aspect, the enhancement of the activity of one or more enzymes involved in the operation of an aromatic amino acid pathway and/or a muconic acid pathway is accomplished through genetic manipulation. In a preferred embodiment described herein, the expression of one or more of the genes coding for enzymes or proteins AroF, AroG, AroH, AroB, TktA, TalB, AroZ, QutC, qa-4, asbF, QuiC, AroY, Rpe, Rpi, Pps, CatA and CatX or their homologs or analogs are enhanced leading to the increased activity of said enzymes. Rpe is a ribulose-5-phosphate epimerase, Rpi is a ribulose-5-phosphate isomerase, and Pps is a phosphoenol pyruvate synthetase (Neidhardt and Curtiss, 1996). If the host strain is a yeast, for example *Saccharomyces cerevisiae,* or a filamentous fungus, for example, *Neurospora crassa,* several of the enzymes that catalyze reactions in the shikimate pathway can be combined into one large protein or polypeptide, called Aro1p, encoded by the ARO1 gene in the case of *S*. *cerevisiae.* Aro1p combines the functions of AroB, AroD, AroE, AroK (or AroL), and AroA). As such, Arolp, and ARO1, or a portion thereof, can be used as a substitute, or in addition to, AroB, AroD, AroE, AroK, and/or AroA.

In yet another embodiment described herein, flux through erythrose-4-phosphate within the bacterial cell is enhanced by means of overexpressing enzymes in the operation of the pentose phosphate pathway. In one aspect, over production of the transaldolase enzyme coded by the *talB* or *talA* gene is engineered. In another aspect, the expression of the genes encoding both transaldolase and transketolase enzymes are enhanced by genetic manipulations. In yet another aspect, the expression of the genes encoding either or both ribulose-5-phosphate epimerase and ribulose-5-phosphate isomerase are enhanced by genetic manipulations.

In another embodiment described herein, the PEP (phosphoenol pyruvate) available for the functioning of the aromatic amino acid pathway is increased through genetic manipulation. In one aspect, competition for the PEP pool is decreased through elimination and/or complementation of the PEP-dependent phosphotransferase system (PTS) for glucose uptake with a PEP independent system for glucose uptake. In yet another embodiment described herein, the availability of PEP is increased by increasing the expression of a gene that encodes a PEP synthetase, such *as pps.*

The figures show:
**FIG. 1****.** Pathway for aromatic amino acid biosynthesis in *E. coli.*

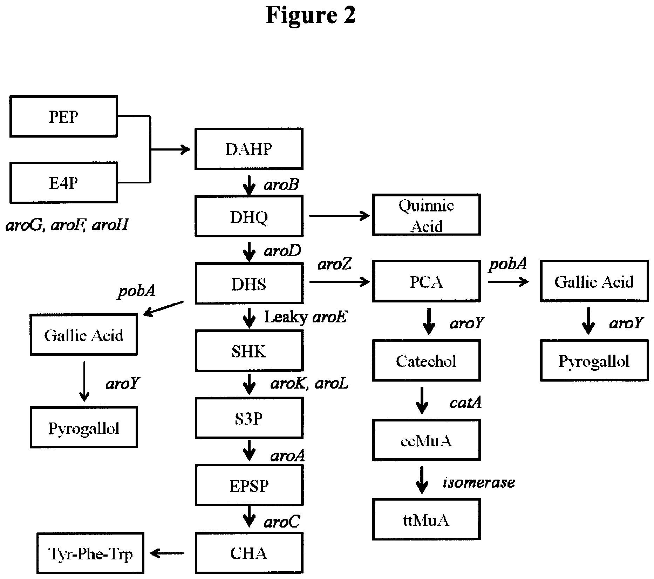

**FIG. 2****.** Pathway for the production of muconic acid in *E. coli.*

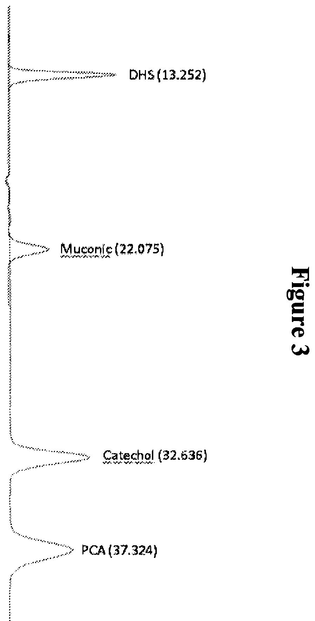

**FIG. 3****.** Chromatograph showing standards used for HPLC analysis of total muconic acid and biochemical intermediates.

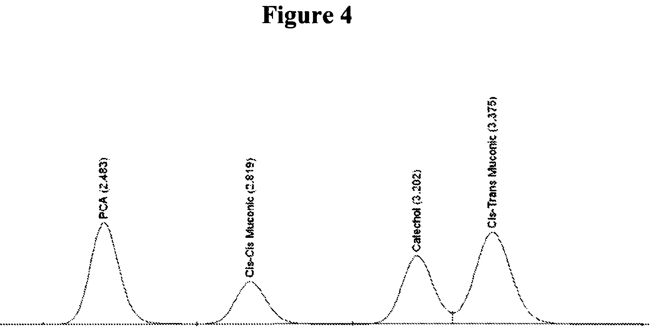

**FIG. 4****.** Chromatograph showing standards used for HPLC analysis of muconic acid isomers.

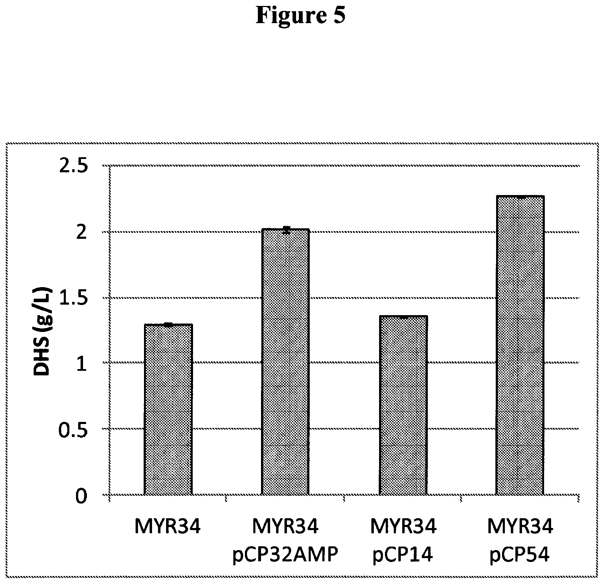

**FIG. 5****.** Titer for the production of DHS in the *E. coli* strain MYR34 transformed with plasmids pCP32AMP, pCP14 and pCP54. MYR34 strain of *E. coli* has a deletion in *aroE* gene. The plasmid pCP32AMP expresses the *aroG* gene coding for DAHP synthase. The plasmid pCP14 expresses the *aroB* gene coding for DHQ synthase. The plasmid pCP54 expresses both the *aroB* and *aroG* genes.

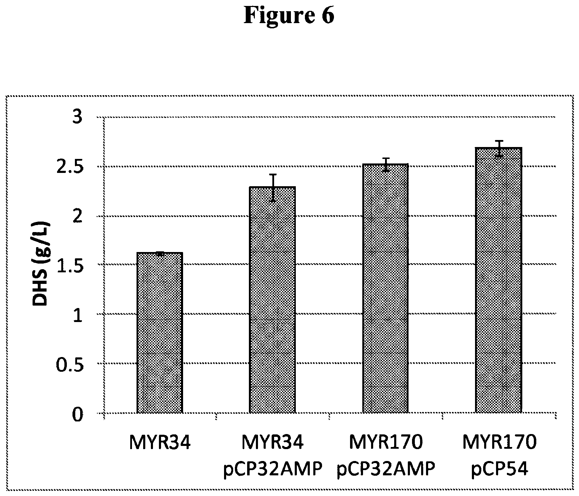

**FIG. 6****.** Titer for the production of DHS in the *E. coli* strains MYR34 and MYR170 transformed with plasmids pCP32AMP and pCP54. The MYR34 strain of *E. coli* has a deletion of the *aroE* gene. The MYR170 strain has a deletion of the *aroE* gene and a second copy of the *aroB* gene under the control of P₁₅ promoter integrated at the *ack* locus of the host chromosomal DNA. The plasmid pCP32AMP expresses the *aroG* gene coding for DAHP synthase. The plasmid pCP54 expresses both *aroB* and *aroG* genes.

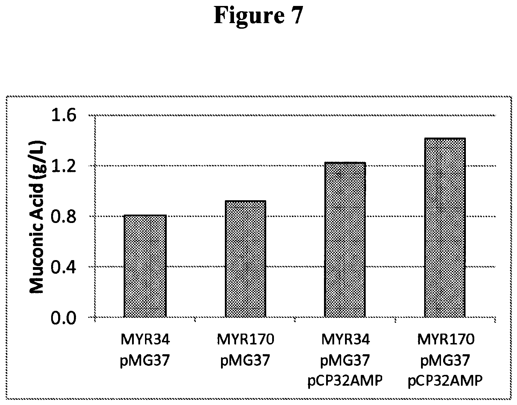

**FIG. 7****.** Titer for the production of cis, cis-muconic acid in the *E. coli* strains MYR34 and MYR170 transformed with plasmid pMG37 alone or with both pMG37 and pCP32AMP plasmids. The MYR34 strain of *E. coli* has a deletion of the *aroE* gene. The MYR170 strain has a deletion of the *aroE* gene and a second copy of the *aroB* gene under the control of P₁₅ promoter integrated at the *ack* locus of the host chromosomal DNA. The plasmid pCP32AMP expresses the *aroG* gene coding for DAHP synthase. The plasmid pMG37 expresses the *aroZ, aroY,* and *catAX,* genes coding for proteins functional in the muconic acid pathway.

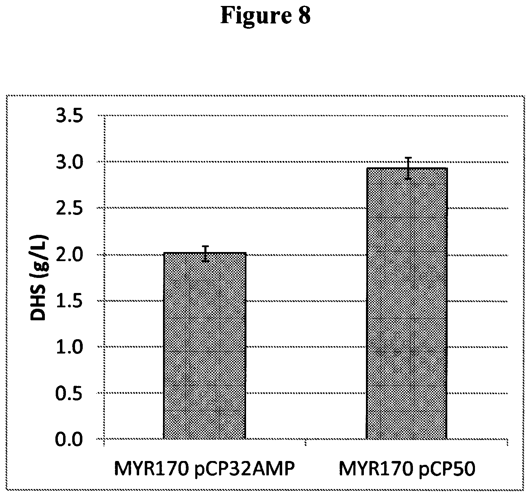

**FIG. 8****.** Titer for the production of DHS in MYR170 strain of *E. coli* transformed with a plasmid expressing *aroG* gene alone (pCP32AMP) or *aroG* and *tktA* genes simultaneously (pCP50). The MYR170 strain has a deletion of the *aroE* gene and a second copy of the *aroB* gene under the control of the P₁₅ promoter integrated at the *ack* locus of the host chromosomal DNA.

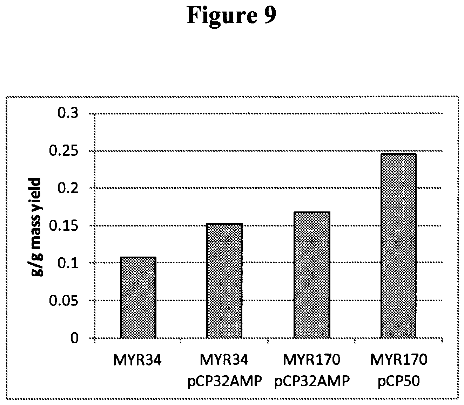

**FIG. 9****.** DHS yield from MYR34 and MYR170 stains of *E. coli* transformed with plasmids pCP32AMP and pCP50. DHS yield is calculated as grams of DHS produced per gram of glucose consumed. The plasmid pCP32AMP expresses the *aroG* gene while pCP50 expresses *aroG* and *tktA.* The bacterial strain MYR34 has a deletion in the *aroE* gene. The MYR170 strain of *E. coli* is derived from MYR34 and has an additional *aroB* gene integrated at the *ack* locus on the chromosomal DNA.

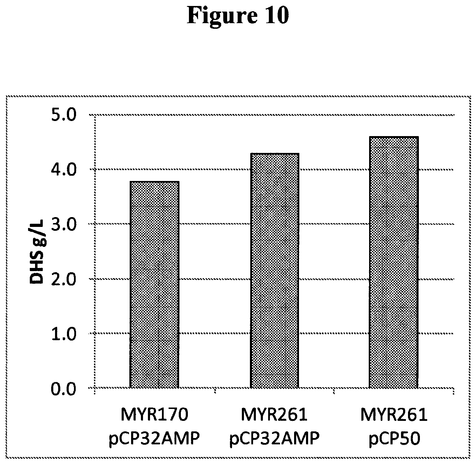

**FIG. 10****.** DHS titer from MYR170 and MYR261 stains of *E. coli* transformed with plasmids pCP32AMP and pCP50. The plasmid pCP32AMP expresses the *aroG* gene while pCP50 expresses the *aroB* and *tktA* genes. The MYR170 strain has a deletion of the *aroE* gene and a second copy of the *aroB* gene under the control of P₁₅ promoter integrated at the *ack* locus of the host chromosomal DNA. MYR261 strain of *E. coli* is derived from the MYR170 strain of *E. coli.* MYR261 strain of *E*. *coli* has a second copy of the *tktA* gene with its native promoter integrated at the *poxB* locus of the chromosomal DNA.

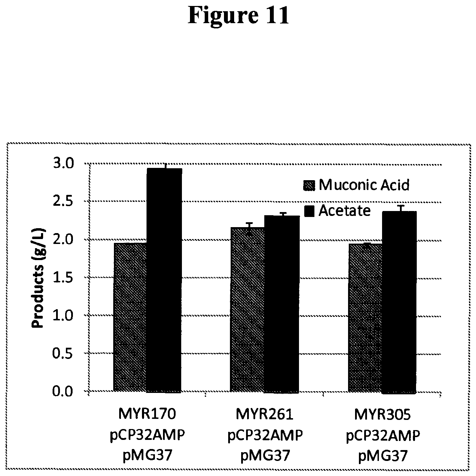

**FIG. 11****.** Muconic acid and acetic acid production in the *E. coli* strains MYR170, MYR261 and MYR305 transformed with the plasmid pCP32AMP expressing *aroG* coding for DAHP synthase in the shikimic acid biosynthetic pathway and plasmid pMG37 expressing *aroZ, aroY* and *catAX* genes coding for proteins functional in the muconic acid pathway. MYR170 strain has a deletion in the *aroE* gene and an additional copy of *aroB* gene under the control of P₁₅ promoter inserted at *ack* locus in the host chromosomal DNA. MYR261 and MYR305 are derivatives of MYR170 strain. MYR261 has an additional copy of *tktA* gene integrated at *poxB* locus on the host chromosomal DNA while MYR305 has a deletion in the *poxB* locus on the host chromosomal DNA.

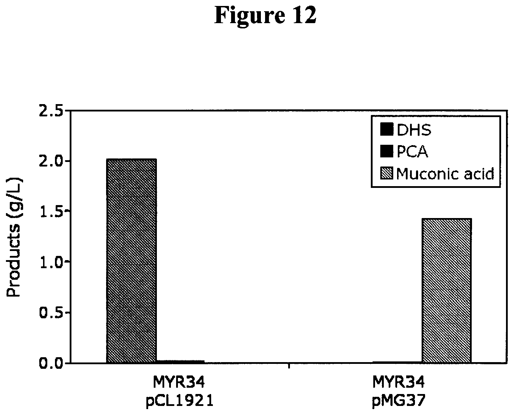

**FIG. 12****.** Conversion of endogenous DHS produced by *E. coli* strain MYR34 into muconic acid. MYR34 strain of *E. coli* has a deletion in the *aroE* gene coding of shikimate dehydrogenase. As a result there is an accumulation of DHS. When MYR34 strain is transformed with a plasmid expressing *aroZ, aroY* and *catAX,* genes coding for proteins functional in muconic acid pathway, there is conversion of DHS into muconic acid. However, no conversion of DHS into muconic acid occurs when MYR34 strain is transformed with the empty plasmid vector (pCL1921) without any exogenous genes.

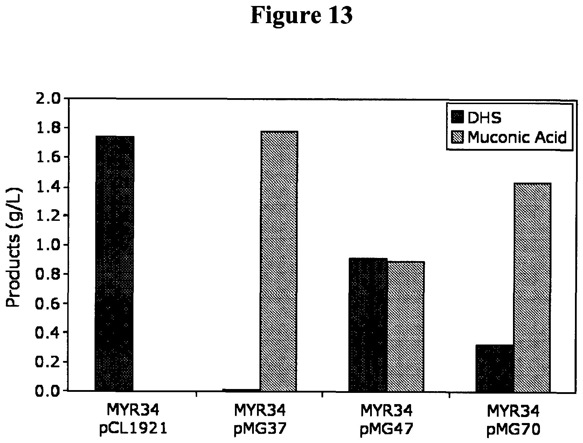

**FIG.13****.** Comparison of *aroZ* homologs for their ability to divert DHS into the muconic acid pathway. Three different *aroZ* homologs, namely *quiC* from *Acinetobacter sp. ADP1, asbF* from *Bacillus thuringiensis,* and *qa-4* from *Neurospora crassa* were cloned under the P₂₆ promoter in a low-copy plasmid which also expressed *catAX* and *aroY* genes from the P₁₅ and lambda P_{R} promoters respectively. These three different plasmid constructs were expressed in MYR34 through transformation and the amount of muconic acid produced was measured.

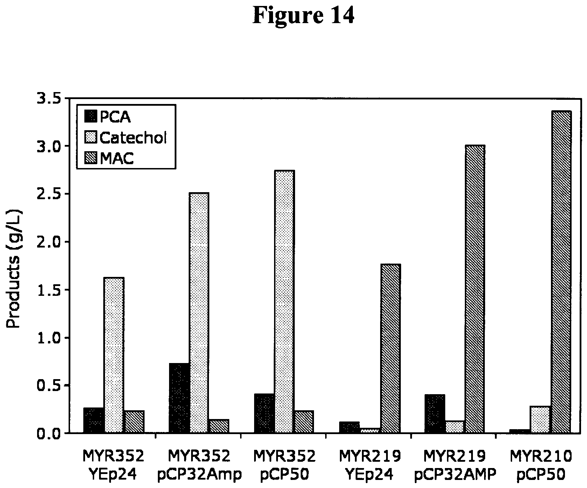

**FIG. 14****.** Single copies of *catAX, aroY* and *quiC* were chromosomally integrated into MYR170 strain of *E. coli* (*ΔaroE, Δack::P₁₅-aroB*) resulting in MYR352 (SEQ ID No. 41). MYR170 was also transformed with low copy plasmid pMG37 carrying all genes necessary for the operation of muconic acid pathway leading to the MYR219 strain. Both MYR352 and MYR219 were transformed with YEp24 (medium-copy empty vector) or pCP32AMP (medium-copy *aroG* expressing plasmid) or pCP50 (medium-copy *aroG* and *tktA* expressing plasmid) and the amount of PCA, catechol and muconic acid produced were quantified using HPLC method.

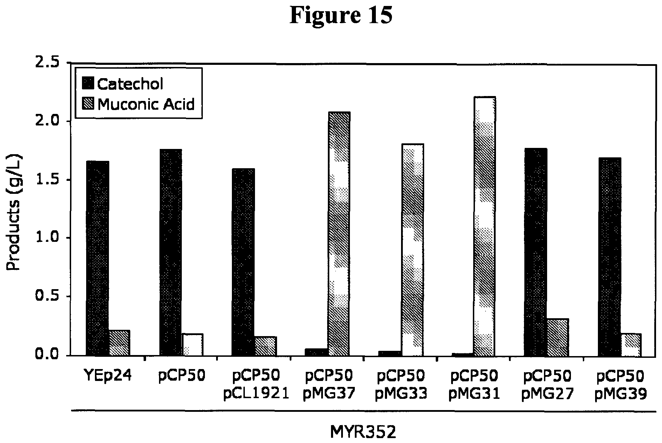

**FIG. 15****.** Removal of catechol accumulation in MYR352 by means of increasing the expression of *catAX.* MYR352 was transformed with a plasmid expressing *aroY* alone ( pMG27) or a plasmid expressing *quiC* alone (pMG39) or a plasmid expressing all three muconic acid pathway genes namely *catAX, aroY* and *quiC* (pMG37) or a plasmid expressing only two genes in the muconic acid pathway namely *catAX* and *aroY* (pMG33). Over expression of *catAX* alone was sufficient to prevent accumulation of catechol.

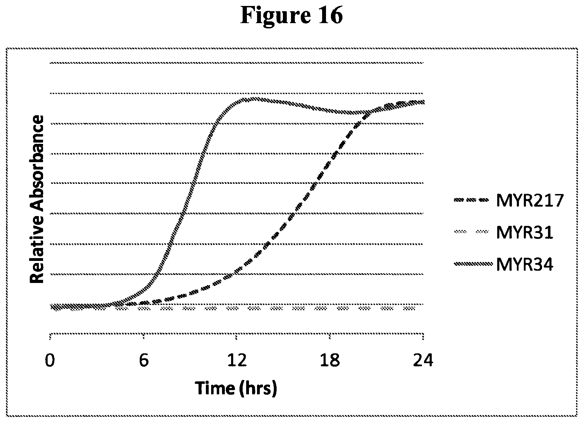

**FIG. 16****.** Growth of strains using different systems for importing glucose. Deletion of *ptsHI* and *galP* (MYR31) leads to lack of growth in minimal glucose medium, while installation of *glf and glk genes* (MYR217) brings back growth. Control strain MYR34 is Δ*aroE,* but otherwise wild type. The three aromatic amino acids and three aromatic vitamins were added to the medium to allow growth of the auxotrophic strains.

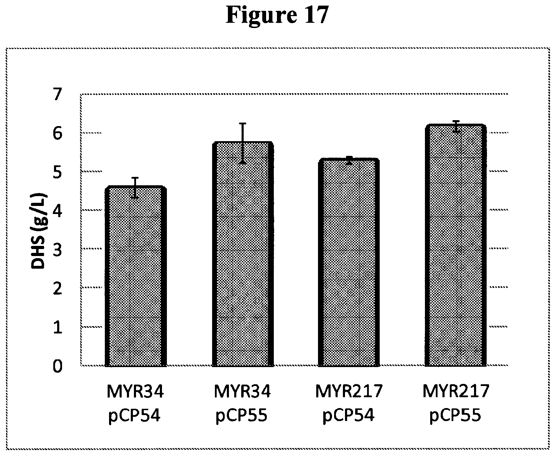

**FIG. 17****.** DHS production in MYR34 and MYR217 strains of *E. coli.* When transformed with plasmids that lead to production of DHS, MYR217, which utilizes *glf-glk* for glucose import, produced a higher titer of DHS than transformants of MYR34, which utilizes the phosphotransferase system (PTS)

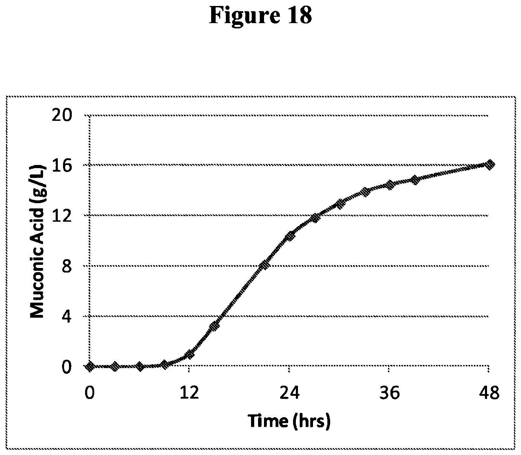

**FIG. 18****.** Production of muconic acid by the MYR428 strain of *E. coli* in a 7 Liter fermentor. The MYR261 strain of *E. coli* with a genotype of Δ*aroE* Δ*ackA*::P₁₅*-aroB* Δ*poxB*::*tktA* was transformed with the plasmids pCP32AMP and pMG37 to generate the MYR428 strain of *E. coli.*

As used in this patent application, the phrase "for example" or "such as" is meant to indicate that there are more than one method, approach, solution, or composition of matter for the subject at hand, and the example given is not meant to be limiting to that example.

The term "heterologous" refers to a gene or protein that is not naturally or natively found in an organism, but which can be introduced into an organism by genetic engineering, such as by transformation, mating, or transduction. A heterologous gene can be integrated (inserted) into a chromosome or contained on a plasmid. The term "exogenous" refers to a gene or protein that has been introduced into, or altered, in an organism for the purpose of increasing, decreasing, or eliminating an activity, by genetic engineering, such as by transformation, mating, transduction, or mutagenesis. An exogenous gene or protein can be heterologous, or it can be a gene or protein that is native to the host organism, but altered by one or more methods, for example, mutation, deletion, change of promoter, change of terminator, duplication, or insertion of one or more additional copies in the chromosome or in a plasmid. Thus, for example, if a second copy of the *aroB* gene is inserted at a site in the chromosome that is distinct from the native site, the second copy would be exogenous.

The term "microorganism" as used herein includes bacteria, archaea, yeast, algae and filamentous fungi that can be used for the commercial production of cis, cis-muconic acid through a fermentation process.

For nomenclature, a gene or coding region is usually named with lower case letters in italics, for example "*aroZ*", while the enzyme or protein encoded by a gene can be named with the same letters, but with the first letter in upper case and without italics, for example "AroZ" or "Arolp", the latter of which is an example of the convention used in yeast for designating an enzyme or protein. The "p" is an abbreviation for protein, encoded by the designated gene. The enzyme or protein can also be referred to by a more descriptive name, for example, AroZ can also be referred to as 3-dehydroshikimate dehydratase. A gene or coding region that encodes one example of an enzyme that has a particular catalytic activity can have several different names because of historically different origins, or because the gene comes from different species. For example the gene that encodes 3-dehydroshikimate dehydratase from *Bacillus thuringiensis* or *Bacillus anthracis* can be named *asbF* instead of *aroZ,* the related gene from *Aspergillus nidulans* can be named *qutC,* the related gene from *Neurospora crassa* can be named *qa-4,* and the related gene from *Acinetobacter baylyi* (also known as *Acinetobacter calcoaceticus* and *Acinetobacter* Sp. ADP1) can be named *quiC.*

A "plasmid" means a circular or linear DNA molecule that is substantially smaller than a chromosome, separate from the chromosome or chromosomes of a microorganism, and that replicates separately from the chromosome or chromosomes. A "plasmid" can be present in about one copy per cell or in more than one copy per cell. Maintenance of a plasmid within a microbial cell in general requires an antibiotic selection, but complementation of an auxotrophy can also be used.

The term "chromosome" or "chromosomal DNA" as used herein in the context of a bacterial cell is a circular DNA molecule that is substantially larger than a plasmid and does not require any antibiotics selection.

An "expression cassette" means a DNA sequence that can be part of a chromosome or plasmid that contains at least a promoter and a gene or region that codes for an enzyme or other protein, such that the coding region is expressed by the promoter, and the enzyme or protein is produced by a host cell that contains the DNA sequence. An "expression cassette" can be at least partly synthetic, or constructed by genetic engineering methods, so that the coding region is expressed from a promoter that is not naturally associated with the coding region. Optionally, the "expression cassette" can contain a transcription terminator that may or may not be a terminator that is naturally associated with the coding region. An "expression cassette" can have coding regions for more than one protein, in which case it can be called an operon, or a synthetic operon.

"Overexpression" of a gene or coding region means causing the enzyme or protein encoded by that gene or coding region to be produced in a host microorganism at a level that is higher than the level found in the wild type version of the host microorganism under the same or similar growth conditions. This can be accomplished by, for example, one or more of the following methods: 1) installing a stronger promoter, 2) installing a stronger ribosome binding site, such as a DNA sequence of 5'-AGGAGG, situated about four to ten bases upstream of the translation start codon, 3) installing a terminator or a stronger terminator, 4) improving the choice of codons at one or more sites in the coding region, 5) improving the mRNA stability, and 6) increasing the copy number of the gene, either by introducing multiple copies in the chromosome or placing the cassette on a multicopy plasmid. An enzyme or protein produced from a gene that is overexpressed is said to be "overproduced". A gene that is being "overexpressed" or a protein that is being "overproduced" can be one that is native to a host microorganism, or it can be one that has been transplanted by genetic engineering methods from a different organism into a host microorganism, in which case the enzyme or protein and the gene or coding region that encodes the enzyme or protein is called "foreign" or "heterologous". Foreign or heterologous genes and proteins are by definition overexpressed and overproduced, since they are not present in the unengineered host organism.

A "homolog" of a first gene, DNA sequence, or protein is a second gene, DNA sequence, or protein that performs a similar biological function to that of said first gene, DNA sequence or protein, and that has at least 25% sequence identity (when comparing protein sequences or comparing the protein sequence derived from gene sequences) with said first gene or protein, as determined by the BLAST computer program for sequence comparison (Altschul et al., 1990; Altschul et al., 1997), and allowing for deletions and insertions. An example of a homolog of the *E. coli aroG* gene would be the *aroG* gene from *Salmonella typhimurium.*

An "analog" of a first gene, DNA sequence, or protein is a second gene, DNA sequence, or protein that performs a similar biological function to that of said first gene, DNA sequence, or protein, but where there is less than 25% sequence identity (when comparing protein sequences or comparing the protein sequence derived from gene sequences) with said first gene, DNA sequence or protein, as determined by the BLAST computer program for sequence comparison (Altschul et al., 1990; Altschul et al., 1997), and allowing for deletions and insertions. An example of an analog of the *Klebsiella pneumoniae* AroZ protein would be the QutC protein from *Aspergillus nidulans,* since both proteins are enzymes that catalyze the 3-dehydroshikimate dehydratase reaction, but there is no significant sequence homology between the two enzymes or their respective genes. A scientist knowledgeable in the art will know that many enzymes and proteins that have a particular biological function, for example DAHP synthase or 3-dehydroshikimate dehydratase, can be found in many different organisms, either as homologs or analogs, and since members of such families of enzymes or proteins share the same function, although they may be slightly or substantially different in structure, different members of the same family can in many cases be used to perform the same biological function using current methods of genetic engineering. Thus, for example, the AroZ enzyme and the QutC enzyme catalyze the same reaction, DHS dehydratase, so either one will result in production of cis, cis-muconic acid in the proper context, and the choice of which one to use ultimately can be made by choosing the one that leads to a higher titer of cis, cis-muconic acid under similar fermentation conditions.

A "non-aromatic carbon source" or a "non-aromatic compound" is a carbon-containing compound that can be used to feed a microorganism described herein as a source of carbon and/or energy, in which the compound does not contain a six-membered ring related to benzene. Examples of non-aromatic carbon sources include glucose, xylose, lactose, glycerol, acetate, arabinose, galactose, mannose, maltose, or sucrose. An "aromatic compound" is a compound that contains one or more six-membered rings related to benzene. An example of an aromatic compound is catechol, or 1,2-dihydroxy benzene.

A "strong constitutive promoter" is a DNA sequence that typically lies upstream (to the 5' side of a gene when depicted in the conventional 5' to 3' orientation), of a DNA sequence or a gene that is transcribed by an RNA polymerase, and that causes said DNA sequence or gene to be expressed by transcription by an RNA polymerase at a level that is easily detected directly or indirectly by any appropriate assay procedure. Examples of appropriate assay procedures include 1) quantitative reverse transcriptase plus PCR, 2) enzyme assay of an encoded enzyme, 3) Coomassie Blue-stained protein gel, or 4) measurable production of a metabolite that is produced indirectly as a result of said transcription, and such measurable transcription occurring regardless of the presence or absence of a protein that specifically regulates level of transcription, a metabolite, or inducer chemical. An example of a promoter that is not a "strong constitutive promoter" is the P_{lac} promoter of *E. coli,* since it is repressed by a repressor in the absence of lactose or the inducer IPTG. By using well known methods in the art, a "strong constitutive promoter" can be used to replace a native promoter (a promoter that is otherwise naturally existing upstream from a DNA sequence or gene), resulting in an expression cassette that can be placed either in a plasmid or chromosome and that provides a level of expression of a desired DNA sequence or gene at a level that is higher than the level from the native promoter. A strong constitutive promoter can be specific for a species or genus, but often a strong constitutive promoter from a bacterium can function well in a distantly related bacterium. For example, a promoter from *Bacillus subtilis* or a phage that normally grows on *B. subtilis* can function well in *E. coli.* A "strong constitutive promoter" is substantially different from inducible promoters, such as P_{tac}, which have been used in the prior art production of cis, cis-muconic acid and typically require an expensive chemical or other environmental change for the desired level of function (Niu et al., 2002).Examples of strong constitutive promoters are P₁₅, P₂₆, from *Bacillus subtilis* phage SP01, and coliphage lambda P_{R} (SEQ ID Nos. 1, 2, and 3).

A "muconic pathway" or "muconic acid pathway" refers to a biochemical pathway from DHS to PCA to catechol to cis, cis-muconic acid, and a "muconic pathway gene" or "muconic acid pathway gene" is a gene that encodes an enzymes that catalyzes a step in a muconic pathway, or encodes an auxiliary function that serves to enhance the activity of one of said enzymes, for example, *aroZ, aroY, catA, catX,* and *qutC.* DHS is an abbreviation for 3-dehydroshikimate, and PCA is an abbreviation for protocatechuic acid. A"muconic plasmid" is a plasmid that contains one or more muconic pathway genes.

Some of the genetic manipulations used herein are centered around the common pathway for aromatic amino acid biosynthesis present in the bacterial cells as shown in Figure 1. The common pathway for aromatic amino acid biosynthesis as depicted in Figure 1 from DAHP synthase to chorismate synthase is also referred as the "shikimic acid pathway".

There is a substantial volume of published work on genetic engineering of microorganisms for the production of the aromatic amino acids, phenylalanine, tyrosine, and tryptophan (US 4,681,852, US 4,753,883, US 6,180,373, European Patent Application 86300748.0). The approaches include using various combinations of feedback resistant enzymes (AroF, AroG, PheA, TyrA), deregulation of repression of transcription *(tyrR),* increasing promoter strength (P_{tac}, P_{lac}) and increasing the copy number of one or more genes (*tktA*). However, many specific combinations of the above approaches were not tried, either because there were too many combinations to try without undue experimentation, or because lack of insight into what would be the best combinations. More importantly, the suitability of any of these combinations of genetic manipulations in developing a biocatalyst for the commercial production of muconic acid using renewable, non-aromatic carbon sources is not yet known.

The aromatic amino acid biosynthetic pathway is well known for many microorganisms, especially for *E. coli* (Neidhardt and Curtiss, 1996). In a wild type cell, the pathway is tightly regulated by both feedback inhibition and repression of transcription. The first committed step is catalyzed by deoxy-arabino-heptulosonate 7-phosphate (DAHP) synthase, of which there are three isozymes encoded by *aroF, aroG, and aroH.* The three isozymes, AroF, AroG, and AroH, are feedback inhibited the products of aromatic amino biosynthetic pathway namely by tyrosine, phenylalanine, and tryptophan, respectively. Feedback resistant mutants of all three are well known (Draths et al., 1992; Lutke-Eversloh and Stephanopoulos, 2007; Hu et al., 2003; Shumilin et al., 1999). One aspect described herein involves use of feedback resistant alleles of *aroF, aroG,* and *aroH* genes in order to express AroF, AroG and AroH enzyme proteins that are resistant to feedback inhibition by the products of aromatic amino acid biosynthetic pathway. Transcription of several of the operons involved in the aromatic pathway is regulated by either the repressor encoded by the *tyrR* gene or the repressor encoded by the *trpR* gene, or both (Neidhardt et al., 1996). Of particular importance is the negative regulation of transcription of *aroG* and *aroF* by the TyrR protein when it is bound with one or more of the aromatic amino acids. One aspect described herein involves the removal of negative regulation by *tyrR* or *trpR* genes by means of eliminating these genes from the chromosome of the host bacterial strain.

The subject of this disclosure is the creation of novel combinations of genetically engineered cassettes with novel genetically engineered elements in order to increase the fermentation parameters and suitability for large scale commercial production of a cis, cis-muconic acid producing strain. In particular, the prior art for production of cis, cis-muconic acid does not teach certain combinations of genetic elements, for example, but not limited to, various combinations of an overproduced feedback resistant AroG, an overproduced feedback resistant AroF, an overexpressed *tktA,* an overexpressed *talA,* chromosomally integrated cassettes for expressing an *aroZ, aroY,* and a *catAX* (or analogs or homologs thereof) from strong constitutive promoters, and a leaky *aroE* allele, which we define as a gene that encodes an AroE enzyme that confers prototrophy for the aromatic amino acids and vitamins, but without leading to significant secretion of aromatic compounds..

All specific examples of strain constructions disclosed herein are derived from a wild type *E. coli* C strain (ATCC 8739), or *E. coli* K-12 strains (YMC9 or MM294) but the genetic elements disclosed herein can be assembled in any other suitable *E. coli* strain, and the expression cassettes or appropriate analogs and homologs of the genetic elements disclosed herein can be assembled in any other suitable microorganism, such as other species of bacteria, archaea, yeast, algae, and filamentous fungi that can be used for the commercial production of cis, cis-muconic acid through a fermentative process.

In *E. coli,* the aromatic amino acid biosynthesis pathway from glucose starts with the non-oxidative branch of the pentose phosphate pathway (PPP). Four key enzymes in the non-oxidative pentose phosphate pathway are transketolase, transaldolase, ribulose-5-phosphate epimerase and ribulose-5-phosphate isomerase. These enzymes catalyze the reactions that lead to the formation of erythrose 4-phosphate (E4P) from hexose or pentose sugars. To increase the availability of E4P in *E. coli,* the *tktA* gene encoding transketolase can be overexpressed (Niu et al., 2002). Similarly, the overexpression of the transaldolase gene is also expected to increase the availability of E4P in some circumstances (Bongaerts et al., 2001). In yet another aspect, the expression of both the transketolase and transaldolase genes are enhanced through genetic manipulations leading to an increase in the activity of transketolase and transaldolase enzymes. In yet another aspect, flux through the non-oxidative branch of the PPP is increased by overproducing ribulose-5-phosphate epimerase and ribulose-5-phosphate isomerase.

The first committed step and most tightly regulated reaction in the common aromatic amino acid pathway is the condensation of phosphoenolpyruvate (PEP) and E4P to produce deoxyarabino-heptulosonate 7-phosphate (DAHP) by DAHP synthase (encoded by *aroG, aroF,* and *aroH*). D-glucose consumed by *E. coli* is brought into aromatic biosynthesis partly through the PPP, and partly through glycolysis. The flow of glucose into the aromatic pathway is greatly increased when transketalose (*tktA*) and an isozyme of DAHP synthase (*aroG*) are amplified through transformation with a plasmid that increases their expression by increasing their copy number (Niu et al., 2002). In a preferred aspect, the exogenous *aroG* and *tktA* genes are integrated into the chromosomal DNA for the purpose of amplification of activities transketolase and DAHP synthase enzymes.

In another embodiment described herein, the flux through PEP within the microbial cell is improved by increasing the PEP available for the synthesis of DAHP. Many genera of bacterial cells consume PEP in the transport of glucose across the cell membrane using a phosphotransferase system (PTS) in which one PEP molecule is consumed for every molecule of glucose transported across the bacterial outer membrane. By replacing or complementing the PEP-dependent PTS with a non-PEP dependent (PEP independent) glucose uptake mechanisms, it is possible to increase the pool size of the PEP available for the aromatic amino acid biosynthetic pathway within the microbial cell. For example, the PTS system for sugar uptake can be replaced or complemented by a GalP-based sugar uptake system or the sugar transporter system based on Glf/Glk proteins (Chandran et al., 2003; Yi et al., 2003). In a preferred aspect, besides deleting the PTS system for sugar uptake for the purpose of conserving PEP pool within the microbial cell, the GalP based sugar uptake system is also inactivated for the purpose of conserving ATP within the microbial cell. In a microbial cell which is defective in the functioning of both PTS system and a Gal-P based sugar uptake system (ΔPTS/Δ*galP*), the sugar uptake can be accomplished by means of introducing an exogenous gene coding for Glf, or exogenous genes encoding both Glf (glucose facilitated diffusion protein) and Glk (glucokinase) proteins. As used herein, the term functional glucose-facilitated diffusion protein refers to any Glf protein as well as any other protein which is functionally equivalent to Glf and functions to transport sugars into the microbial cells by facilitated diffusion. In one aspect, the gene coding for the glucose facilitator protein Glf is introduced into the microbial cell which is ΔPTS/Δ*galP* and the glucose transported into the microbial cell is phosphorylated by endogenous glucose kinase. In another aspect, the genes coding for both Glf and Glk proteins are introduced into a microbial cell which is ΔPTS/Δ*galP*. In a preferred aspect, the exogenous *glf* and *glk* genes introduced into the microbial cell are integrated into the host chromosomal DNA.

In another embodiment described herein, when the carbon source for growth and energy requires gluconeogenesis (for example if the carbon source is acetate or succinate), the PEP pool can be increased by increasing the activity of carboxylating enzymes already present within the cell, for example PEP carboxykinase, which is encoded by *pck* in *E. coli,* or by introducing an exogenous carboxylating enzyme. In a preferred embodiment, the introduced exogenous gene coding for a carboxylating enzyme is stably integrated into the host chromosome. Genes coding for the carboxylating enzyme can be derived from a variety of microbial species. The genes coding for the carboxylating enzymes can further be subjected to genetic manipulations so that the expression of the carboxylating enzyme within the biocatalyst for cis, cis-muconic acid production is significantly enhanced.

In yet another embodiment described herein, the PEP pool inside the microbial cell is increased by decreasing or eliminating the activity of pyruvate kinase enzymes such as PykA and PykF which use PEP as a substrate.

From DAHP, the aromatic amino acid pathway proceeds via a number of intermediates to chorismate (CHA), a branch point for the biosynthesis of three aromatic amino acids namely L-Tyrosine (L-Tyr), L-Phenylalanine (L-Phe), and L-Tryptophan (L-Trp).

In the initial stages of the common aromatic amino acid pathway, 3-dehydroquinate (DHQ) synthase (AroB) removes the phosphate group from DAHP leading to the formation of DHQ. The enzyme DHQ dehydratase (AroD) removes a water molecule from DHQ leading to the formation of 3-dehydroshikimate (DHS) which is subsequently reduced to shikimate (SHK) by shikimate dehydrogenase (AroE). Shikimate kinase I/II (AroK, AroL) phosphorylates shikimate to shikimate 3-phosphate (S3P). There is a condensation of S3P with PEP leading to the formation of 5 enolpyruvoylshikimate 3-phosphate (EPSP). The formation of EPSP is mediated by EPSP synthase (AroA). A phosphate group from EPSP is removed by chorismate synthase (AroC) leading to the formation of chorismate (CHA).

As shown in the Figure 2, the aromatic amino acid pathway can be blocked at the level of conversion of 3-dehydroshikimate (DHS) to shikimate (SHK) due to a mutation in *aroE* gene leading to the accumulation of DHS (Niu et al., 2002). Introduction of an exogenous *aroZ* gene functions to convert DHS into protocatechuate (PCA). PCA is subsequently converted into catechol through a decarboxylation reaction mediated by an AroY enzyme. Catechol is ultimately converted into cis-cis muconic acid (ccMuA) through the action of a *catA* gene product. ccMuA can be acted upon by maleyl acetoacetate isomerase to yield trans-trans muconic acid (ttMuA). The biosynthetic pathway from DHS to ccMuA and ttMuA is referred as muconic acid pathway. The three different genes responsible for the conversion of DHS to ccMuA can be obtained from various microbial species and introduced into a microorganism selected for muconic acid production such as *Escherichia coli.* In a preferred embodiment described herein, the exogenous genes coding for the proteins involved in muconic acid pathway are integrated into host chromosomal DNA.

In redirecting the aromatic amino acid pathway to the production of cis, cis-muconic acid mutation of the *aroE* gene is critical. The *aroE* gene can be completely inactivated leading to a total block in the biosynthesis of aromatic amino acids as was done with the WN1/pWN2.248strain of *E. coli* described for the muconic acid production (Niu et al., 2002). An important drawback with the WN1/pWN2.248 *E*.*coli* strain and related strains is that due to the complete inactivation of the *aroE* gene, this strain has become auxotrophic for the aromatic acids such as phenylalanine, tyrosine and tryptophan, and aromatic vitamins or vitamin-like compounds mentioned above. As a result, this strain during its growth for the production of cis, cis-muconic acid requires the exogenous addition of these six compounds (or a common intermediate such as shikimate), thereby adding substantially to the cost of commercial production of cis, cis-muconic acid using such a strain. A novel approach to overcome this dependency on an exogenous source of aromatic amino acids is to use a strain with a leaky mutation in *aroE.* The leaky *aroE* mutant would allow a limited flow of carbon to shikimic acid while accumulating significant amounts of DHS which is then available for the conversion into PCA by the action of an AroZ enzyme. Thus the use of a leaky mutant form of *aroE* would eliminate the dependence on exogenous aromatic amino acids, while still diverting the flow of carbon to cis, cis-muconic acid.

The genes coding for the synthesis of AroZ, AroY and CatA proteins essential for the conversion of DHS into cis, cis-muconic acid can be derived from many microbial species. In one embodiment, these exogenous genes are integrated into the host chromosome of the biocatalyst being developed. In a preferred embodiment, the expression of these exogenous genes within the biocatalyst is driven by a constitutive promoter without the need for any inducers.

The enzyme 3-dehydroshikimate dehydratase (AroZ; EC 4.2.1.118) is required for biosynthesis of the intermediate protocatechuate. In this specification, "AroZ" shall refer to any enzyme that catalyzes the 3-dehydroshikimate dehydratase reaction. In the *prior art,* this enzyme is expressed from the *aroZ* gene of *Klebsiella pneumoniae* strain A170-40 (ATCC25597) (Niu et al., 2002; Draths and Frost, 1995). However, the specific activity of AroZ varies widely among organisms, from 0.1 to 261 micromoles/min/mg (Wheeler et al, 1996; Fox et al, 2008; Pfleger et al, 2008), so a significant improvement can be had by expressing an *aroZ* gene also known as *asbF* (Fox et al, 2008; Pfleger et al, 2008), *qutC* (Wheeler et al, 1996), *qa-4* (Rutledge, 1984), and *quiC,* from an organism that has a higher specific activity than *K. pneumoniae,* for example *Acinteobacter baylyi, Aspergillus nidulans* (Wheeler et al, 1996), now also known as *Emericella nidulans,* or *Neurospora crassa* (Rutledge, 1984; Stroman et al, 1978), or *Podospora anserina,* also known as *Podospora pauciseta* (Hansen et al, 2009).

As one particular example, the coding sequence for the *qa-4* gene from *N. crassa* that encodes 3-dehydroshikimate dehydratase can be obtained by any of several well known methods, for example whole gene DNA synthesis, cDNA cloning, or by a combination of genomic DNA cloning and PCR or synthetic DNA linker synthesis. Since there are no introns in the *qa-4* gene, the coding region can be obtained by PCR from genomic DNA (Rutledge, 1984). The protein sequence of the *qa-4* enzyme (SEQ ID No. 4) and the DNA sequence of the native gene (SEQ ID No. 5) are known.

Alternatively, an expression cassette can be constructed for the 3-dehydroshikimate dehydratase from *A. nidulans.* The coding sequence for the QutC enzyme from *A. nidulans* can be obtained by any of several well known methods, for example whole gene DNA synthesis, cDNA cloning, or by a combination of genomic DNA cloning and PCR or synthetic DNA linker synthesis. The protein sequence of QutC (SEQ ID No. 6) and the DNA sequence of the native gene, containing no introns, are known (SEQ ID No. 7; GenBank accession number M77665.1). An expression cassette can be obtained by DNA synthesis, or by a combination of genomic cloning and PCR, so that the QutC enzyme can be produced accurately in *E. coli.* By expressing a coding sequence for QutC from a strong, constitutive promoter in *E. coli,* sufficient expression can be obtained from one or two copies of the gene integrated in the chromosome, obviating the need for maintaining more than two copies of the expression cassette on a multicopy plasmid as has been disclosed in the prior art (Niu et al., 2002), and which can lead to instability. The method described above can be used in general to obtain a DNA sequence that codes for a desired enzyme, and that coding sequence can then be used to construct an expression cassette designed to function in *E. coli* or another appropriate microbial host organism.

The specific activity of AroZ can also be improved by using the protein sequence from the prior art (Niu et al., 2002) but constructing an improved expression cassette, for example, in which a stronger promoter and/or ribosome binding site (RBS) has been installed in front of the coding region, as described in Example 4.

The *aroZ* gene encoding AroZ (3-dehydroshikimate dehydratase) from *Klebsiella pneumoniae* strain A170-40 can be obtained as described in the *prior art.* The DNA sequence of the gene and surrounding DNA can be determined by methods well known in the art. A heterologous gene described herein such as *aroZ* can be built into an expression cassette using a native DNA sequence or it can be synthesized with a codon optimized sequence for the intended host organism. An *aroZ* gene can be cloned as described (Draths and Frost, 1995) from any other microbe that contains an active *aroZ* gene, for example *K. pneumoniae* strain *342, Acinetobacer Sp. ADP1 (Acinetobacter baylyi ADP1), Bacillus thuringiensis, Emericella nidulans, Erwinia amylovora, Pseudomonas putida* W619, and many others.

The enzyme protocatechuate decarboxylase (AroY; EC 4.1.1.63) is required for biosynthesis of the intermediate catechol. In this specification, "AroY" shall refer to any enzyme that catalyzes the protocatechuate decarboxylase reaction. In the *prior art,* this enzyme is expressed from the *aroY* gene of *Klebsiella pneumoniae* strain A170-40 (ATCC25597) on a multicopy plasmid (Niu et al., 2002). However, once again an improvement in the process can be gained by producing enough of the enzyme from one or two copies of an expression cassette integrated in the chromosome of the host organism. This can be accomplished by obtaining an *aroY* gene from an organism that naturally produces an AroY enzyme that has higher specific activity than that of the *K. pneumoniae* AroY enzyme of the prior art, or by increasing the level of expression of the *K. pneumoniae* AroY by constructing an expression cassette that, for example, uses a strong constitutive promoter and/or strong RBS as described above under Example 4. The protein sequence for AroY from *K. pneumoniae* strain A170-40 is given in SEQ ID No. 8. The corresponding gene, *aroY,* can be cloned as described above (Draths and Frost, 1995), or based on the protein sequence, it can be synthesized with optimized codons for the intended host organism.

The *aroY* gene can be obtained from any other microorganism that contains a homolog or analog, for example, *K. pneumoniae* strain NCTC418 (ATCC15380), *Klebsiella pneumoniae 342,* and *Arxula adeninivorans* (Sietmann et al, 2010). The DNA sequence of the *aroY* gene from *Klebsiella pneumoniae* 342 and surrounding DNA is given as SEQ ID No. 9.

The enzyme catechol 1,2-dioxygenase (CatA; EC 1.13.11.1) is required for the last step of cis, cis-muconic acid biosynthesis. In this specification, "CatA" shall refer to any enzyme that catalyzes the catechol 1,2-dioxygenase reaction. In the prior art, this enzyme is expressed from the *catA* gene of *Acinetobacter calcoaceticus* strain ADP1 on a multicopy plasmid (Niu et al., 2002). The source strain, *Acinetobacter calcoaceticus* strain ADP1, apparently has been renamed *Acinetobacter Sp.* ADP1 and *Acinetobacter baylyi* ADP1 (Neidle and Ornston, 1986; Barbe et al, 2004; de Berardinis et al, 2008). In this prior art example, the *catA* gene was expressed from a P_{tac} promoter, which requires either lactose or IPTG (isopropylthiogalactoside) as an inducer. These compounds are too expensive for use in commercial fermentations, so again, significant improvements in the process are needed, both to eliminate the need for an expensive inducer and to create a more stable strain by integrating the expression cassette in the chromosome. This can be accomplished by constructing an expression cassette for the *catA* gene that uses a strong constitutive promoter, strong RBS, and/or more stable mRNA as described above in the other Examples.

The DNA sequence of the *catA* gene and surrounding sequences from *Acinetobacter baylyi* ADP1 is given in SEQ ID No. 10. The protein sequence for CatA from the same strain is given in SEQ ID No. 11. In a preferred embodiment, the expression cassette for *catA* contains one or two additional open reading frames that exist naturally downstream from *catA,* in order to increase the expression level of the *catA* gene (Schirmer and Hillen, 1998). Many other organisms can be a source for a *catA* gene, for example *Pseudomonas arvilla, Pseudomonas fluorescens* (Nakazawa et al, 1967; Kojima et al, 1967), *Streptomyces* Sp. Strain 2065 (Iwagami et al, 2000), *Cupriavidus necator* 335T, and many others (Perez-Pantoja et al, 2008).

In order to improve the flow of carbon towards cis, cis-muconic acid, it is necessary to block certain other pathways branching out of the aromatic amino acid pathway, besides reducing the flow of carbon from DHS to shikimate (SHK) by using a leaky *aroE* mutant. Some bacteria, for example in the genus *Acinetobacter* and *Pseudomonas,* contain a gene named *pobA,* which encodes an enzyme, p-hydroxybenzoate hydroxlase, that converts DHS into gallic acid. Although a PobA homolog or analog has not been found in *E. coli,* strains of *E. coli* engineered to produce DHS secrete measurable amounts of gallic acid (Li and Frost, 1999), so it is likely that such an enzyme does exist in *E. coli.* In addition, the PCA derived from DHS can be converted into gallic acid by the action of p-hydroxybenzoate hydroxlase (PobA) enzyme coded by the *pobA* gene. The gallic acid thus produced can be subsequently converted to pyrogallol. One way to block the carbon flow to gallic acid and pyrogallol in the biocatalyst selected for an improved cis, cis-muconic acid is to block or diminish the activity of p-hydrobenzoate hydroxlase (PobA) protein through genetic manipulations. Similarly, DHQ, the precursor to DHS can also be acted upon by shikimate dehydrogenase coded by *aroE* leading to the production of quinnic acid. In an embodiment described herein, the leaky AroE mutant enzyme is additionally selected or screened for its inability or reduced ability to convert DHQ into quinnic acid.

There are several advantages in producing trans, trans-muconic acid in place of cis, cis-muconic acid. Trans, trans-muconic acid is preferred over cis, cis-muconic acid in the Diels Alder reaction with ethylene for the production of terephthalic acid. A biocatalyst with a genetically manipulated aromatic pathway produces cis, cis-muconic acid which can be converted into trans, trans-muconic acid outside the cell using chemical conversion processes. On the other hand by means of introducing a maleylacetoacetate isomerase or similar isomerase enzyme into the biocatalyst, it is possible to convert the cis, cis-muconic acid into trans, trans-muconic acid within the bacterial biocatalyst.

The specification in this patent application provides several different aspects of invention related to the construction of a microbial strain for efficient production of muconic acid. A person skilled in the art can compile several different aspects of the present invention to construct a biocatalyst with very high efficiency for the production of muconic acid.

### EXPERIMENTAL SECTION

### GENERAL REMARKS

**Strain and inoculum preparations:** A list of the bacterial strains and the plasmids used herein is provided in Table 1. All specific examples of strain constructions disclosed herein are derived from a wild type *E. coli* C strain (ATCC 8739), or *E. coli* K-12 strains (YMC9 or MM294) but the genetic elements disclosed herein can be assembled in any other suitable *E. coli* strain, and the expression cassettes or appropriate analogs and homologs of the genetic elements disclosed herein can be assembled in any other suitable microorganism, such as other species of bacteria, archaea, yeast, algae, and filamentous fungi that can be used for the commercial production of cis, cis-muconic acid through a fermentative process.

*E. coli* C is capable of fermenting 10% glucose in AM1 mineral media. AMI medium contains 2.63 g/L (NH₄)₂HPO₄, 0.87 g/L NH₄H₂PO₄, 1.5 mM MgSO₄, 1.0 mM betaine, and 1.5 ml/L trace elements. The trace elements are prepared as a 1000X stock and contained the following components: 1.6 g/L FeCl₃, 0.2 g/L CoCl₂•6H₂O, 0.1 g/L CuCl₂, 0.2 g/L ZnCl₂•4H₂O, 0.2 g/L NaMoO₄, 0.05 g/L H₃BO₃, and 0.33 g/L MnCl₂•4H₂O. The pH of the fermentation broth is maintained at 7.0 with 1.0 - 10.0 M KOH or 1.0 - 9.0 M ammonium hydroxide.

**Fermentations:** Fermentations were started by streaking on a fresh NBS-2% glucose (Jantama et al., 2008a) plate from a 40% glycerol stock of *E. coli* strain genetically engineered and stored in a -80°C freezer. Plasmids, if present, are retained by including the appropriate antibiotic(s) in the agar plates and liquid media. Ampicillin (sodium salt) is used at 150 mg/L, spectinomycin HCL at 100 mg/L, tetracycline HCl at 15 mg/l, and kanamycin sulfate at 50 mg/l. After 24 to 48 hours (37°C), a single colony is picked into 25 ml of the same medium in a shake flask. After shaking at 200 rpm at 37°C until the cells have grown to an OD₆₀₀ of about 1.0, the culture is cooled an ice and an equal volume of sterile 80% glycerol is added. 2 ml aliquots are then frozen at -80°C to be used as inocula for fermentations.

**Cell growth:** Cell mass was estimated by measuring the optical density at 550 nm (OD₅₅₀) or 600 nm (OD₆₀₀) using a Thermo Electronic Spectronic 20 spectrophotometer.

**Analysis of intermediates in shikimic acid pathway and muconic acid pathways:** Total muconic acid produced in fermentation broths, which includes cis, cis-muconic acid and cis, trans-muconic acid, and other biochemical intermediates were assayed by HPLC with a Waters Alliance instrument, and monitoring absorbance at 210 nm or refractive index at 45° C, using standards purchased from Sigma-Aldrich. The column was a BioRad Aminex HPX-87H run at 50°C with 8 mM sulfuric acid as the mobile phase at a flow rate of 0.6 ml/min for 40 minutes. A chromatograph of purchased standards (Sigma-Aldrich) is shown in Figure 3. To prepare for HPLC, fermentation samples are diluted 10 or 100 fold in 0.05 M potassium phosphate buffer, pH 7.0, to preserve the cis, cis- form of muconic acid from isomerizing to the cis, trans-form.

To separate the isomers of muconic acid, the samples prepared as above were run in a second HPLC system. The instrument was an Agilent 1200 HPLC, the column was an Agilent Eclipse XDB-C18, 4.6 x 150 mm run at 30 degrees Centigrade with a mobile phase of 50mM KH₂PO4 in 30% methanol adjusted to pH 3.0 with phosphoric acid. The flow rate was 1 ml/min for 4 minutes, with detection by absorbance at 278 nm. The cis, trans-muconic acid standard was created by dissolving cis, cis-muconic acid in water and allowing it to undergo spontaneous acid catalyzed isomerization for about 2 hours at room temperature, until the HPLC peak had completely shifted to a new position. The other standards were purchased from Sigma-Aldrich. A chromatograph showing standards is shown in Figure 4.

**Composition of muconic acid production medium for the fermentation** process: Each liter of fermentation medium contains 50 ml /L of 1M KH₂PO₄, 10 ml of 200g/L Citric acid + 25 g/L Ferric citrate, 1.2 ml of 98% Sulfuric acid, and a drop of Antifoam 204. These components were mixed with enough water to allow room for addition of other components below. After autoclaving, the following components were added : 10, 20, 30 or 40 ml of 50% glucose (to give 5, 10, 15, or 20 g/l final), 2 ml of 1M MgSO4, 1 ml of 0.1M CaCl2, 10 ml of 1000X Trace elements (Jantama et al. 2008a), 1, 2, 4, or 8 ml of 50g/L Phenylalanine + 50 g/L Tyrosine + 50 g/L Tryptophan (to give 0.5, 0.1, 0.2, or 0.4 g/l final), 10 ml of 1 g/L p-hydroxybenzoic acid + 1 g/l p-aminobenzoic acid + 1 g/L 2,3-dihydroxylbenzoic acid, and, as necessary, 1 ml of 150 mg/ml Ampicillin (sodium salt) and/or 1 ml of 100 mg/ml Spectinomycin HCl.

For fed batch fermentations, the feed bottle contained 600 g/L of anhydrous glucose and 32 ml/L of 50 g/L Phenylalanine + 50 g/L Tyrosine + 50 g/L Tryptophan. 9M NH₄OH was used as a base to maintain the pH of the fermentation medium.

For shake flasks, NBS salts (Jantama et al. 2008a) plus 0.2 M MOPS buffer, pH 7.4 was substituted for the pre-autoclave mix described above, but the glucose and other additives were the same.

Fed-batch fermentations were performed in 7L New Brunswick Scientific Fermentors with pH, DO, temperature, glucose, and feed rate controlled by either DCU controllers or Biocommand Software. The temperature was maintained at 37°C, the pH was maintained at 7.0 by 9N ammonium water, and the dissolved oxygen(DO) was maintained at 30% air saturation while increasing impeller's speed from 750rpm to 1200rpm. The initial glucose concentration in the desired medium was around 5 to 25 g/L. A glucose solution was added to the fermentor when the glucose concentration was dropped to below 5 g/L, and the feed rate of glucose was controlled by the dissolved oxygen level. The total fermentation time was 48 hrs, and the final titer was 16 g/L of muconic acid.

Construction of plasmids expressing muconic acid pathway genes: The three heterologous genes required for conversion of DHS to muconic acid were cloned either singly or in combination into a low-copy plasmid, pCL1921 (Lerner and Inouye, 1990). The DNA sequence of pCL1921 is given in SEQ ID No. 20 in Table 3. Briefly, the coding sequences of *catAX, aroY* and *aroZ* analogs or homologs were codon-optimized for expression in *E. coli* and commercially synthesized (GeneArt, Invitrogen). These sequences were then PCR amplified using a forward primer carrying a unique ribosome-binding site and a reverse primer carrying a unique terminator sequence for each gene. The resulting PCR fragment was digested with restriction enzymes and cloned downstream of a unique constitutive promoter sequence by standard molecular cloning procedures. The promoter sequences were cloned by PCR amplification from source DNA sequences previously described (United States Patent Application 20090191610; United States Patent 7244593) followed by restriction digestion and standard molecular cloning. The promoter-RBS-coding sequence-terminator sequence together constituted an expression cassette. Individual expression cassettes were next combined to generate plasmids expressing one, two or all three muconic acid pathway genes.

### Example 1

### Increasing expression of aroG and aroF.

The *tyrR* gene of *E. coli* can be mutated by any one of a number of well known methods, such as chemical or radiation mutagenesis and screening (for example by PCR and DNA sequencing) or selection for analog resistance (for example, resistance to 4-fluorotyrosine), transposon mutagenesis, bacteriophage Mu mutagenesis, or transformation. In a preferred embodiment, the mutation in *tyrR* gene is a null mutation (a mutation that leaves no detectable activity), and in a more preferable embodiment, at least a portion of the *tyrR* gene is deleted. This can be accomplished, for example, by using a two step transformation method using linear DNA molecules (Jantama et al, 2008a; Jantama et al, 2008b). In the first step, a *cam^{R}, sacB* cassette is integrated at the *tyrR* locus to replace most or all of *tyrR* open reading frame by double recombination and selecting for chloramphenicol resistance. In the second step, a linear DNA comprising a deleted version of the *tyrR* gene is integrated by double recombination, selecting for resistance to 5% sucrose in a rich medium such as LB. Correct deletions are identified and confirmed by diagnostic polymerase chain reaction (PCR). The purpose of deleting *tyrR* is to increase expression of *aroG* and *aroF.* An alternative approach that achieves a similar result is to replace the native promoter in front of *aroG* and/or *aroF* with a strong constitutive promoter and add, if necessary, a transcription terminator. More details on how this is accomplished in general are given in Example 4 below.

The *latter* of the *two* approaches described above for overcoming the repression of AroG and AroF activities by TyrR protein is preferable, since deletion of *tyrR* can cause unwanted overexpression of genes such as *aroLM* (Neidhardt and Curtiss, 1996). More detail on how this is accomplished in general is given in Example 4 below.

### Example 2

### Feedback resistant AroG and AroF

Mutations in the *aroG* gene that lead to a feedback resistant AroG enzyme (3-deoxy-D-arabinoheptulosonate-7-phosphate synthase or DAHPS) are well kn*own* in the art (Shumilin et al, 1999; Kikuchi et al, 1997; Shumilin et al, 2002). Also well known are methods for creating, identifying, and characterizing such mutations (Ger et al., 1994, Hu et al., 2003). A preferable mutation is one that leads to complete resistance to inhibition by phenylalanine. Any of the known published feedback resistant mutations can be introduced into an *aroG* gene contained in the chromosome or on a plasmid by any of a number of well known methods, one example of which is mutagenic PCR in which the desired mutation is synthesized as part of a PCR priming oligonucleotide (Hu et al., 2003). Correct installation of the mutation is confirmed by DNA sequencing. The sequence of the wild type *aroG* gene from *E. coli* C is given in SEQ ID No. 18. A preferred mutation is a point mutation that changes amino acid 150 of AroG from proline to leucine, for example by changing codon 150 from CCA to CTA (Hu et al, 2003). In a more preferred embodiment, codon 150 is changed from CCA to CTG, which is a preferred codon in *E. coli.* This particular allele of *aroG* is preferred, since the encoded DAHP synthase is completely resistant to inhibition by phenylalanine up to 3 mM, and it has a specific activity similar to the wild type enzyme (Hu et al., 2003).

Additional feedback resistant *aroG* alleles can be obtained by mutagenesis and selection for resistance to one or more phenylalanine analogs, such as beta-2-thienylalanine, p-fluorophenylalanine, p-chlorophenylalanine, o-fluorophenylalanine, and o-chlorophenylalanine, followed by demonstrating that the mutation causing the resistance is linked to the *aroG* gene (Ger et al., 1994; US Patent 4,681,852). Linkage to *aroG* can be demonstrated directly by DNA sequencing or enzyme assay in the presence and absence of phenylalanine, (Ger et al., 1994) or indirectly by phage mediated transduction and selection for a genetic marker at or near the *aroG* locus that can be selected, either for or against (US Patent 4,681,852). Such a genetic marker can be a deletion or point mutation in the *aroG* gene itself, or a mutation in any suitable closely linked gene such as *nadA* in case *of E. coli.* For an example in *E. coli,* after mutagenesis and selection for phenylalanine analog resistance, individual mutants or pools of mutants can be used as donors for P1 mediated transduction into a naive recipient that is deleted for all three DAHP synthase genes, *aroG, aroF,* and *aroH,* and selecting for growth on an appropriate minimal medium. The transductants will then be enriched for mutations in the desired gene(s). Alternatively, after mutagenesis and selection for analog resistance, individual mutants or pools of mutants can be used as donors for P1 mediated transduction into a naive recipient strain that contains a null mutation in the *nadA* gene, again selecting for growth on an appropriate minimal medium lacking nicotinamide. Another approach is to select for resistant mutants in a strain background that contains a transposon, for example Tn10, insertion near the *aroG* gene, such as in the *nadA* gene. P1 transduction from analog resistant mutants into a strain background that does not contain said transposon and selecting for tetracycline or other appropriate antibiotic resistance will enrich for the desired *aroG* mutations. In all such approaches, feedback resistance is ultimately confirmed by enzyme assay and DNA sequencing of the gene. We shall refer to alleles of *aroG* that are resistant to feedback inhibition as *aroG**.

Strain WM191 (*ΔtyrR, ΔaroF*) was derived from YMC9 (ATCC 33927). The two step gene replacement method (Jantama et al., 2008a) was used to install clean deletions in both *tyrR* and *aroF,* to give strain WM191. Next, a *nadA::Tn10* allele was transduced in from CAG12147 (CGSC 7351, Coli Genetic Stock Center, Yale University) to give strain WM189 (*ΔtyrR, ΔaroF, nadA::Tn10*). Selection was on LB plus tetracycline HCl (15 mg/l). Strain RY890 (*ΔtyrR::kan, aroF363*) was derived from MM294 (ATCC 33625) in three steps by P1 transduction. The donor strains, in order, were JW1316-1 (CGSC 9179, Coli Genetic Stock Center, Yale University), NK6024 (CGSC 6178, Coli Genetic Stock Center, Yale University), and AB3257 (CGSC 3257, Coli Genetic Stock Center, Yale University), and the three selections, in order, were LB plus kanamycin sulfate (50 mg/l), LB plus tetracycline hydrochloride (15 mg/l), and NBS minimal glucose (Jantama et al., 2008a) with thiamine HCl (5 mg/1).

WM189 was mutagenized with UV light to about 20% survival and plated on NBS minimal glucose medium (Jantama et al., 2008a) containing o-fluorophenylalanine (1 mM), thiamine (5 mg/l), and nicotinamide (1 mM). Colonies from each of several plates were collected into separate pools, and P1vir lysates were made on each pool. These lysates were used to transduce WM191 to tetracycline resistance (15 mg/l) on LB medium, and the colonies obtained were replica plated to NBS minimal glucose medium containing o-fluorophenylalanine at 1 mM, thiamine (5 mg/l), and nicotinamide (1 mM). Colony replicas that survived both tetracycline and analog were assumed to contain a feedback resistant mutation in *aroG.* Eight individual colonies from 5 independent pools were chosen for DNA sequencing. The *aroG* coding regions were amplified by polymerase chain reaction and sequenced. The results, shown in Table 4, revealed that each of the eight strains contained a point mutation in their *aroG* gene. Some of the alleles were identical to published alleles, but some were novel.

A P1vir lysate from one of the pools described above was used to transduce RY890 (which has an *aroG* wild type allele) to tetracycline resistance and resistance to o-fluorophenylalanine (0.3 mM) by replica plating as described above. Four colonies, named RY893, RY897, RY899, and RY901, were picked for DNA sequencing (Table 4), and again, two of the alleles were identical to a published allele, but two were novel. Strain RY902, which is isogenic to the latter four strains, but contains a wild type *aroG* gene, was constructed as a control, by transduction from CAG12147. These five strains were grown overnight in shake flasks in 25 ml NBS minimal glucose (15 g/l) plus thiamine HCl (5 mg/l) and nicotinamide (1 mM). The resulting cells were harvested by centrifugation, resuspended to be rinsed with 10 ml water, re-centrifuged, and resuspended in 0.5 ml of 50 mM potassium phosphate, pH 7.0. The suspended cells were lysed by vortexing with three drops of chloroform, and the crude lysate was assayed for DAHP synthase activity using a method similar to a method described in the literature (Hu et al., 2003), with the following modifications. The phosphate buffer was 50 mM (final concentration), pH 7.0, the final erythrose-4-phosphate concentration was 2 mM, the final phosphoenol pyruvate concentration was 5 mM, the incubation temperature was 30°C, and the reaction was stopped at 10 minutes. We define 1 mU as the activity that produced 1 nMole of DAHP per minute per milligram protein. To test for feedback resistance, each crude lysate was assayed with or without phenylalanine at a final concentration of 18 mM. The assay results are shown in Table 5. The enzymes showed varying specific activity and resistance to phenylalanine, but all of selected mutant versions that were tested were significantly more resistant than the wild type controls.

The *aroG* alleles from RY893, RY899, RY901, and RY902, described above, were introduced into a muconic acid producing strain background as follows. P1vir lysates from the *aroG** and *aroGwt* donor strains were used to transduce MYR219 (*E*. *coli* C, Δ*aroE, Δack::P₁₅-aroB,* pMG37) to tetracycline HCl resistance (15 mg/1), to give new strains RY903, RY909, RY911, and RY912, respectively. Each of these strains was then transduced to kanamycin sulfate resistance (50 mg/l) using a P1vir lysate of JW1316-1, to introduce the Δ*tyrR::kan* allele, to give strains RY913, RY919, RY921, and RY922, respectively. Spectinomycin selection was maintained throughout to maintain the muconic plasmid. The resulting four strains were grown for 48 hours at 37°C in shake flasks in 25 ml NBS minimal medium (Jantama et al., 2008a) containing supplements of 20 g/l glucose, 0.2 M MOPS buffer, pH 7.4, nicotimamide (1 mM), phenylalanine (100 mg/l), tyrosine (100 mg/l), tryptophan (100 mg/1), p-hydroxybenzoic acid (1 mg/l), p-aminobenzoic acid (1 mg/1), 2,3-dihydroxybenzoic acid (1 mg/l), phenol red (10 mg/l), and ammonium sulfate (1 g/l). The pH was kept close to 7 as estimated by eye from the color of the phenol red, against a pH 7.0 standard, by manual addition of 1 ml aliquots of 1.0 M KOH as called for to the shake flasks. The muconic acid produced was assayed by HPLC as described above, and the results are shown in Table 6. All three strains that contain a feedback resistant *aroG** allele produced more muconic acid than the isogenic strain containing the wild type *aroG* allele. In a separate experiment disclosed herein, strain MYR205, containing *aroG* on multicopy plasmid pCP32AMP, produced 1.5 g/l muconic acid in a shake flask. Thus, the inventors have shown that the combination of Δ*tyrR* and single copy chromosomal *aroG** can perform well compared to an isogenic *aroG* plasmid containing strain to produce muconic acid in shake flasks. The inherent superior genetic stability of the chromosomal alleles compared to plasmid alleles, plus the alleviation of the need for a selective medium to hold in a plasmid, makes the novel strains described herein more suitable for large scale commercial fermentations. Furthermore, no chemical inducer was required for expression of muconic acid pathway genes. Thus, strains described above are improved over those of the prior art (Niu et al., 2002), all of which contain the gene for overexpression of DAHP synthase on an undesirable multicopy plasmid.

In a similar fashion to that described above for AroG, a mutation that leads to an AroF or AroH isozyme that is resistant to feedback inhibition by tyrosine can be installed on a plasmid or in the chromosome. A preferred mutation is a point mutation that changes amino acid 148 of AroF from proline to leucine, for example by changing codon 148 from CCG to CTG (Weaver et al., 1990), to give a gene named *aroF*.* Other alleles of *aroF** can be isolated by resistance to tyrosine analogs (for example o-fluorotyrosine, m-fluorotyrosine, p-fluorophenylalanine, etc.) in a fashion analogous to that described above for *aroG** alleles. *aroF** alleles can be selected, enriched for, and transduced by linkage to a transposon or a kanamycin resistance insertion, for example in a closely linked Δ*yfiR::kan* as in a strain such as JW2584 (CGSC 10051, Coli Genetic Stock Center, Yale University).

### Example 3

### Deletion of aroE from chromosomal DNA and muconic acid production

In this example the effect of overexpression of *aroB* and *aroG* on multicopy plasmids as well as the expression of genes coding for proteins functional in the muconic acid pathway was investigated. Strain MYR34 containing a deletion in the *aroE* gene coding for shikimate dehydrogenase was used as parent strain in these studies. The deletion of chromosomal copy of *aroE* was accomplished in a fashion similar to that described above in Example 1. When MYR34 was transformed with the plasmid pCP32AMP overexpressing the *aroG* gene coding for DAHP synthase protein functional in the shikimic acid pathway, there was a significant increase in the accumulation of DHS. When MYR34 was transformed with the plasmid expressing *aroB* from a constitutive promoter, no significant increase in the accumulation of DHS was noticed. However, when the *E. coli* strain MYR34 was transformed with the plasmid expressing both *aroB* and *aroG* genes, there was an increase in the accumulation of DHS than observed with MYR34 transformed with *aroG* alone thus suggesting *aroB* as a secondary bottleneck in DHS production (Figure 5).

In the experiments presented in Figure 6, the effect of an additional copy of the *aroB* gene integrated into the host chromosomal DNA was examined. In the *E. coli* strain MYR170 derived from MYR34, an additional copy of the *aroB* gene under the control of the P₁₅ promoter was integrated into the host chromosome at the *ack* locus. When MYR170 strain was transformed with the pCP32AMP plasmid, there was a slight increase in the DHS accumulation when compared to the DHS accumulation detected in the MYR34 strain transformed with the same plasmid. This slight increase in the accumulation of DHS in the MYR170 can be attributed to an additional copy of *aroB* gene integrated into the host chromosomal DNA. When MYR170 was transformed with pCP54 expressing both *aroB* and *aroG* genes, there was a further increase in the DHS accumulation suggesting *aroB* as a secondary bottleneck in DHS production.

Figure 7 provides the results on muconic acid production with the *E. coli* strains MYR34 and MYR170. Having established that in the *aroE* deletion strains MYR34 and MYR170, with overexpression of *aroB* and *aroG* genes there is an accumulation of DHS, efforts were made to see whether the expression of "muconic pathway" genes coding for the proteins functional in muconic acid production pathway would lead to conversion of DHS into cis, cis-muconic acid. In these experiments, the *E. coli* stains MYR34 and MYR170 were transformed either with the plasmid pMG37 alone or with both plasmids pMG37 and pCP32AMP. The plasmid pMG37 expresses *aroZ, aroY* and *catAX* genes coding for proteins functional in muconic acid pathway. The muconic acid production in both MYR34 and MYR170 increased when these bacterial strains were transformed with both the plasmids pCP32AMP and pMG37 when compared to the muconic acid production in these two strains transformed only with pMG37 plasmid suggesting that in these strains *aroB* expression is the bottleneck for cis, cis-muconic acid production.

### Example 4

### Overexpression of tktA

Transketolase encoded by *tktA* is a key enzyme in the pentose phosphate pathway and is thought to be limiting for the production of erythrose-4-phosphate, one of the key intermediates in the production of muconic acid. Overexpression of *tktA,* which encodes transketolase, by installing the gene with its native promoter on a multicopy plasmid (Sprenger et al, 1995, 1995a), is known to improve flux into the aromatic pathway (Draths et al., 1992). However, such plasmids are unstable, and often require antibiotic selection for maintenance. Another approach in the prior art was to add one additional copy of the *tktA* gene to the chromosome of the host strain (Niu et al., 2002). However, one additional copy of *tktA* with its native promoter is not sufficient to saturate the aromatic pathway with erythrose-4-phosphate, since its native promoter is not very close to the ideal. As such, the process needs substantial improvement.

Improved overexpression of *tktA* can be obtained, for example, by substituting the native *tktA* promoter in the chromosome with a strong constitutive promoter, for example a P₁₅ or P₂₆ promoter from *Bacillus subtilis* phage SPO1 (SEQ ID No. 1 and SEQ ID No. 2, respectively), or the P_{R} promoter from bacteriophage lambda (SEQ ID No. 3). This is accomplished in two steps as described in Example 1, except that the *cam^{R}, sacB* cassette is used to replace the native chromosomal *tktA* promoter in the first step. In the second step, the strong constitutive promoter is installed by transforming with a linear DNA comprising the strong constitutive promoter, flanked by at least 50 bases of the 5' end of the *tktA* coding region on the downstream side and at least 50 base pairs of homology just upstream of the native *tktA* promoter on the upstream side of the strong constitutive promoter, and selecting for sucrose resistance. Improved expression from such an expression cassette is also accomplished by increasing the stability of the mRNA that is transcribed from the expression cassette. Improvement of the mRNA stability is accomplished by adding a stem loop structure at either the 5' end of the mRNA, the 3' end of the mRNA, or both. A stem-loop structure is often present at the end of an mRNA that is naturally terminated by a rho-independent transcription terminator, but if it is not, then a rho-independent transcription terminator can be added to the DNA sequence by well known methods of genetic engineering (ligation, PCR, etc.). Such a terminator can be comprised of an inverted repeat of between 4 and 20 bases in each repeat, separated by a "loop" of 3 or more bases, and followed by a region of one or more bases that is enriched for T's (deoxythymidine). The inverted repeats are rich in G's and C's (deoxyguanidine and deoxycytidine). Similarly, a stem-loop can be constructed into the 5' end of an mRNA by inserting a DNA sequence just downstream from the start point of transcription, but before the ribosome binding site, that contains a stem-loop as described above, but without the T-enriched region. An example of this is given in association with the P₁₅ promoter (SEQ ID No. 1).

In the analysis of the effect of overexpression of the *tktA* gene on the flow of carbon through the shikimic acid pathway, *E. coli* strain MYR170 was used as a parental strain. MYR170 has a deletion in the *aroE* gene coding for shikimate dehydrogenase enzyme and an additional copy of the *aroB* gene at the *ack* locus.

In the experiments described in the Figures 8, 9 and 10 two different plasmids namely pCP32AMP and pCP50 were used. The plasmid pCP32AMP expresses only the DAHP synthase *aroG* gene from its native promoter and the plasmid pCP50 expresses the transketolase gene *tktA* from its native promoter along with *aroG* gene. MYR170, having an *aroE* deletion and an additional copy of *aroB* gene under the control of P₁₅ promoter integrated at the *ack* locus of the chromosomal DNA, was transformed individually with pCP32AMP and pCP50 plasmids. As shown in Figure 8 the DHS accumulation was increased further with the expression of *aroG* gene along with *tktA* gene when compared to the *E. coli* cells expressing only *aroG* gene.

Figure 9 provides data on the DHS yield in two different strains namely MYR34, MYR170 transformed with the plasmid pCP32AMP or pCP50. MYR34 strain having *aroE* gene deletion yielded 0.1 gram of DHS per gram of glucose consumed. The DHS yield in the MYR34 increased to 0.15 gram of DHS per gram of glucose consumed when this strain was transformed with the pCP32AMP plasmid with *aroG* gene overexpression. MYR170 has an additional copy of *aroB* gene inserted at the *ack* locus. As a result of the presence of this additional copy of the *aroB* gene, the yield for DHS production in the MYR170 strain transformed with pCP32AMP was slightly higher than the DHS yield noted in the MYR34 strain transformed with pCP32AMP. Thus the presence of an additional copy of *aroB* in MYR170 caused an increased carbon flow through shikimic acid pathway. Further increase in the DHS yield was observed when the MYR170 strain was transformed with plasmid pCP50 expressing both *aroG* and *tktA* genes. Thus the presence of additional copy of *tktA* accounted for an increase carbon flow through shikimic acid pathway. More specifically, the effect of presence of additional *aroB* and *tktA* genes caused an additive effect on DHS yield.

MYR261 used in the experiments described in Figure 10 was engineered to integrate an additional copy of *tktA* gene into the chromosomal DNA of MYR170 at the *poxB* locus. The desired gene replacement (*poxB::tktA*) in the MYR261 strain was confirmed via PCR. MYR261 was transformed either with pCP32AMP *(aroG* overexpression) plasmid or pCP50 (*aroG* and *tktA* over expression) plasmid. As a control, MYR170 was transformed with pCP32AMP plasmid. As the result shown in Figure 10 indicate, the presence of an additional copy of *tktA* gene in the chromosomal DNA of MYR261 increased the titer for DHS production with pCP32AMP plasmid when compared to the titer for DHS production observed in the MYR170 strain transformed with the same plasmid. Further increase in the transketolase level in the MYR261 strain when transformed with the plasmid pCP50 over expressing transketolase led to further increase in the titer for DHS production. The enzyme encoded by *poxB,* PoxB, or pyruvate oxidase, produces acetate as a reaction product. As such, the deletion of *poxB* that results from the insertion of *tktA* as described herein removes a potentially active pathway for acetate production. Similarly, simultaneous insertion of *P₁₅aroB* and deletion of *ackA,* which encodes AckA, or acetate kinase, as described below in Example 12 below, removes another potentially active pathway to acetate. Production of acetate is generally undesirable in fermentations (Jantama et al., 2008b). As such, these deletions can be useful for reducing acetate production.

Figure 11 provides the titer for muconic acid and acetic acid production in MYR170, MYR261 and MYR305 strains of *E.coli* after transformation with the plasmids pCP32AMP and pMG37. MYR305 is derived from MYR170 by means of deleting *poxB* gene from the chromosomal DNA while MYR261 is a MYR170 derivative wherein the *poxB* gene has been inactivated by means of inserting an additional copy of the *tktA* gene. As mentioned above, the plasmid pCP32AMP expresses the *aroG* gene coding for DAHP synthase protein functioning in the shikimic acid biosynthetic pathway leading to the accumulation of DHS due to the deletion of *aroE* gene in the *E. coli* strains MYR170, MYR261 and MYR305. With the expression of muconic pathway genes namely *aroZ, aroY* and *catAX* on the plasmid pMG37, the DHS is converted into cis, cis-muconic acid as illustrated in Figure 2. With the presence of an additional copy of the *aroB* gene and the *tktA* gene in the MYR261 strain, there was a slight increase in the production of muconic acid accompanied by a decrease in the accumulation of acetic acid.

### Example 5

### Overexpression of talA or talB

The *talB* gene encodes the predominant transaldolase in *E. coli,* but the *talA* gene also encodes a minor transaldolase. Overproduction of transaldolase is known to improve flux into the aromatic pathway (Lu and Liao, 1997; Sprenger, 1995; Sprenger et al, 1995b). In the prior art, this was accomplished by overexpression of the *tal* gene (now known to be the *talB* gene) on a multicopy plasmid from its native promoter (Lu et al., 1997, Sprenger et al., 1995b). However, such plasmids are unstable, and require antibiotic selection for maintenance. Thus, there is a need for an improved process. Improved expression of *talB* can be obtained, for example, by substituting the native *talB* promoter in the chromosome with a strong constitutive promoter, for example a P₁₅ or P₂₆ promoter from *Bacillus subtilis* phage SPO1 (SEQ ID No. 1 and SEQ ID No. 2, respectively), or the P_{R} promoter from bacteriophage lambda (SEQ ID No. 3). This is accomplished in two steps as described in Example 1, except that the *cam^{R}, sacB* cassette is used to replace the native chromosomal *talB* promoter in the first step. In the second step, the strong constitutive promoter is installed by transforming with a linear DNA comprising the strong constitutive promoter, flanked by at least 50 bases of the 5' end of the *talB* coding region on the downstream side and at least 50 base pairs of homology just upstream of the native *talB* promoter on the upstream side of the strong constitutive promoter, and selecting for sucrose resistance. The *talA* gene can also be overexpressed by a similar method, but it is preferred to over express the *talB* gene, since it encodes the predominant activity (Sprenger, 1995; Sprenger et al, 1995b). See Example 4 for more details on construction of the expression cassette designed for overexpression.

### Example 6

### Expression of aroZ, aroY and catAX genes

To demonstrate conversion of endogenous DHS produced by *E. coli* into muconic acid, heterologous genes *catAX* from *Acinetobacter sp. ADP1, aroY* from *Klebsiella pneumoniae,* and *quiC* from *Acinetobacter sp. ADP1,* were cloned under strong constitutive promoters (P₁₅, P_{R}, and P₂₆, respectively) in a low-copy plasmid, pCL1921 (Lerner and Inouye, 1990) to generate a 'muconic plasmid' pMG37. MYR34 strain derivatives carrying the empty vector (pCL1921) or pMG37 were grown at 37°C for 17 hrs. in a shake flask medium (NBS minimal medium supplemented with aromatic amino acids and vitamins) containing 2% glucose. Supernatants were collected and analyzed by HPLC. In contrast to MYR34/pCL1921 which shows accumulation of DHS, MYR34/pMG37 shows production of muconic acid (Figure 12). No significant amount of DHS, or intermediate products such as PCA and catechol were detected from the latter strain, suggesting that the heterologous genes expressed from pMG37 were functional and sufficient.

### Example 7

### Comparison of aroZ homologs

Three different *aroZ* homologs and analogs were compared (Figure 13) for their ability to divert DHS into the muconic acid production pathway. *quiC* from *Acinetobacter sp. ADP1, asbF* from *Bacillus thuringiensis,* and *qa-4* from *Neurospora crassa,* are reported to encode for proteins that have AroZ-like activity (Elsemore and Ornston, 1995; Fox et al, 1995; Rutledge, 1984). Each of these genes was codon-optimized for expression in *E. coli* and synthesized by GeneArt (Invitrogen), and cloned under a strong constitutive P₂₆ promoter in low-copy 'muconic plasmid' which also expressed *catAX* and *aroY* genes from the P₁₅ and P_{R} promoters, respectively. MYR34/pCL1921, MYR34/pMG37 (muconic plasmid with *quiC* as *aroZ*), MYR34/pMG47 (muconic plasmid with *asbF* as *aroZ*), and MYR34/pMG70 (muconic plasmid with *qa-4* as *aroZ*) were grown at 37°C for 48 hrs. in a shake flasks with minimal medium containing 2% glucose, the aromatic amino acids and aromatic vitamins. Supernatants were collected and analyzed by HPLC. As expected, empty vector transformed MYR34 accumulated DHS and produced no muconic acid. The two *aroZ* homologs and the one analog examined were functional in diverting DHS towards muconic acid production, but to a varying degree. The MYR34 derivative expressing *quiC* gene was most robust and showed nearly 100% conversion of DHS to muconic acid with insignificant amount of DHS retention. The MYR34 derivative expressing the fungal *aroZ* homologue, *qa-4,* followed close with about 80% conversion of DHS to muconic acid and 20% DHS retention. Lastly, the MYR34 derivative expressing *asbF* gene showed only 50% conversion of DHS to muconic acid and 50% DHS retention. Taken together, under our shake flask assay conditions, the expression and/or activity of *quiC* gene appeared to be the highest compared to that of other *aroZ* homologs.

### Example 8

### Chromosomal integration of catAX, aroY and quiC

Muconic acid can be produced by strains that contain only chromosomally integrated single copies of *catA-X, aroY* and *quiC* expressed from constitutive promoters at *adhE* locus.

MYR170 (*ΔaroE, Δack::P₁₅-aroB*), a high DHS producer, was the host strain used for integrating the muconic acid pathway genes at the *adhE* locus in the chromosome (SEQ ID No. 41). The resulting strain MYR352 was transformed with plasmids YEp24 (medium-copy, empty vector), pCP32AMP (medium-copy, *aroG* expressed from native promoter), or pCP50 (medium-copy, *aroG* and *tktA* expressed from their respective native promoters) to generate derivative strains. The latter two plasmids were used to increase DHS production. Strains were grown at 37°C for 72 hrs. in shake flask medium containing 2% glucose as described above. Supernatants were collected at 72 hrs. and analyzed by HPLC. As expected, the *aroG* and *aroG*/*tktA* transformed MYR352 derivatives showed an overall increase in total product formation compared to an empty vector control (Figure 14). All of the MYR352 transformants produced measurable titers of muconic acid, demonstrating for the first time that muconic acid can be produced by a strain that contains only integrated "muconic pathway" genes and without a fed chemical inducer of gene expression.

Not all DHS that was produced in any of these MYR352 derivative strains was converted to the end product muconic acid. Instead, there was a significant amount of catechol accumulation (Figure 14), suggesting that expression or activity of *catAX* is limiting when it is expressed from a single copy on chromosome. Since the major accumulating intermediate was catechol, it is likely that *quiC* and *aroY* gene expression and/or activity is sufficient in the MYR352 strain background for muconic acid synthesis.

The MYR352 strain derivatives were compared in parallel with analogous MYR219 strain derivatives. MYR219 strain is same as MYR170 strain but contains low-copy plasmid pMG37 expressing muconic acid pathway genes. Thus, the main difference between MYR352 and MYR219 strains is with reference to the dosage of muconic acid pathway genes (1 copy vs. about 5 copies, respectively). In contrast to MYR352 derivative strains, MYR219 derivative strains showed very little accumulation of catechol or other intermediates, and successfully produced the end product muconic acid. Together, these results indicate the need for increasing *catAX* activity in strains such as MYR352.

### Example 9

### Expression of catAX

Accumulation of catechol and inefficient production of muconic acid in MYR352 strain is due to limiting dosage and/or activity of the *catAX* gene product(s). As described above, MYR352 contains Δ*aroE, Δack::P₁₅-aroB* and chromosomally integrated single copies of *catAX, aroY* and *quiC* genes under strong constitutive promoters. This strain was transformed with medium-copy empty vector control (YEp24) or *aroG*/*tktA* expression plasmid (pCP50) to increase carbon flow into the aromatic amino acid synthesis pathway and produce high amounts of DHS. Growth of transformed strains at 37°C for 72 hrs in shake flask medium supplemented with 2% glucose as described above resulted in accumulation of catechol intermediate. This result suggested that *catAX* activity may be insufficient in MYR352. To confirm this hypothesis, the ability of one or more muconic acid pathway genes expressed from low-copy plasmid to alleviate catechol accumulation in MYR352/pCP50 was tested (Figure 15). Specifically, MYR352/pCP50 was further transformed with low-copy empty vector control (pCL1921) or plasmids expressing all three genes, two genes, or one gene of the muconic acid production pathway. The derivative strains were assayed in a shake flask experiment as described above. While increasing the dosage of *aroY* alone (from pMG27) or *quiC* alone (from pMG39) did not alleviate catechol accumulation , expression of all of the muconic acid pathway genes (from pMG37) or *catAX* and *aroY* together (from pMG33), resulted in successful conversion of catechol to muconic acid. Further, expression of *catAX* alone (from pMG31) was sufficient for production of muconic acid and preventing accumulation of catechol.

### Example 10

### Constructing a leaky aroE mutation

In the prior art process for producing cis, cis-muconic acid, the host strain contains a mutation in the *aroE* gene named *aroE353,* which is a null mutation. As a result, the strain requires the feeding of the aromatic amino acids (phenylalanine, tyrosine, and tryptophan) and aromatic vitamins made from the shikimate pathway (p-hydroxy benzoic acid, p-amino benzoic acid, and 2,3-dihydroxy benzoic acid). The aromatic amino acids are too expensive to be fed in a commercially attractive process. As such, the *prior art* process needs a substantial improvement. This can be accomplished by installing a leaky version of the *aroE* gene, that we shall call *aroE*.* Leaky mutations are obtained by first generating a missense mutation that changes one amino acid in the *aroE* coding sequence that results in a null phenotype. This can be accomplished by any form of mutagenesis and screening for simultaneous auxotrophy for the six aromatic compounds listed above. A preferred method is to create a pool of mutant *aroE* genes by error-prone PCR mutagenesis, using *Taq* DNA polymerase, using wild type *E. coli* C genomic DNA as the template, and using PCR oligonucleotide primers that hybridize about 1000 base pairs upstream and 1000 base pairs downstream of the *aroE* coding region. The resulting pool of linear DNA molecules is used to transform an *E. coli C* derivative that produces cis, cis-muconic acid, and which contains an integrated *cam^{R}, sacB* cassette that has replaced the *aroE* coding region (see Example 4 for a related example), and selecting for sucrose resistance. The transformants are then screened for auxotrophs that have lost chloramphenicol resistance and require the six aromatic compounds listed above. Several independent auxotrophs are picked and tested for revertability by plating about 10⁷,10⁸, or 10⁹ cells (rinsed in minimal glucose medium) on a minimal glucose plate without the six aromatic compounds. Revertants that give rise to colonies on the plates are picked and tested for production of cis, cis-muconic acid, but without production of substantial levels of aromatic amino acids. Among such revertants will be strains that carry one or more mutations in the *aroE* gene, such that the AroE enzyme provides enough aromatic amino acids and vitamins for growth, but not a surplus of these aromatic compounds. Another method to obtain a leaky *aroE* mutant is to install one of the classical revertable *aroE* mutants, such as *aroE353* and *aroE24* (both available from the Coli Genetic Stock Center at Yale University, New Haven, CT, USA), into a cis, cis-muconic acid producing strain, and select for revertants as described above.

### Example 11

### Import of glucose by facilitated diffusion

One of the substrates in the first committed step of the aromatic pathway is phosphoenolpyruvate (PEP). PEP is also the source of phosphate and energy for importing glucose and some other sugars by the bacterial phosphotransferase system (PTS). Thus, when a bacterium is growing on a PTS-dependent sugar, there is competition between the PTS and the aromatic pathway for PEP. As such, a significant improvement in increasing flux to the aromatic pathway can be achieved by deleting the PTS and providing an alternative pathway for sugar uptake. One solution to this problem is to replace the PTS with the *E. coli* GalP permease, a proton symporter that works reasonably well for glucose uptake (US Patent 6,692,794). However, the proton symporter still uses energy to maintain the proton gradient that is necessary to drive the permease. As such, there is a need for even further improvement in the process.

Some sugars, such as xylose, can be imported by a transporter protein that derives energy from hydrolysis of ATP (adenosine triphosphate). Once again, if the energy-dependent transporter can be replaced by a transporter that requires less energy, then an improvement can be made, since the energy inherent in the ATP can be conserved for other beneficial uses.

A significant improvement can be obtained by using a facilitated diffusion transporter, which expends no energy for the importation of the sugar (Parker et al, 1995; Snoep et al, 1994). For example, the glucose facilitator from *Zymomonas mobilis,* encoded by the *glf* gene, can be used in place of, or in addition to, the PTS in 3-dehydroshikimate (DHS) producing strains (Yi et al., 2003). However, these strains still rely at least partly on GalP for glucose import. Since GalP requires energy in the form of a proton gradient for importation of glucose, there is a need for improvements in the efficiency of glucose import for muconic producing strains.

A cassette for expression of *glf* plus a glucokinase gene, *glk,* also from *Z*. *mobilis,* can be assembled with a strong constitutive promoter, for example P₂₆. This cassette can then be integrated into the genome of a host strain at a location that will not interfere with production of the desired compound, which in this case is cis, cis-muconic acid. An example of such a location in the *E. coli* chromosome is the threonine degradation operon, *tdcABCDEFG.* If the growth medium contains no threonine, then this operon is not needed or expressed, so an insertion of an expression cassette in that operon does not interfere with metabolism.

To achieve the above described improvement, one or more of the genes encoding a PTS function are deleted, using a method similar to that disclosed in Example 1. For example, one or more of *ptsH, ptsI, crr,* or *ptsG* can be deleted. Next *galP* is deleted. The P₂₆-*glf, glk* cassette can then be installed in two steps, similar to those described in Example 1. In the first step, a *cam^{R}, sacB* cassette is integrated at the *tdc* operon, using a linear DNA derived from pAC21 (SEQ ID No. 15), and selecting for chloramphenicol (30 mg/l) resistance. In the second step, the P₂₆-*glf*, *glk* cassette is integrated at the *tdc* operon, using a linear DNA derived from pAC19 (SEQ ID No. 15), selecting for sucrose resistance and screening for chloramphenicol sensitivity, and in this case, improved growth on minimal glucose medium.

To test whether facilitated diffusion of glucose could substitute for the conventional glucose import systems in *E. coli,* the *ptsHI* genes and the *galP* gene were deleted from MYR34 (Δ*aroE*), and then the P₂₆-*glf, glk* cassette was integrated at the *tdc* operon, using a linear DNA derived from pAC19 (SEQ ID No. 14), to give strain MYR217. MYR217 grows reasonably well on a minimal glucose medium supplemented with the required three aromatic amino acids and three aromatic vitamin-like compounds (Figure 16). However, strain MYR31, which contains deletions of *ptsHI* and *galP,* but does not contain the *glf, glk* cassette did not show any measurable growth (Figure 16). Thus, facilitated diffusion is sufficient to replace the two conventional glucose import systems in our strain background.

To test whether facilitated diffusion is useful for producing compounds derived from the aromatic pathway, MYR34 and MYR217 were transformed with pCP54 (*aroG, aroB*) and pCP55 (*aroG, aroB, tktA).* Production of the aromatic intermediate 3-dehydroshikimate (DHS) in shake flasks was compared for these two strains (Figure 17). With either pCP54 or pCP55, the strain using facilitated diffusion produced as much or more DHS than the strains using the conventional glucose import systems. Production of DHS is a good proxy for muconic acid production in engineered *E. coli* strains, so we can conclude that facilitated diffusion of glucose is a useful improvement for muconic acid production.

### Example 12

### Overexpression of the aroB gene

Expression of the *aroB* gene is reported to be rate limiting for cis, cis-muconic acid production (Niu et al., 2002). In the prior art, this was allegedly solved by integrating a second copy of the *aroB* gene with its native promoter. However, this is insufficient to alleviate the *aroB* limitation, since the native promoter and ribosome binding site of the *aroB* gene are far from ideal. As such, the process needs substantial improvement.

Improved overexpression of *aroB* can be obtained, for example, by replacing the native *aroB* promoter in the chromosome with a strong constitutive promoter, for example a P₁₅ or P₂₆ promoter from *Bacillus subtilis* phage SPO1 (SEQ ID No. 1 and SEQ ID No. 2, respectively), or the P_{R} promoter from bacteriophage lambda (SEQ ID No. 3). This is accomplished in two steps as described in Example 4, except that the *cam^{R}, sacB* cassette is used to replace the native chromosomal *aroB* promoter and/or ribosome binding site in the first step. In the second step, the strong constitutive promoter is installed by transforming with a linear DNA comprising the strong constitutive promoter, followed by a ribosome binding site and at least 50 bases from the 5' end of the *aroB* coding sequence, including the ATG start codon, on the downstream side, and at least 50 base pairs of homology just upstream of the native *aroB* promoter on the upstream side of the strong constitutive promoter, and selecting for sucrose resistance. In addition to, or instead of, installing a stronger promoter, using a similar method, a stronger ribosome binding site, for example, AGGAGG, can be installed about 4 to 10 base pairs upstream of the ATG translation start codon of *aroB.* A copy of such a synthetic cassette, for example, a *P₁₅-aroB* cassette, can be integrated in the chromosome at a locus distinct from the native *aroB* locus, for example at the *ack* locus. Simultaneous deletion of the *ack* gene, as well as deleting the *poxB* gene as in Example 4 can help to reduce formation of unwanted acetate during fermentations.

### Example 13

### Decreasing flux through the oxidative branch of the pentose phosphate pathway

The erythrose-4-phosphate that is needed for the first committed step in the aromatic pathway is derived from the non-oxidative portion of the pentose phosphate pathway (PPP). There are two different pathways by which carbon can enter the PPP. The first is from glucose-6-phosphate by the enzymes glucose-6-phosphate dehydrogenase (encoded by the *zwf* gene), 6-phosphogluconolactonase (encoded by the *pgl* gene), and 6-phophogluconate dehydrogenase (encoded by the *gnd* gene), to give ribulose-5-phosphate. In the last of these three steps, one carbon is lost as CO₂. This path into the PPP is called the oxidative branch of the PPP. Ribulose-5-phosphate is then converted into a variety of other sugar phosphates by the action of isomerases, epimerases, transketolase, and transaldolase. This group of reversible reactions, starting with ribulose-5-phosphate, is called the non-oxidative branch of the PPP. The second path by which carbon can enter the PPP is through fructose-6-phosphate and glyceraldehye-3-phosphate (both of which come from the Embden-Myerhoff pathway, also known as glycolysis), which are combined and rearranged by transaldolase and transketolase to give the variety of other sugar phosphates, one of which is erythrose-4-phosphate. If carbon enters the PPP through this second route, then no CO₂ is lost. In order to improve the yield of cis, cis-muconic acid from glucose, the loss of CO₂ can be prevented by blocking the oxidative branch of the PPP, such that all carbon entering the PPP must come through a non-oxidative route from fructose-6-phosphate and glyceraldehye-3-phosphate. The blocking of the oxidative branch of the PPP is accomplished by deleting the zwf gene, using a two step method similar to that disclosed in Example 1 for deleting the *tyrR* gene.

### Example 14

### Increasing the flux to and through PEP to the aromatic pathway

It is desirable to ensure that PEP is not a rate limiting intermediate on the pathway to cis, cis-muconic acid. This is accomplished, for example, by increasing the recycling of pyruvate to PEP by the enzyme PEP synthetase, which is accomplished by integrating an overexpression cassette of the *pps* gene as described above in other examples. Another approach is to limit the consumption of PEP by pyruvate kinase, which in *E. coli* is encoded by the *pykA* and *pykF* genes. In this case, the approach is to decrease the activity of the enzyme(s). This is accomplished by deleting one or more genes that encode pyruvate kinase (as described in Example 1 for *tyrR*), or reducing the strength of expression of one or more of these genes, for example, by mutating the promoter, ribosome binding site, or coding sequence, such that the level of pyruvate kinase activity is decreased. For example, the RBS in front of the *E. coli pykA* gene is 5'CGGAGTATTACATG. The ATG translation start codon is underlined. This sequence can be mutated to CaGAGTATTACATG, CaaAGTATTACATG, CaatGTATTACATG, CaataTATTACATG, and so on, such that the RBS sequence is made less like the consensus RBS of AGGAGG by one base change at a time. Each mutated version is then introduced into the chromosome at the *pykA* locus, replacing the wild type, and cis, cis-muconic acid production levels are measured for improvement.

### Example 15

### Conferring growth on sucrose

Strains derived from *E. coli* C do not grow on sucrose as a sole carbon source. However, they can be genetically engineered to do so as disclosed in PCT Patent Application PCT/US11/064598. As such, a cis, cis-muconic acid producing strain can be engineered to grow on sucrose as disclosed in the above mentioned application.

### Example 16

### An improved producer of cis, cis-muconic acid

All of the features described in Examples 1 - 15 can be combined in one strain of *E. coli* by installing the features one after another. The resulting strain comprises an improved cis, cis-muconic acid producer. The resulting strain can then be even further improved by integrating a second copy of each overexpression cassette described above, one at a time, at a location separate from the location of the first copy. An example of a convenient and safe location is at a *Bsr*B1 restriction site just downstream from the terminator of *rrfF,* which encodes a ribosomal RNA. The desired cassette is ligated as a blunt linear DNA into the unique *Bsr*B1 site of plasmid pMH17F (SEQ ID No. 17). An example is the ligation of the *catAX* expression cassette to give a plasmid named *pcatAX.* In parallel, a *cam^{R}, sacB* cassette is ligated as a blunt fragment into pMH17F to give pMH28F (SEQ ID No. 19). A linear DNA derived from pMH28 by PCR or by restriction enzyme cutting is used to deposit the *cam^{R}, sacB* cassette at the *rrfF* site. Next, a linear DNA derived from *pcatAX* by PCR or by restriction enzyme cutting is used to install the second copy of the *catAX* cassette at the *rrfF* locus, using selection on sucrose. The resulting strain is then compared with its grandparent strain for cis, cis-muconic acid production to determine that *catAX* was a limiting step. By a similar method, each cassette from Examples 2 - 15 is tested for a rate limiting step. If a step is found to be rate limiting, then one or more additional copies of the relevant cassette is/are integrated at yet other appropriate locations in the chromosome, leading to still further improvements in cis, cis-muconic acid production, without the need for plasmids or inducible promoters.

### Example 17

### Production of cis, cis-muconic acid by fermentation

Cis, cis-muconic acid can be produced by genetically engineered microorganisms disclosed in the above Examples 1-15. The growth medium can vary widely and can be any medium that supports adequate growth of the microorganism. A preferred medium is a minimal medium containing mineral salts and a non-aromatic carbon source, such as glucose, xylose, lactose, glycerol, acetate, arabinose, galactose, mannose, maltose, or sucrose (see above for an example of a preferred minimal growth medium). For each combination of engineered microorganism and growth medium, appropriate conditions for producing cis, cis-muconic acid are determined by routine experiments in which fermentation parameters are systematically varied, such as temperature, pH, aeration rate, and compound or compounds used to maintain pH. As cis, cis-muconic acid is produced, one or more compounds must be fed into the fermentor to prevent pH from going too low. Preferred compounds for neutralizing the acid include alkaline salts such as oxides, hydroxides, carbonates, and bicarbonates of ammonium, sodium, potassium, calcium, magnesium, or a combination two or more of such alkaline salts.

Muconic acid production by MYR428 strain of *E. coli* in a 7 Liter fermentor is shown in Figure 18. MYR261 strain *of E. coli* with a genotype of Δ*aroE* Δ*ackA*::*P₁₅-aroB* Δ*poxB::tktA* was transformed with the plasmids pCP32AMP and pMG37 to generate MYR428. MYR428 was grown in a 7 liter fermentor as described above with glucose feeding for 48 hours. The final muconic acid titer was 16 g/l (see Figure 18).

After fermentation is complete, cells are removed by flocculation, centrifugation, and/or filtration, and the cis, cis-muconic acid is then purified from the clarified broth by a combination of one or more subsequent steps, for example precipitation, crystallization, electrodialysis, chromatography (ion exchange, hydrophobic affinity, and/or size based), microfiltration, nanofiltration, reverse osmosis, and evaporation.

| Table 1. Bacterial strains and plasmids used herein | |
|---|---|
| Bacterial strain / Plasmid | Characteristics |
| Bacterial Strains | |
| ATCC8739 | *Escherichia coli* "C" wild type |
| MYR34 | ATCC8739 *ΔaroE* |
| MYR170 | ATCC8739 *ΔaroE, ΔackA::P₁₅aroB* |
| MYR261 | ATCC8739 *ΔaroE, ΔackA::P₁₅aroB, ΔpoxB::tktA* |
| MYR305 | ATCC8739 *ΔaroE, ΔackA::P₁₅aroB, ΔpoxB* |
| MYR31 | ATCC8739 *ΔptsHI, ΔgalP* |
| MYR217 | ATCC8739 *ΔptsHI, ΔgalP, Δtdc::glf-glk, ΔaroE* |
| MYR352 | ATCC8739 *ΔaroE, ΔackA::P₁₅aroB, ΔadhE::P₁₅-catAX, P_{R}-aroY, P₂₆*-*quiC* |
| RY903 | *ΔaroE, ΔackA::P₁₅aroB,* pMG37, *aroG*20-893* |
| RY909 | *ΔaroE, ΔackA::P₁₅aroB,* pMG37, *aroG*20-899* |
| RY911 | *ΔaroE, ΔackA::P₁₅aroB,* pMG37, *aroG*20-901* |
| RY912 | *ΔaroE, ΔackA::P₁₅aroB,* pMG37, *aroGwt* |
| RY913 | *ΔaroE, ΔackA::P₁₅aroB,* pMG37, *aroG*20-893, ΔtyrR::kan* |
| RY919 | *ΔaroE, ΔackA::P₁₅aroB,* pMG37, *aroG*20-899, ΔtyrR::kan* |
| RY921 | *ΔaroE, ΔackA::P₁₅aroB,* pMG37, *aroG*20-901, ΔtyrR::kan* |
| RY922 | *ΔaroE, ΔackA::P₁₅aroB,* pMG37, *aroGwt, ΔtyrR::kan* |

| Plasmids | |
|---|---|
| YEp24 | 2µ yeast origin, *URA3*, Tc^{R}, pMB1 replicon, Ap^{R} |
| pCP32AMP | 2µ yeast origin, *URA3*, Tc^{R}, pMB1 replicon, Ap^{R}, *aroG* |
| pCP14 | 2µ yeast origin, *URA3*, Tc^{R}, pMB1 replicon, Ap^{R}, *P₁₅aroB* |
| pCP54 | 2µ yeast origin, *URA3*, Tc^{R}, pMB1 replicon, Ap^{R}, *P₁₅aroB, aroG* |
| pCP50 | 2µ yeast origin, *URA3*, Tc^{R}, pMB1 replicon, Ap^{R}, *aroG*, t*ktA* |
| pCP55 | 2µ yeast origin, *URA3*, Tc^{R}, pMB1 replicon, Ap^{R}, *aroG*, *aroB*, *tktA* |
| pCL1921 | pSC101 replicon, Spc^{R} |
| pMG27 | pSC101 replicon, Spc^{R}, *P_{R}-aroY* |
| pMG31 | pSC101 replicon, Spc^{R} , *P₁₅-catAX* |
| pMG33 | pSC101 replicon, Spc^{R}, *P₁₅-catAX, P_{R}-aroY* |
| pMG37 | pSC101 replicon, Spc^{R} , *P₁₅-catA-CatX, P_{R}-aroY, P₂₆-quiC* |
| pMG39 | pSC101 replicon, Spc^{R}, *P₂₆-quiC* |
| pMG47 | pSC101 replicon, Spc^{R}, *P₁₅-catAX, P_{R}-aroY, P₂₆-asbF* |
| pMG70 | SC101 replicon, Spc^{R} , *P₁₅-catAX, P_{R}-aroY, P₂₆-qa-4* |

| Table 2 Sequence Information | | |
|---|---|---|
| No. | Name | Description |
| 1 | SEQ ID No. 1 | The P₁₅ promoter from *Bacillus subtilis* phage SP01, with a stem and loop added just downstream from the transcription start site. |
| 2 | SEQ ID No. 2 | The P₂₆ promoter from *Bacillus subtilis* phage SP01 |
| 3 | SEQ ID No. 3 | The P_{R} promoter from *Escherichia coli* phage |
| 4 | SEQ ID No. 4 | Protein sequence of 3-dehydroshikimate dehydratase from *Neurospora crassa* encoded by the *qa-4* gene. |
| 5 | SEQ ID No. 5 | Genomic DNA sequence of the *qa-4* gene from *Neurospora crassa* plus surrounding sequences. |
| 6 | SEQ ID No. 6 | Protein sequence of 3-dehydroshikimate dehydratase from *Aspergillus nidulans.* encoded by the *qutC* gene |
| 7 | SEQ ID No. 7 | Genomic DNA sequence of the *qutC* gene from *Aspergillus nidulans* plus surrounding sequences |
| 8 | SEQ ID No. 8 | Protein sequence of protocatechuate decarboxylase (AroY) from *Klebsiella pnemoniae* A TCC25597 |
| 9 | SEQ ID No. 9 | DNA sequence of the *aroY* gene of *Klebsiella pneumoniae* 342 plus 2 kilobases of surrounding DNA sequences |
| 10 | SEQ ID No. 10 | DNA sequence of the *catA* gene from *Acinetobacter baylyi ADP1*, including 410 bases of upstream sequence and two open reading frames downstream |
| 11 | SEQ ID No. 11 | Protein sequence of CatA (catechol 1,2-dioxygenase) from *Acinetobacter baylyi ADP1* |
| 12 | SEQ ID No. 12 | DNA sequence of the *quiC* (3-dehydroshikimate dehydratase)gene from *Acinetobacter sp. ADP1* |
| 13 | SEQ ID No. 13 | Codon-optimized DNA sequence of the *quiC* (3-dehydroshikimate dehydratase)gene from *Acinetobacter sp. ADP1* |
| 14 | SEQ ID No. 14 | Protein sequence of QuiC (3-dehydroshikimate dehydrogenase from *Acinetobacter sp. ADP1* |
| 15 | SEQ ID No. 15 | DNA sequence of the plasmid pAC21 |
| 16 | SEQ ID No. 16 | DNA sequence of the plasmid pAC19 |
| 17 | SEQ ID No. 17 | DNA sequence of the plasmid pMH17F |
| 18 | SEQ ID No. 18 | DNA sequence of the coding region of the wild type *aroG* gene |
| 19 | SEQ ID No. 19 | DNA sequence of the plasmid pMH28F |

| Table 3. Sequence Information - cont. | | |
|---|---|---|
| No. | Name | Description |
| 20 | SEQ ID No. 20 | DNA sequence of the plasmid pCL1921 |
| 21 | SEQ ID No. 21 | DNA sequence of the plasmid pMG27 |
| 22 | SEQ ID No.2 2 | DNA sequence of the plasmid pMG31 |
| 23 | SEQ ID No. 23 | DNA sequence of the plasmid pMG33 |
| 24 | SEQ ID No. 24 | DNA sequence of the plasmid pMG37 |
| 25 | SEQ ID No. 25 | DNA sequence of the plasmid pMG39 |
| 26 | SEQ ID No. 26 | DNA sequence of the plasmid pMG47 |
| 27 | SEQ ID No. 27 | DNA sequence of the plasmid pMG70 |
| 28 | SEQ ID No. 28 | DNA sequence of the plasmid pCP32AMP |
| 29 | SEQ ID No. 29 | DNA sequence of the plasmid pCP14 |
| 30 | SEQ ID No. 30 | DNA sequence of the plasmid pCP50 |
| 31 | SEQ ID No. 31 | DNA sequence of the plasmid pCP54 |
| 32 | SEQ ID No. 32 | DNA sequence of the plasmid pCP55 |
| 33 | SEQ ID No. 33 | DNA sequence of the plasmid YEP24 |
| 34 | SEQ ID No. 34 | DNA sequence of the deleted *aroE* region |
| 35 | SEQ ID No. 35 | DNA sequence of the integrated cassette *Δack::P₁₅aroB* |
| 36 | SEQ ID No. 36 | DNA sequence of the *ΔpoxB* region |
| 37 | SEQ ID No. 37 | DNA sequence of the integrated cassette *ΔpoxB::tktA* |
| 8 | SEQ ID No. 38 | DNA sequence of the *ΔptsHI* region |
| 39 | SEQ ID No. 9 | DNA sequence of the integrated cassette *Δtdc::glf-glk* |
| 40 | SEQ ID No. 40 | DNA sequence of the *ΔgalP* region |
| 41 | SEQ ID No. 41 | MYR352 *ΔadhE::P₁₅-catAX, P_{R}-aroY, P₂₆-quiC* |

| Table 4. *aroG**mutant alleles that lead to resistance to phenylalanine feedback inhibition | | | |
|---|---|---|---|
| Strain | Allele number | Nucleotide mutation | Amino acid mutation |
| RY893 | *aroG*20-893* | C449T | Pro150Leu |
| RY897 | *aroG*20-897* | C449T | Pro150Leu |
| RY899 | *aroG*20-899* | T538C | Ser180Pro |
| RY901 | *aroG*20-901* | C438T | Pro150Ser |
| MYR450 | *aroG*111* | C55T | Pro19Ser |
| MYR451 | *aroG*211* | G533A | Gly178Glu |
| MYR452 | *aroG*212* | C540T | Ser180Phe |
| MYR453 | *aroG*311* | Deletion from base pair 36 to 44bp | Deletion of Glu-Ile-Lys |
| MYR454 | *aroG*312* | C632T | Ser211Phe |
| MYR455 | *aroG*411* | T29A | Ile10Asn |
| MYR456 | *aroG*412* | G533A | Gly178Glu |
| MYR457 | *aroG*511* | C448T | Pro150Ser |

| Table 5. AroG activity measurement in crude extract from various recombinant *E. coli* strains | | | |
|---|---|---|---|
| Strain | *aroG** allele | Specific activity mU | % of activity resistant to phenylalanine |
| | | (One mU = one nM product made per milligram protein per minute) | |
| RY893 | *aroG*20-893* | 62 | 34 |
| RY897 | *aroG*20-897* | 55 | 77 |
| RY899 | *aroG*20-899* | 92 | 113 |
| RY901 | *aroG*20-901* | 78 | 76 |
| RY902 | *aroG wild type* | 38 | 6 |
| RY890 | *aroG wild type* | 54 | 7 |

| Table 6. Muconic acid production in shake flasks by strains containing feedback resistant aroG* alleles | |
|---|---|
| Strain | Muconic acid titer g/l |
| RY913 | 3.04 |
| RY919 | 3.11 |
| RY921 | 2.99 |
| RY922 | 1.45 |

### REFERENCES

U.S. Patent No. 4,480,034
**(0135)** U.S. Patent No. 4,535,059
(0136) U.S. Patent No. 4,588,688
(0137) U.S. Patent No. 4,608,338
**(0138)** U.S. Patent No. 4,681,852
(0139) U.S. Patent No. 4,753,883
**(0140)** U.S. Patent No. 4,833,078
**(0141)** U.S. Patent No. 4,968,612
**(0142)** U.S. Patent No. 5,168,056
**(0143)** U.S. Patent No. 5,272,073
**(0144)** U.S. Patent No. 5,487,987
**(0145)** U.S. Patent No. 5,616,496
**(0146)** U.S. Patent No. 6,600,077
**(0147)** U.S. Patent No. 6,180,373
**(0148)** U.S. Patent No. 6,210,937
**(0149)** U.S. Patent No. 6,472,169
**(0150)** U.S. Patent No. 6,613,552
(0151) U.S. Patent No. 6,962,794
**(0152)** U.S. Patent No. 7,244,593
(0153) U.S. Patent No. 7,638,312
**(0154)** U.S. Patent No. 7,790,431
(0155) US Patent Application Publication No. US 2009/0191610
(0156) U.S. Patent Application Publication No. US 2010/0314243 A1
**(0157)** European Patent Application No. 86300748.0
**(0158)** International Patent Application Publication No. WO 2011/017560
**(0159)** International Patent Application Publication No. WO 2011/085311
**(0160)** International Patent Application Publication No. WO 2011/123154
**(0161)** Altschul, S. F., Gish, W., Miller, W., Myers, E. W., and Lipman, D. J. (1990) Basic local alignment search tool, J Mol Biol 215, 403-410.
**(0162)** Altschul, S. F., Madden, T. L., Schaffer, A. A., Zhang, J., Zhang, Z., Miller, W., and Lipman, D. J. (1997) Gapped BLAST and PSI-BLAST: a new generation of protein database search programs, Nucleic Acids Res 25, 3389-3402.
**(0163)** Barbe, V., Vallenet, D., Fonknechten, N., Kreimeyer, A., Oztas, S., Labarre, L., Cruveiller, S., Robert, C., Duprat, S., Wincker, P., Ornston, L. N., Weissenbach, J., Marliere, P., Cohen, G. N., and Medigue, C. (2004) Unique features revealed by the genome sequence of Acinetobacter sp. ADP1, a versatile and naturally transformation competent bacterium, Nucleic Acids Res 32, 5766-5779.
**(0164)** Bird, J. A. and Cain, R. B. (1968) cis-cis-muconate, the product inducer of catechol 1,2-oxygenase in Pseudomonas aeruginosa. Biochem. J. 109, 479-481.
**(0165)** Bongaerts, J., Kramer, M., Muller, U., Raven, L. and Wubbolts, M. (2001) Metabollic engineering for microbial producitnof aromatic acids and derived compunds. Met. Eng. 3, 289-300.
**(0166)** Chandran, S. S., Yi, J., Draths, K. M., von Daeniken, R., Weber, W. and Frost, J. W. (2003) Phosphoenolpyruvate availability and the biosynthesis of shikimic acid. Biotechnol. Prog. 19, 808-814.
**(0167)** Chen, R., Hatzimanikatis, V., Yap, W. M. G. J., Potma, P. W. and Bailey, J. E. (1997) Metabolic consequences of phosphotransferase (PTS) mutation in a phenylalanie-producing recombinant Escherichia coli. Biotechnol. Prog. 13, 768-775.
**(0168)** Chen, K., Dou, J., Tang, S., Yang, Y., Wang, H., Fang, H. and Zhou, C. (2012) Deletion of the aroK gene is essential for high shikimic acid accumulation through the shikimate in E. coli. Bioresource Technol, 119, 141-147.
**(0169)** Choi, W. J., Lee, E. Y., Cho, M. H., and Choi, C. Y. (1997) Enhanced production of cis, cis-muconate in a cell-recycle bioreactor. J. Fermentation and Bioengineering. 84, 70-76.
**(0170)** Curran, K. A., Leavitt, J. M., Karim, A. S. and Alper, H. S. (2012) Metabolic engineering of muconic acid production in Saccharomyces cerevisiae. Metabol. Engineer. 15, 55-66.
**(0171)** de Berardinis, V., Vallenet, D., Castelli, V., Besnard, M., Pinet, A., Cruaud, C., Samair, S., Lechaplais, C., Gyapay, G., Richez, C., Durot, M., Kreimeyer, A., Le Fevre, F., Schachter, V., Pezo, V., Doring, V., Scarpelli, C., Medigue, C., Cohen, G. N., Marliere, P., Salanoubat, M., and Weissenbach, J. (2008) A complete collection of single-gene deletion mutants of Acinetobacter baylyi ADP1, Mol Syst Biol 4, 174.
(0172) Draths, K. M., Pompliano, D. L., Conley, D. L., Frost, J. W., Berry, A., Disbrow, G. L., Staversky, R. J., and Lievense, J. C. (1992) Biocatalytic Synthesis of Aromatics from D-Glucose - the Role of Transketolase, Journal of the American Chemical Society 114, 3956-3962.
(0173) Draths, K. M., and Frost, J. W. (1995) Environmentally Compatible Synthesis of Catechol from D-Glucose, Journal of the American Chemical Society 117, 2395-2400.
**(0174)** Elsemore, D. A., and Ornston, L. N. (1995) Unusual ancestry of dehydratases associated with quinate catabolism in Acinetobacter calcoaceticus, J Bacteriol 177, 5971-5978.
(0175) Escalante, A., Calderon, R., Valdiva, A., de Anda, R., Hernandez, G., Ramirez, O. T., Gosset, G. and Boliver, F. (2010) Metabolic engineering for the production of shikimic acid in an evolved Escherichia coli strain lacking the phosphoenolpyrvate: carbohydrate phosphotransferase system. Microbial Cell Factories 9, 21-33.
**(0176)** Flores, N., Xiao, J., Berry, A., Bolivar, F. and Valle, F. (1996) Pathway engineering for the production of aromatic compounds in Escherichia coli. Nature Biotechn. 14, 620 - 623.
(0177) Fox, D. T., Hotta, K., Kim, C. Y., and Koppisch, A. T. (2008) The missing link in petrobactin biosynthesis: asbF encodes a (-)-3-dehydroshikimate dehydratase, Biochemistry 47, 12251-12253.
(0178) Ger, Y., Chen, S., Chiang, H., and Shiuan, D. (1994) A Single Ser-180 Mutation Desensitizes Feedback Inhibition of the Phyenylalanine-Sensitive 3-Deoxy-D-Arabino-Hepulosonate 7-Phosphate (DAHP) Synthetase in Eschericia coli, J Biochem 116, 986-990.
(0179) Grant, D. J., and Patel, J. C. (1969) The non-oxidative decarboxylation of p-hydroxybenzoic acid, gentisic acid, protocatechuic acid and gallic acid by Klebsiella aerogenes (Aerobacter aerogenes), Antonie Van Leeuwenhoek 35, 325-343.
(0180) Hansen, E. H., Moller, B. L., Kock, G. R., Bunner, C. M., Kristensen, C., Jensen, O. R., Okkels, F. T., Olsen, C. E., Motawia, M. S., and Hansen, J. (2009) De novo biosynthesis of vanillin in fission yeast (Schizosaccharomyces pombe) and baker's yeast (Saccharomyces cerevisiae), Appl Environ Microbiol 75, 2765-2774.
(0181) Hu, C., Jiang, P., Xu, J., Wu, Y., and Huang, W. (2003) Mutation analysis of the feedback inhibition site of phenylalanine-sensitive 3-deoxy-D-arabino-heptulosonate 7-phosphate synthase of Escherichia coli, J Basic Microbiol 43, 399-406.
**(0182)** Iwagami, S. G., Yang, K., and Davies, J. (2000) Characterization of the protocatechuic acid catabolic gene cluster from Streptomyces sp. strain 2065, Appl Environ Microbiol 66, 1499-1508.
**(0183)** Jantama, K., Haupt, M. J., Svoronos, S. A., Zhang, X., Moore, J. C., Shanmugam, K. T., and Ingram, L. O. (2008a) Combining metabolic engineering and metabolic evolution to develop nonrecombinant strains of Escherichia coli C that produce succinate and malate, Biotechnol Bioeng 99, 1140-1153.
(0184) Jantama, K., Zhang, X., Moore, J. C., Shanmugam, K. T., Svoronos, S. A., and Ingram, L. O. (2008b) Eliminating side products and increasing succinate yields in engineered strains of Escherichia coli C, Biotechnol Bioeng 101, 881-893.
(0185) Kaneko, A., Ishii, Y., and Kirimura, K. (2011) High-yield production of cis, cis-muconic acid from catechol in aqueous solution by biocatalyst. Chem. Lett. 40, 381-383.
(0186) Kikuchi, Y., Tsujimoto, K., and Kurahashi, O. (1997) Mutational analysis of the feedback sites of phenylalanine-sensitive 3-deoxy-D-arabino-heptulosonate-7-phosphate synthase of Escherichia coli, Appl Environ Microbiol 63, 761-762.
(0187) Kojima, Y., Fujisawa, H., Nakazawa, A., Nakazawa, T., Kanetsuna, F., Taniuchi, H., Nozaki, M., and Hayaishi, O. (1967) Studies on pyrocatechase. I. Purification and spectral properties, J Biol Chem 242, 3270-3278.
(0188) Kramer, M., Bongaerts, J., Bovenberg, R., Kremer, S., Muller, U., Orf, S., Wubbolts, M. and Raeven, L. (2003) Metabolic engineering for microbial production of shikimic acid. Metabol. Eng. 5, 277-283.
(0189) Lerner, C. G., and Inouye, M. (1990) Low copy number plasmids for regulated low-level expression of cloned genes in Escherichia coli with blue/white insert screening capability, Nucleic Acids Res 18, 4631.
**(0190)** Li, K. and Frost, J.W. (1999) Microbial synthesis of 3-dehydroshikimic acid: A comparative analysis of D-xylose, L-arabinose, and D-glucose carbon sources. Biotechnol. Prog. 15, 876-883.
**(0191)** Lu, J. L., and Liao, J. C. (1997) Metabolic engineering and control analysis for production of aromatics: Role of transaldolase, Biotechnol Bioeng 53, 132-138.
**(0192)** Lu, J., Tang, J., Liu, Y., Zhu, X. (2012) Combinatorial modulation of galP and glk gene expression for improves alternative glucose utilization. Appl. Microbiol. Biotechnol. 93, 2455-2462
**(0193)** Lutke-Eversloh, T., and Stephanopoulos, G. (2007) L-tyrosine production by deregulated strains of Escherichia coli, Appl Microbiol Biotechnol 75, 103-110.
**(0194)** Mizuno, S., Yoshikawa, N., Seki, M., Mikawa, T., and Imada, Y. (1988) Microbial production of cis, cis- muconic acid from benzoic acid. Appl Microbiol Biotechnol. 28, 20-25.
**(0195)** Nakazawa, A., Kojima, Y., and Taniuchi, H. (1967) Purification and properties of pyrocatechase from Pseudomonas fluorescens, Biochim Biophys Acta 147, 189-199.
(0196) Neidhardt, F. C., and Curtiss, R. (1996) Escherichia coli and Salmonella : cellular and molecular biology, 2nd ed., ASM Press, Washington, D.C.
**(0197)** Neidle, E. L., and Ornston, L. N. (1986) Cloning and expression of Acinetobacter calcoaceticus catechol 1,2-dioxygenase structural gene catA in Escherichia coli, J Bacteriol 168, 815-820.
**(0198)** Niu, W., Draths, K. M., and Frost, J. W. (2002) Benzene-free synthesis of adipic acid, Biotechnol Prog 18, 201-211.
**(0199)** Parker, C., Barnell, W. O., Snoep, J. L., Ingram, L. O., and Conway, T. (1995) Characterization of the Zymomonas mobilis glucose facilitator gene product (glf) in recombinant Escherichia coli: examination of transport mechanism, kinetics and the role of glucokinase in glucose transport, Mol Microbiol 15, 795-802.
**(0200)** Parsek, M. R., Shinabarger, D..L., Rithmel, R. K. and Chakrabarty, A. M. (1992) Roles of CatR and cis, cis-Muconate in activation of the catBC operson, which is involved in benzoate degradationin Pseudomonas putida. J. Bacteriol. 174, 7798-7806.
**(0201)** Patnaik, R. and Liao, J. C. (1994) Engineering of Escherichia coli central metabolism for aromatic metabolite with near theoretical yiled. App. Env. Microbiol. 60, 3903-3908.
**(0202)** Pfleger, B. F., Kim, Y., Nusca, T. D., Maltseva, N., Lee, J. Y., Rath, C. M., Scaglione, J. B., Janes, B. K., Anderson, E. C., Bergman, N. H., Hanna, P. C., Joachimiak, A., and Sherman, D. H. (2008) Structural and functional analysis of AsbF: origin of the stealth 3,4-dihydroxybenzoic acid subunit for petrobactin biosynthesis, Proc Natl Acad Sci U S A 105, 17133-17138.
**(0203)** Perez-Pantoja, D., De la Iglesia, R., Pieper, D. H., and Gonzalez, B. (2008) Metabolic reconstruction of aromatic compounds degradation from the genome of the amazing pollutant-degrading bacterium Cupriavidus necator JMP134, FEMS Microbiol Rev 32, 736-794.
**(0204)** Perez-Pantoja, D., Donoso, R., Agullo, L., Cordova, M., Seeger, M., Pieper, D. H., and Gonzalez, B. (2011) Genomic analysis of the potential for aromatic compounds biodegradation in Burkholderiales, Environ Microbiol.
**(0205)** Pittard, J. and Wallace, B. J. (1966) Distribution and function of genes concerned with aromatic biosynthesis in Escherichia coli. J. Bacteriol. 91, 1494-1508.
**(0206)** Polen, T., Spelberg, M. and Bott, M. (2012) toward bitechnological produciton of adipic acid and precursors from biorenewables, J. Biotechnol, http://dx.doi.org/10.1016/j.biotec.2012-07.008.
**(0207)** Rutledge, B. J. (1984) Molecular characterization of the qa-4 gene of Neurospora crassa, Gene 32, 275-287.
**(0208)** Schirmer, F., and Hillen, W. (1998) The Acinetobacter calcoaceticus NCIB8250 mop operon mRNA is differentially degraded, resulting in a higher level of the 3' CatA-encoding segment than of the 5' phenolhydroxylase-encoding portion, Mol Gen Genet 257, 330-337.
**(0209)** Shumilin, I. A., Kretsinger, R. H., and Bauerle, R. H. (1999) Crystal structure of phenylalanine-regulated 3-deoxy-D-arabino-heptulosonate-7-phosphate synthase from Escherichia coli, Structure 7, 865-875.
**(0210)** Shumilin, I. A., Zhao, C., Bauerle, R., and Kretsinger, R. H. (2002) Allosteric inhibition of 3-deoxy-D-arabino-heptulosonate-7-phosphate synthase alters the coordination of both substrates, J Mol Biol 320, 1147-1156.
(0211) Shumilin, I. A., Bauerle, R., Wu, J., Woodard, R. W., and Kretsinger, R. H. (2004) Crystal structure of the reaction complex of 3-deoxy-D-arabino-heptulosonate-7-phosphate synthase from Thermotoga maritima refines the catalytic mechanism and indicates a new mechanism of allosteric regulation, J Mol Biol 341, 455-466.
**(0212)** Shumkova, E. S., Solyanikova, I. P., Plotnikova, E. G. and Golovleva, L. A. (2009) Phenol degrdation by Rhodococcus opacus Strain 1G. App. Biocehm. Microbiol. 45, 43-49.
**(0213)** Sietmann, R., Uebe, R., Boer, E., Bode, R., Kunze, G., and Schauer, F. (2010) Novel metabolic routes during the oxidation of hydroxylated aromatic acids by the yeast Arxula adeninivorans, J Appl Microbiol 108, 789-799.
**(0214)** Smith, M. R. and Ratledge, C. (1989) Quantitative biotransformation of catechol to cis, cis -muconate. Biotech. Lett. 11, 105-110.
(0215) Snoep, J. L., Arfman, N., Yomano, L. P., Fliege, R. K., Conway, T., and Ingram, L. O. (1994) Reconstruction of glucose uptake and phosphorylation in a glucose-negative mutant of Escherichia coli by using Zymomonas mobilis genes encoding the glucose facilitator protein and glucokinase, J Bacteriol 176, 2133-2135.
(0216) Sprenger, G. A. (1995) Genetics of pentose-phosphate pathway enzymes of Escherichia coli K-12, Arch Microbiol 164, 324-330.
(0217) Sprenger, G. A., Schorken, U., Sprenger, G., and Sahm, H. (1995a) Transketolase A of Escherichia coli K12. Purification and properties of the enzyme from recombinant strains, Eur J Biochem 230, 525-532.
(0218) Sprenger, G. A., Schorken, U., Sprenger, G., and Sahm, H. (1995b) Transaldolase B of Escherichia coli K-12: cloning of its gene, talB, and characterization of the enzyme from recombinant strains, J Bacteriol 177, 5930-5936.
(0219) Stroman, P., Reinert, W. R., and Giles, N. H. (1978) Purification and characterization of 3-dehydroshikimate dehydratase, an enzyme in the inducible quinic acid catabolic pathway of Neurospora crassa, J Biol Chem 253, 4593-4598.
(0220) Tang, J., Zhu, X., Lu, J. and Liu, P. (2012) Recruiting alternative glucose utilization pathways for improving succinate production. App Microbiol Biotechnol DOI 10, 1007/s00253-012-434.1
(0221) Tateoka, T., and Yasuda, I. (1995) 3-Dehydroshikimate dehydratase in mung hean cultured cells, Plant Cell Reports 15, 212-217.
(0222) Weaver, L. M., and Herrmann, K. M. (1990) Cloning of an aroF allele encoding a tyrosine-insensitive 3-deoxy-D-arabino-heptulosonate 7-phosphate synthase, J Bacteriol 172, 6581-6584.
(0223) Weber, C., Bruckner, C., Weinreb, S., Lehr, C., Essl, C. and Bole, E. (2012) Biosynthesis of cis, cis-muconic acid and its aromatic precursors catechol and proteocatechuic acid, from renewable feedstocks by Saccharomyces cerevisiae, App Environ Microbiol. 78, 8421-8430.
(0224) Wheeler, K. A., Lamb, H. K., and Hawkins, A. R. (1996) Control of metabolic flux through the quinate pathway in Aspergillus nidulans, Biochem J 315 (Pt 1), 195-205.
(0225) Wu, C-M., Wu, C-C., Su, C-C., Lee, S-N., Lee, Y-A. and Wu, J-Y. (2006) Microbial synthesis of cis,cis-muconic acid form benzoate by Sphingobacterium sp. Mutants. Biochem. Eng. J. 29, 35-40.
(0226) Xie, N., Tang, H., Feng, J., Tao, F., Ma, C. and Xu, P. (2009) Characterization of benzoate degradationby newly isolated bacterium Pseudomonas sp. XP-M2. Biochem. Eng. J. 46, 79-82.
(0227) Yi, J., Draths, K. M., Li, K. and Frost, J. W. (2003)Altered Glucose Transport and Shikimate Pathway Product Yields in E. coli. Biotechnol. Prog. 2003, 19, 1450-1459
(0228) Yoshikawa, N., Mizuno, S., Ohta, K., and Suzuki, M. (1990) Microbial production of cis, cis-muconic acid. J. Biotechnol. 14, 203-210

## Claims

1. A genetically engineered *Escherichia coli* in which the PEP-dependent phosphotransferase system and the GalP-based system for glucose import are eliminated, and which comprises an exogenous glucose facilitator system comprising a protein encoded by an exogenous *glf* gene.

2. A genetically engineered *Escherichia coli* as in claim 1 further comprising a *pck* gene coding for phosphoenol pyruvate carboxykinase enzyme with increased activity.

3. A genetically engineered *Escherichia coli* as in claim 1 further comprising a deletion in the *pykA* gene coding for pyruvate kinase enzyme activity.

4. A genetically engineered *Escherichia coli* as in claim 1 further comprising a deletion in the *pykF* gene coding for pyruvate kinase enzyme activity.

5. A genetically engineered *Escherichia coli* as in claim 1 further comprising one or more exogenous genes selected from the group consisting of *aroZ, qa-4, asbF, aroY, quiC* and *catAX,* wherein said one or more exogenous genes code for proteins functional in a muconic acid pathway.

6. A genetically engineered *Escherichia coli* as in claim 1 further comprising one or more exogenous genes selected from the group consisting of *aroB, aroD, aroF, aroG, aroH, tktA, talB, rpe,* and *rpi,* wherein said one or more exogenous genes code for proteins functional in a shikimic acid pathway.

7. A genetically engineered *Escherichia coli* as in claim 1 wherein the activity of a negative regulator protein of aromatic amino acid biosynthesis encoded by a *tyrR* gene or its homolog is substantially reduced or eliminated.

8. A genetically engineered *Escherichia coli* as in claim 1 further comprising an *aroG** gene which codes for a DAHP synthase enzyme that is substantially resistant to inhibition by phenylalanine.

9. A genetically engineered *Escherichia coli* of claim 8 further comprising an *aroG** gene that codes for a DAHP synthase enzyme that is selected from the group consisting of *aroG*20-893, aroG*20-897, aroG*20-899, aroG*20-901, aroG*111, aroG*211, aroG*212, aroG*311, aroG*312, aroG*411, aroG*412,* and *aroG*511.*

10. A genetically engineered *Escherichia coli* of claim 1 further comprising an exogenous gene that codes for a QuiC enzyme of a bacterium of the genus *Acinetobacter,* or a homolog of said QuiC enzyme.

## Patentansprüche

1. Gentechnisch verändertes *Escherichia coli,* bei dem das PEP-abhängige Phosphotransferasesystem und das GalP-basierte System für den Glukoseimport eliminiert sind, und das ein exogenes Glukose-Fazilitatorsystem umfasst, welches ein Protein umfasst, das durch ein exogenes *glf*-Gen kodiert wird.

2. Gentechnisch verändertes *Escherichia coli* nach Anspruch 1, weiter umfassend ein *pck*-Gen, das ein Phosphoenolpyruvat-Carboxykinase-Enzym mit erhöhter Aktivität kodiert.

3. Gentechnisch verändertes *Escherichia coli* nach Anspruch 1, weiter umfassend eine Deletion im *pykA-*Gen, das Pyruvatkinase-Enzymaktivität kodiert.

4. Gentechnisch verändertes *Escherichia coli* nach Anspruch 1, weiter umfassend eine Deletion im *pykF*-Gen, das Pyruvatkinase-Enzymaktivität kodiert.

5. Gentechnisch verändertes *Escherichia coli* nach Anspruch 1, weiter umfassend ein oder mehrere exogene Gene, ausgewählt aus der Gruppe, bestehend aus *aroZ, qa-4, asbF, aroY, quiC* und *catAX,* wobei das eine oder die mehreren exogenen Gene Proteine kodieren, die in einem Muconsäureweg funktionsfähig sind.

6. Gentechnisch verändertes *Escherichia coli* nach Anspruch 1, weiter umfassend ein oder mehrere exogene Gene, ausgewählt aus der Gruppe, bestehend aus *aroB, aroD, aroF, aroG, aroH, tktA, talB, rpe* und *rpi,* wobei das eine oder die mehreren exogenen Gene Proteine kodieren, die in einem Shikimisäureweg funktionsfähig sind.

7. Gentechnisch verändertes *Escherichia coli* nach Anspruch 1, wobei die Aktivität eines negativen Regulatorproteins der Biosynthese aromatischer Aminosäuren, das durch ein *tyrR-Gen* oder dessen Homolog kodiert wird, im Wesentlichen reduziert oder eliminiert ist.

8. Gentechnisch verändertes *Escherichia coli* nach Anspruch 1, weiter umfassend ein *aroG**-Gen, das ein DAHP-Synthase-Enzym kodiert, welches im Wesentlichen resistent gegen Hemmung durch Phenylalanin ist.

9. Gentechnisch verändertes *Escherichia coli* nach Anspruch 8, weiter umfassend ein *aroG*-*Gen, das ein DAHP-Synthase-Enzym kodiert, welches ausgewählt ist aus der Gruppe, bestehend aus *aroG*20-893, aroG*20-897, aroG*20-899, aroG*20-901, aroG*111, aroG*211, aroG*212, aroG*311, aroG*312, aroG*411, aroG*412* und *aroG*511.*

10. Gentechnisch verändertes *Escherichia coli* nach Anspruch 1, weiter umfassend ein exogenes Gen, das ein QuiC-Enzym eines Bakteriums der Gattung *Acinetobacter* oder ein Homolog des QuiC-Enzyms kodiert.

## Revendications

1. *Escherichia coli* génétiquement modifiée dans laquelle le système de phosphotransférase dépendant de PEP et le système à base de GaIP pour l'importation de glucose sont éliminés, et qui comprend un système facilitateur de glucose exogène comprenant une protéine codée par un gène *glf* exogène.

2. *Escherichia coli* génétiquement modifiée selon la revendication 1, comprenant en outre un gène *pck* codant pour une enzyme de phosphoénol pyruvate carboxykinase avec une activité accrue.

3. *Escherichia coli* génétiquement modifiée selon la revendication 1, comprenant en outre une délétion dans le gène *pykA* codant pour une activité enzymatique de pyruvate kinase.

4. *Escherichia coli* génétiquement modifiée selon la revendication 1, comprenant en outre une délétion dans le gène *pykF* codant pour une activité enzymatique de pyruvate kinase.

5. *Escherichia coli* génétiquement modifiée selon la revendication 1, comprenant en outre un ou plusieurs gènes exogènes choisis dans le groupe consistant en *aroZ, qa-4, asbF, aroY, quiC* et *catAX,* dans laquelle lesdits un ou plusieurs gènes exogènes codent pour des protéines fonctionnelles dans une voie d'acide muconique.

6. *Escherichia coli* génétiquement modifiée selon la revendication 1, comprenant en outre un ou plusieurs gènes exogènes choisis dans le groupe consistant en *aroB, aroD, aroF, aroG, aroH, tktA, talB, rpe,* et *rpi,* dans laquelle lesdits un ou plusieurs gènes exogènes codent pour des protéines fonctionnelles dans une voie d'acide shikimique.

7. *Escherichia coli* génétiquement modifiée selon la revendication 1, dans laquelle l'activité d'une protéine régulatrice négative d'une biosynthèse d'acide aminé aromatique codée par un gène *tyrR* ou son homologue est sensiblement réduite ou éliminée.

8. *Escherichia coli* génétiquement modifiée selon la revendication 1, comprenant en outre un gène *aroG** qui code pour une enzyme de synthèse DAHP qui est sensiblement résistante à une inhibition par la phénylalanine.

9. *Escherichia coli* génétiquement modifiée selon la revendication 8, comprenant en outre un gène *aroG** qui code pour une enzyme de synthèse DAHP qui est choisie dans le groupe consistant en *aroG*20-893, aroG*20-897, aroG*20-899, aroG*20-901, aroG*111, aroG*211, aroG*212, aroG*311, aroG*312, aroG*411, aroG*412,* et *aroG*511.*

10. *Escherichia coli* génétiquement modifiée selon la revendication 1, comprenant en outre un gène exogène qui code pour une enzyme QuiC d'une bactérie du genre *Acinetobacter,* ou un homologue de ladite enzyme QuiC.
